(19) Europäisches Patentamt
European Patent Office
Office européen des brevets

(11) Publication number: **0 396 427 B1**

(12) # EUROPEAN PATENT SPECIFICATION

(45) Date of publication of patent specification: **13.09.95**

(51) Int. Cl.[6]: **C07D 233/64**, C07D 233/66, C07D 233/84, C07D 233/70, A01N 43/50

(21) Application number: **90304875.9**

(22) Date of filing: **04.05.90**

(54) **Pesticidal 1-arylimidazoles.**

(30) Priority: **05.05.89 US 348682**

(43) Date of publication of application:
**07.11.90 Bulletin 90/45**

(45) Publication of the grant of the patent:
**13.09.95 Bulletin 95/37**

(84) Designated Contracting States:
**AT BE CH DE DK ES FR GB GR IT LI LU NL SE**

(56) References cited:
**EP-A- 0 283 173** **EP-A- 0 289 919**
**DE-A- 3 614 364** **US-A- 4 608 437**
**US-A- 4 728 653** **US-A- 4 755 213**

**CHEMICAL ABSTRACTS, VOL. 80, 1974, ABSTRACT NR. 82809J**

(73) Proprietor: **RHONE-POULENC AGROCHIMIE**
**14-20, rue Pierre Baizet**
**F-69009 Lyon (FR)**

(72) Inventor: **Powell, Gail Scotton**
**6508 Bakersfield Drive**
**Raleigh,**
**North Carolina 27606 (US)**
Inventor: **Sinodis, David Neal**
**1212 Nottingham Drive**
**Cary,**
**North Carolina 27511 (US)**
Inventor: **Timmons, Philip Reid**
**704 Duluth Street**
**Durham,**
**North Carolina 27705 (US)**
Inventor: **Wu, Tai-Teh**
**115 Summerline Drive**
**Chapel Hill,**
**North Carolina 27514 (US)**

(74) Representative: **Bentham, Stephen et al**
**J.A. KEMP & CO.**
**14 South Square**
**Gray's Inn**
**London WC1R 5LX (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services
(3.10/3.09/3.3.3)

## Description

The present invention relates to new 1-arylimidazoles and intermediates and processes to make the compounds. The invention further pertains to compositions of said compounds and methods, using said compounds, for the control of arthropod, nematode, helminth or protozoan pests. In particular, it pertains to the application of compounds or compositions thereof in agricultural and methods of use, particularly as pesticides, for controlling arthropods, especially mites or foliar or soil insects, without causing injury to crop plants.

Various substituted imidazole compounds are known to exhibit a number of different types of pesticidal activity, including activity as herbicides, plant growth regulators, fungicides, nematicides, insecticides and biocides. Included among these are the following: European Patent Application No. EP 270061A discloses as insecticides 1-arylimidazoles that are unsubstituted in the 2 and 4 positions of the imidazole ring, which additionally has a second phenyl substituent in the 5 position. US Patent No. 4755213 discloses as plant growth regulators 1-arylimidazoles which are likewise unsubstituted in the 2 and 4 positions of the imidazole ring and further substituted by a carboxamide (aminocarbonyl) group in the 5 position. European Patent Application Nos. EP 277384A and EP 289066A discloses as herbicides 1-arylimidazoles which are only substituted in the 2 and 5 positions and again unsubstituted in the 4 position of the imidazole ring. Other 1-substituted imidazoles are described as insecticides in European Patent Application No. 289919A, in which case the 1-substituent is aralkyl or aralkoxy (ie, an alkyl or alkoxy bridging group between the imidazole and aryl rings). European Patent Application No. 283173A discloses as insecticides, etc. 2-arylimidazoles in which the aryl ring is attached to the imidazole ring at a carbon atom (2-position) rather than a nitrogen atom and the 1-position nitrogen atom is substituted by hydrogen or an optionally substituted alkyl group. Australian Patent Application No. 8812-883A discloses as fungicides, insecticides, nematicides, etc., imidazole compounds which may be substituted at the 4 or 5 or both 4 and 5 positions of the imidazole ring (ie, attachment to carbon rather than nitrogen) by an optionally substituted phenyl ring and are substituted on the 1-position nitrogen atom by a hydrogen atom or a sulfonyl group.

The present invention pertains to new and novel 1-arylimidazole compounds which exhibit outstanding pesticidal properties, especially as insecticides or miticides or both.

The compounds, including their stereo isomers, e.g. diastereomers and optical isomers, have the following general formula (I)

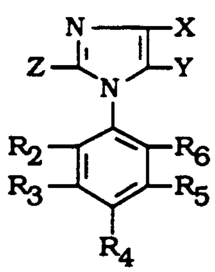

(I)

wherein:

| | |
|---|---|
| X | is a group selected from haloalkyl or haloalkoxy or an unsubstituted or halo-substituted group selected from alkylsulfenyl, alkylsulfinyl or alkylsulfonyl, wherein the defined alkyl and alkoxy moieties of each group are a linear or branched chain, containing one to four carbon atoms, and the halo-substitution of each group comprises one or more halogen atoms, which are the same or different, up to full substitution of the alkyl or alkoxy moiety; |
| Y and Z | are each individually selected from: a hydrogen or a halogen atom; a group selected from nitro, cyano, hydroxyl (and acceptable salts thereof), sulfhydryl (and acceptable salts thereof), formyl, hydroxycarbonyl (and acceptable salts thereof), alkoxycarbonyl, aminocarbonyl, alkylaminocarbonyl, dialkylaminocarbonyl, amino, alkylamino, dialkylamino, a trialkylammonium salt, cyanoalkyl, alkoxycarbonylamino, arylcarbonylamino alkylaminocarbonylamino, dialkylaminocarbonylamino, aminosulfonyl, alkylaminosulfonyl, dialkylaminosulfonyl, or alkoxyalkylideneimino, in which the defined alkyl and alkoxy moieties of each group are a linear or branched chain containing one to four carbon |

atoms; a linear or branched chain alkenyl or alkynyl group containing two to four carbon atoms; or a group selected from an unsubstituted or halo-substituted alkyl, alkoxy, alkylcarbonyl, alkylcarbonylamino, alkylsulfenyl, alkylsulfinyl or alkylsulfonyl, in which the defined alkyl and alkoxy moieties of each group are a linear or branched chain containing one to four carbon atoms and the halo-substitution comprises one or more halogen atoms, which are the same or different, up to full substitution of the alkyl or alkoxy moiety; and only one of Y and Z is a sulfur containing group; and

$R_2$, $R_3$, $R_4$, $R_5$ and $R_6$ are individually selected from: a hydrogen or a halogen atom; a group selected from nitro, cyano, amino, alkylamino or dialkylamino, in which the alkyl moiety of each group is a linear or branched chain containing one to four carbon atoms; a linear or branched chain alkenyl or alkynyl group containing two to four carbon atoms, which may be substituted by one or more halogen atoms, which are the same or different, up to full substitution; or a group selected from an unsubstituted or halo-substituted alkyl, alkoxy, alkylsulfenyl, alkylsulfinyl or alkylsulfonyl, in which the defined alkyl and alkoxy moieties of each group are a linear or branched chain containing one to four carbon atoms and the halo-substitution comprises one or more halogen atoms, which are the same or different, up to full substitution of the alkyl or alkoxy moiety. provided that

(i) X is other than trifluoromethyl when $R_3$ represents methyl, $R_4$ represents nitro or amino and the other symbols represent hydrogen and

(ii) X is other than chloromethyl when $R_4$ represents methyl, methoxy or ethoxy and the other symbols represent hydrogen.

According to a preferred feature of the invention, the pesticidal compounds are selected from amongst the compounds of formula (I), wherein X is $S(O)_nR_1$, having the formula (II)

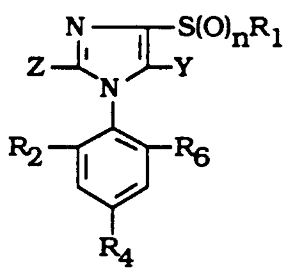

(II)

wherein:

Y and Z are individually selected from a hydrogen or halogen atom; a group selected from nitro, cyano, hydroxyl, sulfhydryl, amino, alkylamino or dialkylamino, in which the defined alkyl moiety of each group is a linear or branched chain containing one to four carbon atoms; or a group selected from an unsubstituted or fully halo-substituted alkyl, alkoxy, alkylcarbonyl, alkylcarbonylamino, alkylsulfenyl, alkylsulfinyl or alkylsulfonyl, in which the defined alkyl and alkoxy moieties of each group are a linear or branched chain, containing one to four carbon atoms, and the full halo-substitution of the alkyl or alkoxy moiety is by the same or different halogen atoms; and only one of Y and Z is a sulfur containing group;

$R_1$ is a linear or branched alkyl group of one to four carbon atoms which are unsubstituted or halo-substituted by one or more halogen atoms, which are the same or different;

$R_2$ is a hydrogen or a halogen atom or an alkyl, alkoxy, methylsulfenyl, methylsulfinyl or methylsulfonyl group;

$R_4$ is selected from a halogen atom or a group selected from trifluoromethyl, trifluoromethoxy, difluoromethoxy, trifluoromethylsulfenyl, trifluoromethylsulfinyl, trifluoromethylsulfonyl, or a linear or branched chain alkyl group containing one to four carbon atoms;

$R_6$ is a halogen atom (i.e., fluorine, chlorine or bromine); and

n is 0, 1 or 2.

Amongst the compounds of formula II which are preferred are those compounds wherein:

Y is a hydrogen atom, a halogen atom, amino, hydroxy, alkoxy of one to four carbon atoms,

methylsulfenyl, methylsulfinyl or methylsulfonyl;

Z is a hydrogen atom, a halogen atom, or a linear or branched alkyl group of one to four carbon atoms which is optionally fully substituted by halogen atoms which are the same or different;

$R_1$ is a methyl group fully substituted by halogen atoms which are the same or different;

$R_2$ is a hydrogen atom, a halogen atom or methylsulfenyl;

$R_4$ is a halogen atom, trifluoromethyl or trifluoromethoxy; and

$R_6$ is a fluorine, chlorine or bromine atom; and

n is 0, 1 or 2.

Even more specifically preferred compounds of formula II are those wherein:

Y is a hydrogen atom, a chlorine atom, a bromine atom, methylsulfenyl, methylsulfinyl or methoxy;

Z is a hydrogen atom, a chlorine atom, a bromine atom or methyl;

$R_1$ is trifluoromethyl, dichlorofluoromethyl or chlorodifluoromethyl;

$R_2$ is a hydrogen atom, a chlorine atom, a bromine atom or methylsulfenyl;

$R_4$ is a chlorine atom, a bromine atom, trifluoromethyl or trifluoromethoxy; and

$R_6$ is a chlorine or bromine atom; and

n is 0, 1 or 2.

The following are some of the representative preferred compounds of formula (II) (described subsequently in **EXAMPLES 1-165**) in the categories identified below.

- High broad spectrum insecticidal activity:

  Compounds of **EXAMPLES 4, 9, 20, 23, 25, 28, 29, 31, 32, 33, 34, 35, 36, 37, 38, 42, 44, 45, 60, 61, 70, 144**, and **146**.

  Typically wherein:
  Y is H;
  Z is Cl or Br (or optionally may be H or $CH_3$);
  $R_1$ is $CF_3$ , $CCl_2F$ or $CClF_2$;
  n is O, 1 or 2;
  $R_2$ is Cl (or optionally may be $SCH_3$);
  $R_3$ and $R_5$ are H; and
  $R_4$ is $CF_3$ (or optionally may be $OCF_3$).
- Good aphicidal activity:

  Compounds of **EXAMPLES 20, 21, 41, 42, 44, 48, 122, 131, 132**, and **144**.

  Typically wherein:
  Y and Z are H (or optionally may be Cl);
  $R_1$ is $CF_3$, $CCl_2F$ or $CClF_2$;
  n is 0, 1 or 2;
  $R_2$ and $R_6$ are Cl;
  $R_3$ and $R_5$ are H; and
  $R_4$ is $CF_3$ (or optionally may be $OCF_3$).
- High aphicidal plus broad spectrum insecticidal activity:

  Compounds of **EXAMPLES 10, 59, 60, 61, 68**, and **69**.

  Typically wherein:
  Y is H;
  Z is $CH_3$;
  $R_1$ is $CF_3$, $CCl_2F$ or $CClF_2$;
  n is 0, 1 or 2;
  $R_2$ and $R_6$ are Cl;
  $R_3$ and $R_5$ are H; and
  $R_4$ is $CF_3$.
- Good to high miticidal activity:

  Compounds of **EXAMPLES 9, 18, 60, 61, 70, 91, 92, 95, 96, 104, 106**, and **109**.

  Typically wherein:
  Y and Z are H (or optionally Z may be Cl, Br or $CH_3$ and Y optionally may be Br);
  $R_1$ is $CF_3$, $CCl_2F$ or $CClF_2$;
  n is 0, 1 or 2;
  $R_2$ and $R_6$ are Cl (or optionally $R_2$ may be $SCH_3$);
  $R_3$ and $R_5$ are H; and
  $R_4$ is Cl or Br (or optionally may be $CF_3$).

- Good to high soil insecticide (corn rootworm) activity:
  Compounds of **EXAMPLES 3, 4, 5, 6, 8, 9, 12, 16, 23, 25, 26, 28, 31, 33, 34, 35, 36, 37** and **38**.
  Typically wherein:
  Y is H, Cl, or Br (or optionally may be $SCH_3$ or $N = CHOC_2H_5$);
  Z is H, Cl or Br;
  $R_1$ is $CF_3$, $CCl_2F$ or $CClF_2$;
  n is 0, 1 or 2;
  $R_2$ and $R_6$ are Cl (or optionally $R_2$ is $SCH_3$);
  $R_3$ and $R_5$ are H; and
  $R_4$ is $CF_3$.

It is an object of the present invention to provide new compounds of the imidazole family together with processes for their preparation and intermediates thereto.

A second object of the present invention is to provide, for example, agronomically or medicinally acceptable compositions.

A third object of the present invention is to provide highly active compounds for use against: arthropods, especially mites, aphids or insects; plant nematodes; or helminth or protozoan pests. The compounds are thus advantageously used, for example, in agricultural or horticultural crops, forestry, veterinary medicine or livestock husbandry, or in public health.

A further object of the present invention is to provide compounds with broad spectrum activity as insecticides, miticides, aphicides or nematicides, by either soil, foliar application or seed treatment, including via systemic action.

An additional object of the present invention is to provide compounds having high arthropod toxicity, for example: to insects in the Coleoptera order, particularly Diabrotica spp. (corn rootworm) or Diptera order, particularly Musca domestica (housefly); to mites in the subclass Acari, particularly Tetranychus urticae - (two-spotted spider mite); or to aphids in the super family Aphidoidea, particularly Aphis nasturtii (buckthorn aphid).

These and other objects of the invention shall become readily apparent from the detailed description of the present invention.

The compounds of general formula (I) can be prepared by the application or adaptation of known methods (i.e. methods heretofore used or described in the chemical literature): generally imidazole ring formation followed wherein necessary by changing substituents. It is to be also understood that, in the description of the following process methods, the sequences for the introduction of the various groups on the imidazole ring may be performed in a different order and that suitable protecting groups may be required as will be apparent to those skilled in the art. Also compounds of general formula (I) may be converted by known methods into other compounds of general formula (I).

In the following description of process methods when symbols appearing in formulae are not specifically defined, it is to be understood that they are "as hereinbefore defined" in accordance with the first definition of each symbol in this specification. The term "protection" shall include conversion to a suitable non-reactive group which may be reconverted when desired, as well as the addition of groups which render the functionality non-reactive. Within the process definitions, unless otherwise stated, amino refers to the unsubstituted amino group.

The invention embraces particular intermediate compounds, useful to make certain of the herein contemplated compounds. Such preferred intermediate compounds, prepared as described herein, are defined in the following methods. In particular, intermediates that are more preferred have $R_2$ to $R_6$ as defined by formula (II) of the invention or more specifically preferred $R_2$, $R_4$ and $R_6$ definitions therein.

The following synthetic Methods I to VI generally describe alternative cyclization procedures beginning with appropriately substituted N-phenylimino compounds which are cyclized by means of a basic reagent to useful and novel intermediate N-phenylimidazole compounds. This reaction (including subsequent initial derivatization of the Z and Y substituents) can be generally represented by the reaction of a compound of formula (III) with a basic agent to give a compound of formula (IV) as follows:

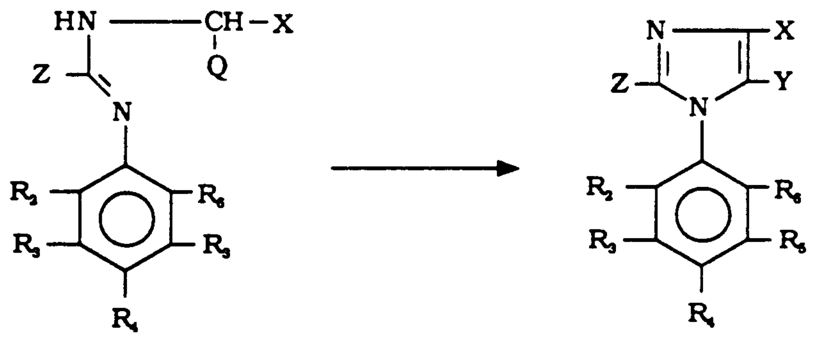

(III) (IV)

wherein for formula (III):
$R_2$, $R_3$, $R_4$, $R_5$ and $R_6$ are as defined in formula (I);
X is hydrogen or haloalkyl, particularly trifluoromethyl;
Z is hydrogen, halogen, alkyl, haloalkyl, or hydroxy, optionally existing in its isomeric keto form; and
Q is cyano or lower alkoxycarbonyl.
wherein for formula (IV):

| | |
|---|---|
| $R_2$, $R_3$, $R_4$, $R_5$ and $R_6$ | are as defined in formula (I); |
| X | is hydrogen or haloalkyl, particularly trifluoromethyl; |
| Y | is amino; hydroxy, optionally existing in its isomeric keto form when X is hydrogen; or alkoxy or haloalkoxy, obtained by alkylation of hydroxy; and |
| Z | is hydrogen; halogen; alkyl; haloalkyl; hydroxy, optionally existing in its isomeric keto form when X is hydrogen and Y is imino; or alkoxy or haloalkoxy, obtained by alkylation of hydroxy. |

Compounds of formula (I) of the invention can then be prepared by reaction of compounds of formula (IV) according to the subsequently described Methods introducing the various substituents, particularly X, Y and Z.

Particularly useful and novel intermediate phenyl imidazole compounds, discussed in the Methods herein for the preparation of compounds of the invention of formula (I), are specifically compounds of formulae (IV), (5), (17), (22), (27), (30)/(29), (37)/(34), (Ia), (Ib), and (Ic). Additionally, compounds of formula (III) which are novel and useful are specifically compounds of formulae (4), (16), (21), (26), (28), and (33).

In particular, the more preferred 4-sulfenated 1-arylimidazoles (X = $S(O)_nR_1$ wherein n and $R_1$ are previously defined) of this invention can be prepared by a variety of methods. Two preferred methods are illustrated by reaction SCHEMES I and II (METHODS I and II).

Method I

According to Method I, a particularly useful compound of formula (I), namely (Ia),

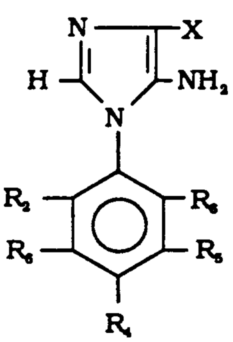

(Ia)

can be prepared wherein X, $R_2$, $R_3$, $R_4$, $R_5$ and $R_6$ are as defined in formula (I).

Method IA Compounds of general formula (I), in which X is alkylsulfenyl, haloalkylsulfenyl, alkylsulfinyl, haloalkylsulfinyl, alkylsulfonyl, and haloalkylsulfonyl, Y is amino, hydrogen, halogen, alkylsulfenyl, haloalkylsulfenyl, alkylsulfinyl, haloalkylsulfinyl, alkylsulfonyl, haloalkylsulfonyl, cyano or nitro, Z is hydrogen or halogen, and $R_2$, $R_3$, $R_4$, $R_5$ and $R_6$ are as defined in formula (I) can be prepared by procedures described in SCHEME I.

In SCHEME I the starting material, alkyl orthoformate (1), in which R′ is a $C_1$ to $C_4$ alkyl group, is generally commercially available and the aniline (2) is usually also a commercial product or else it may be prepared following well-known literature procedures. The catalyst used for formimidate (3) formation is generally an inorganic acid such as hydrochloric acid or an organic acid such as p-toluenesulfonic acid. The reaction may be carried out at a temperature between about -20 °C and about 180 °C, preferably between about 0 °C and about 120 °C, in the presence of an inert organic solvent such as a hydrocarbon, a chlorinated hydrocarbon, an aromatic, an ether, an alcohol or the alkyl orthoformate itself may be used as the solvent. The formimidate (3) may exist as a mixture of regioisomers.

The intermediate formimidine (4) is prepared by reaction of the formimidate (3) with aminoacetonitrile or the hydrochloride salt thereof in the presence of a base and in an inert organic solvent preferably capable of providing a homogeneous solution for the reactants. Typical organic and inorganic bases are alkoxides, hydroxides, hydrides, carbonates of alkali or alkaline earth metals, and amines. Solvents which may be employed include inert organic solvents such as alcohols (e.g., methanol or ethanol), ethers (e.g., diethyl ether, tetrahydrofuran, dioxane or diglyme), amines (e.g., triethylamine or pyridine) or water or combinations of these solvents. The reaction is usually conducted at a temperature between about -20 °C and about 180 °C, preferably between about 20 °C and about 120 °C.

The intermediate formimidine (4) may either be isolated or cyclized in situ to imidazole (5) without isolation by further treatment with a base and under the conditions as described above, preferably using sodium methoxide in methanol at about 20-25 °C. Compounds of formulae (4) and (5) are new and are within the scope of the invention as intermediates in the methods or processes of synthesis of compounds of formula (I) of the invention.

The reaction of imidazole (5) with a sulfenyl halide, preferably chloride, $R_1SHalo$, in which $R_1$ is alkyl or haloalkyl, to give (6) conveniently may be conducted in an inert aprotic organic solvent such as a chlorinated hydrocarbon, a hydrocarbon, an ether, etc., preferably

## SCHEME I

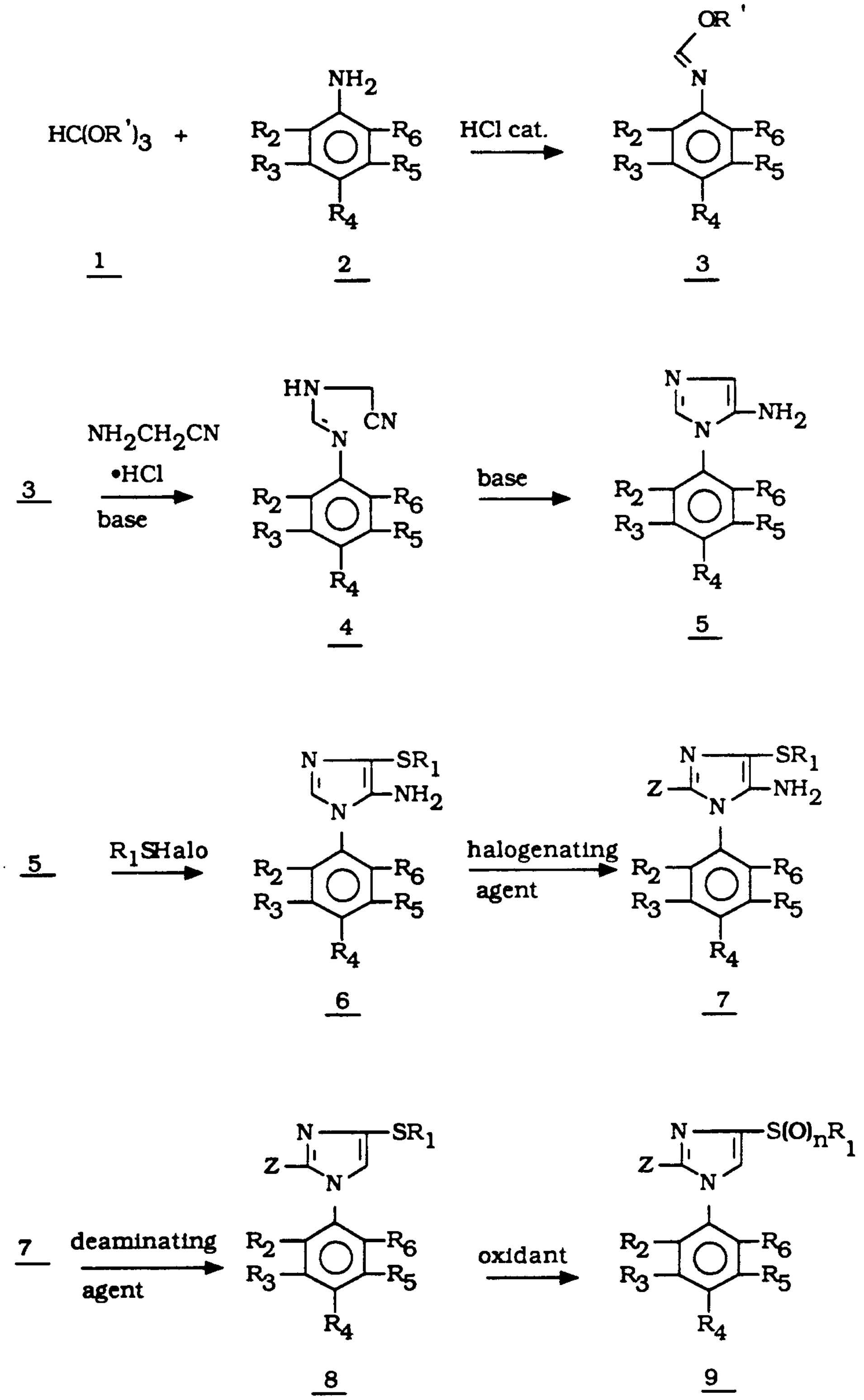


1 2 3 4 5 6 7 8 9

in dichloromethane, with or without an acid acceptor such as pyridine, any tertiary amine or an alkali metal carbonate. The reaction may be carried out between about -25°C and about 100°C depending on the boiling point of the sulfenyl halide reagent and the solvent.

The aminoimidazole (6) can be halogenated to the corresponding haloimidazole (7), Z is halogen, by reacting (6) with a halogenating agent such as sulfuryl chloride, thionyl chloride, chlorine or bromine and

with or without an acid acceptor or a catalyst such as a Lewis acid. The reaction is conducted in an inert aprotic organic solvent such as a chlorinated hydrocarbon or an ether. The reaction may be carried out between about -50 °C and about 150 °C, preferably between about -10 °C to about 110 °C, depending on the reactivity of aminoimidazole (6) and the reactivity of the halogenating agent used.

The desaminoimidazole (8) may be prepared by reacting the aminoimidazole (7) with an organic nitrite, such as t-butyl nitrite, in an organic solvent such as tetrahydrofuran between about -20 °C to about 180 °C, preferably between about 10 °C to about 100 °C.

The oxidation of the sulfide (8), n = 0, to the sulfoxide, n = 1, or sulfone (9), n = 2, may be carried out by using an appropriate quantity of peracetic acid, trifluoroperacetic acid, m-chloroperbenzoic acid, hydrogen peroxide, a combination of peracetic acid and hydrogen peroxide, or potassium peroxymonosulfate which is commercially available as Oxone®. The reaction is usually conducted in an inert organic solvent typically between about -30 °C to about 180 °C.

Additionally, compounds of formula (7) of SCHEME I can be converted to other compounds of the present invention. In a first case of substitutive deamination, (7) is initially reacted with a deaminating agent, such as described for the conversion of (7) to (8), and then it is immediately reacted with a quenching agent such as bromoform, cupric chloride or dimethyl disulfide to produce a compound of general formula (I) of the invention, wherein Y is a halogen atom or an alkylsulfenyl (n = 0) group, in which the alkyl is optionally halo-substituted, and Z is a halogen atom. The reaction is usually conducted in an inert organic solvent such as anhydrous acetonitrile, typically at a temperature between about -20 ° and about 180 °C, preferably between about 10 °C and about 100 °C. Further compounds, wherein Y are namely sulfoxides (an = 1) and sulfones (n = 2), of the invention can then be prepared by an oxidation reaction conducted in a similar manner for the conversion of (8) to (9).

In an alternative synthesis, a compound of formula (7) can be converted to a diazonium compound by reaction of the 5-amino substituent with nitrous acid at a temperature below about 5 °C. Subsequent decomposition of the diazonium compound in the presence of, for example, cuprous chloride, bromide, cyanide or nitrite via a Sandmeyer reaction provides compounds of general formula (I) of the invention, wherein Y is, for example, a chlorine or bromine atom or a cyano or nitro group and Z is a halogen atom.

Method IB A compound of formula (I), in which X is haloalkoxy, Y is as previously defined in Method IA, preferably a hydrogen or an optionally protected amino group, Z is hydrogen or halogen, preferably hydrogen, and $R_2$, $R_3$, $R_4$, $R_5$ and $R_6$ have the meanings given in the general definition of the invention may be prepared by the following procedures:

a) A useful intermediate compound, wherein X is halogen, such as bromo, chloro or iodo, Y is preferably hydrogen, amino or a protected amino group, and $R_2$, $R_3$, $R_4$, $R_5$ and $R_6$ are as defined above, can be prepared by a commonly utilized halogenation method from a compound of formula (5) with an appropriate amount of halogenating agent such as bromine, chlorine, sulfuryl chloride, N-chlorosuccinimide or N-bromosuccinimide in a suitable solvent such as haloalkane, ether, tetrahydrofuran or acetonitrile at a reaction temperature from about -25 °C to about 100 °C, preferably from about -10 °C to about 85 °C. To prevent further halogenation at the 2-position of imidazolyl ring, a stoichiometric amount of halogenating agent may be used. The compound obtained may be deaminated following a procedure similar to that described in Method I to give an intermediate compound wherein Y is hydrogen and X is halogen.

b) An intermediate compound, in which X is hydroxy, Y is preferably hydrogen or a protected amino, Z is preferably hydrogen, and $R_2$, $R_3$, $R_4$, $R_5$ and $R_6$ are as defined above, may be prepared by converting the intermediate, in which X is halogen, into the corresponding Grignard reagent or the corresponding lithium derivative following commonly known procedures, then followed by treatment with oxodiperoxymolybdenum(pyridine)(hexamethylphosphoric triamide) (MoOPH) by a procedure similar to that described by N. J. Lewis et. al. in J. Org. Chem., 1977, 42, 1479. Alternatively, the Grignard reagent or the lithium derivative described above may be reacted with a trialkyl borate followed by oxidation with hydrogen peroxide or other oxidizing agents to produce the hydroxy analog by a procedure similar to that reported by M. F. Hawthorne, J. Org. Chem., 1957, 22, 1001 or R. W. Hoffmann and K. Ditrich, Synthesis, 1983, 107.

c) A compound of formula (I), in which X is haloalkoxy, Y is preferably hydrogen or a protected amino, Z is preferably hydrogen, and $R_2$, $R_3$, $R_4$, $R_5$ and $R_6$ are as defined above, may be prepared from a corresponding compound in which X is hydroxy, Y is preferably hydrogen or protected amino, Z is preferably hydrogen, and $R_2$, $R_3$, $R_4$, $R_5$ and $R_6$ are as defined above, by various haloalkylating methods described in Synthesis of Fluoroorganic Compounds; Knunyants, I, L, and Yakobson, G. G., Ed.; Springer-Verlag: Berlin, 1985; pp 263-269, followed by a deprotection step, if necessary.

Method IC A compound of formula (I), in which X is haloalkyl, Y is as defined previously in Method IA, preferably an amino or protected amino, Z is hydrogen or halogen, preferably hydrogen, and $R_2$, $R_3$, $R_4$, $R_5$ and $R_6$ are as defined above, can be prepared from a compound of formula (5), following the sequence below:

a) Preparation of an intermediate compound, i.e. formula (11), in which X is formyl, Y is preferably amino or protected amino, Z is preferably hydrogen, and $R_2$, $R_3$, $R_4$, $R_5$, $R_6$ are as defined before, can be prepared by various methods of synthesis such as the Gattermann and Koch reaction, the Reimer-Tiemann reaction, the Vilsmeier-Haack reaction or modificaton of these methods. Under Vilsmeier conditions, the formylation can be carried out by treating a compound of formula (5), in which Z is hydrogen, with a disubstituted formamide, such as dimethyl formamide or N-phenyl-N-methylformamide, and phosphorous oxychloride which may be replaced with a halogen acid anhydride such as thionyl chloride, oxalyl chloride or phosgene. The reaction temperature may be from about -10 °C to about 200 °C, preferably from about room temperature to about 100 °C. Solvents to be used are those inert to the Vilsmeier Reaction and to the reagents involved, such as dichlorobenzene, carbon tetrachloride or dichloromethane. Another method of formylating a compound of formula (5) is to hydrolyze an intermediate compound, i.e. formula (10), in which X is bis(alkylthio)methyl or bis(arylthio)methyl (Ra is alkyl or aryl), by treating with an alkylnitrite, preferably isoamyl nitrite, in a suitable solvent such as a halogenated alkane, preferably dichloromethane, followed by a hydrolysis procedure similar to that reported by E. Fujita et. al, Tet. Let., 1978, 3561. Protection of the amino group with an appropriate protecting group may be necessary during the reaction with alkyl nitrites. The process for conversion of (10) to (11) may be generally represented as follows:

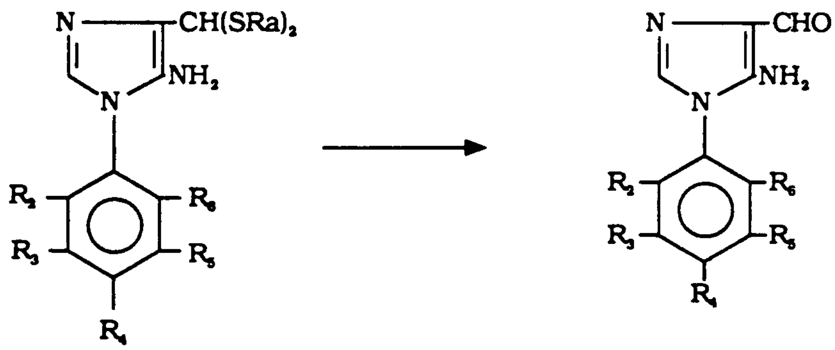

10 11

An intermediate compound, i.e. formula (10), in which X is a bis(alkylthio)methyl or bis(arylthio)-methyl group, Y is preferably amino, Z is preferably hydrogen, and $R_2$, $R_3$, $R_4$, $R_5$,and $R_6$ are those herein above defined for the definition of the invention, can be prepared by reaction of a compound of the formula (5) with tris(alkylthio)methane or tris(arylthio)methane, $(RaS)_3CH$, in the presence of a thiophilic Lewis Acid, preferably a sulfonium salt, such as dimethyl-(methylthio)-sulfonium tetrafluoroborate in an aprotic solvent, at a temperature between about -10 °C and about 100 °C, optionally in the presence of an acid accepter such as pyridine. A more preferred process employs acetonitrile or dichloromethane as solvent at about 25 °C with tris(methylthio)methane as the tris-(alkylthio)methane and dimethyl(methylthio)sulfonium tetrafluoroborate as the Lewis acid without an acid acceptor. A typical procedure is reported by R. A. Smith et. al., Synthesis, 166, 1984. The process is represented as shown below:

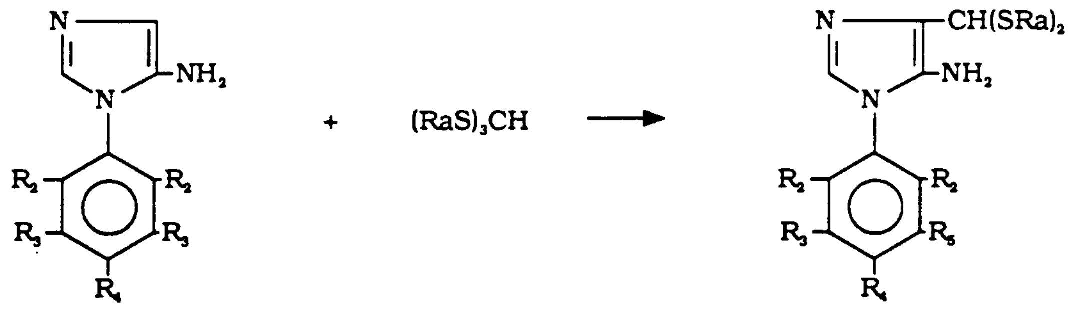

5 10

b) Preparation of an intermediate compound, i.e. formula (12), in which X is hydroxymethyl, Y is preferably amino or protected amino, Z is preferably hydrogen, and $R_2$, $R_3$, $R_4$, $R_5$ and $R_6$ have the meanings given in the definition of the invention, may be prepared by reduction of compounds of the formula (11). The reduction can be conducted with a reducing agent such as lithium aluminum hydride, sodium bohydride, aluminum isoproxide, borane and substituted boranes, and other metal hydrides in a suitable aprotic or protic solvent. For more reactive hydrides, e.g. lithium aluminum hydride, the reaction may be conducted in an inert solvent such as tetrahydrofuran, ethyl ether or dimethoxyethane, at a reaction temperature from about -10 ° C to about 20 ° C, preferably at a temperature from about 20 ° C to about 100 ° C. For milder hydrides, such as sodium borohydride, the reaction may be conducted in an alcohol such as methanol at a temperature from about -10 ° C to about 100 ° C, preferably from about room temperature to about 75 ° C.

c) A compound, i.e. formula (13), wherein X is haloalkyl, specifically chloromethyl, fluoromethyl, bromomethyl or iodomethyl, Y is preferably amino or protected amino, Z is preferably hydrogen, and $R_2$, $R_3$, $R_4$, $R_5$, and $R_6$ have the meanings given in the definition of the invention, can be prepared from intermediate compounds of formula (12), wherein X is hydroxymethyl, by using an appropriate

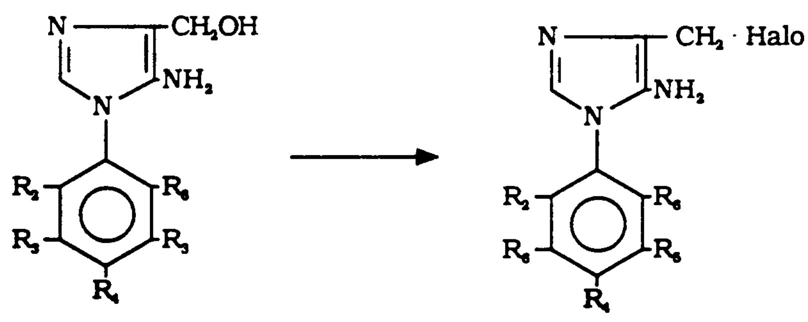

12 13

chlorinating, fluoronating or brominating agent. For chlorination, the reaction may be carried out with reagents such as thionyl chloride, phosphorous trichloride, phosphorous pentachloride or phosphorous oxychloride in dichloromethane or ethyl ether at a reaction temperature from about -20 ° C to about 100 ° C. The reaction can be carried out with or without the presence of an acid acceptor such as triethylamine or pyridine. For fluorination, the reaction can be conducted with dialkylaminosulfur trifluoride in a solvent such as acetonitrile, dichloromethane or glyme at a reaction temperature from about -20 ° C to about 100 ° C. A more preferable condition uses diethylaminosulfur trifluoride in acetonitrile at about room temperature. A representative procedure is given by W. J. Middletown , J. Org. Chem., (1975), 42, 5, 574. Other fluororinating reagents that may be used are sulfur trifluoride, bis-(dialkylamino)-sulfur trifluoride or sodium or potassium fluoride in a polyhydrogen fluoride-pyridine solution. The procedure is similar to that reported by Olah and Welch, Synthesis, 653, (1974). For bromination, the reaction may be conducted with brominating agents such as bromine, n-bromosuccinimide, phosphorous tribromide or hydrogen bromide in an inert solvent such as dichloromethane or

ethyl ether at a temperature from about -20°C to about 100°C. For iodidation, the reaction may be performed with hydrogen iodide in an inert solvent such as dichloromethane at a reaction temperature from about -20°C to about 100°C. The above-mentioned halogenations can be carried out with a deactivating group attached to the amino function such as an acyl group to prevent the additional halogenation at the 2-position of the imidazolyl ring.

d) Alternatively, a compound of formula (I), in which X is a haloalkyl group, Y is preferably amino, Z is preferably hydrogen, and $R_2$, $R_3$, $R_4$, $R_5$ and $R_6$ are as defined above, may be prepared from the corresponding compound in which X is a formyl group or a carboxylic function and the Y amino group is optionally protected. For example, treatment of the formyl compound with diethylaminosulfur trifluoride in a manner analogous to that described by W. J. Middleton in J. Org. Chem, 1975, 40, 574 provides the compound of formula (I) in which X is a difluoromethyl group and the other substituents are as defined above. Oxidation of the above mentioned intermediate compound, wherein X is formyl, with an oxidizing agent such as potassium permanganate in acetone-water or chromium trioxide in sulfuric acid, known as Jones' reagent, gives an intermediate compound, wherein X is carboxyl, Y is preferably amino, Z is preferably hydrogen, and $R_2$, $R_3$, $R_4$, $R_5$ and $R_6$ are as defined above. Reaction of the compound above in which X is carboxyl with sulfur tetrafluoride, similar to that described by G. A. Boswell et. al. Org. Reaction, 1974, 21, 1-124, gives the compound of formula (I) in which X is a trifluoromethyl group and the other groups are as defined above.

## Method II

A compound of formula (I) of the invention, wherein X and Y are as defined and prepared by the Methods IA, IB, and IC, Z is halogen, preferably chlorine, and n, $R_1$, $R_2$, $R_3$, $R_4$, $R_5$ and $R_6$ are as previously defined, can be prepared by procedures described in SCHEME II.

According to SCHEME II, intermediates of formulae (14) and (15) may be prepared in a similar manner to the method described in GB Patent Specification No. 2,203,739, incorporated herein by reference.

For the subsequent reactions, the conditions used in the alkylation of (15) to (16), the ring closure of (16) to (17) and the preferred sulfenylation substitution of (17) to (18) are similar to the ranges of reaction parameters described for related compounds, i.e., compounds of formulae (3) to give (4), (4) to give (5), and (5) to give (6), respectively, prepared according to SCHEME I. Compounds of formulae (17) and (18) of SCHEME II are analogous to compounds of formulae (5) to (7) of SCHEME I and thus compounds of formulae (17) and (18) can be converted to other compounds of the invention, wherein Z is halogen and X, Y, n, $R_1$ to $R_6$ are as defined in Method I, in a similar manner as described in SCHEME I and Method I or alternatives thereto. Compounds of formulae (16) and (17) are new and are within the scope of the invention as intermediates in the methods or processes of synthesis of compounds of formula (I) of the invention.

## Method III

A compound of the formula (I), in which Z is an alkyl or halogen substituted alkyl group, and X, Y, n, $R_1$, $R_2$, $R_3$, $R_4$, $R_5$ and $R_6$ are as defined in Method I or in the definition of formula (I), can be prepared according to the SCHEME (III). The amide (19) can be prepared by well known methods using an acyl halide, anhydride or ester. When reacting with an acyl halide, a base may be used as a catalyst or the aniline is converted to the corresponding amide anion with metal hydride or metal alkane. The reaction temperature may be from about 4°C to about 100°C for the acyl halide reaction. When using an anhydride, the reaction may be conducted with various inorganic or organic acid catalysts, Lewis acids or basic catalysts, such as pyridine or triethylamine. The reaction temperature may be from about -10° to about 150°C. This reaction may be enhanced with a metal catalyst, such as zinc dust.

The amide (19) can be halogenated into an imido halide (20) using a halogenating agent such as phosphorous pentahalide in an inert solvent such as dichloromethane, acetonitrile or chloroform. The

## SCHEME II

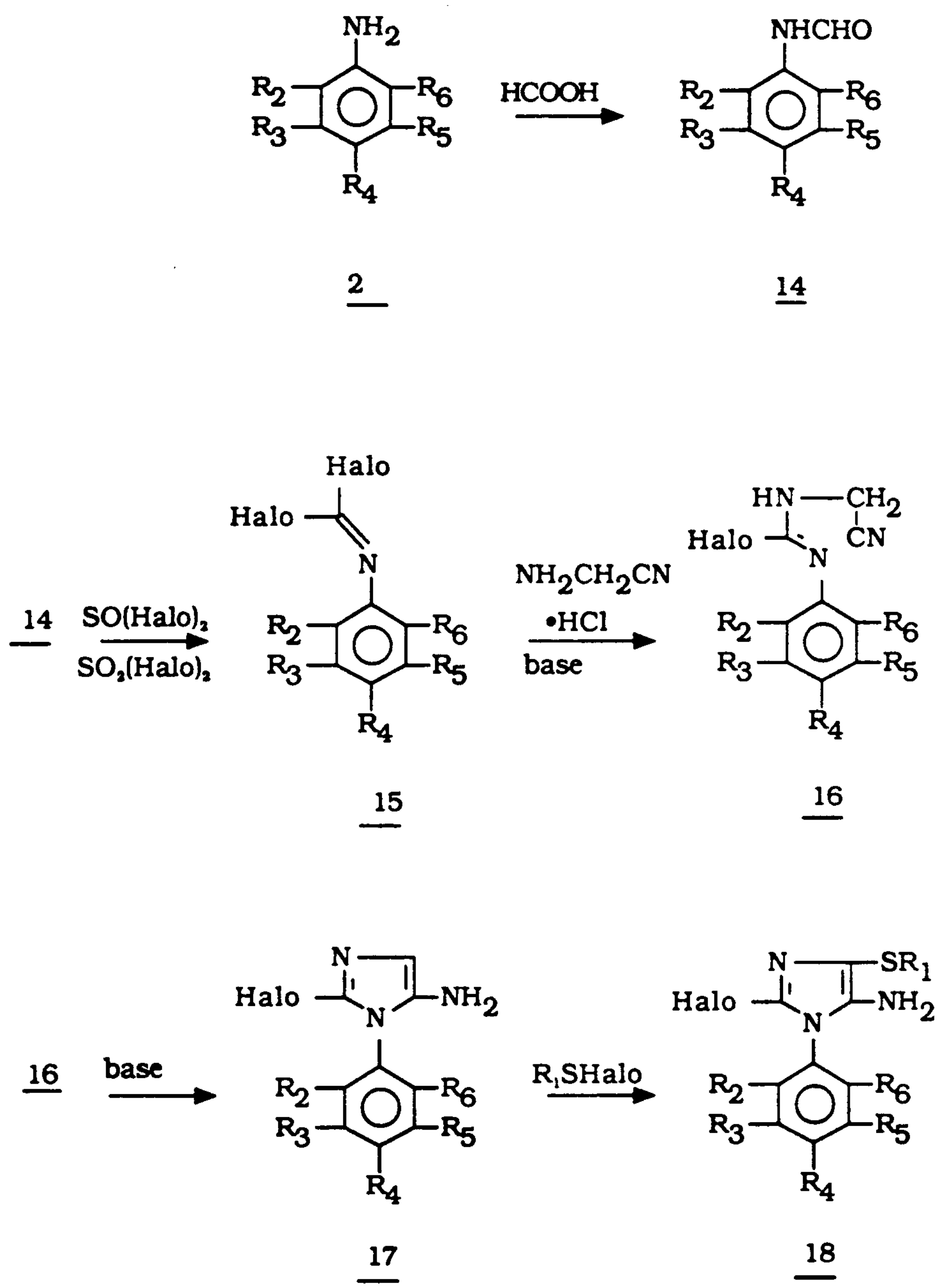


preferred solvents are halogented alkanes, such as chloroform and dichloromethane. The alkylation to (21) may be conducted with aminoacetonitrile or its hydrochloride salt in the presence of a base, such as a carbonate, hydroxide or trialkylamine, preferably potassium carbonate in an appropriate solvent, such as tetrahydrofuran, acetonitrile or chloroform. The ring closure to (22) can be achieved by treating the amidine (21) with a catalytic amount of a base, such as an amine or alkali, hydroxide or alkoxide in a suitable solvent, such as an alcohol or halogenated alkane. The reaction is preferably carried out with sodium methoxide in anhydrous methanol at ambient temperature. The ring closure to (22) can also be achieved in a one step reaction from (20) via (21) by using more than one equivalent of aminoacetonitrile in a suitable solvent such as chloroform at reflux temperature.

A compound of formula (23), wherein Z is alkyl or haloalkyl, Y is amino, $R_2$ to $R_6$ are as defined for general formula (I), and X is alkylsulfenyl, haloalkylsulfenyl, alkylsulfinyl, haloalkylsulfinyl, alkylsulfonyl, haloalkylsulfonyl, haloalkyl or haloalkoxy, can be prepared by the procedures described in Method I.

Further compounds of the invention, wherein Y is defined by formula (I), can be prepared from a compound of formula (23) by the Methods herein described for the conversion of Y is amino into other defined Y substituents of formula (I).

Method IV

A compound of formula (I), in which X is haloalkyl, particularly perfluoroalkyl, Y is amino or may be additionally other Y substituents defined by formula (I), Z is halogen, alkyl or haloalkyl, and $R_2$, $R_3$, $R_4$, $R_5$

## SCHEME III

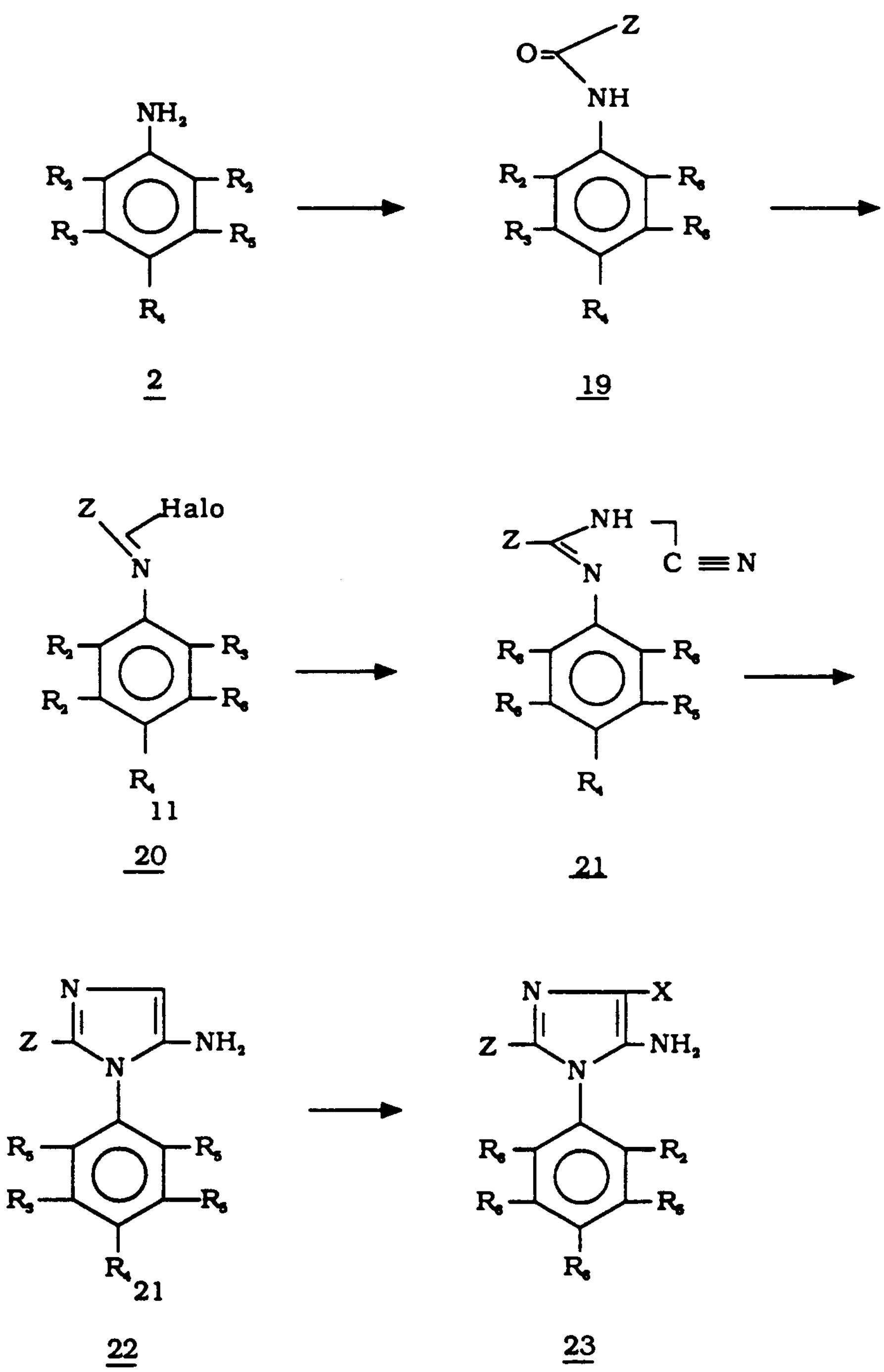

2

19

20

21

22

23

and $R_6$ have the meanings given in the definition of formula (I), may be prepared by the sequences described below:

The intermediate compound of formula (25) can be prepared by reacting the known iminolperfluoronitrile, (24), with a compound of formula (20) in the presence of a base catalyst such as pyridine at a reaction temperature from about -75 °C to about 100 °C, preferably at the temperature from about 0 °C to about 85 °C. Iminoperfluoronitriles are known compounds and various compounds of this type can be prepared according to the procedure reported by W. J. Middleton and C. G. Krespan J. Org. Chem., 33, 9, 3625, (1968). The nucleophilic property of iminoperfluoronitrile with a basic catalyst has also been demonstrated in the same report. The transformation is represented by the following equation:

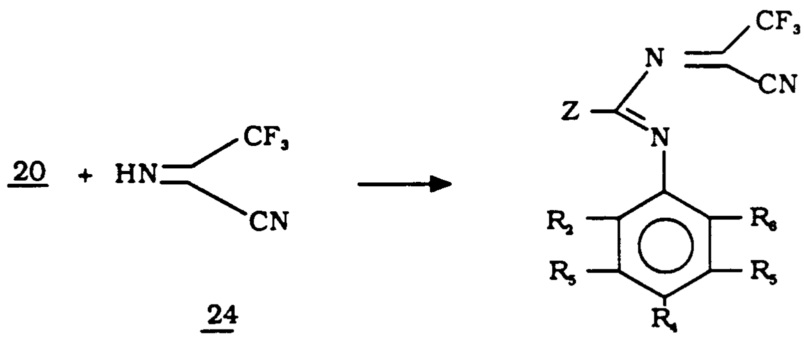

20

24

25

The intermediate compound of formula (25), described above, can be treated with a reducing agent, such as sodium borohydride in an inert solvent such as an alcohol or ether, at a reaction temperature from about 0 °C to about 85 °C to produce an intermediate compound of formula (26). Sodium borohydride, in general, reduces an imino function, but keeps the nitrile function unaffected (see Jerry March, "Advanced Organic Chemistry, McGraw-Hill Book Company, p. 834-835, 2nd Edition and references cited therein).

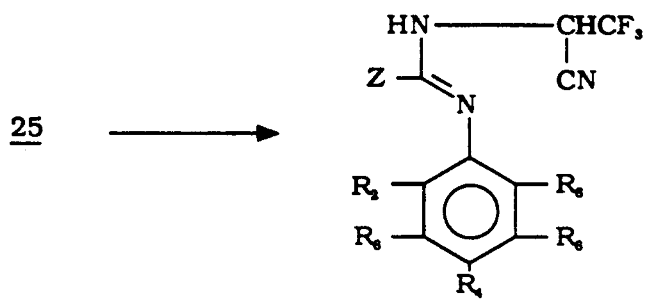

25

26

The intermediate of formula (26) can then be ring-closed in the same fashion as that described in Method I to give a compound of the invention of formula (27), wherein Z is halogen, alkyl or haloalkyl, and $R_2$ to $R_6$ are as defined in formula (I) of the invention.

26

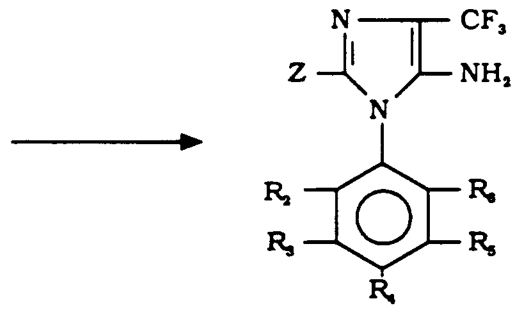

27

Further compounds of the invention, wherein Y is defined by formula (I), can be prepared from a compound of formula (27) by Methods described herein for the conversion of Y is amino into other defined Y substituents of formula (I).

Method V

A compound of formula (I), wherein Y is a hydroxy, alkoxy or haloalkoxy, Z is alkyl, haloalkyl or halogen, X is as defined for formula (I), preferably perhaloalkylsulfenyl, perhaloalkylsulfinyl, perhaloalkylsulfonyl, and $R_1$, n, $R_2$, $R_3$, $R_4$, $R_5$ and $R_6$ have the meanings given previously, can be prepared by the following processes:

a) A compound of the formula (28), in which Z is alkyl, haloalkyl, halogen, and $R_2$, $R_3$, $R_4$, $R_5$, and $R_6$ are herein above defined for the general definition of formula (I), can be prepared by alkylation with glycine or a glycine ester of an appropriate imino halide, such as the compound indicated by the formula (20), from SCHEME III, wherein Z is halogen, alkyl or haloalkyl. The reaction can be conducted in an inert organic solvent, such as dichloromethane, chloroform, tetrahydrofuran or ethyl ether at a reaction temperature from about -20°C to about 150°C, depending on the size and the electronic effect of the Z group. In the subsequent reactions, the conditions for the ring closure to the compound of formula (29) - (or its enolate form 30)) or salts thereof, and the sulfenylation of a compound of formula (30) or salts thereof to a compound of formula (31) and salts thereof are similar to the ranges of reaction parameters described for related compounds, i.e., compounds of formula (4) to give (5), and (5) to give (6), respectively according to SCHEME I, Method I. The corresponding compound, in which Y is alkoxy or haloalkoxy, can be prepared following the well known Williamson synthesis. The ether formation can be achieved by reacting the preformed alkoxide in an inert solvent, such as ethyl ether or tetrahydrofuran, with an appropriate alkylating agent such as an alkyl halide or alkyl sulfate at a reaction temperature of about -10°C to about 100°C, preferably at a temperature from about 4°C to about 50°C. The ether formation may be more efficiently carried out in two phases involving use of a phase-transfer catalyst. An example of the reaction system is: water, dichloromethane, a quaternary ammonium hydroxide, a compound of formula (31) and an alkyl halide. The procedure may be similar to the one reported by Freeman and Dubois, Tet. Let, 3251 (1975). The intermediate compound of formula (30), before sulfenylation, may be optionally alkylated or haloalkylated by the methods described above followed by alkylsulfenylation or haloalkylsulfenylation according to procedures parallel to those described in Method I to obtain a compound of formula (32). Compounds of formulae (31) and (32) may be oxidized by the procedures also outlined in Method I to prepare the corresponding sulfoxide (n = 1) and sulfone (n = 2) compounds, X = $S(O)_nR_1$, in which $R_1$ is as previously defined.

Additionally, a compound of formula (I), wherein Z is alkyl, haloalkyl or halogen, Y is hydroxy, alkoxy, or haloalkoxy and X, and $R_2$ to $R_6$ are as defined for formula (I), may be prepared from the compound of formula (30), or optionally alkoxylated or haloalkoxylated analogs thereof, by appropriate conversion of the compound in which X is hydrogen, to an X substituent defined for formula (I) by the Methods describe herein.

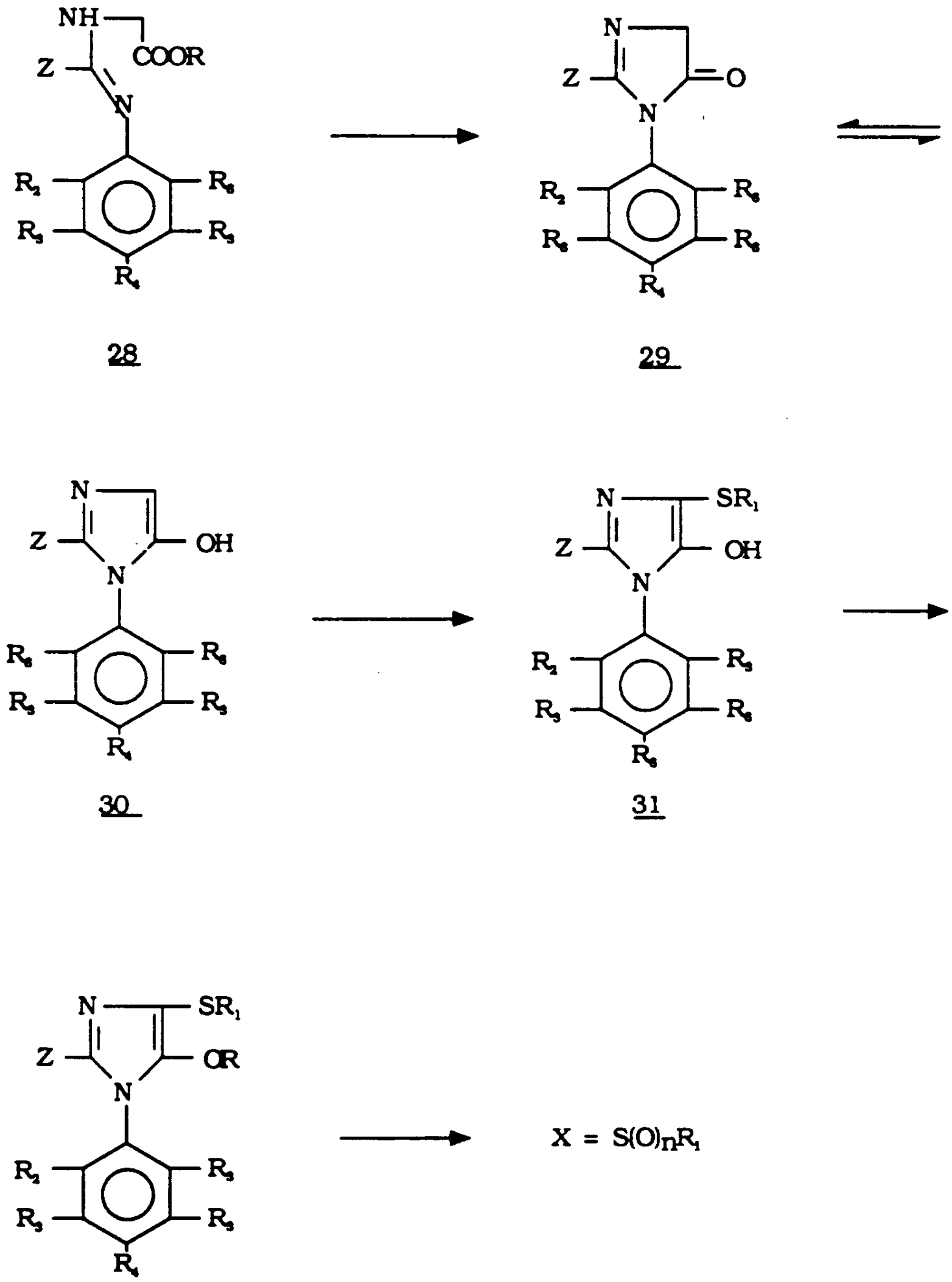


28

29

30

31

32

Method VI

A compound of formula (I), wherein Z is hydroxy, alkoxy, haloalkoxy or halogen, Y is the substituent defined in formula (I), particularly amino, X is the substituent defined in formula (I), particularly $S(O)_nR_1$, and n, $R_1$ and $R_2$ to $R_6$ are as previously defined, can be prepared according to the following synthetic sequences:

a) The appropriate aniline is first converted into the corresponding isocyanate by treatment of the aniline with phosgene or oxalyl chloride in an inert solvent such as dichloromethane or chloroform. The isocyanate compound is then to reacted with aminoacetonitrile to give the urea of formula (33). The urea compound of formula (33) can be ring-closed into the corresponding iminohydantoin of formula (34) or

salts thereof in the presence of a base such as an alkali alkoxide or amine. The iminohydantoin can then be chlorinated with chlorinating agents such as phosphorous pentachloride, thionyl chloride, phosphorous oxychloride or phosphorous pentachloride, preferably with phosphorous pentachloride, at a reaction temperature from about -10 °C to about 180 °C, preferably from about room temperature to about 100 °C. The 2-halogenated imidazole (Z is halogen), (35), or salts thereof can then be alkylsulfenylated into the desired alkyl or haloalkyl sulfenyl products of formula (36), in which X is $SR_1$. These sulfenylated compounds (36) can then be further oxidized into other compounds of the invention, namely sulfoxides or sulfones, $S(O)_nR_1$ in which n is 1 or 2 and $R_1$ is as defined. The procedures for sulfenylation and oxidation are similar to those described in Method I.

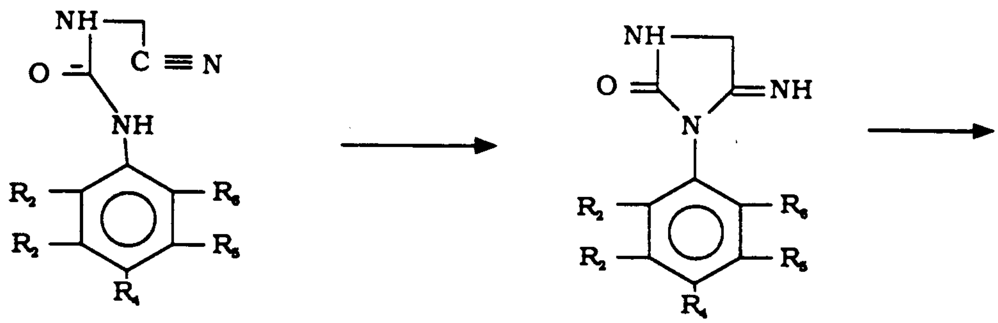

33

34

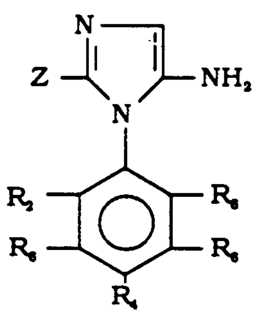

35

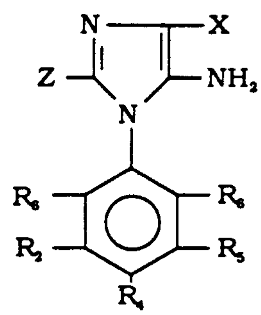

36

b) A compound of formula (I), in which Z is hydroxy or salts thereof, alkoxy, haloalkoxy, Y is amino, hydrogen or halogen, and X, $R_2$, $R_3$, $R_4$, $R_5$ and $R_6$ have the meanings described in the definition of the invention, may be prepared from a compound of formula (34) wherein $R_2$, $R_3$, $R_4$, $R_5$ and $R_6$ are as defined above, by the scheme described below:

The iminohydantoin (34) may be aromatized into its corresponding 2-hydroxy-5-aminoimidazole (37) or salts thereof by an appropriate PH control in a suitable solvent. The hydroxy imidazole of formula (37) or salts thereof can be sulfenylated with an appropriate sulfenyl halide, $R_1$SHalo, preferably chloride, to give a compound where Z is hydroxy, Y is amino, and X is $S(O)_nR_1$, in which n is 0 and $R_1$ is as defined, by procedures similar to those described in Method I. The corresponding desamino analog (Y is hydrogen) may be prepared by deamination with t-butylnitrite or via the diazonium intermediate following a procedure similar to that described in Method I. By the Sandmeyer reaction, the 5-halo-2-hydroxyimidazole may thus be prepared. Additionally, the sulfenylated analogs above may be deaminated to give compounds wherein X is $S(O)_nR_1$, Y is alkylsulfenyl or halogen, and Z is hydroxy or halogen.

The 2-alkoxy- or 2-haloalkoxy-3-sulfenylated-imidazole analogs (Z is alkoxy or haloalkoxy) of formula (39) may be prepared via the intermediate compound (38) which may be prepared by direct alkylation with an appropriate alkylating agent, such as alkyl iodide, haloalkyl iodide, alkyl bromide and dialkylsulfate, of a

compound of formula (34)/37) in a suitable solvent, such as tetrahydrofuran, alcohol, acetonitrile, acetone, etc., at a reaction temperature from about room temperature to about 150 °C, preferably from about room temperature to about 100 °C. The subsequent sulfenylation to (39) can be conducted according to a procedure similar to that described in Method I for general sulfenylation. Alternatively, the alkylation step to a compound in which Z is alkoxy or haloalkoxy may be carried out after the sulfenylation and deamination by procedures similar to those described above. If the O-alkylation is to be carried out prior to deamination an appropriate amino protection group (W) may be introduced before the O-alkylation reaction and then subsequently removed.

Additionally, from the various above compounds, wherein Z is hydroxy or salts thereof, alkoxy or haloalkoxy, X is hydrogen, and Y is

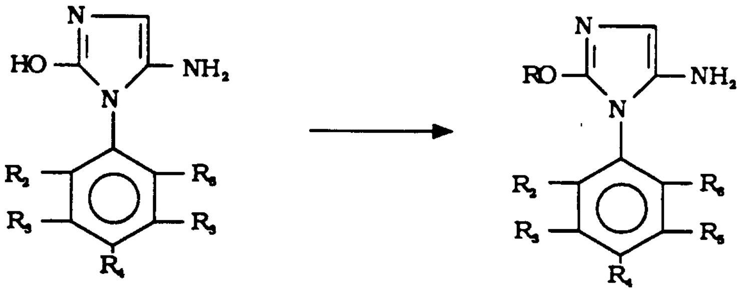

37 38

38

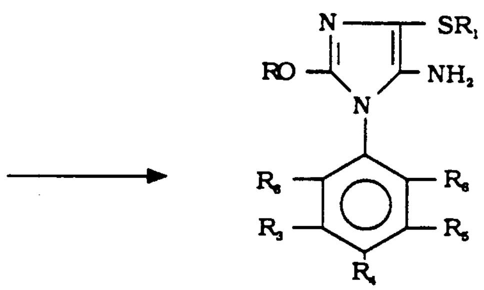

39

37

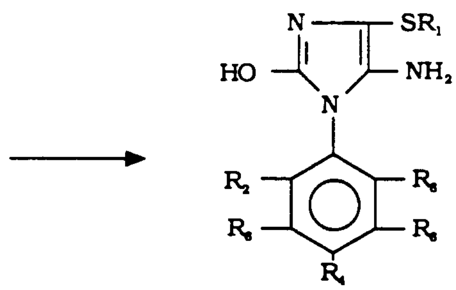

40

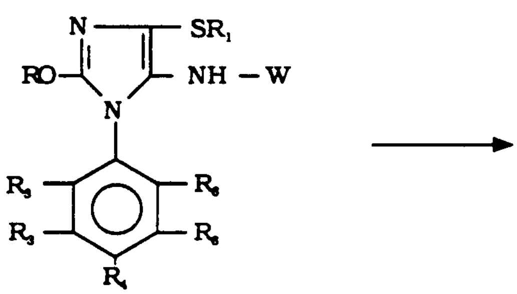

39

41

amino or hydrogen, other compounds of the invention of formula (I), wherein X and Y are defined in formula (I), can be prepared according to the Methods described herein, specific for X and Y.

Methods VII to XXVIII Generalization

The following Methods VII to XXVIII detail specific procedures for introducing a Z substituent into a particular compound of formula (Ia) to provide a further useful compound of formula (Ib).

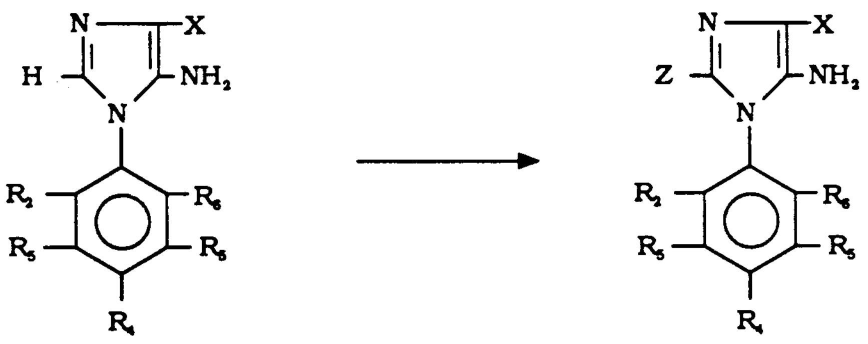

(Ia) (Ib)

Method VII

A compound of formula (Ib), in which Z is aminosulfonyl, alkylaminosulfonyl or dialkylaminosulfonyl, Y is $NH_2$, and X, $R_2$, $R_3$, $R_4$, $R_5$ and $R_6$ have the meanings given in the definition of formula (I), can be prepared from a compound of formula (I), wherein Z is hydrogen, Y is amino, and X, $R_2$, $R_3$, $R_4$, $R_5$ and $R_6$ have the meanings herein above defined, by the following sequence:

a) An intermediate compound of formula (Ib), in which Z is chlorosulfonyl, Y is amino, and X, $R_2$, $R_3$, $R_4$, $R_5$ and $R_6$ have the meanings given in the definition of formula (I) may be prepared by treating a compound of formula (Ia), wherein Z is hydrogen and X, Y, and $R_2$ to $R_6$ are as defined above, with chorosulfonic acid or dichlorosulfonic acid.

b) The compound of formula (Ib), wherein Z is aminosulfonyl, alkylamino-sulfonyl or dialkylaminosulfonyl, can be prepared by reacting the chlorosulfonyl intermediate with ammonia or an appropriate alkylamine or dialkylamine in a suitable solvent such as halogenated alkane, ether, tetrahydrofuran or hexane, at a reaction temperature from about -50°C to about 50°C, preferably from about -20°C to about room temperature.

Method VIII

A compound of formula (Ib), in which Z is nitro or halogen, Y is amino or protected amino, and X, $R_2$, $R_3$, $R_4$, $R_5$ and $R_6$ have the meanings given in the definition of formula (I), can be prepared by direct nitration or halogenation of a compound of formula (Ia), wherein Z is hydrogen, and X, Y, $R_2$, $R_3$, $R_4$, $R_5$ and $R_6$ are as defined above.

The nitration may be conducted with variety of nitrating agents, such as a mixture of concentrated nitric acid and sulfuric acid in acetic acid or acetic anhydride, dinitrogen pentaoxide in halogenated alkane, an ester of nitric acid such as ethyl nitrate, a mixed anhydride such as acetyl nitrate, nitryl halide with or without a Friedel-Crafts catalyst such as ferric chloride or methyl nitrate, or a nitronium salt such as nitronium tetrafluoroborate. The reaction may be conducted in a suitable solvent, such as acetic acid, acetic anhydride, tetramethylene sulfone, tetrahydrofuran or water under neutral, basic or acidic conditions at a reaction temperature from about -50°C to about 155°C. A preferred procedure is to conduct the nitration using nitryl chloride in the presence of titanium tetrachloride in tetramethylene sulfone at a reaction temperature from about -10°C to about 25°C.

The corresponding amino derivative of formula (Ib), Z is amino, may then be conveniently prepared by a standard reduction of the above-mentioned nitro analog. A variety of reducing agents are well known. Examples are zinc, tin, or iron with hydrochloric acid reduction, catalylic hydrogenation and sulfides such as NaHS, $(NH)_4S$ or polysulfide.

The compound of formula (Ib), in which Z is halogen, may be obtained from a compound of (Ia), wherein Z is hydrogen according to halogenation procedures similar to those in Method IB.

Method IX

A compound of formula (Ib), in which Z is alkyl, hydroxyl and salts thereof, alkoxy or haloalkoxy, Y is amino or protected amino, and X, $R_2$, $R_3$, $R_4$, $R_5$ and $R_6$ have meanings given in the definition of formula (I), may be prepared from a compound of formula (Ia), wherein Z is hydrogen and the other groups are as defined above, by treatment with a strong base, preferably an organic base such as lithium diisopropylamide or n-butyllithium in a suitable solvent such as tetrahydrofuran or ethyl ether to give an organometalic carbanion. By quenching the carbanion with an appropriate alkylating agent such as alkyl halide or dialkylsulfate the compound in which Z is alkyl is obtained. Alternatively, the carbanion can be reacted, according to procedures similar to those described in Method IB, to first give a compound wherein Z is hydroxyl and then by standard alkylating conditions, the compound in which Z is alkoxy or haloalkoxy is obtained.

Method X

A compound of the formula (Ib), wherein Z is formyl, Y is amino and X, $R_2$, $R_3$, $R_4$, $R_5$ and $R_6$ have the meanings given in the definition of formula (I), that is to say a compound of the formula (42), may be prepared by the Vilsmeier-Haack Reaction or modifications thereof. This formylation can be carried out by treating a compound of formula (Ia), e.g. (6) in which Z is hydrogen, with a disubstituted formamide, such as dimethyl formamide or N-phenyl-N-methyl-formamide, and phosphorous oxychloride which may be replaced with a halogen acid anhydride such as thionyl chloride, oxalyl chloride or phosgene. The reaction temperature may be from about -10 °C to about 200 °C, preferably from about room temperature to about 100 °C. Solvents to be used are those inert to the Vilsmeier Reaction and to the reagents involved, such as dichlorobenzene, carbon tetrachloride or dichloromethane.

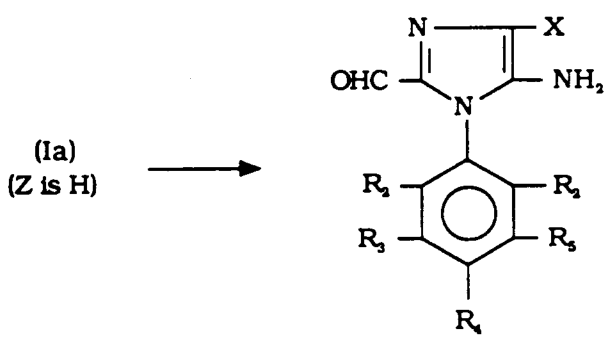

42

Method XI

Another method of formylating to give a compound of formula (Ib), wherein Z is formyl, Y is amino, and X, $R_2$, $R_3$, $R_4$, $R_5$ and $R_6$ are as defined in formula (I), is described as follows.

A compound of formula (42), wherein Z is formyl, can be prepared by hydrolyzing a compound of formula (43), wherein Z is a bis(alkylthio)- or bis(arylthio)methyl group. This is done by treating with an alkylnitrite in a suitable solvent such as a halogenated alkane, preferably isoamyl nitrite in dichloromethane, followed by hydrolysis similar to the procedure reported by E. Fujita et. al., Tet. Let., 1978, 3561. Protection of the amino group with an appropriate protecting group may be necessary during the reaction with alkyl nitrites. The process may be generally represented as follows:

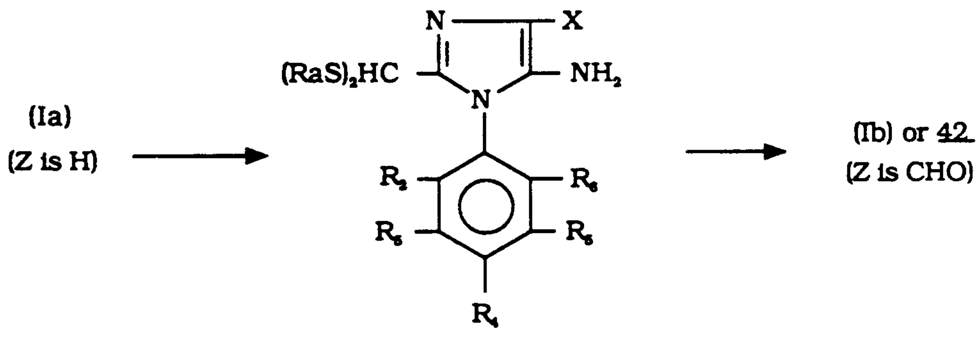

(Ia) (Z is H)

(Ib) or 42 (Z is CHO)

43

Method XII

An intermediate compound of formula (43), in which Z is a bis(alkylthio)methyl or bis(arylthio)methyl group, Y is amino, and X, $R_2$, $R_3$, $R_4$, $R_5$, and $R_6$ are those herein above defined for the definition of formula (I), can be prepared by reaction of a compound of the formula (Ia), e.g. (6), in which Z is hydrogen and X, Y, and $R_2$ to $R_6$ are as defined above, with tris(alkylthio)methane or tris(arylthio)methane, $(RaS)_3CH$, in the presence of a thiophilic Lewis Acid, preferably a sulfonium salt such as dimethyl(methylthio)sulfonium tetrafluoroborate in an aprotic solvent at a temperature between about -10 °C and about 100 °C, optionally in the presence of an acid accepter such as pyridine. A more preferred process employs acetonitrile or dichloromethane as solvent at about 25 °C with tris(methylthio)methane as the tris(alkylthio)methane and dimethyl(methylthio)sulfonium tetrafluoroborate as the Lewis acid, without an acid acceptor. A typical procedure is reported by R. A. Smith et. al., Synthesis, 166, 1984.

Method XIII

A compound of formula (Ib), in which Z is methyl, Y is amino, and X, $R_2$, $R_3$, $R_4$, $R_5$ and $R_6$ have the meanings given in the definition of formula (I), may be conveniently prepared by reduction of a compound of formula (Ia), i.e. (42), wherein Z is formyl and the other groups are as defined above. The reduction may be conducted with sodium borohydride in a suitable solvent such as an alcohol at a reaction temperature from about -10 °C to about 120 °C, preferably in methanol at a temperature from about room temperature to about 80 °C. Alternatively, the analog wherein Z is methyl may be prepared by a sequential treatment of the formyl compound, (42), with p-toluenesulfonylhydrazine and sodium cyanoborohydride according to a method similar to that described in J. Am. Chem. Soc. 1971, 93, 1793.

Method XIV

A compound of formula (Ib), i.e. (44), in which Z is a carboxylic group or salts thereof, Y is amino or protected amino, and X, $R_2$, $R_3$, $R_4$, $R_5$ and $R_6$ have the meanings given in the definition of formula (I), can be prepared from a compound of formula (42), in which Z is formyl, by treatment with a variety of oxidizng agents such as potassium permangante in acid, basic or neutral solution, chromic acid, bromine, silver oxide or molecular oxygen in a suitable solvent. Selection of solvent will depend on the solubility of oxidizing agent and the substrate. Examples of solvents are acetone, water, alcohol, tetrahydrofuran, dimethoxyethane acetonitrile or a halogenated hydrocarbon such as dichloromethane or chloroform. The reaction temperature may range from about -20 °C to about 150 °C, preferably from about room temperature to about 100 °C.

Method XV

A compound of formula (Ib), i.e. (45), in which Z is cyano, Y is amino or protected amino, and X, $R_2$, $R_3$, $R_4$, $R_5$ and $R_6$ have the meanings given in the general definition of formula (I), may be prepared by reaction of a compound of formula (44), in which Z is carboxyl, with isophthalonitrile at a reaction temperature from about 100 °C to about 300 °C. A representative example of a procedure for the

transformation is given in J. Org. Chem, 1958, 23, 1350.

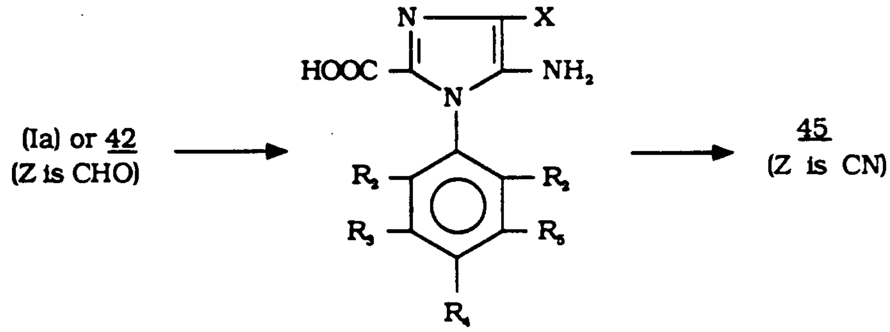


44

Method XVI

Alternatively, the cyano analog of formula (45), wherein Z is cyano, Y is amino or protected amino, and X, $R_2$, $R_3$, $R_4$, $R_5$ and $R_6$ are as defined by formula (I), can be prepared by the sequential transformation of a formyl compound of formula (42), in which Z is formyl, to its corresponding aldoxime of formula (46), in which all other substituents are as defined in formula (42), followed by a dehydration reaction. The dehydration reaction may be achieved with variety of dehydrating agents, such as acetic anhydride, diphenyl hydrogen phosphonate, 2,4,6-trichlorotriazene or ethylorthoformate and acid. Preferably the dehydrating agent is acetic anhydride at a reaction temperature from about -10 °C to about 180 °C. The aldoxime intermediate of formula (46) can be prepared by reacting an aldehyde of formula (42) with hydroxyamine in a suitable solvent such as an alcohol, tetrahydrofuran, water, a halogenated hydrocarbon or a mixture solvent of halogenated hydrocarbon, alcohol and water. The reaction temperature may range from about -10 °C to about 120 °C, preferably from about 4 °C to about 50 °C.

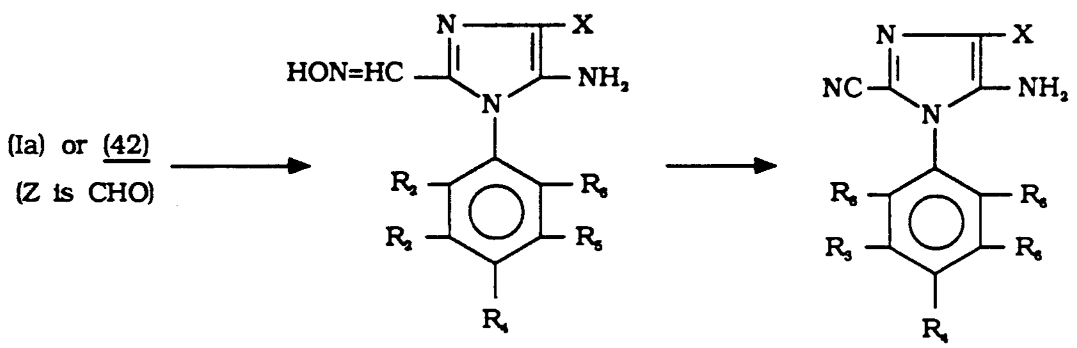


46 45

Method XVII

A compound of formula (Ib), i.e. (48), in which Z is aminocarbonyl, alkylaminocarbonyl, dialkylanimocarbonyl or alkoxycarbonyl (Z is $COZ^1$ in which $Z^1$ is amino, aklylamino, dialkylamino or alkoxy), Y is amino or protected amino, and X, $R_2$, $R_3$, $R_4$, $R_5$ and $R_6$ have the meanings given in the definition of formula (I), may be prepared by sequential transformation from a compound of formula (44), in which Z is carboxy, to the corresponding intermediate acid halide of formula (47) such as an acid chloride, then followed by reaction of the acid halide with ammonia or an appropriate alkylamine, dialkylamine or alkyl alcohol. The chlorination can be achieved by reacting the acid with a chlorinating agent such as thionyl chloride, hydrogen chloride,

oxalyl chloride, phosphorous trichloride, phosphorous pentachloride or triphenylphosphine in carbon tetrachloride in the presence of a base as catalyst such as pyridine or triethylamine in an inert solvent such as dichloromethane, ethyl ether, acetonitrile, carbon tetrachloride or tetrahydrofuran at a reaction temperature from about -20 ° C to about 150 ° C. The preferred conditions are thionyl chloride in dichloromethane at reflux temperature. The reaction between the acid halide and the appropriate amine or alcohol can be carried out in an inert solvent such as dichloromethane, chloroform, toluene, acetonitrile or tetrahydrofuran at a reaction temperature from about -20 ° C to about 120 ° C, preferably at the temperature from about -20 ° C to about room temperature.

(44)

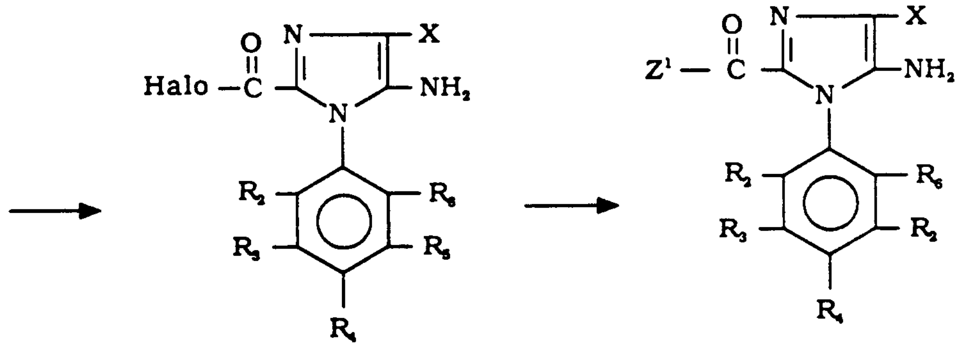

47

48

Method XVIII

A compound of formula (Ib), in which Z is amino, alkylamino, dialkylamino or trialkylammonium salt, Y is amino or protected amino, and X, $R_2$, $R_3$, $R_4$, $R_5$ and $R_6$ have the meaning given in the definition of formula (I), may be synthesized from a compound of formula (44), in which Z is carboxyl, by the method of the Curtius reaction or a modification thereof such as the Yamada modification. By the conventional Curtius rearrangement, the desired amino derivative may be obtained by a sequential transformation from an acyl halide of formula (47) to an azide of formula (49) by treating the acyl halide with sodium aside or tetramethylguanidinium aside which can then be pyrolyzed into its corresponding isocyanate (50). The isocyanate (50) can then be hydrolyzed into the corresponding amine (51) in which Z is amino. By the Yamada modification, the reaction may be accomplished by treating an acid of formula (44), in which Z is carboxyl, with diphenylphosphoryl azide in the presence of a base such as triethylamine in an inert solvent such as toluene, benzene or tetrahydrofuran at a reaction temperature from about 0 ° C to about 150 ° C to give the isocyanate intermediate (50) which can then be hydrolyzed with water to afford the compound of the formula (51). A representative procedure is given in Shioro et. al. J. Am. Chem. Soc. 1972, 94, 6203. The corresponding compound of formula (Ib), wherein Z is alkylamino, dialkylamino or trialkylammonium salt, namely (52), can be conveniently prepared by monoalkylation, dialkylation and trialkylation using a alkylating agent such as an alkyl iodide or dialkyl sulfate in an inert solvent such as acetonitrile, tetrahydrofuran or dimethoxyethane at a reaction temperature from about 0 ° C to about 160 ° C optionally in the presence of a base such as potassium carbonate or triethylamine. Alternatively, for methylation of the compound which Z is amino, an Eschweiler-Clark Reaction may be utilized to achieve the desired N-methylation. This reductive methylation can be conveniently conducted by reacting an amine of formula (51) with formaldehyde and formic acid. The procedure is similar to that reported by H. T. Clark et. al. J. Am. Chem. Soc., 55, 4571, 1933.

44

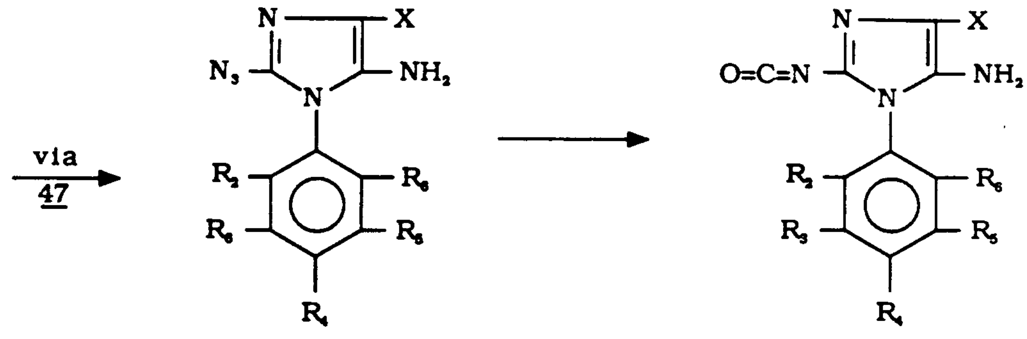


49

50

50

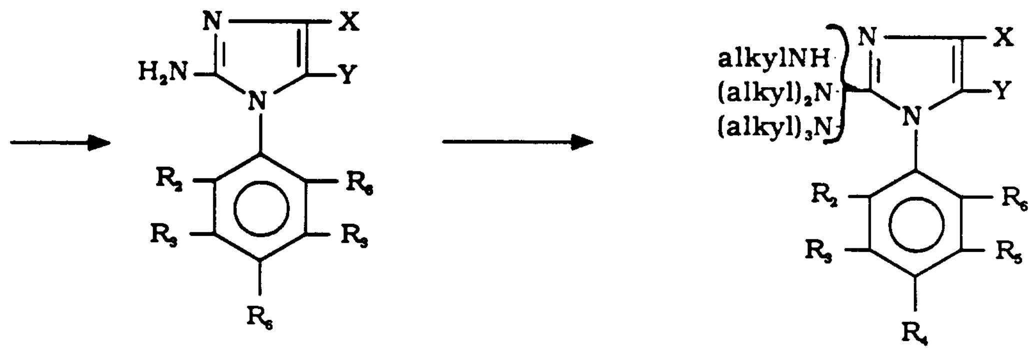

51

52

Method XIX

A compound of formula (Ib), in which Z is alkoxycarbonylamino, alkylaminocarbonylamino, dialkylaminocarbonyl- amino, Y is amino or protected amino, and X, $R_2$, $R_3$, $R_4$, $R_5$ and $R_6$ have the meanings given in the general definition of formula (I), may be conveniently prepared by a two step sequence involving the first step of converting a compound of formula (51), in which Z is amino, into its corresponding chlorocarbonylamino or isocyanate intermediate by a treatment with phosgene. The reaction can be carried out in an inert organic solvent such as toluene, dichloromethane or tetrahydrofuran at a reaction temperature from about -15 °C to about 100 °C, preferably from about -15 °C to about 50 °C. The second step is to react the chlorocarbonylamino or isocyanate intermediate compound with an appropriate alkyl alcohol, alkylamine or dialkylamine. The reaction can be carried out in an inert organic solvent such as a halogenated alkane, toluene, ether or tetrahydrofuran at a reaction temperature from about -20 °C to about 100 °C, preferably from about 0 °C to about 50 °C, optionally in the presence of a base such as an amine.

Method XX

A compound of formula (Ib), in which Z is alkoxyalkylideneimino, Y is amino or protected amino, and X, $R_2$, $R_3$, $R_4$, $R_5$ and $R_6$ have the meanings given in the definition of formula (I), can be prepared by reacting a compound of formula (51), in which Z is amino, with an appropriate alkyl orthoformate. The catalyst, solvent and conditions for the transformation are similar to that described for the preparation of compounds of formula (3) from (2) in Method I. For a compound in which Y is an amino group, an appropriate protection group may be introduced before the transformation is carried out.

Method XXI

A compound of formula (Ib), In which Z is alkylcarbonylamino, haloalkylcarbonylamino or arylcarbonylamino group, Y is amino or protected amino, and X, $R_2$, $R_3$, $R_4$, $R_5$ and $R_6$ have the meanings given in the definition of formula (I), can be conveniently prepared from a compound of formula (51), in which Z is amino, by a reaction with an appropriate alkyl, haloalkyl or aryl carbonyl halide, such as acetyl chloride, chloroacetyl chloride, benzoyl chloride or toluoyl chloride in a suitable solvent, such as dichloromethane, ethyl ether or tetrahydrofuran, optionally in the presence of an acid acceptor such as pyridine or triethylamine, at a reaction temperature from about -10 °C to about 100 °C, preferably from about -10 °C to about 50 °C.

Method XXII

An intermediate compound of the formula (Ib), namely formula (53), in which Z is hydroxymethyl, Y is amino or protected amino, and X, $R_2$, $R_3$, $R_4$, $R_5$ and $R_6$ have the meanings given in the definition of formula (I), may be prepared by reduction of a compound of the formula (42), in which Z is formyl. The reduction can be conducted with a reducing agent such as lithium aluminum hydride, sodium borohydride, aluminum isoproxide, borane or substituted borane or another metal hydride in a suitable aprotic or protic solvent. For a more reactive hydride, e.g. lithium aluminum hydride, the reaction may be conducted in an inert solvent such as tetrahydrofuran, ethyl ether or dimethoxyethane at a reaction temperature from about -10 °C to about 120 °C, preferably at a temperature from about 20 °C to about 100 °C. For a milder hydride, such as sodium borohydride, the reaction may be conducted in an alcohol such as methanol at a temperature from about -10 °C to about 100 °C, preferably from about room temperature to about 75 °C.

42

(Z is CHO)

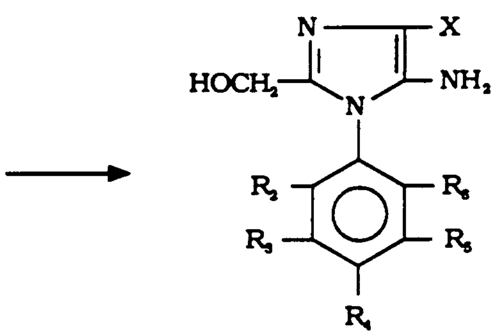

53

Method XXIII

A compound of formula (Ib), i.e. (54), wherein Z is haloalkyl, particularly chloromethyl, fluoromethyl, bromomethyl or iodomethyl, Y is amino or protected amino, and X, $R_2$, $R_3$, $R_4$, $R_5$ and $R_6$ have the meanings given in the definition of formula (I), can be prepared from the intermediate compound of formula (53), in which Z is hydroxymethyl, by using an appropriate chlorinating, fluorinating or brominating agent. For chlorination, the reaction may be carried out with reagents such as thionyl chloride, phosphorous trichloride, phosphorous pentachloride, phosphorous oxychloride in dichloromethane or ethyl ether at a reaction temperature from about -20 °C to about 100 °C. The reaction can be carried out optionally in the presence of an acid acceptor such as triethylamine or pyridine. For fluorination, the reaction can be conducted with dialkylaminosulfur trifluoride in a solvent such as acetonitrile, dichloromethane or glyme at a reaction temperature from about -20 °C to about 100 °C. A more preferable condition is using diethylaminosulfur fluoride in acetronitrile at room temperature. A representative procedure is given in, W. J. Middletown , J. Org. Chem., (1975), 42, 5, 574. Other fluorinating reagents may also be used, such as sulfur trifluoride, bis(dialkylamino)sulfur trifluoride or sodium or potassium fluoride in a polyhydrogen fluoride-pyridine solution, which procedure is that reported by Olah and Welch, Synthesis, 653, (1974). For bromination, the reaction may be conducted with a brominating agent such as bromine, N-bromosuccinimide, phosphorous tribromide or hydrogen bromide in an inert solvent such as dichloromethane or ethyl ether at a temperature from about -20 °C to about 100 °C. For iodidation, the reaction may be performed with hydrogen iodide in an inert solvent such as dichloromethane at a reaction temperature from about -20 °C to about 100 °C.

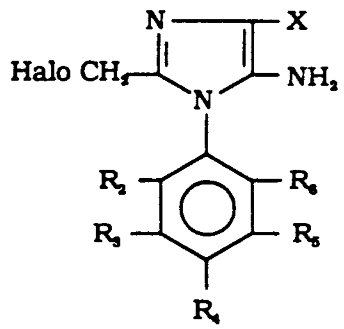

54

Method XXIV

A compound of formula (Ib), in which Z is cyanoalkyl, particularly cyanomethyl, Y is amino or protected amino, and X, Y, $R_2$, $R_3$, $R_4$, $R_5$ and $R_6$ have the meanings given in the definition of formula (I), may be prepared from the corresponding halomethyl compound of formula (54), the preparation of which is described above in Method XXIII, by cyanation with a metal cyanide such as copper cyanide, an alkali cyanide or alkaline metal cyanide such as sodium cyanide or potassium cyanide in a suitable solvent such as dimethylformamide, tetrahydrofuran, acetonitrile, diglyme or tetramethylenesulfone at a reaction temperature from about room temperature to about 250 °C, preferably from about 70 °C to about 150 °C.

Method XXV

A compound of formula (Ib), in which Z is alkenyl or alkynyl, Y is amino or protected amino, and X, $R_2$, $R_3$, $R_4$, $R_5$ and $R_6$ have the meanings given in the definition of formula (I), may be prepared from formula (42), in which Z is formyl, by employing the Wittig reaction or modifications thereof such as the Wadsworth-Emmons (Horner) Modification. The Wittig reagents may be those which are commercially available or those that can be prepared according to well-known literature procedures. The reaction may be conducted in inert solvents such as tetrahydrofuran, dimethoxyethane or toluene at a reaction temperature from about -30 °C to about 180 °C. Examples of the Wittig reagents that may be employed are an alkyl triphenyl phosphonium halide such as methyl triphenylphosphonium iodide, isopropyl triphenylphosphonium iodide, allyl triphenyl phosphonyl halide or trialkyl phosphonoacetate. A representative example of the procedure for the Wittig reaction is given in Org. Synth. Coll. Vol. 5, 751 (1973). In case that the Wittig reagent employed contains an alkynyl group such as propargyl triphenylphosphonium bromide, which is commercially available, the compound obtained is where Z is an alkynyl substituent. Additionally, the alkynyl analog, formula (55), with alkynyl directly attached to 2-carbon of the imidazolyl ring, can be introduced from the corresponding Z is halogen analog, such as an iodo analog, by a reaction with a copper acetylide using a procedure similar to that described by R.E. Atkinson et. al., J. Chem. Soc.(C), 2173, 1969 or the references cited therein.

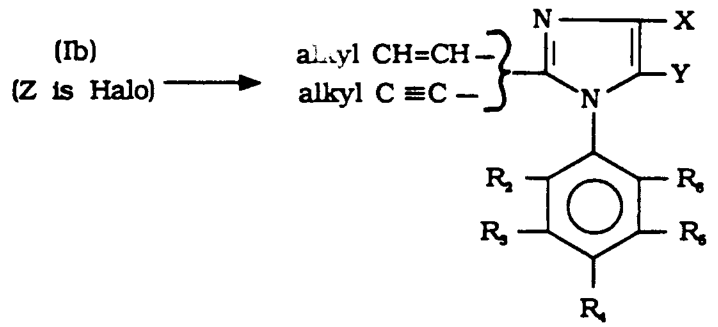


55

Method XXVI

A compound of formula (Ib), in which Z is alkylcarbonyl or haloalkylcarbonyl, Y is amino or protected amino, and X, $R_2$, $R_3$, $R_4$, $R_5$ and $R_6$ have the meanings given in the definition of formula (I), can be prepared by an alkylation of a compound of formula (42), in which Z is formyl, with a carbanion such as a Grinard Reagent or a metal alkane such as a lithium alkane in an inert solvent such as tetrahydrofuran, ethyl ether, hexane, dimethoxyethane or a combination thereof at a reaction temperature from about -70 °C to about 100 °C to give the intermediate, (56), with a secondary hydroxy alkyl methyl at the Z position. This intermediate is then subsequently oxidized with an oxidizing agent such as manganese dioxide, dichromate, permanganate or molecular oxygen in a suitable solvent such as dichloromethane, alcohol, acetone or water at a reaction temperature from about -10 °C to about 175 °C, preferably from about about 4 °C to about 50 °C, to the compound of formula (57). Specifically, the methylcarbonyl analog at the Z position may be alternatively prepared in one step by treating a compound of formula (42), in which Z is formyl with $AlMe_2$-(BHT) $(OEt)_2$ in a suitable solvent such as toluene at a reaction temperature from about -20 °C to about 55 °C, preferably at about room temperature. A representative procedure is reported in M. B. Power and A. R. Barron Tet. Let., 31, 3, 323, 1990 and references cited therein. The corresponding compound in which Z is haloalkylcarbonyl can be conveniently prepared by the typical method of halogenating a ketone, such as using bromine, chlorine, iodine, N-chlorosuccinimide or N-bromosuccinimide to provide a compound wherein Z is haloalkylcarbonyl.

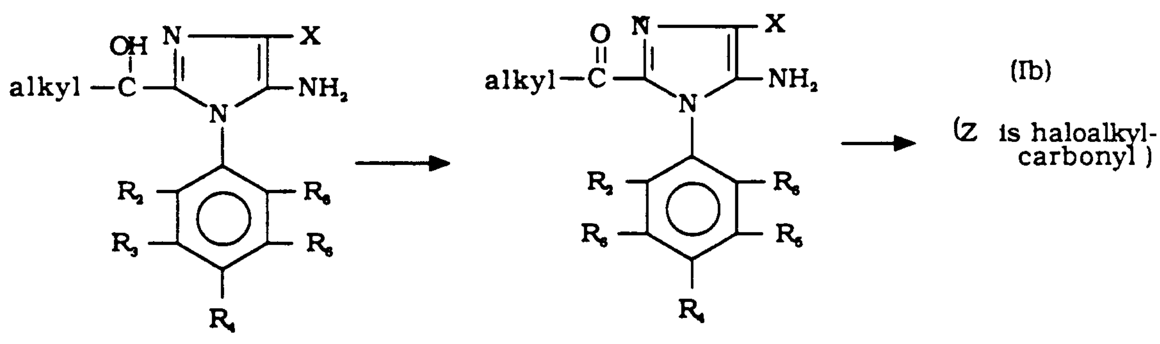


56 57

Method XXVII

A compound of formula (Ib), in which Z is alkylsulfenyl, haloalkylsulfenyl, alkylsulfinyl, haloalkylsulfinyl, alkylsulfonyl, or haloalkylsulfonyl, Y is amino, and X, $R_2$, $R_3$, $R_4$, $R_5$ and $R_6$ are those defined for the definition of formula (I), can be prepared by the following sequential steps:

a) A useful intermediate compound of formula (Ib), i.e. (58), in which Z is thiocyano, Y is amino or protected amino, and X, $R_2$, $R_3$, $R_4$, $R_5$ and $R_6$ have the meanings given in the definition of formula (I), can be prepared by reacting a compound of formula (Ia), in which Z is hydrogen, with a mixture of bromine and a metal thiocyanate in suitable solvent such as methanol or ethanol at a temperature from about -78 ° C to about 100 ° C, preferably from about -78 ° C to about room temperature.

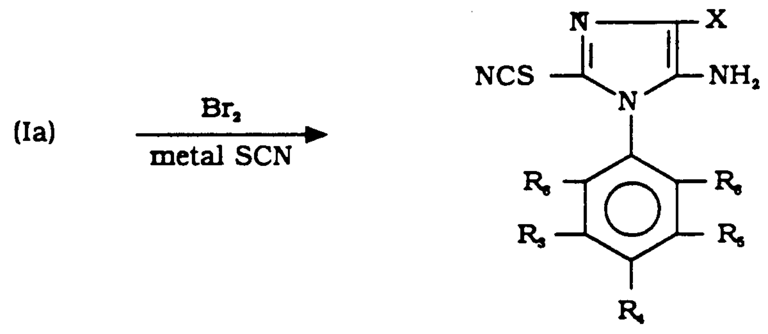


58

b) A compound of formula (IB), namely (59), in which Z is alkylsulfenyl or haloalkylsulfenyl, Y is amino or protected amino, and X, $R_2$, $R_3$, $R_4$, $R_5$ and $R_6$ have the meanings given in the definition of formula (I), can be prepared from a compound of formula (58), in which Z is thiocyano, by treatment with an alkylating agent, in a suitable solvent such as an alcohol, acetonitrile, tetrahydrofuran, dimethoxyethane or water with or without the presence of a base such as an alkali hydroxide or a alkali carbonate at a reaction temperature from about -20 ° C to about 150 ° C, preferably from about 0 ° C to about 85 ° C.

58

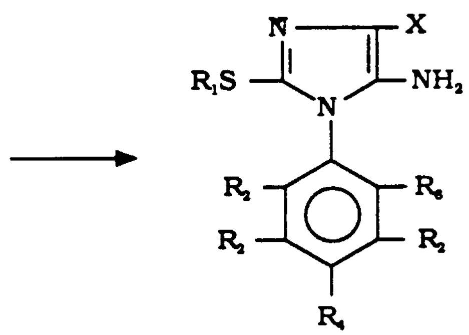

59

c) A compound of formula (Ib), in which Z is alkylsulfinyl haloalkylsulfinyl, alkylsulfonyl or haloalkylsulfonyl, Y is amino or protected amino, and X, $R_2$, $R_3$, $R_4$ and $R_5$ have the meanings given in the definition of formula (I), can be prepared from a sulfenyl compound of formula (59) by treatment with a stoichiometric amount of an appropriate oxidizing agent. The procedures of these transformations are similar to those described for the oxidation of compounds of formula (8) to (9) in Method I.

d) Additionally, an intermediate compound of formula (Ib), i.e. (60), in which Z is thiocyano, Y is hydrogen, and X, $R_2$, $R_3$, $R_4$, $R_5$ and $R_6$ are as defined in formula (I), can be prepared by deamination of a compound of formula (58), in which Z is thiocyano, Y is hydrogen and X and $R_2$ to $R_6$ are as defined in formula (I), following the procedure similar to that described in Method I. This can then be further alkylated to an alkyl- or haloalkyl sulfenyl compound and then oxidized by the above procedure to give a compound of formula (I), wherein Y is hydrogen, Z is as defined in parts b) or c) above, and X, $R_2$, $R_3$, $R_4$, $R_5$ and $R_6$ are as defined for formula (I).

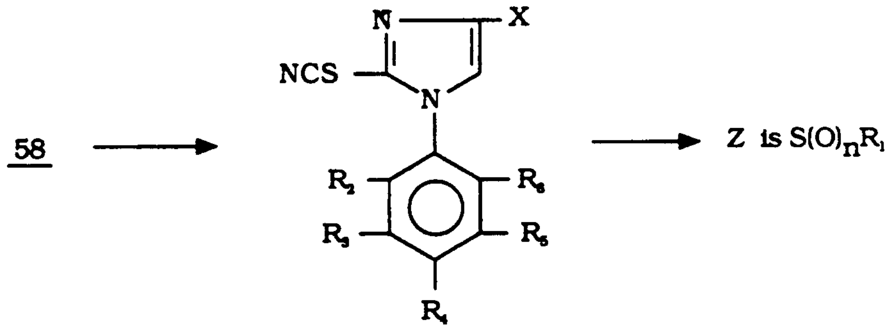


60

e) Furthermore, a compound of formula (Ib), in which Z is haloalkylsulfenyl, and X, Y, $R_2$, $R_3$, $R_4$, $R_5$ and $R_6$ are as defined above may be prepared from a compound of formula (58) or (60), wherein Z is thiocyano, via the corresponding disulfide. According to the procedures similar to those described in Method XLIV below. These compounds may then be oxidized to the corresponding sulfoxide (n = 1) or sulfone (n = 2) compounds, in which Z is $S(O)_nR_1$ as previously defined, according to methods as described above, i.e. Method I.

Method XXVIII

A compound of formula (Ib), in which Z is sulfhydryl or salts thereof, Y is amino or protected amino, and X, $R_2$, $R_3$, $R_4$, $R_5$ and $R_6$ have the meanings given in the definition of formula (I), can be prepared by a free radical-promoted sulfur-carbon cleavage of a compound of formula (58), wherein Z is thiocyano. The reaction may be conducted with a free radical promoter such as potassium ferricyanide in a suitable solvent such as an alcohol, tetrahydrofuran, water or a mixture thereof, in an appropriate proportion, under neutral or basic conditions at a reaction temperature from about -10 °C to about 180 °C. A preferred procedure is to carry out the reaction using potassium ferricyanide in methanol and water in the presence of potassium hydroxide at refluxing conditions.

Alternatively, an analogous compound of formula (60), wherein Z is thiocyano, Y is hydrogen, and X and $R_2$ to $R_6$ are as defined in formula (I), can be converted by procedures similar to those above to a compound wherein Z is sulfhydryl or salts thereof.

Methods XXIX to XLIII Generalization

The following Methods XXIX to XLIII detail specific procedures, for introducing a Y substituent into a particular compound of formula (Ib) to provide a compound of the invention of formula (I).

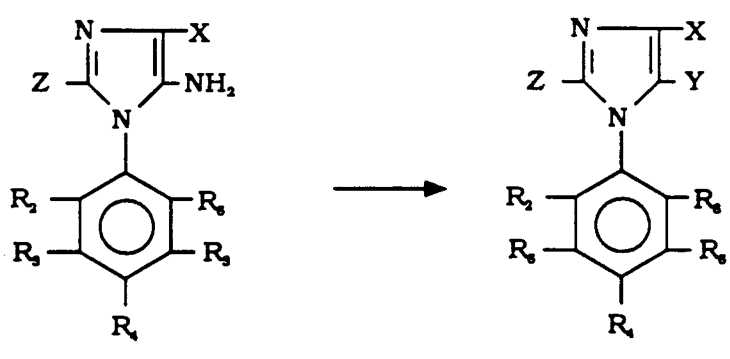

(Ib) (I)

Method XXIX

A compound of formula (I), in which Y is alkoxycarbonylamino, alkylaminocarbonylamino, dialkylaminocarbonylamino, and X, Z, $R_2$, $R_3$, $R_4$, $R_5$ and $R_6$ have the meanings given in the definition of formula (Ib), may be prepared from a compound of formula (Ib), in which Y is amino and the other substituents are defined as above by procedures similar to those described in Method XIX.

Method XXX

A compound of formula (I), in which Y is alkoxyalkylideneimino and X, Z, $R_2$, $R_3$, $R_4$, $R_5$ and $R_6$ have the meanings given in the definition of formula (I), can be prepared from a corresponding compound of formula (Ib), wherein Y is amino, by a procedure similar to that described in Method XX.

Method XXXI

A compound of formula (I), in which Y is alkylcarbonylamino, haloalkylcarboxylamino or arylcarbonylamino group and X, Z, $R_2$, $R_3$, $R_4$, $R_5$ and $R_6$ have the meanings given in the definition of formula (I), may be prepared from the corresponding compound of formula (Ib), in which Y is amino by a sequence of procedures similar to that described in Method XXI.

Method XXXII

A compound of formula (I), in which Y is sulfhydryl or salts thereof, and X, Z, $R_2$, $R_3$, $R_4$, $R_5$ and $R_6$ have the meanings given by the definition of formula (I), can be prepared by the sequence described below:

a) The intermediate compound, in which Y is thiocyano and X, Z, $R_2$, $R_3$, $R_4$, $R_5$ and $R_6$ have the meanings given in the definition of formula (I), can be prepared from a compound of formula (I), i.e. (61) herein below, in which Y is hydrogen, optionally obtained via Method I, and X, Z, $R_2$, $R_3$, $R_4$, $R_5$ and $R_6$ are as defined above. The transformation may be achieved by a procedure similar to that described in Method XXVII.

b) The thiocyano intermediate compound obtained by the method described above can be converted into the corresponding compound of formula (I), wherein Y is sulfhydryl and salts thereof, using a procedure similar to that described in Method XXVIII.

Method XXXIII

A compound of formula (I), i.e. (62), in which Y is alkyl, haloalkyl, alkenyl, alkynyl, cyanoalkyl or formyl and X, Z, $R_2$, $R_3$, $R_4$, $R_5$ and $R_6$ are defined in the definition of formula (I), except those base-sensitive in nature, can be prepared from a compound of formula (61), in which Y is hydrogen, by treatment with a strong base, preferably an organic base such as lithium diisopropylamide, n-butyl lithium or sec-butyl lithium, in an appropriate solvent, such as tetrahydrofuran or ethyl ether at a reaction temperature from about -75°C to about room temperature, followed by quenching the metal carbanion, with an appropriate electrophile, e.g. alkyl halide or N-formyl piperidine, to obtain the corresponding substituent at the Y position. This method of synthesis is generally known as a directed ortho metalation reaction. Examples of the procedure are described by V. Snieckus in Bull. Soc. Chim. Fr., 1988, (1), 67-78 and references cited therein.

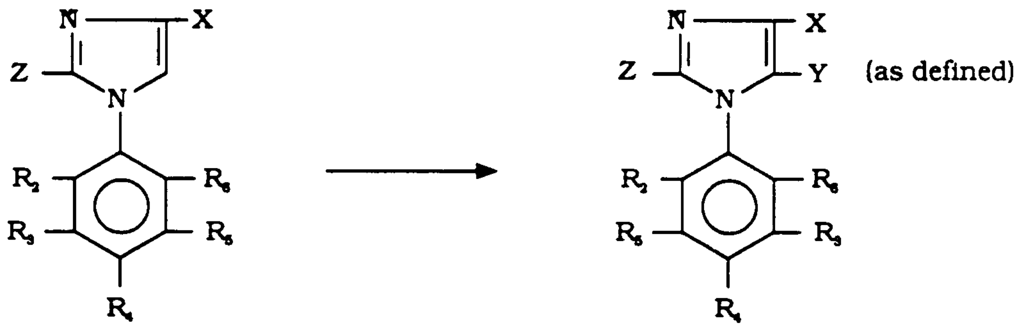

61

62

Method XXXIV

A compound of formula (I), in which Y is a carboxylic group or a carboxylate salt and X, Z, $R_2$, $R_3$, $R_4$, $R_5$ and $R_6$ have the meanings given in the definition of the invention, can be prepared from a compound of formula (I), in which Y is formyl and X, Z, $R_2$, $R_3$, $R_4$, $R_5$ and $R_6$ are defined as above, by a procedure similar to that described in Method XIV.

Method XXXV

A compound of formula (I), in which Y is cyano and X, Z, $R_2$, $R_3$, $R_4$, $R_5$ and $R_6$ have the meanings given in the definition of formula (I), can be prepared from a compound of formula (I), in which Y is a carboxylic group and the other substituents are as defined above, by a procedure similar to that described in Method XV or Method XVI.

Method XXXVI

A compound of formula (I), in which Y is aminocarbonyl, alkylaminocarbonyl, dialkylaminocarbonyl or alkoxycarbonyl and X, Z, $R_2$, $R_3$, $R_4$, $R_5$ and $R_6$ have the meanings given in the definition of formula (I), can be prepared from a compound of formula (I), in which Y is carboxyl and X, Z, $R_2$, $R_3$, $R_4$, $R_5$ and $R_6$ are as defined above, by a procedure similar to that described in Method XVII.

Method XXXVII

A compound of formula (I), in which Y is alkylamino, dialkylamino or trialkylammonium salt and X, Z, $R_2$, $R_3$, $R_4$, $R_5$ and $R_6$ have the meanings given in the definition of formula (I), can be prepared from a compound of formula (I), i.e. (Ib), in which Y is amino and the other substituents are as defined above, by monoalkylating, dialkylation and trialkylation with an appropriate alkylating agent, The solvent, reaction temperature and alkylating agent may be chosen based on the general procedures described in Method XVIII. For N-methylation, the Eschweiler-Clark reaction may be employed by a procedure similar to that described in Method XVIII.

Method XXXVIII

A compound of formula (I), in which Y is haloalkyl, particularly halomethyl, including fluoro, chloro, bromo and iodoalkyl and X, Z, $R_2$, $R_3$, $R_4$, $R_5$ and $R_6$ have the meanings given in the definitions of formula (I), can be prepared from a compound of formula (I), in which Y is formyl and the other substituents are as defined above, by a sequence of transformations via the corresponding hydroxymethyl intermediate which is then converted into the halomethyl analogs. The sequence and procedures of the transformations are similar to those described in Method XXII and XXIII.

Method XXXIX

A compound of formula (I), in which Y is alkenyl or alkynyl and X, Z, $R_2$, $R_3$, $R_4$, $R_5$' and $R_6$ have the meanings given in the definition of formula (I), can be prepared from a compound of formula (I), in which Y is formyl (or optionally Y is halogen obtained via Method I) and the other substituents are as defined above, by a procedure similar to that described in Method XXV.

Method XL

A compound of formula (I), in which Y is alkylcarbonyl or haloalkylcarbonyl and X, Z, $R_2$, $R_3$, $R_4$, $R_5$ and $R_6$ have the meanings given in the definition of formula (I), can be prepared from the corresponding compound of formula (I), in which Y is formyl, following a procedure similar to that described in Method XXVI. The transformation is achieved via an intermediate which bears a secondary hydroxyalkylmethyl at the Y position or by a direct transformation using $AlMe_2(BHT)(OEt)_2$ to give the compound in which Y is alkylcarbonyl, followed by halogenation procedures as in Method XXVI to give the comound in which Y is haloalkylcarbonyl.

Method XLI

A compound of formula (I), in which Y is aminosulfonyl, alkylaminosulfonyl or dialkylaminosulfonyl and X, Z, $R_2$, $R_3$, $R_4$, $R_5$ and $R_6$ have the meanings given in the definition of formula (I), can be prepared from a compound of formula (I), i.e, (61), wherein Y is hydrogen, optionally obtained via Method I, and X, Z, $R_2$, $R_3$, $R_4$, $R_5$ and $R_6$ have the meanings herein above defined by the following sequence:

a) An intermediate compound of formula (64), in which Y is chlorosulfonyl and X, Z, $R_2$, $R_3$, $R_4$, $R_5$ and $R_6$ have the meanings given in the definition of formula (I), may be prepared by treating a compound of formula (61), in which Y is hydrogen, optionally obtained via Method I, with an alkyl lithium, such as n-butyllithium or sec-butyllithium in an inert solvent such as ethyl ether, hexane, tetrahydrofuran or a mixed solvent combination thereof at a temperature from about -78°C to about room temperature, preferably from about -78°C to about -30°C, followed by quenching the carbanion (63) with sulfuryl chloride in an inert solvent, such as hexane or ethyl ether at a temperature from about -78°C to about room temperature, preferably from about -78°C to about -20°C. A similar procedure is reported by S. N. Bhattacharya, et. al., J. Chem. Soc. (C), 1968, 1265.

Alternatively, the carbanion intermediate (63) may be prepared by a similar method from a compound of formula (I), in which Y is a halogen, optionally obtained via Method I, such as chlorine, bromine or iodine, by treatment with magnesium or alkyl lithium in an inert solvent at a temperature similar to that described above.

b) The compound of formula (65), in which Y is aminosulfonyl, alkylaminosulfonyl or dialkylaminosulfonyl, can be prepared by reacting the chlorosulfonyl intermediate (64) with ammonia or an appropriate alkylamine or dialkylamine in a suitable solvent such as a halogenated alkane, ether, tetrahydrofuran or hexane at a reaction temperature from about -50°C to about 50°C, preferably from about -20°C to about room temperature.

61

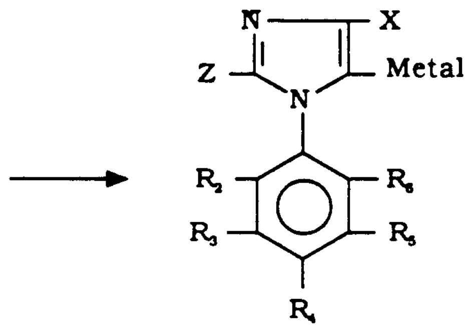

63

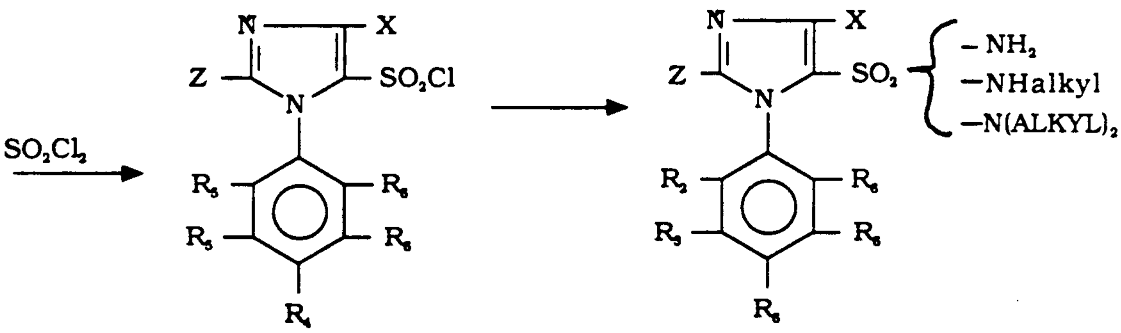


64

65

Method XLII

A compound of formula (I), in which Y is nitro or amino, and X, Z, $R_2$, $R_3$, $R_4$, $R_5$ and $R_6$ have the meanings given in the definition of formula (I), can be prepared by a direct nitration from a compound of general formula (I), i.e. (61), wherein Y is hydrogen, optionally obtained via Methods I, and X, Z, $R_2$, $R_3$, $R_4$, $R_5$ and $R_6$ are as defined above. The nitration reaction and subsequent reduction to the compound wherein Y is amino may be conducted by a procedure similar to that described in Method VIII.

Method XLIII

A compound of formula (I), in which Y is hydroxy and salts thereof, alkoxy or haloalkoxy and X, Z, $R_2$, $R_3$, $R_4$ and $R_5$ have the meanings given in the definition of formula (I), may be prepared from a compound of formula (I), wherein Y is halogen, optionally obtained via Methods I, and the other groups are as defined above, by converting the halo compound into the corresponding Grignard reagent or lithum carbanion, followed by treatment with oxodiperoxymolybdenum(pyridine)(hexamethylphosphorictriamide) (MoOPH) to a compound wherein Y is hydroxyl, using a procedure similar to that described in Method IB. The corresponding alkoxy or haloalkoxy compound can then be conveniently prepared utilizing a procedure similar to that described in Method IB.

Method XLIV

A compound of formula (I), wherein X is particularly alkylsulfenyl, haloalkylsulfenyl, alkylsulfinyl, haloalkylsulfinyl, alkylsulfonyl or haloalkylsulfonyl and Y, Z, $R_2$, $R_3$, $R_4$, $R_5$ and $R_6$ have the meanings of the definition of formula (I), can be alternatively prepared by the following procedures starting from the compound in which X is hydrogen to give intermediate, wherein X is thiocyano, (71), or X is chlorosulfonyl, (67). Either of these interemediates may be converted to the corresponding disulfide intermediate which is

then converted to the sulfenyl compound, in which Y is $SR_1$ and in which $R_1$ is as previously defined, which in turn may be oxidized to the corresponding sulfoxide or sulfone, X is $S(O)_nR_1$, in which n is 1 or 2.

a) An intermediate of the formula (67), in which X is chlorosulfonyl, Y, Z, $R_2$, $R_3$, $R_4$, $R_5$ and $R_6$ have the meanings defined in the definition of formula (I), can be prepared from an intermediate compound of the formula (Ic), namely (66), in which X is hydrogen and Y, Z, $R_2$, $R_3$, $R_4$, $R_5$ and $R_6$ are defined herein above, by treatment with chlorosulfonic or dichlorosulfonic acid. The reaction can be carried out in the presence of organic solvents such as methylene chloride, chloroform, carbon tetrachloride or dimethylformamide or using chlorosulfonic acid as solvent at a reaction temperature from about -10 °C to about 160 °C. A representative procedure for chlorosulfonation of an aromatic compound is reported in J. March, "Advanced Organic Chemistry", McGraw-Hill publ. (1968), p. 402.

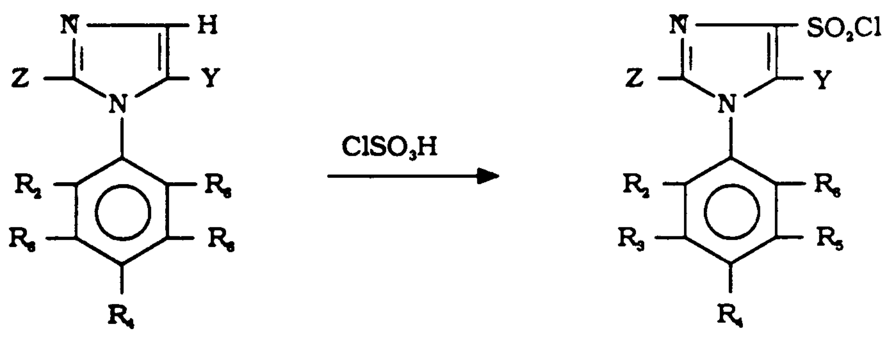


(Ic) or 66 67

b) An intermediate disulfide compound of the formula (68), in which X is disulfide and the definitions of Y, Z, $R_2$, $R_3$, $R_4$, $R_5$ and $R_6$ are those given for the definition of formula (I), can be prepared from the compound of the formula (67) by treatment with a reducing agent, such as triphenylphosphine, in the presence of an organic solvent, such as tetrahydrofuran, dichloromethane or toluene at a reaction temperature from about -10 °C to about 120 °C. A representative example of a procedure for the reduction to p-tolyldisulfide is reported in J. Org. Chem, 1980, 45, 4792. Alternatively, disulfenylation can be effected using a metal carbonyl such as hexacarbonylmolybedium in anhydrous tetramethyurea. The procedure of this reaction is reported by H. Alper, Angew. Chem. Internat. Edit, 8, 677, 1969.

67

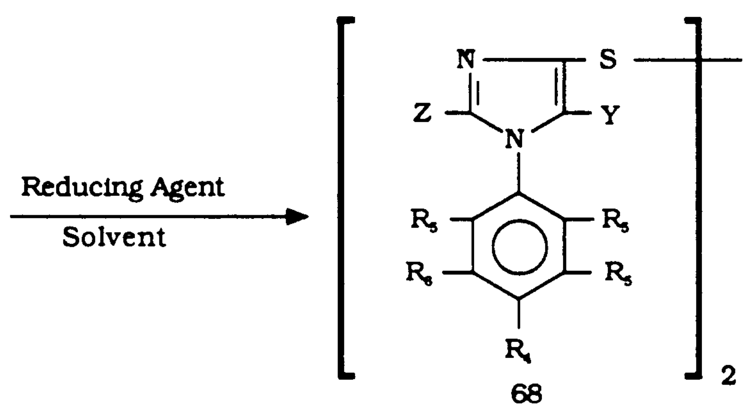


68

c) A compound of the formula (I), namely (70), wherein the definition of Y, Z, $R_2$, $R_3$, $R_4$, $R_5$ and $R_6$ are those given for the definition of formula (I), and X is haloalkylsulfenyl, preferably perhaloalkylsulfenyl, $R_7S$, in which $R_7$ is $CFR_8R_9$ and $R_8$ and $R_9$ are F, Cl, Br or a perfluoroalkyl group, can be prepared by the reaction of a compound of the formula (68) and a perhaloalkane compound of the formula (69), Halo-$CFR_8R_9$, wherein Halo is Cl , Br or I, $R_8$ is F, Cl or Br, and $R_9$ is F, C, Br or a perfluoroalkyl group, with a reducing agent which can promote the formation of the free radical $CFR_8R_9$ (from Halo-$CFR_8R_9$). The

reducing agent is preferably chosen from a metal consisting of zinc, aluminum, cadmium, manganese or a compound with an oxide of sulfur, e.g., a dithionite or a hydroxymethylsulfinate. The alkaline dithonite, alkaline earth or the metal dithionite corresponds to the formula $M_n(S_2O_4)$, in which n can be 1 or 2 depending upon the valence of the metal M. When a dithionite or a hydroxymethylsulfinate is used, a base is needed. The base can be chosen from among an alkaline hydroxide, alkali earth hydroxide, ammonia, alkylamine, triethylbenzylammonium or the salt of weak acids such as disodium phosphate, sodium metabisulfite, sodium hydrogen sulfite or sodium borate. The solvents used for the reaction are those which can solubilize the dithionite or the hydroxymethylsulfinate, and the compounds (68) and (69). Useful solvents are acetonitrile, dimethylformamide, formamide, dimethylacetamide, hexamethylphosphoramide, N-methylpyrrolidone, dimethylsulfoxide or sulfolane. The reaction temperature is from about 10 °C to about 100 °C. Typical procedures are similar to those reported by A. Maggiolo, J. Am. Chem. Soc., 1951, 5815 and by P.W. Feit, Acta. Chem. Scan., 16, 1962, 297. The reaction is represented by the following equation:

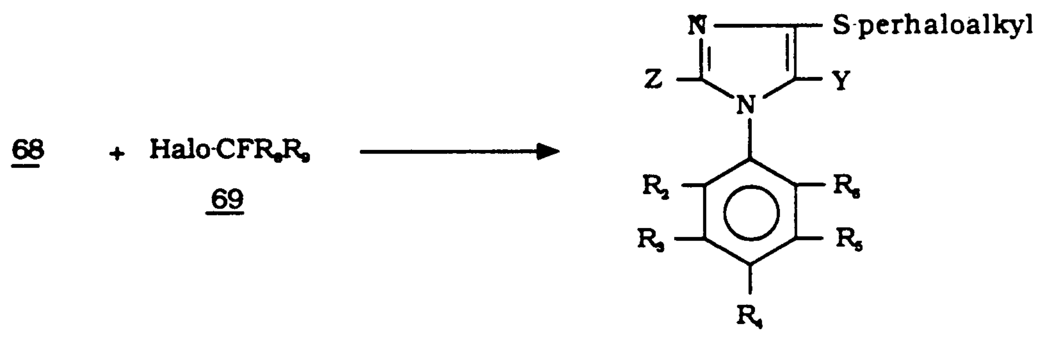


68 + 69 → 70

d) The intermediate compound of formula (I), namely (71), in which X is cyanothio and Y, Z, $R_2$, $R_3$, $R_4$, $R_5$ and $R_6$ have the meanings given in the definition of formula (I), may be prepared from a compound of formula (Ic), i.e. (66), by treatment with bromine and an alkali thiocyanate such as potassium thiocyanate in a suitable solvent such as methanol at a temperature from about -78 °C to about room temperature. The solvent should be inert to and capable of solvolyzing the reactants.

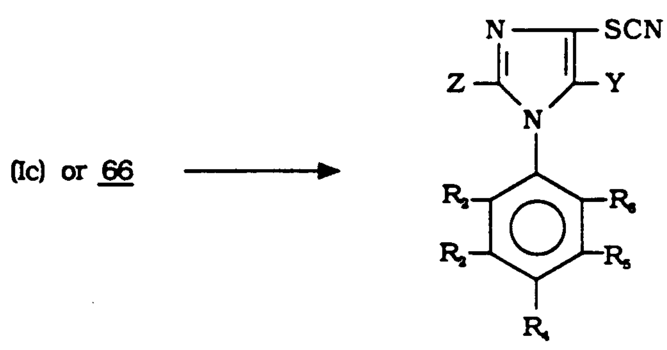

(Ic) or 66 → 71

e) Alternatively, the compound of formula (70), wherein X is haloalkylsulfenyl, preferably perhaloalkylsulfenyl, may be prepared by a sequence of oxidation of a compound of formula (71) to form an intermediate disulfide compound of formula (68), which can then be converted into its corresponding haloalkylsulfenyl compound of formula (70). The oxidation can be achieved using an oxidizing agent such as hydrogen peroxide in the presence of an alkali hydroxide, such as sodium hydroxide, or an amine such as ammonia in a suitable solvent, such as an alcohol, water, tetrahydrofuran, a halogenated alkane or mixed solvent thereof, at a reaction temperature from about -70 °C to about 55 °C. Typical procedures are reported by A. Maggiolo, J. Am. Chem. Soc., 1951, 5815 and by P. W. Feit, Acta. Chem. Scan., 16,

1962, 297. The haloalkylsulfenyl compound of formula (70) can be prepared by reacting the disulfide intermediate compound with an appropriate perhaloalkane, optionally in the presence of a reducing agent such as a metal consisting of zinc, aluminum, cadmium or manganese.

71

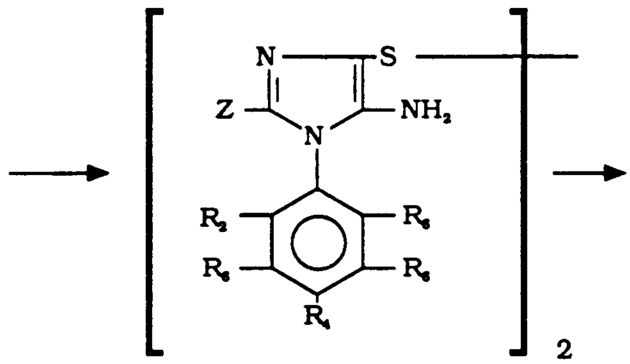

70

68

f) A further compound of formula (I), i.e. (72), wherein X is alkylsulfenyl or haloalkylsulfenyl and Y, Z, $R_2$, $R_3$, $R_4$, $R_5$ and $R_6$ are as defined by formula (I), can be prepared by treating a compound of formula (71) with an appropriate alkyl halide, $R_1$ Halo, in which $R_1$ is alkyl or haloalkyl, preferably an alkyl iodide or an alkyl bromide in a suitable solvent such as an alcohol, preferably the corresponding alkyl alcohol, in the presence of a base catalyst such as an alkali hydroxide or alkali carbonate at a reaction temperature from about -20 °C to about 75 °C.

71

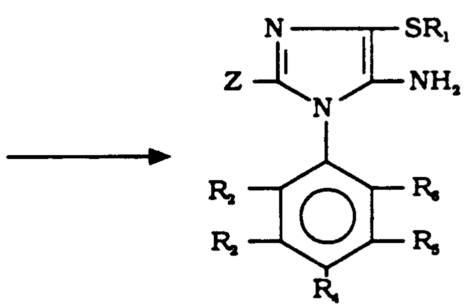

72

g) A compound of formula (I), wherein X is alkylsulfinyl, haloalkylsulfinyl, alkylsulfonyl or haloalkylsulfonyl and Y, Z, $R_2$, $R_3$, $R_4$, $R_5$ and $R_6$ are as defined in formula (I), can be prepared from a compound of formulae (70) or (72) by the oxidation procedures described, for example in Method I.

Method XLV

Still other processes to make compounds of formula (I), which are contemplated within the present invention include, for example, an aromatic nucleophilic displacement reaction of a halogen atom on the phenyl ring by an alkylthiol or anion thereof. In this way, compounds of formula (I) (e.g., compounds of formulae (6), (7), (8), (9) and (18) provide other new compounds of formula (I), wherein one or more of $R_2$ to $R_6$ is an alkylsulfenyl group, which may be further oxidized to the corresponding sulfoxide or sulfone, in a manner similar to that of oxidation of (8) to (9) in Method (I). This reaction, as appropriate, may also be conducted with starting materials or intermediates in the above described processes to introduce into said compounds an alkylsulfenyl, alkylsulfinyl or alkylsulfonyl group on the phenyl ring prior to formation of compounds of formula (I) of the invention.

This process may be exemplified by the following equation in which a compound of formula (73) is reacted to give a compound of formula (74). Compounds of formulae (73) and (74) are preferred examples

of compounds of the invention of formula (I) or (II) wherein: $R_3$ and $R_5$ are each a hydrogen atom; $R_2$ is a halogen atom (e.g., F. Cl or Br) in the case of compound (73) or in the case of compound (74). $R_2$ is an alkylsulfenyl group, wherein the alkyl moiety is a linear or branched chain containing one to four carbon atoms; $R_4$ and $R_6$ are as defined in formula (I), preferably electron withdrawing groups such as trifluoromethyl, cyano, nitro or a halogen atom; and X, Y and Z are as defined in formula (I) or (II).

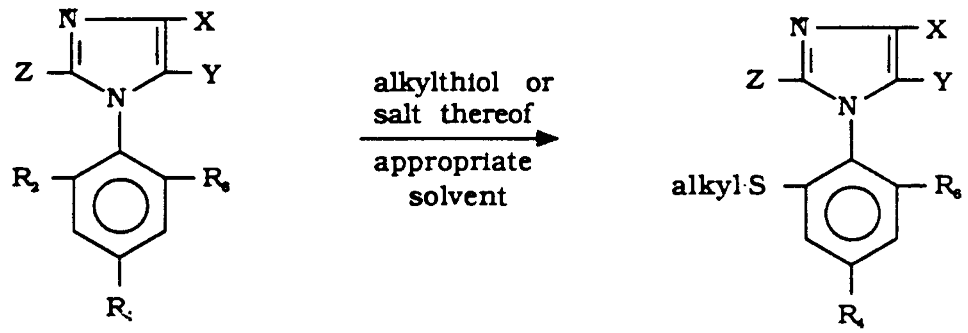


73

74

The process is preferably conducted in a solvent which is capable of solvolyzing the 1-phenyl imidazole compound and the alkylthiol or thiolate salt thereof, which is, for example, an alkali metal, alkaline earth metal or tetraalkylammonium salt, but preferably a sodium or potassium salt. Preferred solvents are ethers (e.g., tetrahydrofuran or diglyme), alcohols (e.g., methanol or ethanol), amines (e.g., triethylamine or pyridine), aprotic solvents such as dimethylformamide, or water or combinations of these solvents. The more preferred solvent systems are water-tetrahydrofuran or water-tetrahydrofuran-methanol. The reaction is generally conducted at a temperature between about -20 °C and about 180 °C. preferably between about 0 °C and about 120 °C.

Method Generalizations

The above methods or processes of synthesis are not to be construed as limiting and therefore, compounds of the present invention, as well as intermediates and starting materials (particularly the anilines), can be prepared by application or adaptation of synthesis methods, which are apparent to one skilled in the art, and are commonly known, used or described in the chemical literature. In this regard, it is understood that, for example, the sequence of the synthetic chemical steps may be performed in a different order as appropriate, suitable protecting groups may be employed, and substituent groups may be incorporated when convenient. In the description of process methods, when symbols appearing in a formula are not specifically defined, it is also to be understood that they are "as herein before defined" in accordance with the first definition of each symbol in this specification.

In an overall/global manner the foregoing Methods of synthesis may be represented by the following processes of the invention which are described as follows:

**$P_1$**. A process of preparation of a compound of formula (Ia),

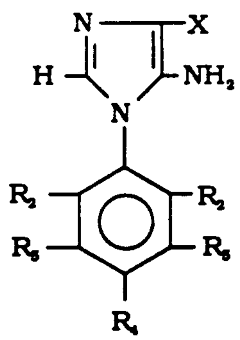

(Ia)

wherein $R_2$, $R_3$, $R_4$, $R_5$ and $R_6$ are as defined for formula (I) and X is alkylsulfenyl, haloalkylsulfenyl, alkylsulfinyl, haloalkylsulfinyl, alkylsulfonyl, haloalkylsulfonyl, haloalkyl or haloalkoxy, wherein a compound of formula (5),

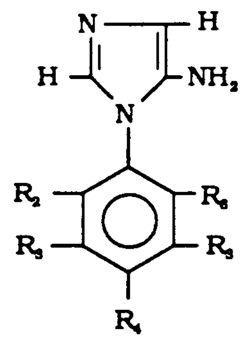

5

in which amino is optionally protected as required:

a) is first reacted with a sulfenyl halide, $R_1$SHalo in which $R_1$ is alkyl or haloalkyl, in an organic reaction medium, optionally in the presence of an acid acceptor such as a tertiary amine to obtain a compound of formula (Ia), wherein X is alkylsulfenyl or haloalkylsulfenyl, which is then optionally oxidized by known methods such as by a peroxide, to obtain a compound of formula (Ia), wherein X is $S(O)_nR_1$ in which n is 1 or 2 and $R_1$ is as defined above, that is to say X is alkylsulfinyl, haloalkylsulfinyl, alkylsulfonyl or haloalkylsulfonyl;

b) is first reacted with a tris(alkylthio)methane or tris(arylthio)methane in an organic reaction medium in the presence of a Lewis Acid and optionally in the presence of an acid acceptor, then the obtained intermediate compound of formula (10), in which X is bis(alkylthio)methyl or bis(arylthio)methyl is reacted in an organic reaction medium with a suitable alkylnitrite followed by a hydrolysis procedure to obtain an intermediate compound of formula (Ia), in which X is formyl, then is followed by known reduction procedures to give the intermediate compound, in which X is hydroxymethyl, and then is finally halogenated by known procedures to give a compound of formula (Ia), in which X is haloalkyl, more specifically halomethyl;

c) is first formylated by well known procedures such as that of Vilsmeier-Haack and the like to give the compound of formula (Ia), in which X is formyl, then is reacted following the procedures above in part b) to likewise obtain the compound of formula (Ia) , in which X is haloalkyl;

d) is reacted by the procedures of part b) or c) above to obtain an intermediate compound of formula (Ia), in which X is formyl, which compound may be optionally oxidized to an intermediate compound of formula (Ia), in which X is carboxyl, then finally the intermediate compound, in which X is formyl, is reacted with a halogenating agent such as diethylaminosulfur trifluoride or the intermediate compound, in which X is carboxyl, is reacted with sulfur tetrafluoride to give a compound of formula (Ia), in which X is haloalkyl, more specifically difluoromethyl or trifluoromethyl; or

e) is first halogenated by well known procedures to obtain an intermediate compound, in which X is halogen, from which an organomagnesium or -lithium derivative is prepared, then said organometallic is reacted with oxodiperoxymolybdenum(pyridine)(hexamethylphosphoric triamide) or a trialkyl borate and an oxidizing agent such as hydrogen peroxide to obtain an intermediate compound of formula (Ia), in which X is hydroxy, then is finally reacted by known haloalkylating procedures to obtain a compound of formula (Ia) in which X is haloalkoxy.

**$P_2$**. A process of preparation of a compound of formula (Ib),

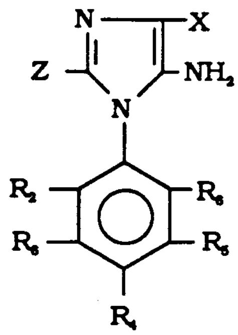

(Ib)

wherein X, $R_2$, $R_3$, $R_4$, $R_5$ and $R_6$ are as defined for formula (I) and Z is aminosulfonyl, alkylaminosulfonyl, dialkylaminosulfonyl, nitro, amino, halogen, alkynyl, alkyl, hydroxy and salts thereof, alkoxy, haloalkoxy, formyl, alkylsulfenyl, haloalkylsulfenyl alkylsulfinyl, haloalkylsulfinyl, alkylsulfonyl, haloalkylsulfonyl, or sulfhydryl and salts thereof, wherein a compound of formula (Ia) ,

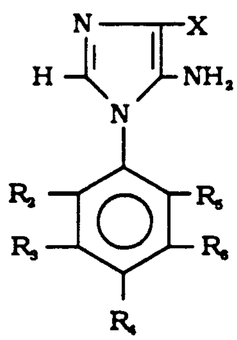

(Ia)

in which X and amino are optionally protected as required:

a) is first reacted with chlorosulfonic or dichlorosulfonic acid to give an intermediate compound, wherein Z is chlorosulfonyl, which compound is reacted with ammonia, an alkylamine or dialkylamine to give a compound of formula (Ib), in which Z is aminosulfonyl, alkylaminosulfonyl or dialkylaminosulfonyl;

b) is halogenated or nitrated by known procedures to give a compound of formula (Ib), in which Z is halogen or nitro, then the compound in which Z is nitro is optionally reduced to Z is amino by known procedures, or optionally the compound, in which Z is halogen, is treated by known procedures with a copper acetylide to give the compound in which Z is alkynyl;

c) is reacted with a strong base such as an organolithium reagent to give an intermediate organometallic carbanion, which is then quenched with an alkylating agent to give a compound of formula (Ib), in which Z is alkyl, or optionally the carbanion is reacted in a manner similar to that described in process $P_1$e to first give a compound of formula (Ib), in which Z is hydroxyl or salts thereof, or then optionally the compound in which Z is hydroxy is converted to a compound wherein Z is alkoxy or haloalkoxy by known alkylation or haloalkylation procedures;

d) is reacted by formylation procedures similar to those described in process $P_1$b or $P_1$c , wherein the compound, in which Z is formyl, is prepared directly by conditions such as the Vilsmeier-Haack

Reaction or via hydrolysis of an intermediate compound in which Z is bis(alkythio)methyl or bis-(arylthio)methyl;

e) is first reacted with a mixture of bromine and a metal thiocyanate to give an intermediate compound of formula (Ib), in which Z is thiocyano, which then is treated with an alkylating agent, optionally in the presence of a base to directly give a compound of formula (Ib), in which Z is alkylsulfenyl or haloalkylsulfenyl, or optionally the intermediate compound in which Z is thiocyano is first oxidized to a corresponding intermediate disulfide compound which is then reacted with a perhaloalkane, optionally in the presence of a reducing agent, to give a compound of formula (Ib) in which Z is haloalkylsulfenyl, particularly perhaloalkylsulfenyl, finally the compound, in which Z is alkylsulfenyl or haloalkylsulfenyl, is optionally oxidized by known methods similar to those of process $P_1a$ to give a compound of formula (Ib), in which Z is alkylsulfinyl, haloalkylsulfinyl, alkylsulfonyl or haloalkylsulfonyl; or

f) is first reacted as above in part e) to give the intermediate compound, in which Z is thiocyano, which is then cleaved by a free radical promoter, such as potassium ferricyanide, to give a compound of formula (Ib), in which Z is sulfhydryl or salts thereof.

**$P_3$**. A process of preparation of a compound of formula (Ib),

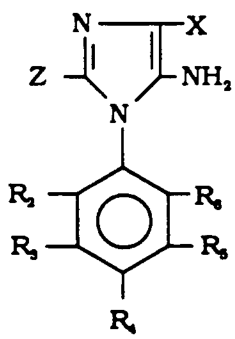

(Ib)

wherein X, $R_2$, $R_3$, $R_4$, $R_5$ and $R_6$ are as defined for formula (I) and Z is amino, alkyl, cyano, carboxyl and salts thereof, aminocarbonyl, alkylaminocarbonyl, dialkylaminocarbonyl, alkoxy-carbonyl, haloalkyl, cyanoalkyl, alkenyl, alkynyl, alkylcarbonyl or haloalkylcarbonyl, wherein a compound of formula (Ib), in which Z is formyl, prepared via procedures described in process $P_2d$, and in which X and amino are optionally protected as required:

a) is reduced to a compound of formula (Ib), in which Z is alkyl, particularly methyl, by known reducing agents such as sodium borohydride or p-toluenesulfonylhydrazine and sodium cyanoborohydride;

b) is reacted with a standard known oxidizing agent to give a compound of formula (Ib), in which Z is carboxyl or salts thereof, then optionally the carboxyl compound can be converted to a compound of formula (Ib), in which Z is amino, by a Curtius rearrangement via an intermediate acid halide, azide, and isocyanate or optionally the compound in which Z is carboxyl is treated with isophthalonitrile to give a compound of formula (Ib), in which Z is cyano, or optionally the compound in which Z is formyl is reacted with hydroxylamine to give an intermediate aldoxime compound which is then dehydrated by standard procedures to give the compound of formula (Ib), in which Z is cyano;

c) is converted to the compound in which Z is carboxyl by the procedure b) above, then the carboxyl is converted by standard procedures to an intermediate acid halide compound, which then is reacted with ammonia, an alkylamine, dialkylamine or alkyl alcohol to give a compound of formula (Ib), in which Z is aminocarbonyl, dialkylaminocarbonyl or alkoxycarbonyl;

d) is first reduced to an intermediate hydroxymethyl compound by procedures similar to those in process $P_1b$, followed by halogenation procedures also similar to those of process $P_1b$, to give a compound of formula (Ib), in which Z is haloalkyl, more specifically halomethyl, or optionally the haloalkyl compound, specifically halomethyl, is treated with a metal cyanide to give a compound of formula (Ib), in which Z is cyanoalkyl, more specifically cyanomethyl;

e) is reacted in a Wittig or modified Wittig reaction to give a compound of formula (Ib), in which Z is alkenyl or alkynyl;

f) is reacted with a Grignard reagent or alkyllithium reagent to give an intermediate compound of formula (Ib), in which Z is α-hydroxyalkyl, then is oxidized with known reagents to give a compound of formula (Ib), in which Z is alkylcarbonyl, then the alkylcarbonyl compound is optionally halogenated to a compound of formula (Ib), in which Z is haloalkylcarbonyl; or

g) is first converted according to the procedures above in parts b) and c) to the acid chloride intermediate, obtained via the compound in which Z is carboxyl, then by conventional Curtius rearrangement procedures, the acid halide intermediate is converted via azide and isocyanate intermediates to the compound of formula (Ib), in which Z is amino.

**$P_4$**. A process of preparation of a compound of formula (Ib),

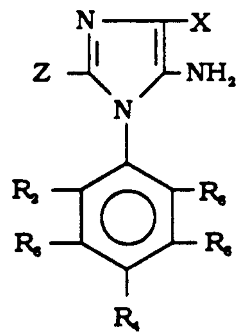

(Ib)

wherein X, $R_2$, $R_3$, $R_4$, $R_5$ and $R_6$ are as defined for formula (I) and Z is alkylamino, dialkylamino, trialkylammonium salt, alkoxycarbonylamino, alkylaminocarbonylamino, dialkylaminocarbonylamino, alkoxyalkylideneimino, alkylcarbonylamino, haloalkylcarbonylamino or arylcarbonylamino, wherein a compound of formula (Ib), in which Z is amino, prepared via procedures described in process $P_2$b or $P_2$g and in which X and Y is amino are optionally protected as required:

a) is first reacted with phosgene to give an intermediate compound of formula (Ib), in which Z is chlorocarbonylamino or isocyanato, which then is reacted with an alkyl alcohol, alkylamine or dialkylamine to give a compound of formula (Ib), in which Z is alkoxycarbonylamino, alkylaminocarbonylamino or dialkylaminocarbonylamine ;

b) is reacted with an alkylating agent, such as an alkyl iodide or dialkyl sulfate or optionally by known reductive methylation using formaldehyde and formic acid to give a compound of formula (Ib), in which Z is alkylamino, dialkylamino or trialkylammonium salt;

c) is reacted with an alkyl orthoformate to give a compound of formula (Ib), in which Z is alkoxyalkylideneimino, particularly alkoxymethylideneimino; or

d) is reacted with an alkyl-, haloalkyl- or arylcarbonyl halide, optionally in the presence of an acid acceptor, to give a compound of formula (Ib), in which Z is alkylcarbonylamino, haloalkylcarbonylamino or arylcarbonylamino.

**$P_5$**. A process of preparation of a compound of formula (I),

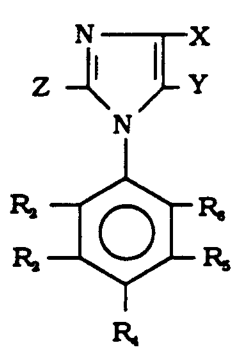

(I)

wherein X, Z, $R_2$, $R_3$, $R_4$, $R_5$ and $R_6$ are as defined for formula (I) and Y is hydrogen, amino, halogen, alkylsulfenyl, haloalkylsulfenyl, alkylsulfinyl, haloalkylsulfinyl, alkylsulfonyl, haloalkylsulfonyl, cyano or nitro, wherein a compound of formula (Ib),

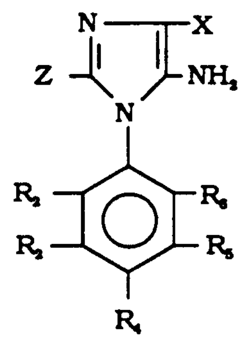

(Ib)

in which X, Z and $R_2$ to $R_6$ are as defined above and in which X, Z, and amino are optionally protected as required:

a) is deaminated by known procedures, such as with an alkylnitrite to convert the compound, in which Y is amino, into its corresponding diazonium salt, followed by quenching the diazonium salt with a quenching agent according to known procedures to obtain a compound of formula (I), in which Y is hydrogen, halogen, cyano, nitro, alkylsulfenyl, or haloalkylsulfenyl and then the compound, in which Y is alkylsulfenyl or haloalkylsulfenyl is optionally oxidized to a compound of formula (I), in which Y is alkylsulfinyl, haloalkylsulfinyl, alkylsulfonyl or haloalkylsulfonyl.

**$P_6$**. A process of preparation of a compound of formula (I),

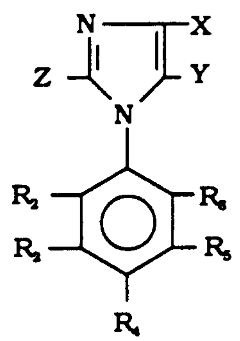

(I)

wherein X, Z, $R_2$, $R_3$, $R_4$, $R_5$ and $R_6$ are as defined for formula (I) and Y is alkoxycarbonylamino, alkylaminocarbonylamino, dialkylaminocarbonylamino, alkoxyalkylideneimino, alkylcarbonylamino, haloalkylcarbonylamino, arylcarbonylamino, alkylamino, dialkylamino or trialkylammonium salt, wherein a compound of formula (Ib),

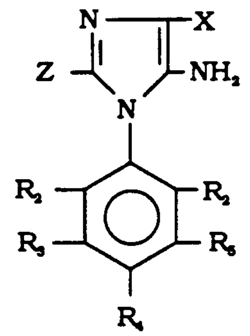

(Ib)

in which X, Z and $R_2$ to $R_6$ are as defined above and in which X, Z and amino are optionally protected as required:

a) is reacted in a manner similar to that described in process $P_4$a via a chlorocarbonylamino or isocyanato intermediate, obtained by reaction with phosgene, which then is by reacted with an alkyl alcohol, alkylamine, or dialkylamine to give a compound of formula (I), in which Y is alkoxycarbonylamino, alkylaminocarbonylamino or dialkylaminocarbonylamino;

b) is reacted in a manner similar to that described in process $P_4$c with an alkylorthoformate to give a compound of formula (I), in which Y is alkoxyalkylideneimino, particularly alkoxymethylideneimino;

c) is reacted in a similar manner to that described in process $P_4$b by alkylation or reductive methylation to give a compound of formula (I), in which Y is alkylamino, dialkylamino or trialkylammonium salt; or

d) is reacted in a similar manner to that described in process $P_4$d with an alkyl-, haloalkyl- or arylcarbonyl halide to give a compound of formula (I), in which Y is alkylcarbonylamino, haloalkylcarbonylamino or arylcarbonylamino.

**$P_7$**. A process of preparation of a compound of formula (I),

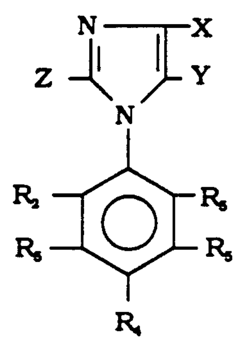

(I)

wherein X, Z, $R_2$, $R_3$, $R_4$, $R_5$ and $R_6$ are as defined for formula (I) and Y is nitro, sulfhydryl and salts thereof, hydroxyl and salts thereof, alkoxy, haloalkoxy, aminosulfonyl, alkylaminosulfonyl, dialkylaminosulfonyl, alkyl, haloalkyl, alkenyl, alkynyl, cyanoalkyl or formyl, wherein a compound of formula (Ib),

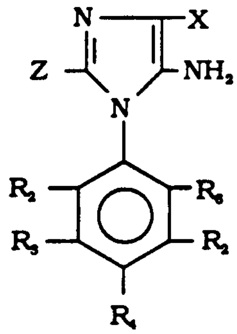

(Ib)

in which X, Z and $R_2$ to $R_6$ are as defined above and in which X and Z are optionally protected as required is deaminated according to the procedure described in process $P_5$ to give a compound of formula (I), in which Y is hydrogen, then said compound, in which X and Z are optionally protected as required:

a) is nitrated by procedures similar to those described in process $P_5$b to give a compound of formula (I), in which Y is nitro;

b) is reacted in a similar manner by the procedures described in process $P_5$f to first give an intermediate compound, in which Y is thiocyano, which is then reacted to give a compound of formula (I), in which Y is sulfhydryl and salts thereof;

c) is first reacted with a strong base such as an organolithium reagent to give an intermediate metal carbanion, which is then quenched with an electrophile to give a compound of formula (I), in which Y is alkyl, haloalkyl, alkenyl, alkynyl, cyanoalkyl or formyl;

d) is converted to the carbanion, as above in part c), and then quenched with sulfuryl chloride to give an intermediate compound, in which Y is chlorosulfonyl, which then is reacted with ammonia or an alkyl- or dialkylamine to give a compound of formula (I), in which Y is aminosulfonyl, alkylaminosulfonyl or dialkylaminosulfonyl;

e) is converted to the carbanion as above in part c) or optionally the carbanion is prepared via the compound in which Y is halogen, obtained by the procedure of process $P_5$, and then the carbanion is reacted in a similar manner to the procedure described in process $P_5$c to give a compound of formula (I), in which Y is hydroxyl and salts thereof, alkoxy or haloalkoxy.

**$P_8$**. A process of preparation of a compound of formula (I),

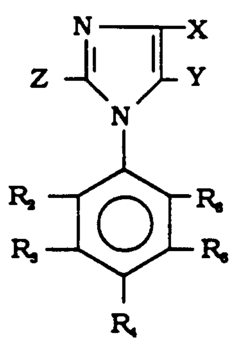

(I)

wherein X, Z, $R_2$, $R_3$, $R_4$, $R_5$ and $R_6$ are as defined for formula (I) and Y is carboxyl and salts thereof, cyano, aminocarbonyl, alkylaminocarbonyl, dialkylaminocarbonyl, alkoxycarbonyl, haloalkyl, alkenyl, alkynyl, alkylcarbonyl or haloalkylcarbonyl, wherein a compound of formula (Ib),

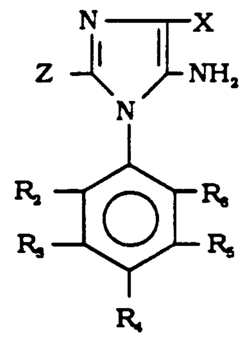

(Ib)

in which X, Z and $R_2$ to $R_6$ are as defined above is first deaminated according to the procedures described in process $P_5$ to give a compound, in which Y is hydrogen, which is then converted by procedures described in process $P_7$c to give a compound of formula (I), in which Y is formyl, then said formyl compound, in which X and Z are optionally protected as required:

a) is reacted by similar procedures described in process $P_3$b to give a compound of formula (I), in which Y is carboxyl and salts thereof or cyano;

b) is reacted by similar procedures described in process $P_3$c to give a compound of formula (I), in which Y is aminocarbonyl, alkylaminocarbonyl, dialkylaminocarbonyl or alkoxycarbonyl;

c) is reacted by similar procedures described in process $P_3$d to give a compound of formula (I), in which Y is haloalkyl, more specifically halomethyl;

d) is reacted by similar procedures described in process $P_2$b and $P_3$e to give a compound of formula (I), in which Y is alkenyl or alkynyl; or

e) is reacted by similar procedures described in process $P_3$f to give a compound of formula (I), in which Y is alkylcarbonyl or haloalkylcarbonyl.

**$P_9$**. A process of preparation of a compound of formula (I),

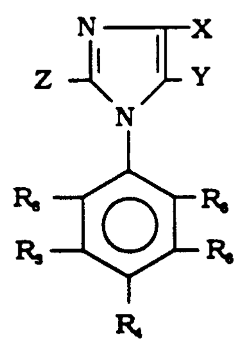

(I)

wherein Y, Z, $R_2$, $R_3$, $R_4$, $R_5$ and $R_6$ are as defined for formula (I) and X is alkylsulfenyl, haloalkylsulfenyl, alkylsulfinyl, haloalkylsulfinyl, alkylsulfonyl or haloalkylsulfonyl, wherein a compound of formula (Ic),

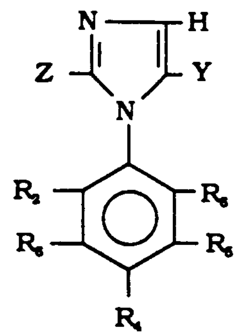

(Ic)

in which Y and Z are optionally protected as required:

a) is first reacted according to procedures similar to those described in process $P_2e$ to convert a compound of formula (Ic), in which X is hydrogen, to an intermediate compound of formula (I), in which X is successively thiocyano and then a disulfide, then also by procedures similar to those described in process $P_2e$, the thiocyano or disulfide intermediates are converted to a compound of formula (I), in which X is alkylsulfenyl or haloalkylsulfenyl, particularly perhaloalkylsulfenyl, which compound then is optionally oxidized by procedures similar to those in process $P_2e$, to obtain the sulfoxide or sulfone analogs, that is a compound of formula (I), in which X is alkylsulfinyl, haloalkylsulfinyl, preferably perhaloalkylsulfinyl, alkylsulfonyl or haloalkylsulfonyl, particularly perhaloalkylsulfonyl; or

b) is first reacted according to procedures similar to those described in process $P_2a$ to convert the compound of formula (Ic), in which X is hydrogen to an intermediate compound of formula (I), in which X is chlorosulfonyl, then the chlorosulfonyl compound is reacted with a reducing agent such as triphenylphosphine to give the same disulfide intermediate described above in part a), then finally the disulfide is converted by the procedures described above in part a) to give a compound of formula (I), in which X is alkylsulfenyl or haloalkylsulfenyl, particularly perhaloalkylsulfenyl or optionally the sulfenyl compound is oxidized to give a compound of formula (I), in which X is alkylsulfinyl, haloalkylsulfinyl, particularly perhaloalkylsulfinyl, alkylsulfonyl or haloalkylsulfonyl, particularly perhaloalkylsulfonyl.

**$P_{10}$**. A process of preparation of a compound of formula (IV),

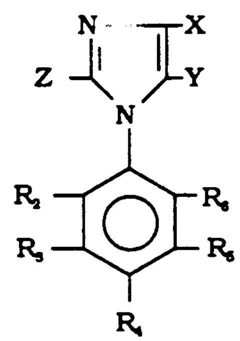

(IV)

wherein:

| | |
|---|---|
| $R_2$, $R_3$, $R_4$, $R_5$ and $R_6$ | are as defined in formula (I); |
| X | is hydrogen or haloalkyl, particularly trifluoromethyl; |
| Y | is amino; hydroxy optionally existing in its isomeric keto form when X is hydrogen; alkoxy or haloalkoxy; |
| Z | is hydrogen; halogen; alkyl; haloalkyl; hydroxy, optionally esixting in its isomeric keto form when X is hydrogen and Y is imino; alkoxy or haloalkoxy; |

whereby a compound of formula (III),

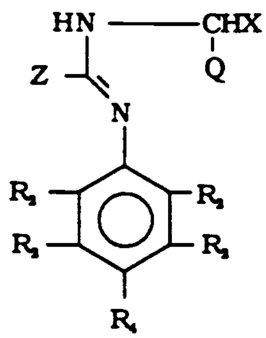

(III)

wherein:

| | |
|---|---|
| $R_2$, $R_3$, $R_4$, $R_5$ and $R_6$ | are as defined above; and |
| X | is hydrogen or haloalkyl, particularly trifluoromethyl; |
| Z | is hydrogen, halogen, alkyl, haloalkyl or hydroxy, optionally existing in its isomeric keto form; and |
| Q | is cyano or lower alkoxycarbonyl; |

is reacted with a basic agent in a suitable reaction medium to give a compound of formula (IV) which when Y or Z is hydroxy, is then optionally alkylated or haloalkylated to Y or Z is alkoxy or haloalkoxy.

**P**$_{11}$. The process of preparation of a compound of formula (IV), according to process $P_{10}$, whereby the compound of formula (IV) is:

a) a compound of formula (5), in which X and Z are each hydrogen and Y is amino;

b) a compound of formula (17), in which X is hydrogen, Y is amino, and Z is halogen, particularly chlorine;

c) a compound of formula (22), in which X is hydrogen, Y is amino, and Z is alkyl or haloalkyl;

d) a compound of formula (27), in which X is haloalkyl, particularly trifluoromethyl, Y is amino, and Z is halogen, alkyl, or haloalkyl;

e) a compound of formula (30), optionally existing in its isomeric keto form (29), in which X is hydrogen, Y is hydroxyl, which is optionally alkylated to Y is alkoxy or haloalkoxy, and Z is halogen, alkyl or haloalkyl; or

f) a compound formula (37), optionally existing in its isomeric keto-imino form (34), in which X is hydrogen, Y is amino, and Z is hydroxy, which is optionally alkylated to Z is alkoxy or haloalkoxy or optionally halogenated to Z is halogen.

**P**$_{12}$. A process of preparation of a compound of formula (I), wherein X, Y, Z, $R_2$, $R_3$, $R_4$, $R_5$ and $R_6$ are as defined in formula (I), whereby a compound of formula (5) is reacted according to the process of preparation of any of processes $P_1$ to $P_9$ for introduction of the X, Y and Z substituents.

**P**$_{13}$. A process of preparation of a compound of formula (I), wherein X, Y, $R_2$, $R_3$, $R_4$, $R_5$ and $R_6$ are as defined in formula (I) and Z is halogen, whereby a compound of formula (17) is reacted according to the process of preparation of any of processes $P_1$ to $P_9$ for introduction of the X and Y substituents.

**P**$_{14}$. A process of preparation of a compound of formula (I), wherein X, Y, $R_2$, $R_3$, $R_4$, $R_5$ and $R_6$ are as defined in formula (I) and Z is alkyl or haloalkyl, whereby a compound of formula (22) is reacted according to the process of preparation of any of processes $P_1$ to $P_9$ for introduction of the X and Y substituents.

**P**$_{15}$. A process of preparation of a compound of formula (I), wherein Y, $R_2$, $R_2$, $R_4$, $R_5$ and $R_6$ are as defined in formula (I), X is haloalkyl, particularly trifluoromethyl, and Z is halogen, alkyl or haloalkyl, whereby a compound of formula (27) is reacted according to the process of any of processes $P_1$ to $P_9$ for introduction of the Y substituent.

**P**$_{16}$. A process of preparation of a compound of formula (I), wherein X, $R_2$, $R_3$, $R_4$, $R_5$ and $R_6$ are as defined in formula (I), Y is hydroxy, alkoxy or haloalkoxy, and Z is halogen, alkyl or haloalkyl, whereby a compound of formula (30), optionally existing in its isomeric keto form (29), in which Y is hydroxy, optionally alkylated to Y is alkoxy or haloalkoxy, is reacted according to the process of preparation of any of processes $P_1$ to $P_9$ for introduction of the X substituent.

**P**$_{17}$. A process of preparation of a compound of formula (I), wherein X, Y, $R_2$, $R_3$, $R_4$, $R_5$ and $R_6$ are as defined in formula (I) and Z is hydroxy, alkoxy, haloalkoxy or halogen, whereby a compound of formula (37), optionally existing in its isomeric keto-imino form (34), in which Z is hydroxy, optionally alkylated to Z is alkoxy or haloalkoxy or optionally halogenated to Z is halogen, is reacted according to the process

of preparation of any of processes $P_1$ to $P_9$ for introduction of X and Y substituents.

**P**$_{18}$. The invention is also related to intermediate compounds as follows: a compound of formula (Ia), (Ib), (Ic), (IV), 5, 17, 22, 27, (30)/(29), or (37)/(34), wherein the substituents X, Y, Z, $R_2$, $R_3$, $R_4$, $R_5$ and $R_6$ are as defined in processes $P_1$ to $P_{17}$, which compound is useful for the preparation of a compound of formula (I), according to any of processes $P_1$ to $P_{17}$.

**P**$_{19}$. The invention is also related to intermediate compounds as follows: a compound of formula (III),

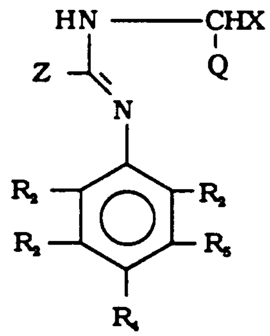

(III)

wherein:

| | |
|---|---|
| $R_2$, $R_3$, $R_4$, $R_5$ and $R_6$ | are as defined in formula (I); |
| X | is hydrogen or haloalkyl, particularly trifluoromethyl; |
| Z | is hydrogen, halogen, alkyl, haloalkyl or hydroxy, optionally existing in its isomeric keto form; and |
| Q | is cyano or lower alkoxycarbonyl, |

which compound is useful for the preparation of an intermediate N-phenylimidazole used in the preparation of a compound of formula (I), according to any of processes $P_1$ to $P_{17}$. Specific compounds of formula (III) are compounds of formula (4), (16), (21), (26), (28) or (33).

REPRESENTATIVE COMPOUNDS OF THE INVENTION

The compounds in **TABLE 1** are illustrative of some of the preferred compounds within the purview of the above generic formula (I) or (II) and can be prepared by the herein described methods or processes of synthesis, by the appropriate selection of reactants, conditions and procedures, which are commonly known and apparent to one skilled in the art.

**TABLE 1**

**REPRESENTATIVE 1-ARYLIMIDAZOLE COMPOUNDS OF FORMULA (I)**

**SUBSTITUENT GROUPS**

| No. | X | Y | Z | $R_2$ | $R_3$ | $R_4$ | $R_5$ | $R_6$ |
|---|---|---|---|---|---|---|---|---|
| 1 | $SCF_3$ | H | H | Cl | H | $OCF_3$ | H | Cl |
| 2 | $SCF_3$ | H | $CH_3$ | Cl | H | $OCF_3$ | H | Cl |
| 3 | $SCF_3$ | H | Cl | Cl | H | $OCF_3$ | H | Cl |
| 4 | $SCF_3$ | H | Br | Cl | H | $OCF_3$ | H | Cl |
| 5 | $SOCF_3$ | H | Cl | Cl | H | $OCF_3$ | H | Cl |
| 6 | $SO_2CF_3$ | H | Cl | Cl | H | $OCF_3$ | H | Cl |
| 7 | $SOCF_3$ | H | Br | Cl | H | $OCF_3$ | H | Cl |
| 8 | $SO_2CF_3$ | H | Br | Cl | H | $OCF_3$ | H | Cl |
| 9 | $SCF_3$ | H | $CF_3$ | Cl | H | $OCF_3$ | H | Cl |
| 10 | $SOCF_3$ | H | $CF_3$ | Cl | H | $OCF_3$ | H | Cl |
| 11 | $SO_2CF_3$ | H | $CF_3$ | Cl | H | $OCF_3$ | H | Cl |
| 12 | $SCF_3$ | H | $CF_3$ | Cl | H | $CF_3$ | H | Cl |
| 13 | $SOCF_3$ | H | $CF_3$ | Cl | H | $CF_3$ | H | Cl |
| 14 | $SO_2CF_3$ | H | $CF_3$ | Cl | H | $CF_3$ | H | Cl |
| 15 | $SCF_3$ | H | $OCH_3$ | Cl | H | $CF_3$ | H | Cl |
| 16 | $SOCF_3$ | H | $OCH_3$ | Cl | H | $CF_3$ | H | Cl |
| 17 | $SOCF_3$ | H | SH | Cl | H | $CF_3$ | H | Cl |

**TABLE 1**

**REPRESENTATIVE 1-ARYLIMIDAZOLE COMPOUNDS OF FORMULA (I)**

| No. | SUBSTITUENT GROUPS | | | | | | | |
|---|---|---|---|---|---|---|---|---|
| | X | Y | Z | $R_2$ | $R_3$ | $R_4$ | $R_5$ | $R_6$ |
| 18 | $SCF_3$ | H | $SCF_3$ | Cl | H | $CF_3$ | H | Cl |
| 19 | $SOCF_3$ | H | $SCF_3$ | Cl | H | $CF_3$ | H | Cl |
| 20 | $SOCF_3$ | H | $SOCF_3$ | Cl | H | $CF_3$ | H | Cl |
| 21 | $SOCF_3$ | H | $SO_2CF_3$ | Cl | H | $CF_3$ | H | Cl |
| 22 | $SCF_3$ | H | $SCF_3$ | Cl | H | $OCF_3$ | H | Cl |
| 23 | $SCF_3$ | H | $SCH_3$ | Cl | H | $CF_3$ | H | Cl |
| 24 | $SOCF_3$ | H | $SCH_3$ | Cl | H | $CF_3$ | H | Cl |
| 25 | $SO_2CF_3$ | H | $SCH_3$ | Cl | H | $CF_3$ | H | Cl |
| 26 | $SCF_3$ | H | $SOCH_3$ | Cl | H | $CF_3$ | H | Cl |
| 27 | $SOCF_3$ | H | $SOCH_3$ | Cl | H | $CF_3$ | H | Cl |
| 28 | $SOCF_3$ | H | $SO_2CH_3$ | Cl | H | $CF_3$ | H | Cl |
| 29 | $SCF_3$ | H | CN | Cl | H | $CF_3$ | H | Cl |
| 30 | $SOCF_3$ | H | CN | Cl | H | $CF_3$ | H | Cl |
| 31 | $SO_2CF_3$ | H | CN | Cl | H | $CF_3$ | H | Cl |
| 32 | $SCF_3$ | H | F | Cl | H | $CF_3$ | H | Cl |
| 33 | $SOCF_3$ | H | F | Cl | H | $CF_3$ | H | Cl |
| 34 | $SO_2CF_3$ | H | F | Cl | H | $CF_3$ | H | Cl |

**TABLE 1**

**REPRESENTATIVE 1-ARYLIMIDAZOLE COMPOUNDS OF FORMULA (I)**

| No. | SUBSTITUENT GROUPS | | | | | | | |
|---|---|---|---|---|---|---|---|---|
| | X | Y | Z | $R_2$ | $R_3$ | $R_4$ | $R_5$ | $R_6$ |
| 35 | $SCF_3$ | H | Cl | Br | H | $CF_3$ | H | Cl |
| 36 | $SCF_3$ | H | Cl | Cl | H | $CF_3$ | H | H |
| 37 | $SCF_3$ | H | Cl | Br | H | $CF_3$ | H | H |
| 38 | $SOCF_3$ | H | Br | Cl | H | $CF_3$ | H | H |
| 39 | $SO_2CF_3$ | H | Br | Cl | H | $CF_3$ | H | H |
| 40 | $SOCF_3$ | H | Cl | Cl | H | Br | H | Cl |
| 41 | $SCF_3$ | H | Cl | F | F | $CF_3$ | F | F |
| 42 | $SOCF_3$ | H | Cl | F | F | $CF_3$ | F | F |
| 43 | $SO_2CF_3$ | H | Cl | F | F | $CF_3$ | F | F |
| 44 | $SCF_3$ | H | Cl | Cl | H | $t\text{-}C_4H_9$ | H | Cl |
| 45 | $SCF_2Cl$ | H | Cl | Cl | H | $OCF_3$ | H | Cl |
| 46 | $SOCF_2Cl$ | H | Cl | Cl | H | $OCF_3$ | H | Cl |
| 47 | $SO_2CF_2Cl$ | H | Cl | Cl | H | $OCF_3$ | H | Cl |
| 48 | $SCF_2Cl$ | H | Br | Cl | H | $OCF_3$ | H | Cl |
| 49 | $SOCF_2Cl$ | H | Br | Cl | H | $OCF_3$ | H | Cl |
| 50 | $SO_2CF_2Cl$ | H | Br | Cl | H | $OCF_3$ | H | Cl |
| 51 | $SCF_2Cl$ | H | $SCF_2Cl$ | Cl | H | $OCF_3$ | H | Cl |

**TABLE 1**

**REPRESENTATIVE 1-ARYLIMIDAZOLE COMPOUNDS OF FORMULA (I)**

| No. | SUBSTITUENT GROUPS | | | | | | | |
|---|---|---|---|---|---|---|---|---|
| | X | Y | Z | $R_2$ | $R_3$ | $R_4$ | $R_5$ | $R_6$ |
| 52 | $SCF_2Cl$ | H | CN | Cl | H | $CF_3$ | H | Cl |
| 53 | $SOCF_2Cl$ | H | CN | Cl | H | $CF_3$ | H | Cl |
| 54 | $SO_2CF_2Cl$ | H | CN | Cl | H | $CF_3$ | H | Cl |
| 55 | $SCF_2Cl$ | H | F | Cl | H | $CF_3$ | H | Cl |
| 56 | $SOCF_2Cl$ | H | F | Cl | H | $CF_3$ | H | Cl |
| 57 | $SO_2CF_2Cl$ | H | F | Cl | H | $CF_3$ | H | Cl |
| 58 | $SCF_2Cl$ | H | $CH_3$ | Cl | H | $CF_3$ | H | Cl |
| 59 | $SCF_2Cl$ | H | $SCH_3$ | Cl | H | $CF_3$ | H | Cl |
| 60 | $SOCF_2Cl$ | H | $SCH_3$ | Cl | H | $CF_3$ | H | Cl |
| 61 | $SO_2CF_2Cl$ | H | $SCH_3$ | Cl | H | $CF_3$ | H | Cl |
| 62 | $SOCF_2Cl$ | H | $SOCH_3$ | Cl | H | $CF_3$ | H | Cl |
| 63 | $SCF_2Cl$ | H | Cl | Cl | H | $CF_3$ | H | H |
| 64 | $SOCF_2Cl$ | H | Cl | Cl | H | $CF_3$ | H | H |
| 65 | $SOCF_2Cl$ | H | Br | Cl | H | $CF_3$ | H | H |
| 66 | $SOCF_2Cl$ | H | Cl | Br | H | $CF_3$ | H | H |
| 67 | $SCF_2Cl$ | H | $OCH_3$ | Cl | H | $CF_3$ | H | H |
| 68 | $SOCF_2Cl$ | H | $OCH_3$ | Cl | H | $CF_3$ | H | H |

**TABLE 1**

**REPRESENTATIVE 1-ARYLIMIDAZOLE COMPOUNDS OF FORMULA (I)**

| No. | SUBSTITUENT GROUPS | | | | | | | |
|---|---|---|---|---|---|---|---|---|
| | X | Y | Z | $R_2$ | $R_3$ | $R_4$ | $R_5$ | $R_6$ |
| 69 | $SCCl_2F$ | H | $SCH_3$ | Cl | H | $CF_3$ | H | Cl |
| 70 | $SOCCl_2F$ | H | $SCH_3$ | Cl | H | $CF_3$ | H | Cl |
| 71 | $SO_2CCl_2F$ | H | $SCH_3$ | Cl | H | $CF_3$ | H | Cl |
| 72 | $SCCl_2F$ | H | $SOCH_3$ | Cl | H | $CF_3$ | H | Cl |
| 73 | $SOCCl_2F$ | H | $SOCH_3$ | Cl | H | $CF_3$ | H | Cl |
| 74 | $SO_2CCl_2F$ | H | $SOCH_3$ | Cl | H | $CF_3$ | H | Cl |
| 75 | $SCCl_2F$ | H | Cl | Cl | H | $OCF_3$ | H | Cl |
| 76 | $SOCCl_2F$ | H | Cl | Cl | H | $OCF_3$ | H | Cl |
| 77 | $SO_2CCl_2F$ | H | Cl | Cl | H | $OCF_3$ | H | Cl |
| 78 | $SCCl_2F$ | H | CN | Cl | H | $CF_3$ | H | Cl |
| 79 | $SOCCl_2F$ | H | CN | Cl | H | $CF_3$ | H | Cl |
| 80 | $SO_2CCl_2F$ | H | CN | Cl | H | $CF_3$ | H | Cl |
| 81 | $SCCl_2F$ | H | $NO_2$ | Cl | H | $CF_3$ | H | Cl |
| 82 | $SOCCl_2F$ | H | $C_2H_5$ | Cl | H | $CF_3$ | H | Cl |
| 83 | $SCCl_2F$ | H | $OCH_3$ | Cl | H | $CF_3$ | H | Cl |
| 84 | $SOCCl_2F$ | H | $CF_3$ | Cl | H | $CF_3$ | H | Cl |
| 85 | $SCCl_2F$ | H | $SCCl_2F$ | Cl | H | $CF_3$ | H | Cl |

**TABLE 1**

**REPRESENTATIVE 1-ARYLIMIDAZOLE COMPOUNDS OF FORMULA (I)**

| | SUBSTITUENT GROUPS | | | | | | | |
|---|---|---|---|---|---|---|---|---|
| **No.** | **X** | **Y** | **Z** | **$R_2$** | **$R_3$** | **$R_4$** | **$R_5$** | **$R_6$** |
| **86** | $SCCl_2F$ | H | $NH_2$ | Cl | H | $CF_3$ | H | Cl |
| **87** | $SCCl_2F$ | $NHCOCH_3$ | Cl | Cl | H | $CF_3$ | H | Cl |
| **88** | $SCCl_2F$ | $NHCOCF_3$ | Cl | Cl | H | $CF_3$ | H | Cl |
| **89** | $SOCCl_2F$ | $NHCOCH_3$ | Br | Cl | H | $CF_3$ | H | Cl |
| **90** | $SOCCl_2F$ | $N=CHOC_2H_5$ | Cl | Cl | H | $CF_3$ | H | Cl |
| **91** | $SOCCl_2F$ | $NHCOOCH_3$ | Cl | Cl | H | $CF_3$ | H | Cl |
| **92** | $SOCCl_2F$ | $NHCONHCH_3$ | Cl | Cl | H | $CF_3$ | H | Cl |
| **93** | $SOCCl_2F$ | $NHCON(CH_3)_2$ | Cl | Cl | H | $CF_3$ | H | Cl |
| **94** | $SOCCl_2F$ | H | $CH_2CH=CH_2$ | Cl | H | $CF_3$ | H | Cl |
| **95** | $SOCCl_2F$ | H | $OCF_3$ | Cl | H | $CF_3$ | H | Cl |
| **96** | $SCCl_2F$ | H | $COCF_3$ | Cl | H | $CF_3$ | H | Cl |
| **97** | $SCCl_2F$ | H | CHO | Cl | H | $CF_3$ | H | Cl |
| **98** | $SCCl_2F$ | H | $COCH_3$ | Cl | H | $CF_3$ | H | Cl |
| **99** | $SCCl_2F$ | H | $COOC_2H_5$ | Cl | H | $CF_3$ | H | Cl |
| **100** | $SOCCl_2F$ | COOH | H | Cl | H | $CF_3$ | H | Cl |
| **101** | $SOCCl_2F$ | $CONH_2$ | H | Cl | H | $CF_3$ | H | Cl |
| **102** | $SOCCl_2F$ | $CONHCH_3$ | H | Cl | H | $CF_3$ | H | Cl |

**TABLE 1**

**REPRESENTATIVE 1-ARYLIMIDAZOLE COMPOUNDS OF FORMULA (I)**

**SUBSTITUENT GROUPS**

| No. | X | Y | Z | $R_2$ | $R_3$ | $R_4$ | $R_5$ | $R_6$ |
|---|---|---|---|---|---|---|---|---|
| **103** | $SOCCl_2F$ | $CON(CH_3)_2$ | H | Cl | H | $CF_3$ | H | Cl |
| **104** | $SOCCl_2F$ | H | $CH_2CH=CH_2$ | Cl | H | $CF_3$ | H | Cl |
| **105** | $SOCCl_2F$ | H | $CH_2CN$ | Cl | H | $CF_3$ | H | Cl |
| **106** | $SOCCl_2F$ | Br | H | Cl | H | $OCF_3$ | H | Cl |
| **107** | $SOCCl_2F$ | H | Cl | Cl | H | $CF_3$ | H | Cl |
| **108** | $SOCCl_2F$ | H | Cl | Cl | H | $CF_3$ | H | $NO_2$ |
| **109** | $SOCCl_2F$ | H | Cl | Cl | H | $CF_3$ | H | $NH_2$ |
| **110** | $SOCCl_2F$ | H | Cl | Cl | H | $CF_3$ | H | $NHCH_3$ |
| **111** | $SOCCl_2F$ | H | Cl | Cl | H | $CF_3$ | H | CN |
| **112** | $SOCCl_2F$ | H | Cl | Cl | H | $CF_3$ | H | $CH_3$ |
| **113** | $SOCCl_2F$ | H | Cl | Cl | H | $CF_3$ | H | $OCH_3$ |
| **114** | $SOCCl_2F$ | H | Br | Cl | H | $CF_3$ | H | Cl |
| **115** | $SOCCl_2F$ | H | Br | Br | H | $CF_3$ | H | Cl |
| **116** | $SCCl_2F$ | H | F | Cl | H | $CF_3$ | H | Cl |
| **117** | $SOCCl_2F$ | H | F | Cl | H | $CF_3$ | H | Cl |
| **118** | $SO_2CCl_2F$ | H | F | Cl | H | $CF_3$ | H | Cl |
| **119** | $SOCCl_2F$ | H | Cl | Cl | H | $t\text{-}C_4H_9$ | H | Cl |

**TABLE 1**

**REPRESENTATIVE 1-ARYLIMIDAZOLE COMPOUNDS OF FORMULA (I)**

**SUBSTITUENT GROUPS**

| No. | X | Y | Z | $R_2$ | $R_3$ | $R_4$ | $R_5$ | $R_6$ |
|---|---|---|---|---|---|---|---|---|
| 120 | $SOCCl_2F$ | H | Cl | Cl | H | Br | H | Cl |
| 121 | $SCCl_2F$ | H | Cl | Cl | H | $SCF_3$ | H | Cl |
| 122 | $SCCl_2F$ | H | Cl | Cl | H | $SOCF_3$ | H | Cl |
| 123 | $SCCl_2F$ | H | Cl | Cl | H | $SO_2CF_3$ | H | Cl |
| 124 | $SCCl_2F$ | H | Cl | Cl | Cl | $CF_3$ | H | Cl |
| 125 | $SCCl_3$ | H | Cl | Cl | H | $CF_3$ | H | Cl |
| 126 | $SOCCl_3$ | H | Cl | Cl | H | $CF_3$ | H | Cl |
| 127 | $SO_2CCl_3$ | H | Cl | Cl | H | $CF_3$ | H | Cl |
| 128 | $SCCl_3$ | Cl | Br | Cl | H | $CF_3$ | H | Cl |
| 129 | $SOCCl_3$ | Cl | Br | Cl | H | $CF_3$ | H | Cl |
| 130 | $SO_2CCl_3$ | Cl | Br | Cl | H | $CF_3$ | H | Cl |
| 131 | $SOCCl_3$ | $SCH_3$ | H | Cl | H | $CF_3$ | H | Cl |
| 132 | $SOCCl_3$ | H | $SCH_3$ | Cl | H | $CF_3$ | H | Cl |
| 133 | $SCCl_3$ | H | $SOCH_3$ | Cl | H | $CF_3$ | H | Cl |
| 134 | $SCF_2CFCl_2$ | H | Cl | Cl | H | $CF_3$ | H | Cl |
| 135 | $SOCF_2CFCl_2$ | H | Cl | Cl | H | $CF_3$ | H | Cl |
| 136 | $SO_2CF_2CFCl_2$ | H | Cl | Cl | H | $CF_3$ | H | Cl |

**TABLE 1**

**REPRESENTATIVE 1-ARYLIMIDAZOLE COMPOUNDS OF FORMULA (I)**

**SUBSTITUENT GROUPS**

| No. | X | Y | Z | $R_2$ | $R_3$ | $R_4$ | $R_5$ | $R_6$ |
|---|---|---|---|---|---|---|---|---|
| 137 | $SOCF_2CFCl_2$ | H | Br | Cl | H | $CF_3$ | H | Cl |
| 138 | $SCH_3$ | H | Cl | Cl | H | $CF_3$ | H | Cl |
| 139 | $SOCH_3$ | H | Cl | Cl | H | $CF_3$ | H | Cl |
| 140 | $SO_2CH_3$ | H | Cl | Cl | H | $CF_3$ | H | Cl |
| 141 | $CF_3$ | H | Cl | Cl | H | $CF_3$ | H | Cl |
| 142 | $OCF_3$ | H | Cl | Cl | H | $CF_3$ | H | Cl |
| 143 | $SCF_3$ | OH | Cl | Cl | H | $CF_3$ | H | Cl |
| 144 | $SOCCl_2F$ | $NHCH_3$ | Cl | Cl | H | $CF_3$ | H | Cl |
| 145 | $SOCCl_2F$ | $NH(CH_3)_2$ | Cl | Cl | H | $CF_3$ | H | Cl |
| 146 | $SOCCl_2F$ | $CH_2C{\equiv}CH$ | Cl | Cl | H | $CF_3$ | H | Cl |
| 147 | $SOCCl_2F$ | H | OH | Cl | H | $CF_3$ | H | Cl |
| 148 | $SOCCl_2F$ | Cl | $NHCH_3$ | Cl | H | $CF_3$ | H | Cl |
| 149 | $SOCCl_2F$ | H | $N(CH_3)_2$ | Cl | H | $CF_3$ | H | Cl |
| 150 | $SOCCl_2F$ | H | $CH_2C{\equiv}CH$ | Cl | H | $CF_3$ | H | Cl |
| 151 | $SOCCl_2F$ | H | Cl | Cl | H | $SOCH_3$ | H | Cl |
| 152 | $SOCCl_2F$ | H | Cl | Cl | H | $SOt\text{-}C_4H_9$ | H | Cl |
| 153 | $SCCl_2F$ | H | Cl | Cl | H | $SO_2t\text{-}C_4H_9$ | H | Cl |

**TABLE 1**

**REPRESENTATIVE 1-ARYLIMIDAZOLE COMPOUNDS OF FORMULA (I)**

| No. | X | Y | Z | $R_2$ | $R_3$ | $R_4$ | $R_5$ | $R_6$ |
|---|---|---|---|---|---|---|---|---|
| | SUBSTITUENT GROUPS | | | | | | | |
| 154 | $SOCF_3$ | H | Cl | Cl | H | Br | H | Cl |
| 155 | $SOCF_2Br$ | H | Cl | Cl | H | Cl | H | Cl |
| 156 | $SOCCl_2F$ | $SO_2NH_2$ | Cl | Cl | H | Cl | H | Cl |
| 157 | $SOCCl_2F$ | $SO_2NHCH_3$ | Cl | Cl | H | $CF_3$ | H | Cl |
| 158 | $SOCCl_2F$ | $SO_2N(CH_3)_2$ | Cl | Cl | H | $CF_3$ | H | Cl |
| 159 | $SCF_3$ | $N(CH_3)_3^+$ | Cl | Cl | H | $CF_3$ | H | Cl |
| 160 | $SOCCl_2F$ | SNa | Cl | Cl | H | $CF_3$ | H | Cl |
| 161 | $SOCCl_2F$ | ONa | Cl | Cl | H | $CF_3$ | H | Cl |
| 162 | $SOCCl_2F$ | COONa | Cl | Cl | H | $CF_3$ | H | Cl |
| 163 | $SOCCl_2CF_3$ | H | Cl | Cl | H | $CF_3$ | H | Cl |
| 164 | $SOCF_3$ | H | Cl | Cl | H | $CH_2CF_3$ | H | Cl |
| 165 | $SCF_3$ | H | $CH_3$ | Cl | H | $OCF_3$ | H | Cl |
| 166 | $SOCF_3$ | H | $CH_3$ | Cl | H | $OCF_3$ | H | H |
| 167 | $SOCCl_2F$ | H | $CH_3$ | Cl | H | $OCF_3$ | H | Cl |
| 168 | $SOCF_3$ | H | $CH_3$ | Br | H | $OCF_3$ | H | Cl |
| 169 | $SOCCl_2F$ | H | $CH_3$ | Br | H | $OCF_3$ | H | Cl |
| 170 | $SOCClF_2$ | H | Cl | Br | H | $OCF_3$ | H | Cl |

**TABLE 1**

**REPRESENTATIVE 1-ARYLIMIDAZOLE COMPOUNDS OF FORMULA (I)**

**SUBSTITUENT GROUPS**

| No. | X | Y | Z | $R_2$ | $R_3$ | $R_4$ | $R_5$ | $R_6$ |
|---|---|---|---|---|---|---|---|---|
| 171 | $SOCF_3$ | H | H | $t-C_4H_9$ | H | Cl | H | H |
| 172 | $SOCl_2F$ | H | Cl | $t-C_4H_9$ | H | Br | H | H |
| 173 | $SOClF_2$ | H | $CH_3$ | $t-C_4H_9$ | H | Cl | H | H |
| 174 | $SOCF_3$ | H | Cl | $t-C_4H_9$ | H | Cl | H | Cl |
| 175 | $SOCCl_2F$ | H | H | $t-C_4H_9$ | H | Cl | H | Cl |
| 176 | $SCCl_2F$ | H | H | $t-C_4H_9$ | H | Cl | H | H |
| 177 | $SOCH_3$ | H | $CH_3$ | Cl | H | $OCF_3$ | H | Cl |
| 178 | $SOCH_3$ | H | Cl | $t-C_4H_9$ | H | Cl | H | H |
| 179 | $SO_2CCl_2F$ | H | $CH_3$ | Cl | H | $OCF_3$ | H | Cl |
| 180 | $SOCCl_2F$ | H | $CHF_2$ | Cl | H | $CF_3$ | H | Cl |
| 181 | $SOCCl_2F$ | H | $CH_2Cl$ | Cl | H | $CF_3$ | H | Cl |
| 182 | $SOCCl_2F$ | H | $CH_2CH_2CH_2CH_3$ | Cl | H | $CF_3$ | H | Cl |
| 183 | $SCCl_2F$ | H | $CH=CH_2$ | Cl | H | $CF_3$ | H | Cl |
| 184 | $SOCCl_2F$ | H | $CH=CH_2-CH_3$ | Cl | H | $CF_3$ | H | Cl |
| 185 | $SOCClF_2$ | H | $CH-(CH_3)_2$ | Cl | H | $CF_3$ | H | Cl |
| 186 | $SOCCl_2F$ | H | COOH | Cl | H | $CF_3$ | H | Cl |
| 187 | $SOCCl_2F$ | $CF_3$ | H | Cl | H | $CF_3$ | H | Cl |

**TABLE 1**

**REPRESENTATIVE 1-ARYLIMIDAZOLE COMPOUNDS OF FORMULA (I)**

**SUBSTITUENT GROUPS**

| No. | X | Y | Z | $R_2$ | $R_3$ | $R_4$ | $R_5$ | $R_6$ |
|---|---|---|---|---|---|---|---|---|
| 188 | $SOCCl_2F$ | $CH=CH_2$ | H | Cl | H | $CF_3$ | H | Cl |
| 189 | $SOCCl_2F$ | $CH=CH_2$-$CH_3$ | H | Cl | H | $CF_3$ | H | Cl |
| 190 | $SCCl_2F$ | CN | H | Cl | H | $CF_3$ | H | Cl |
| 191 | $SCCl_2F$ | H | $CH_3$ | Cl | H | Cl | H | Cl |
| 192 | $S(O)CCl_2F$ | H | $CH_3$ | Cl | H | Br | H | Cl |
| 193 | $SCCl_2F$ | H | $CH_3$ | Cl | H | Cl | H | H |

The following **EXAMPLES 1** to **164** further illustrate some of the more preferred compounds of formula (I) and (II) of the invention that were prepared. Details of typical methods of synthesis utilized in the preparation of intermediates and compounds of the invention are specifically provided below for compounds of **EXAMPLES 1** to **10**. The other compounds were prepared using similar methods of synthesis or modifications thereof of the detailed procedures as applicable to a given compound. These compound

**EXAMPLES 11** to **164** are listed in **TABLE 2**, wherein the compounds are grouped by the phenyl ring substitution shown below; $R_1$, n, Y, and Z are as defined. Reported melting points for compounds represent the average value of an observed melting point range determined for a compound or furthermore represent the average value of a number of separate melting point determinations. Additionally, one or more spectroscopic analyses (IR, NMR, GC/MS, etc.) have been performed on each compound for characterization and confirmation of the chemical structure.

| GROUP | PHENYL RING SUBSTITUTION IN TABLE 2 | | |
|---|---|---|---|
| | **$R_2$** | **$R_4$** | **$R_6$** |
| **1** | Cl | $CF_3$ | Cl |
| **2a** | $SCH_3$ | $CF_3$ | Cl |
| **2b** | $SC_2H_5$ | $CF_3$ | Cl |
| **2c** | $SOCH_3$ | $CF_3$ | Cl |
| **2d** | $SO_2CH_3$ | $CF_3$ | Cl |
| **3** | H | $CF_3$ | Cl |
| **4a** | Cl | Cl | Cl |
| **4b** | Cl | Br | Cl |
| **5a** | Cl | $OCF_3$ | Cl |
| **5b** | Br | $OCF_3$ | Br |
| **5c** | Br | $OCF_3$ | Cl |

**EXAMPLE 1**

Preparation of 1-(2,6-dichloro-4-trifluoromethylphenyl)-5-amino-4-trifluoromethylsulfenylimidazole.

Process SCHEME I:

a) Preparation of intermediate: ethyl N-(2,6-dichloro-4-trifluoromethylphenyl)formimidate.

To 1.09 g (4.6 mmole) of 2,6-dichloro-4-trifluoromethylaniline was added conc. HCl (0.46 mmole) and 1.04 g (7.0 mmole) of triethylorthoformate. The resulting mixture was stirred and then it was heated to 85 °C and evaporated under vacuum. The residue was analyzed by $^1H$ NMR which indicated the desired structure-$^1H$ NMR ($CDCL_3$) : $\delta$1.42 (t, J = 7.0 Hz, 3H). 4.47 (q, J = 7.0 Hz, 2H), 7.57 (s, 3H). This compound was used in the next step without further purification.

b) Preparation of intermediate: cyanomethyl N-(2,6-dichloro-4-trifluoromethylphenyl)formimidine.

To a solution of 20.20 g (0.218 mole) of aminoacetonitrile hydrochloride in 500 ml of methanol was added at 0 °C 11.79g (0.218 mole) of sodium methoxide. 'The mixture was stirred at RT for 30 min. and then evaporated to dryness under vacuum. The residue was extracted twice with 400 ml of diethyl ether and the ethereal solution was added to 62.45g (0.218 mole) of ethyl N-(2,6-dichloro-4-trifluoromethylphenyl)-formimidate at RT. The solvent was evaporated, 400ml of tetrahydrofuran was added, and the mixture was heated to reflux for 18 h. The solvent was then evaporated and the residue partitioned between water and methylene chloride. The organic layer was dried over anhydrous sodium sulfate and the solvent was evaporated. The residue was finally purified by flash column chromatography using 20% ethyl acetate in hexane, followed by elution with 30% ethyl acetate in hexane to give 24 g (37.25% yield) of the desired product. $^1H$ NMR ($CDCL_3$): $\delta$ 4.40 (s, 2H), 7.55 (s, 2H), 7.59 (s, 1H).

c) Preparation of intermediate: 1-(2,6-dichloro-4-trifluoromethylphenyl)-5-aminoimidazole.

To a solution of 4.4g (14.91 mmole) of cyanomethyl N-(2,6-dichloro-4-trifluoromethylphenyl)formimidine in 400 ml of methanol was added 81 mg (14.91 mmole) of sodium methoxide at 4 °C. The mixture was stirred at RT for 3h. The mixture was then evaporated to dryness to give the desired product (100% yield)-$^1$HNMR ($CDCL_3$/acetone-$d_6$): $\delta$ 3.43 (s, 2H), 6.68 (s, 1H), 7.28 (s, 1H), 7.88 (2H).

d) Preparation of 1-(2,6-dichloro-4-trifluoromethylphenyl)-5-amino-4-trifluoromethylsulfenylimidazole.

To a solution of 4.8g (14.91 mmole) of 1-(2,6-dichloro-4-trifluoromethylphenyl)-5-aminoimidazole in 400 ml of dichloroethane was added 1.3 ml (14.91 mmole) of trifluoromethanesulfenyl chloride at 0 °C. The mixture was stirred at 0 °C for 4 h and then at RT for 15 h. Water was added and the mixture was partitioned between water and methylene chloride. The organic layer was dried over anhydrous sodium sulfate and the solvent removed. The residue was recrystallized from methylene chloride to give 3.36g (52.51% yield) of the desired product., mp 134 °C.

| Anal.: $C_{11}H_5Cl_2F_6N_3S$. | | | | |
|---|---|---|---|---|
| Calc.:<br>Found: | C, 33.35;<br>C, 33.54; | H, 1.27;<br>H, 1.20; | N, 10.61;<br>N, 10.67; | S 8.09.<br>S 8.37. |

**EXAMPLE 2**

Preparation of 1-(2,6-dichloro-4-trifluoromethylphenyl)-5-amino-2-chloro-4-trifluoromethylsulfenylmidazole.

To a solution of 6.0 g (15.15 mmole) of 1-(2,6-dichloro-4-trifluoromethylphenyl)-5-amino-4-trifluoromethylsulfenylimidazole in 100 ml of methylene chloride was added 1.70 ml (18.18 mmole) of sulfuryl chloride at 0 °C. The resulting mixture was stirred at RT for 5 days under a nitrogen atmosphere. The mixture was quenched with water, then partitioned between methylene chloride and aqueous sodium bicarbonate. The organic layer was dried over anhydrous sodium sulfate and the solvent was removed. The residue was purified by column chromatography using 20% ethyl acetate in hexane to give 1.9 g (31.62% yield) of the desired product, mp 172.5 °C.

**EXAMPLE 3**

Preparation of 1-(2,6-dichloro-4-trifluoromethylphenyl)-2-chloro-4-trifluoromethylsulfenylimidazole.

To a solution of 2.0g (4.64 mmole) of 1-(2,6-dichloro-4-trifluoromethylphenyl)-5-amino-2-chloro-4-trifluoromethylsulfenylimidazole in 40 ml of tetrahydrofuran was added 2.76 ml (23.2 mmole) of t-butylnitrite. The resulting mixture was heated to reflux under a nitrogen atmosphere for 2 h. The mixture was evaporated to dryness and the residue was purified by column chromatography using 10% ethyl acetate in hexane to give 1.6g (83.0% yield) of the desired product, mp 112 °C.

| Anal.: $C_{11}H_3Cl_3F_6N_2S$. | | | | |
|---|---|---|---|---|
| Calc:<br>Found: | C, 31.79;<br>C, 31.71; | H, 0.73;<br>H, 0.68; | N, 6.74;<br>N, 6.75; | F, 27.43.<br>F, 27.65. |

**EXAMPLE 4**

Preparation of 1-(2,6-dichloro-4-trifluoromethylphenyl)-2-chloro-4-trifluoromethylsulphinylimidazole.

To a solution of 800 mg (1.93 mmole) of 1-(2,6-dichloro-4-trifluoromethylphenyl)-2-chloro-4-trifluoromethylsulfenylimidazole in trifluoroacetic acid was added 0.20 ml of 30% hydrogen peroxide at 0 °C. The resulting mixture was stirred at 0 °C for 4 h and then at RT for 50 h. The mixture was evaporated at RT and the residue was partitioned between methylene chloride and a saturated aqueous sodium bisulfite solution. The organic layer was washed with an aqueous sodium bicarbonate solution and the organic layer was evaporated. The residue was purified by flash column chromatography on silica gel using 5% ethyl acetate in hexane. After the solvent was removed there was obtained 300 mg (36.02% yield) of the desired product as a white solid, mp 147.5 °C.

| Anal.: $C_{11}H_3Cl_3F_6N_2OS$. | | | | | | |
|---|---|---|---|---|---|---|
| Calc.:<br>Found: | C, 30.61;<br>C, 30.63; | H, 0.70;<br>H, 0.83; | N, 6.49;<br>N, 6.48; | Cl, 24.64;<br>Cl, 24.83; | F, 26.41;<br>F, 26.53; | S, 7.43<br>S, 7.78. |

**EXAMPLE 5**

Preparation of 1-(2,6-dichloro-4-trifluoromethylphenyl)-2-chloro-4-trifluoromethylsulfonylimidazole.

To a solution of 300 mg (0.72 mmole) of 1-(2,6-dichloro-4-trifluoromethylphenyl)-2-chloro-4-trifluoromethylsulfenylimidazole in 5 ml of trifluoroacetic acid was added 0.15 ml (1.44 mmole) of 30% hydrogen peroxide at 0°C. The resulting mixture was stirred at RT for 4 days. The mixture was evaporated to remove trifluoroacetic acid and the residue was partitioned between methylene chloride and a saturated aqueous sodium bisulfite solution. The organic layer was washed with an aqueous sodium bicarbonate solution. The organic layer was dried over anhydrous sodium sulfate and the solvent was removed. The residue was purified by preparative TLC using 100% methylene chloride to give 190 mg (59.03% yield) of the desired product as white solid, mp 182.5°C.

**EXAMPLE 6**

Preparation of 1-(2,6-dichloro-4-trifluoromethylphenyl)-2-chloro-5-methylsulfenyl-4-trifluoromethylsulfenylimidazole.

To a solution of 700 mg (1.77 mmole) of 1-(2,6-dichloro-4-trifluoromethylphenyl)-5-amino-2-chloro-4-trifluoromethylsulfenylimidazole in 8 ml of chloroform was added 0.26 ml (2.54 mmole) of dimethyl disulfide and 0.32 ml (0.89 mmole) of t-butylnitrite at 0°C. The resulting mixture was stirred at 0°C for 15 min. and then at RT for 45 min.. The mixture was diluted with 75 ml of methylene chloride and partitioned between water and methylene chloride. The organic layer was dried over anhydrous sodium sulfate and the solvent was evaporated. The residue was purified by preparative TLC using 5% ethyl acetate in here to give 480 mg (58.74% yield) of the desired product. $^1H$ NMR ($CDCl_3$) : δ 2.26 (s, 3H), 7.82 (s, 2H).

**EXAMPLE 7**

Preparation of 1-(2,6-dichloro-4-trifluoromethylphenyl)-5-amino-2-bromo-4-trifluoromethylsulfenylimidazole.

To a solution of 1.35g (3.40 mmole) of 1-(2,6-dichloro-4-trifluoromethylphenyl)-5-amino-4-trifluoromethylsulfenylimidazole in 20 ml of chloroform was added 0.5 ml (9.76 mmole) of bromine. The resulting mixture was stirred at RT under a nitrogen atmosphere for 2 hr. The mixture was then evaporated to remove the excess of bromine and the residue was partioned between water and methylene chloride. The organic layer was dried over anhydrous sodium sulfate and solvent was removed. The residue was purified by flash column chromatography on silica gel using 7% ethyl acetate in hexane to give 200 mg (13.62% yield) of the desired product, mp 154°C.

**EXAMPLE 8**

Preparation of 1-(2,6-dichloro-4-trifluoromethylphenyl)-5-bromo-4-trifluoromethylsulfenylimidazole.

To a solution of 2.0g (5.05 mmole) of 1-(2,6-dichloro-4-trifluoromethylphenyl)-5-amino-4-trifluoromethylsulfenylimidazole in 10 ml of acetonitrile was added 1 ml of bromoform and 1.20 ml (10.10 mmole) of t-butylnitrite at 0°C. The resulting mixture was stirred at RT under a nitrogen atmosphere for 1.5 h. Ten ml of toluene was added and the mixture was evaporated to dryness under vacuum. The residue was purified by column chromatography on silica gel using 5% ethyl acetate in hexane to give 800 mg (34.44% yield) of the desired product, mp 87.5°C.

| Anal.: $C_{11}H_3BrCl_2F_6N_2S$. | | | | | |
|---|---|---|---|---|---|
| Calc.:<br>Found: | C, 28.72;<br>C, 29.06; | H, 0.66;<br>H, 0.69; | N, 6.09;<br>N, 6.20; | F, 24.78;<br>F, 24.2; | S, 6.97.<br>S, 7.48. |

**EXAMPLE 9**

Preparation of 1-(6-chloro-2-methylsulfenyl-4-trifluoromethylphenyl)-2-bromo-4-chlorodifluoromethylsulfonylimidazole.

To a solution of 500 mg (0.984 mmole) of 1-(2,6-dichloro-4-trifluoromethylphenyl)-2-bromo-4-chlorodifluoromethylsulfonyl-imidazole in 2 ml of tetrahydrofuran was added a solution of 69 mg (0.984 mmole) of sodium methanethiolate in 0.3 ml of water. The resulting mixture was stirred at RT for 14 hours, after which it was partitioned between water and diethyl ether. The organic layer was separated, dried over anhydrous sodium sulfate and stripped of solvent. The residue was purified by preparative TLC using 20% ethyl acetate in hexane to give 180 mg (35% yield) of the product, mp 116 °C.

**EXAMPLE 10**

Preparation of 1-(2,6-dichloro-4-trifluoromethylphenyl)-2-methyl-4-chlorodifluoromethylsulfenylimidazole.

a) Preparation of intermediate: N-acetyl-2,6-dichloro-4-trifluoromethylaniline.

To 10.6 g (0.26 mole) of dry potassiumin hydride in THF (150 ml) was added 20 g (87.3 mmole) of 2,6-dichloro-4-trifluoromethylaniline at 0 °C under a nitrogen atmosphere. The resulting mixture was stirred and warmed to room temperature for 3.5 h. The mixture was cooled to 0 °C and 6.6 ml (92.8 mmole) of acetyl chloride was added dropwise. The mixture was stirred at 0 °C for 30 min. The mixture was warmed to room temperature under a nitrogen atmosphere overnight. The mixture was quenched with satd. $NH_4Cl$ (150 ml). The mixture was evaporated to remove THF and the suspension was filtered and the solid washed with hexane, followed by a wash with dichloromethane to give 14.5 g( 61%) of the desired product. $^1H$ NMR, ($CDCl_3/CD_3OD$): $\delta$ 2.12(s, 3H), 7.60(s,2H).

b) Preparation of intermediate: 1-chloro-1-methyl- N-(2,6-dichloro-4-trifluoromethylphenyl)formimine.

To a suspension of 4.3 g (15.8 mmole) of N-acetyl-2,6-dichloro-4-trifluoromethylaniline in 50 ml of chloroform was added 3.3 g (15.8 mmole) of phosphorous pentachloride at RT. The mixture was heated to reflux under a nitrogen atmosphere for 1 h. The mixture was evaporated to dryness. To the residue was added 50 ml of benzene. The resulting mixture was heated to reflux for 1 h. under a nitrogen atmosphere. The mixture was evaporated to dryness and the residue purified by a column chromatography on silica gel using 10% ethyl acetate in hexane to yield 4.3 g (93.7% yield) of the desired product as an oil. $^1H$ NMR, ($CDCl_3$): $\delta$ 2.70(s, 3H), 7.58(s, 2H).

c) Preparation of intermediate : 1-(2,6-dichloro-4-trifluoromethylphenyl)-5-amino-2-methylimidazole.

To a solution of 9.6g (33.0 mmole) of 1-chloro-1-methyl-N-(2,6-dichloro-4-trifluoromethylphenyl)-formimine in 300 ml of chloroform was added 3.7g (66.0 mmole) of aminoacetonitrile at RT. The resulting mixture was heated to reflux under a nitrogen atmosphere for 60 h. The reaction mixture was used in the following step without purification. The $^1H$ NMR spectrum indicated approximately 60% conversion based on the starting iminochloride. $^1H$ NMR, ($CDCl_3$) : $\delta$ 2.13(s, 3H), 6.58(s, 1H), 7.76(s, 2H).

d) Preparation of 1-(2,6-dichloro-4-trifluoromethylphenyl)5-amino-2-methyl-4-chlorodifluoromethylsulfenylimidazole.

To the reaction mixture, described above in (c), was added 5.8 ml (57.7 mmole) of chlorodifluoromethanesulfenyl chloride at RT. The mixture was stirred at RT for 3.5 h. The mixture was quenched with water. The mixture was partitioned between water and dichloro methane. The organic layer was dried over anhydrous sodium sulfate and the solvent evaporated to give the desired product. The crude

product was used in the following step without further purification.

e) Preparation of the 1-(2,6-dichloro-4-trifluoromethylphenyl)-2-methyl-4-chlorodifluoromethylsulfenylimidazole.

To the crude product, described above in (d), was added 100 ml of THF, followed by addition of 19.6 ml (165 mmole) of t-butylnitrite. The mixture was stirred at RT under a nitrogen atmosphere with protection from light overnight. The mixture was evaporated to dryness. The residue was purified by a flash column chromatography using 10% ethyl acetate in hexane to give 1.3 g (9.46% yield from the imino chloride, described in (b)) of the desired product, mp 118.5 °C.

**TABLE 2**

**ADDITIONAL SYNTHESIZED IMIDAZOLE COMPOUNDS OF FORMULA (I) AND (II)**

| CMPD. OF EXAMPLE | Substituent | | | | M.P.,°C |
|---|---|---|---|---|---|
| | $R_1$ | n | Y | Z | |
| | **Group 1: $R_2$ and $R_6$ are Cl, and $R_4$ is $CF_3$** | | | | |
| **11** | $CF_3$ | 0 | H | H | 63.5 |
| **12** | $CF_3$ | 0 | Cl | H | 82.5 |
| **13** | $CF_3$ | 0 | $SCH_3$ | H | 85.5 |
| **14** | $CF_3$ | 0 | Cl | Cl | OIL |
| **15** | $CClF_2$ | 0 | $NH_2$ | H | 166.5 |
| **16** | $CClF_2$ | 0 | $N=CHOC_2H_5$ | H | OIL |
| **17** | $CCl_2F$ | 0 | $NH_2$ | H | 177 |
| **18** | $CCl_2F$ | 0 | Br | H | 105.5 |
| **19** | $CCl_2F$ | 0 | $SCH_3$ | H | 99 |
| **20** | $CCl_2F$ | 0 | H | Cl | 120 |
| **21** | $CCl_2F$ | 0 | Cl | Cl | OIL |
| **22** | $CCl_2F$ | 0 | $NH_2$ | Cl | 176 |
| **23** | $CCl_2F$ | 0 | H | Br | 123 |
| **24** | $CCl_2F$ | 0 | $NH_2$ | Br | 133 |
| **25** | $CF_3$ | 1 | H | H | 98 |
| **26** | $CF_3$ | 2 | H | H | 170.5 |
| **27** | $CF_3$ | 2 | Br | H | 152.5 |
| **28** | $CCl_2F$ | 1 | H | Cl | 171 |
| **29** | $CCl_2F$ | 1 | H | Br | 181.5 |
| **30** | $CCl_2F$ | 2 | Br | H | 175.5 |
| **31** | $CCl_2F$ | 2 | H | Cl | 171 |
| **32** | $CCl_2F$ | 2 | H | Br | 180.5 |
| **33** | $CClF_2$ | 1 | H | Br | 155.5 |
| **34** | $CClF_2$ | 2 | H | Br | 160.5 |
| **35** | $CClF_2$ | 0 | H | Br | 104.5 |
| **36** | $CClF_2$ | 0 | H | Cl | 92.5 |
| **37** | $CClF_2$ | 1 | H | Cl | 145.5 |

**TABLE 2**

**ADDITIONAL SYNTHESIZED IMIDAZOLE COMPOUNDS OF FORMULA (I) AND (II)**

| CMPD. OF EXAMPLE | Substituent $R_1$ | n | Y | Z | M.P.,°C |
|---|---|---|---|---|---|
| | **Group 1: $R_2$ and $R_6$ are Cl, and $R_4$ is $CF_3$** | | | | |
| **38** | $CClF_2$ | 2 | H | Cl | 159.5 |
| **39** | $CCl_2F$ | 1 | $SO_2CH_3$ | H | 162.5 |
| **40** | $CCl_2F$ | 1 | $SOCH_3$ | H | OIL |
| **41** | $CClF_2$ | 0 | H | H | 69.5 |
| **42** | $CCl_2F$ | 0 | H | H | 67.5 |
| **43** | $CF_3$ | 0 | H | $SCH_3$ | OIL |
| **44** | $CCl_2F$ | 1 | H | H | 141.5 |
| **45** | $CCl_2F$ | 2 | H | H | 188 |
| **46** | $CCl_2F$ | 0 | H | $SCH_3$ | OIL |
| **47** | $CClF_2$ | 2 | H | H | 164 |
| **48** | $CClF_2$ | 1 | H | H | 109.5 |
| **49** | $CCl_2F$ | 0 | H | $SCH(CH_3)_2$ | OIL |
| **50** | $CCl_2F$ | 0 | H | $SOCH(CH_3)_2$ | OIL |
| **51** | $CCl_2F$ | 1 | H | $SOCH(CH_3)_2$ | 148 |
| **52** | $CCl_2F$ | 1 | H | $SOCH(CH_3)_2$ | 149 |
| **53** | $CH_3$ | 0 | H | $SCH_3$ | 85 |
| **54** | $CH_3$ | 0 | H | H | OIL |
| **55** | $CH_3$ | 1 | H | H | 129.5 |
| **56** | $CH_3$ | 2 | H | H | 220.5 |
| **57** | $CH(CH_3)_2$ | 1 | H | H | 170.5 |
| **58** | $CH(CH_3)_2$ | 2 | H | H | 206.5 |
| **59** | $CCl_2F$ | 0 | H | $CH_3$ | 129 |
| **60** | $CCl_2F$ | 1 | H | $CH_3$ | 154 |
| **61** | $CCl_2F$ | 2 | H | $CH_3$ | 198 |
| **63** | $CCl_2F$ | 0 | H | $CF_3$ | 88.5 |
| **64** | $CCl_2F$ | 1 | H | $CF_3$ | 139.5 |
| **65** | $CF_3$ | 0 | H | $CH_3$ | 127.5 |

**TABLE 2**

**ADDITIONAL SYNTHESIZED IMIDAZOLE COMPOUNDS OF FORMULA (I) AND (II)**

| CMPD. OF EXAMPLE | Substituent | | | | M.P.,°C |
|---|---|---|---|---|---|
| | $R_1$ | n | Y | Z | |
| **Group 1: $R_2$ and $R_6$ are Cl, and $R_4$ is $CF_3$** | | | | | |
| 66 | $CF_3$ | 1 | H | $CH_3$ | 140.5 |
| 67 | $CF_3$ | 2 | H | $CH_3$ | 180.5 |
| 68 | $CClF_2$ | 1 | H | $CH_3$ | 143.5 |
| 69 | $CClF_2$ | 2 | H | $CH_3$ | 172.5 |
| **Group 2a: $R_2$ is $SCH_3$, $R_6$ is Cl, and $R_4$ is $CF_3$** | | | | | |
| 70 | $CCl_2F$ | 2 | H | Cl | OIL |
| 71 | $CCl_2F$ | 0 | H | Cl | OIL |
| 72 | $CCl_2F$ | 1 | H | Cl | 136 |
| 73 | $CClF_2$ | 0 | H | Cl | OIL |
| 74 | $CF_3$ | 0 | H | Cl | OIL |
| **Group 2b: $R_2$ is $SC_2H_5$, $R_6$ is Cl, and $R_4$ is $CF_3$** | | | | | |
| 75 | $CCl_2F$ | 0 | H | Cl | OIL |
| **Group 2c: $R_2$ is $SOCH_3$, $R_6$ is Cl, and $R_4$ is $CF_3$** | | | | | |
| 76 | $CClF_2$ | 2 | H | Cl | OIL |
| *77 | $CF_3$ | 0 | H | Cl | 192.5 |
| *78 | $CF_3$ | 0 | H | Cl | 112.5 |

*isomeric compounds

| CMPD. OF EXAMPLE | $R_1$ | n | Y | Z | M.P.,°C |
|---|---|---|---|---|---|
| **Group 2d: $R_2$ is $SO_2CH_3$, $R_6$ is Cl, and $R_4$ is $CF_3$** | | | | | |
| 79 | $CClF_2$ | 2 | H | Cl | OIL |

**TABLE 2**

**ADDITIONAL SYNTHESIZED IMIDAZOLE COMPOUNDS OF FORMULA (I) AND (II)**

| CMPD. OF EXAMPLE | Substituent $R_1$ | n | Y | Z | M.P.,°C |
|---|---|---|---|---|---|
| | **Group 3: $R_2$ is H, $R_6$ is Cl and $R_4$ is $CF_3$** | | | | |
| **80** | $CCl_2F$ | 0 | H | H | OIL |
| **81** | $CCl_2F$ | 1 | H | H | 109.5 |
| **82** | $CCl_2F$ | 0 | Cl | H | OIL |
| **83** | $CCl_2F$ | 1 | H | Cl | 111 |
| **84** | $CF_3$ | 0 | H | H | OIL |
| **85** | $CF_3$ | 0 | H | Br | 117 |
| **86** | $CF_3$ | 1 | H | H | 87.5 |
| **87** | $CF_3$ | 2 | H | H | 137 |
| **88** | $CCl_2F$ | 0 | Br | Br | 108.5 |
| | **Group 4a: $R_2$, $R_4$, and $R_6$ are Cl** | | | | |
| **89** | $CCl_2F$ | 1 | $NH_2$ | H | 209 |
| **90** | $CCl_2F$ | 0 | H | Cl | 117.5 |
| **91** | $CClF_2$ | 0 | H | H | 47 |
| **92** | $CCl_2F$ | 0 | H | H | OIL |
| **93** | $CClF_2$ | 2 | H | H | 141 |
| **94** | $CCl_2F$ | 2 | H | H | 159.5 |
| **95** | $CCl_2F$ | 1 | H | H | 93.5 |
| **96** | $CClF_2$ | 1 | H | H | 87.5 |
| **97** | $CF_3$ | 0 | H | H | 65.5 |
| **98** | $CF_3$ | 1 | H | H | 101 |
| **99** | $CF_3$ | 2 | H | H | 129.5 |
| **100** | $CF_3$ | 0 | $NH_2$ | H | 144 |
| **101** | $CF_3$ | 0 | H | H | 65.5 |
| **102** | $CF_3$ | 1 | H | H | 101 |
| **103** | $CF_3$ | 2 | H | H | 129.5 |

**TABLE 2**

**ADDITIONAL SYNTHESIZED IMIDAZOLE COMPOUNDS OF FORMULA (I) AND (II)**

| CMPD. OF EXAMPLE | Substituent $R_1$ | n | Y | Z | M.P.,°C |
|---|---|---|---|---|---|
| | **Group 4b: $R_2$ and $R_6$ are Cl and $R_4$ is Br** | | | | |
| **104** | $CCl_2F$ | 0 | H | H | 72 |
| **105** | $CCl_2F$ | 0 | $NH_2$ | H | 202.5 |
| **106** | $CCl_2F$ | 1 | H | H | 129.5 |
| **107** | $CCl_2F$ | 2 | H | H | 175 |
| **108** | $CClF_2$ | 0 | $NH_2$ | H | 154 |
| **109** | $CClF_2$ | 0 | H | H | 47 |
| **110** | $CClF_2$ | 2 | H | H | 156.5 |
| **111** | $CClF_2$ | 1 | H | H | 101 |
| **112** | $CF_3$ | 0 | H | H | 68 |
| **113** | $CF_3$ | 1 | H | H | 115.5 |
| **114** | $CF_3$ | 2 | H | H | 144 |
| **115** | $CF_3$ | 0 | $NH_2$ | H | 161.5 |
| **116** | $CF_3$ | 0 | H | H | 68 |
| **117** | $CF_3$ | 1 | H | H | 115.5 |
| **118** | $CF_3$ | 2 | H | H | 144 |
| | **Group 5a: $R_2$ and $R_6$ are Cl and $R_4$ is $OCF_3$** | | | | |
| **119** | $CCl_2F$ | 0 | $NH_2$ | H | OIL |
| **120** | $CCl_2F$ | 0 | H | H | OIL |
| **121** | $CCl_2F$ | 1 | H | H | 108.5 |
| **122** | $CF_3$ | 0 | $NH_2$ | H | 111 |
| **123** | $CF_3$ | 0 | $NH_2$ | Br | 115 |
| **124** | $CF_3$ | 0 | H | H | OIL |
| **125** | $CF_3$ | 0 | H | Br | 85.5 |
| **126** | $CClF_2$ | 0 | $NH_2$ | H | 112 dec. |
| **127** | $CF_3$ | 2 | H | H | 127.5 |

**TABLE 2**

**ADDITIONAL SYNTHESIZED IMIDAZOLE COMPOUNDS OF FORMULA (I) AND (II)**

| CMPD. OF EXAMPLE | Substituent | | | | |
|---|---|---|---|---|---|
| | $R_1$ | n | Y | Z | M.P.,°C |
| | **Group 5a: $R_2$ and $R_6$ are Cl and $R_4$ is $OCF_3$** | | | | |
| **128** | $CF_3$ | 1 | H | H | 65 |
| **129** | $CF_3$ | 1 | H | Br | 137 |
| **130** | $CClF_2$ | 0 | H | H | OIL |
| **131** | $CClF_2$ | 1 | H | H | 59.5 |
| **132** | $CF_3$ | 2 | H | Br | 138.5 |
| **133** | $CClF_2$ | 0 | $NH_2$ | Br | 157 |
| **134** | $CClF_2$ | 2 | H | H | 130.5 |
| **135** | $CClF_2$ | 0 | H | Br | 112 |
| **136** | $CClF_2$ | 2 | H | Br | 156 |
| **137** | $CClF_2$ | 0 | $N=CHOC_2H_5$ | Br | OIL |
| **138** | $CClF_2$ | 1 | H | Br | 158 |
| **139** | $CCl_2F$ | 0 | $NH_2$ | Cl | 179 |
| **140** | $CCl_2F$ | 0 | H | Cl | 141 |
| **141** | $CClF_2$ | 0 | $NHCH_3$ | Br | 108 |
| **142** | $CCl_2F$ | 1 | H | Cl | 185 |
| **143** | $CCl_2F$ | 2 | H | H | 122 |
| **144** | $CCl_2F$ | 0 | $NH_2$ | Br | 177.5 |
| **145** | $CCl_2F$ | 0 | H | Br | 141.5 |
| **146** | $CCl_2F$ | 1 | H | Br | 181 |
| **147** | $CCl_2F$ | 2 | H | Br | 188 |
| **148** | $CCl_2F$ | 2 | H | Cl | 185.5 |
| **149** | $CH_3$ | 0 | H | H | 60.5 |
| **150** | $CH_3$ | 2 | H | H | 171 |
| **151** | $CH_3$ | 1 | H | H | 131 |

**TABLE 2**

**ADDITIONAL SYNTHESIZED IMIDAZOLE COMPOUNDS OF FORMULA (I) AND (II)**

| CMPD. OF EXAMPLE | Substituent $R_1$ | n | Y | Z | M.P.,°C |
|---|---|---|---|---|---|
| | **Group 5b: $R_2$ and $R_6$ are Br and $R_4$ is $OCF_3$** | | | | |
| **152** | $CCl_2F$ | 0 | $NH_2$ | H | 141 |
| **153** | $CCl_2F$ | 0 | H | H | OIL |
| **154** | $CCl_2F$ | 1 | H | H | 115 |
| **155** | $CCl_2F$ | 2 | H | H | 124.5 |
| **156** | $CClF_2$ | O | $NH_2$ | H | 135 |
| **157** | $CClF_2$ | 0 | H | H | 51 |
| **158** | $CClF_2$ | 2 | H | H | 146.5 |
| **159** | $CClF_2$ | 1 | H | H | 103.5 |
| **160** | $CClF_2$ | 1 | $NH_2$ | H | 135 dec. |
| | **Group 5c: $R_2$ is Br, $R_6$ is Cl, and $R_4$ is $OCF_3$** | | | | |
| **161** | $CCl_2F$ | 0 | $NH_2$ | H | 150 |
| **162** | $CCl_2F$ | 0 | H | H | 68.5 |
| **163** | $CCl_2F$ | 1 | H | H | 87 |
| **164** | $CCl_2F$ | 2 | H | H | 142.5 |

**EXAMPLE 165**

MITICIDE, INSECTICIDE, NEMATICIDE USE

The following test procedures, using the compounds of **EXAMPLES 1-164**, were conducted to determine the pesticidal use and activity of compounds of the invention against: mites; certain insects, including an aphid, a caterpillar, a fly, and two species of beetle larvae (one foliar feeding and the other root feeding); and nematodes. The specific species tested were as follows:

| GENUS, SPECIES | COMMON NAME | (ABBREVIATION) |
|---|---|---|
| Tetranychus urticae | two-spotted spider mite | TSM |
| Aphis nasturtii | buckthorn aphid | BA |
| Spodoptera eridania | southern armyworm | SAW |
| Epilachna varivestis | Mexican bean beetle | MBB |
| Musca domestica | housefly | HF |
| Diabrotica u. howardi | southern corn rootworm | SCRW |
| Meloidogyne incognita | southern root-knot nematode | SRKN |

Formulations:

The test compounds (**EXAMPLES 1-164**) were formulated for use according to the following methods used for each of the test procedures.

For mite, aphid, southern armyworm, and Mexican bean beetle tests, a solution or suspension was prepared by adding 10 mg of the test compound to a solution of 160 mg of dimethylformamide, 838 mg of acetone, 2 mg of a 3:1 ratio of Triton X-172 : Triton X-152 (respectively, mainly anionic and nonionic low foam emulsifiers which are each anhydrous blends of alkylaryl polyether alcohols with organic sulfonates), and 98.99 g of water. The result was a concentration of 100 ppm of the test compound.

For housefly tests, the formulation was initially prepared in a similar manner to the above, but in 16.3 g of water with corresponding adjustment of other components, providing a 200 ppm concentration. Final dilution with an equal volume of a 20% by weight aqueous solution of sucrose provided a 100 ppm concentration of the test compound. When necessary, sonication was provided to insure complete dispersion.

For southern corn rootworm tests, a solution or suspension was prepared in the same manner as that used for the initial 200 ppm concentration for housefly. Aliquots of this 200 ppm formulation were then used by dilution with water according to the required test concentration.

For southern root-knot nematode and southern armyworm systemic tests, a stock solution or suspension was prepared by adding 15 mg of the test compound to 250 mg of dimethylformamide, 1250 mg of acetone and 3 mg of the emulsifier blend referenced above. Water was then added to bring the total volume to 45 ml and a test compound concentration of 333 ppm. When necessary, sonication was provided to insure complete dispersion.

Test Procedures:

The above formulated test compounds were then evaluated for their pesticidal activity at the specified concentrations, in ppm (parts per million) by weight, according to the following test procedures:

Two-spotted spider mite: Leaves infested with adult and nymphal stages of the two-spotted spider mite, obtained from a stock culture were placed on the primary leaves of two bean plants growing in a 6 cm. peat pot. A sufficient number of mites (150-200) for testing were transferred to the fresh plants within a period of twenty-four hours. The potted plants (one pot per compound) were placed on a revolving turntable and sprayed, sufficient to wet the plants to runoff, with 100 ml of the 100 ppm test compound formulation by use of a DeVilbiss spray gun set at 40 psig. air pressure. As an untreated control, 100 ml of the water-acetone-DMF-emulsifier solution, containing no test compound, were also sprayed on infested plants. A treated control with a commercial technical compound, either dicofol or hexythiazox, formulated in the same manner, was tested as a standard. The sprayed plants were held for six days, after which a mortality count of motile forms was made.

Two-spotted spider mite (ovicide test): Eggs were obtained from adults of the two-spotted spider mite from a stock culture. Heavily infested leaves from the stock culture were placed on uninfested bean plants. Females were allowed to oviposit for a period of about 24 hours, after which the leaves of the plant were dipped into a solution of TEPP (tetraethyl diphosphate) in order to kill the motile forms and prevent additional egg laying. This dipping procedure, which was repeated after the plants dried, did not affect the viability of the eggs. The potted plants (one pot per compound) were placed on a revolving turntable and sprayed, sufficient to wet the plants to runoff, with 100 ml of the 100 ppm test compound formulation by use of a DeVilbiss spray gun set at 40 psig. air pressure. As an untreated control, 100 ml of the water-acetone-DMF-emulsifier solution, containing no test compound, were also sprayed on infested plants. A treated control with a commercial technical compound, typically demeton, formulated in the same manner, was tested as a standard. The sprayed plants were held for seven days, after which a mortality count of egg forms was made along with notations on residual activity on hatched larvae.

Buckthorn aphid: Adult and nymphal stages of buckthorn aphid were reared on potted dwarf nasturtium plants. The potted plants (one pot per compound tested) infested with 100-150 aphids, were placed on a revolving turntable and sprayed with 100 ml of the 100 ppm test compound formulation by use of a DeVilbiss spray gun set at 40 psig air pressure. As an untreated control, 100 ml of a water-acetone-DMF-emulsifier solution, containing no test compound, were also sprayed on infested plants. A treated control with a commercial technical compound, malathion, formulated in the same manner, was tested as a standard. After spraying, the pots were stored for one day after which the dead aphids were counted.

Southern armyworm: Potted bean plants, were placed on a revolving turntable and sprayed with 100 ml of the 100 ppm test compound formulation by use of a DeVilbiss spray gun set at 40 psig air pressure. As

an untreated control, 100ml of a water-acetone-DMF-emulsifier solution, containing no test compound, were also sprayed on plants. A treated control with a commercial technical compound, either cypermethrin or sulprofos, formulated in the same manner, was tested as a standard. When dry, the leaves were placed in plastic cups lined with moistened filter paper. Five randomly selected second instar Southern armyworm larvae were introduced into each dish which was closed and held for five days. Larvae which were unable to move the length of the body, even upon stimulation by prodding, were considered dead.

Southern armyworm - systemic evaluation: This test was conducted in conjunction with the southern root-knot nematode evaluation (discussed below). The tomato plants, grown in the soil (at an initial compound test screening rate of 13.2 ppm soil concentration) for nematode evaluation, were then utilized for evaluation of a compound's uptake via roots and subsequent systemic transport to the tomato foliage. At the termination of the nematode test, the tomato foliage was excised, placed into a plastic container, and infested with second instar larvae of southern armyworm. After about 5 days, the larvae were examined for percent mortality.

Mexican bean beetle: Potted bean plants were placed on a revolving turntable and sprayed with 100 ml of the 100 ppm test compound formulation, sufficient to wet the plants to runoff, by use of a DeVilbiss spray gun set at 40 psig air pressure. As an untreated control, 100 ml of a water-acetone-DMF-emulsifier solution, containing no test compound, were also sprayed on plants. A treated control with a commercial technical compound, either cypermethrin or sulprofos, formulated in the same manner, was tested as a standard. When dry, the leaves were placed in plastic cups lined with moistened filter paper. Five randomly selected second instar Mexican bean beetle larvae were introduced into each dish which was closed and held for five days. Larvae which were unable to move the length of the body, even upon stimulation by prodding, were considered dead.

House fly: Four to six day old adult house flies were reared according to the specifications of the Chemical Specialties Manufacturing Association (Blue Book, McNair-Dorland Co., N.Y. 1954; pages 243-244, 261) under controlled conditions. The flies were immobilized by anesthetizing with carbon dioxide and twenty five immobilized individuals, males and females, were transferred to a cage consisting of a standard food strainer and a wrapping-paper-covered surface. Ten ml of the 100 ppm test compound formulation were added to a soufflé cup containing an absorbent cotton pad. As an untreated control, 10 ml of a water-acetone-DMF-emulsifier-sucrose solution, containing no test compound, were applied in a similar manner. A treated control with a commercial technical compound, malathion, formulated in the same manner, was tested as a standard. The bait cup was introduced inside the food strainer prior to admitting the anesthetized flies. After 24 hours, flies which showed no sign of movement on stimulation were considered dead.

Southern corn rootworm: Into a jar containing 60g of sandy loam soil was added 1.5 ml of an aqueous formulation consisting of an aliquot of the 200 ppm test compound formulation diluted with water as appropriate for the final soil concentration of the test compound, 3.2 ml of water and five pregerminated corn seedlings. The jar was shaken thoroughly to obtain an even distribution of the test formulation. Following this, twenty southern corn rootworm eggs were placed into a cavity, which was made in the soil. Vermiculite(1 ml) and water (1.7ml) were then added to this cavity. In a similar manner, an untreated control was prepared by application of the same size aliquot of a water-acetone-DMF-emulsifier solution, containing no test compound. Additionally, a treated control with a commercial technical compound (selected typically from terbufos, fonofos, phorate, chlorpyrifos, carbofuran, isazophos, or ethoprop), formulated in the same manner was used as a test standard. Alter 7 days, the living rootworm larvae were counted using a well known "Berlese" funnel extraction method.

Southern root-knot nematode: Infected roots of tomato plants, containing egg masses of southern root-knot nematode, were removed from a stock culture and cleaned of soil by shaking and washing with tap water. The nematode eggs were separated from the root tissue and rinsed with water. Samples of the egg suspension were placed on a fine screen over a receiving bowl, in which the water level was adjusted to be in contact with the screen. From the bowl, juveniles were collected on a fine screen. The bottom of a cone-shaped container was plugged with coarse vermiculite and then filled to within 1.5 cm of the top with about a 200 ml volume of pasturized soil. Then into a hole made in the center of the soil in the cone was pipetted an aliquot of the 333 ppm test compound formulation. A treated control with a commerical technical compound, fenamifos, formulated in a similar manner, was tested as a standard. As an untreated control, an aliquot of a water-acetone-DMF-emulsifiersolution, containing no test compound, was applied in a similar manner. Immediately after treatment of the soil with the test compound there were added to the top of each cone 1000 second stage juvenile southern root-knot nematodes. After 3 days, a single healthy tomato seedling was then transplanted into the cone. The cone, containing the infested soil and tomato seedling, was kept in the greenhouse for 3 weeks. At the termination of the test, roots of the tomato seedling were

removed from the cone and evaluated for galling on a rating scale relative to the untreated control as follows:

1- severe galling, equal to untreated control
3- light galling
4- very light galling
5- no galling, ie, complete control

These results were then converted to an $ED_3$ or $ED_5$ value (effective dose to provide a 3 or 5 gall rating).

Use Results: Results of miticidal, insecticidal, and nematicidal activity for some of the representative compound **EXAMPLES 1-164** of the invention are discussed below or some compound **EXAMPLES** are set forth in **TABLE 3** against the indicated test species (BA, SAW, MBB, HF, TSM, SCRW: designated by common name abbreviations) and at the indicated dosage rates. The results in **TABLE 3** are presented (by an X) as compounds which provide a 70-100% mortality against the indicated test species. The compounds of the invention also provide some other control of mites (TSM) where, for example, compounds of **EXAMPLES 9, 18, 19, 30, 70, 71** and **92**, all at 100 ppm except compound of **EXAMPLE 30** at 25 ppm, gave 50-100% residual toxicity (mortality) to hatched larvae in the mite ovicide test. Nematicidal activity is additionally provided by compounds of the invention where, for example, compounds of **EXAMPLES 25, 86, 130**, and **131**, gave ED3 values on SRKN of between about 7 to 21 kg/ha. Furthermore, compounds of the invention exhibit reduced or antifeeding properties for some pest species, for example for foliar pests such as Southern armyworm and Mexican bean beetle. Some of the compounds additionally exhibit systemic control of Southern armyworm via root uptake. These compounds are **EXAMPLES 4, 25, 40, 44, 48, 72, 81, 86, 87, 106, 121, 128, 131**, and **143**. The compounds of the invention have utility against various pest species at even lower rates, for example: for foliar application, rates in the range of about 50-0.5ppm, or less, may be useful; for bait application, rates in the range of about 50-0.05ppm, or less, may be useful; and for soil application, rates in the range of about 1.0-0.01ppm, or less, may be useful.

In the above discussion and the results reported in **TABLE 3**, compounds according to the invention are applied at various concentrations. The use of a 1 ppm (concentration of the compound in parts per million of the test solution applied) foliar solution or suspension or emulsion corresponds approximately to an application of 1 g/ha of active ingredient, based upon an approximate spray volume of 1000 liters/ha (sufficient to run off). Thus applications of foliar sprays of from about 6.25 to 500 ppm would correspond to about 6-500 g/ha. For soil applications, a 1 ppm soil concentration, on the basis of about a 7.5 cm soil depth, corresponds to an approximate 1000 g/ha broadcast field application.

**TABLE 3**

**USE EXAMPLE OF PESTICIDAL ACTIVITY OF REPRESENTATIVE IMIDAZOLE COMPOUNDS PROVIDING 70-100% PEST MORTALITY**

| CMPD. OF | Foliar or Bait Application at 100 ppm | | | | | Soil conc.-1.45 ppm |
|---|---|---|---|---|---|---|
| EXAMPLE | BA | SAW | MBB | HF | TSM | SCRW |
| 1 | X | | | X | | |
| 2 | | | X | | | X |
| 3 | | X | | X | | X |
| 4 | X | X | | X | | X |
| 5 | | X | | X | | X |
| 6 | | | | X | | X |
| 7 | | | | | | X |
| 8 | | | | X | | X |
| 9 | X | X | X | X | X | X |
| 10 | X | X | | X | | |
| 11 | X | | | X | | |
| 12 | | | | X | | X |
| 13 | | X | | X | | X |
| 14 | | | | X | | X |
| 15 | | | | | | |
| 16 | X | | | | | |
| 17 | | | | | | |

**TABLE 3**

**USE EXAMPLE OF PESTICIDAL ACTIVITY OF REPRESENTATIVE IMIDAZOLE COMPOUNDS PROVIDING 70-100% PEST MORTALITY**

| CMPD. OF | Foliar or Bait Application at 100 ppm | | | | | Soil conc.-1.45 ppm |
|---|---|---|---|---|---|---|
| EXAMPLE | BA | SAW | MBB | HF | TSM | SCRW |
| 18 | | X | | X | X | X |
| 19 | X | X | | X | | X |
| 20 | X | X | | X | | |
| 21 | X | | | X | | X |
| 22 | | X | X | | | |
| 23 | X | X | X | X | | X |
| 24 | | | X | | | X |
| 25 | X | X | | X | | X |
| 26 | | | | X | | X |
| 27 | | X | | X | | X |
| 28 | X | X | X | X | | X |
| 29 | X | X | X | X | | X |
| 30 | X | | | X | X | X |
| 31 | | X | X | X | | X |
| 32 | X | X | X | X | | X |
| 33 | X | X | X | X | | X |
| 34 | | X | X | X | | X |

**TABLE 3**

**USE EXAMPLE OF PESTICIDAL ACTIVITY OF REPRESENTATIVE IMIDAZOLE COMPOUNDS PROVIDING 70-100% PEST MORTALITY**

| CMPD. OF EXAMPLE | Foliar or Bait Application at 100 ppm | | | | | Soil conc.-1.45 ppm |
|---|---|---|---|---|---|---|
| | BA | SAW | MBB | HF | TSM | SCRW |
| 35 | X | X | | X | | X |
| 36 | | X | | X | | X |
| 37 | | X | X | X | | X |
| 38 | | X | X | X | | X |
| 39 | | X | X | X | | X |
| 40 | | X | | X | X | X |
| 41 | X | X | | X | | X |
| 42 | X | X | | X | | X |
| 44 | X | X | | X | | |
| 45 | X | X | X | X | | X |
| 47 | | X | X | X | | |
| 48 | X | X | | X | | |
| 59 | X | X | | X | X | |
| 60 | X | X | X | X | X | |
| 61 | X | X | X | X | | |
| 65 | X | X | | X | | |
| 66 | X | X | X | X | | |

## TABLE 3

**USE EXAMPLE OF PESTICIDAL ACTIVITY OF REPRESENTATIVE IMIDAZOLE COMPOUNDS PROVIDING 70-100% PEST MORTALITY**

| CMPD. OF | Foliar or Bait Application at 100 ppm | | | | | Soil conc.-1.45 ppm |
|---|---|---|---|---|---|---|
| EXAMPLE | BA | SAW | MBB | HF | TSM | SCRW |
| 67 | X | X | | X | | |
| 68 | X | X | X | X | | |
| 69 | X | X | X | X | | |
| 70 | | X | X | X | X | X |
| 71 | | X | | X | | X |
| 72 | X | | X | X | | X |
| 73 | | X | | X | | X |
| 78 | X | X | X | X | | |
| 81 | X | X | X | X | | |
| 82 | | | | X | X | |
| 86 | | | X | | | |
| 87 | | X | X | | | |
| 88 | | | | X | X | |
| 90 | X | X | | X | X | |
| 91 | | | | X | X | |
| 92 | | | X | | X | |
| 95 | | | X | X | X | |

**TABLE 3**

**USE EXAMPLE OF PESTICIDAL ACTIVITY OF REPRESENTATIVE**

**IMIDAZOLE COMPOUNDS PROVIDING 70-100% PEST MORTALITY**

| CMPD. OF EXAMPLE | Foliar or Bait Application at 100 ppm | | | | | Soil conc.-1.45 ppm |
|---|---|---|---|---|---|---|
| | BA | SAW | MBB | HF | TSM | SCRW |
| 96 | | | | X | X | |
| 101 | X | | | X | X | |
| 102 | | | | X | X | |
| 104 | | X | | X | X | |
| 106 | | | | X | X | |
| 109 | | X | | X | X | |
| 111 | | | | X | X | |
| 121 | X | X | X | X | | |
| 130 | X | | | X | | |
| 131 | X | X | | X | | |
| 135 | X | X | | X | | |
| 143 | X | X | X | X | | |
| 145 | X | X | | X | | |
| 146 | X | X | X | X | | |
| 147 | | X | | X | | |

## METHODS AND COMPOSITIONS

As is evident from the foregoing pesticidal uses, the present invention provides pesticidally active compounds and methods of use of said compounds for the control of a number of pest species which

includes: arthropods, especially insects or mites; plant nematodes; or helminth or protozoan pests. The compounds thus are advantageously employed in practical uses, for example, in agricultural or horticultural crops, forestry, veterinary medicine or livestock husbandry, or in public health.

A feature of the present invention therefore provides a method of control of pests at a locus which comprises the treatment of the locus (e.g., by application or administration) with an effective amount of a compound of general formula (I) and more preferably a compound of formula (II), wherein the substituent groups are as hereinbefore defined. The locus includes, for example, the pest itself or the place (plant, animal, person, field, structure, premises, forest, orchard, waterway, soil, plant or animal product, or the like) where the pest resides or feeds.

The compounds of this invention are preferably used to control soil insects, such as corn rootworm, termites (especially for protection of structures), root maggots, wireworms, root weevils, stalkborers, cutworms, root aphids, or grubs. They may also be used to provide activity against plant pathogenic nematodes, such as root-knot, cyst, dagger, lesion, or stem or bulb nematodes, or against mites. For the control of soil pests, for example corn rootworm, the compounds are advantageously applied to or incorporated at an effective rate into the soil in which crops are planted or to be planted or to the seeds or growing plant roots.

Furthermore, these compounds may be useful in the control via foliar application or systemic action of some arthropods, especially some insects or mites, which feed on the above ground portions of plants. Control of foliar pests may additionally be provided by application to the plant roots or plant seeds with subsequent systemic translocation to the above ground portions of the plants.

In the area of public health, the compounds are especially useful in the control of many insects, especially filth flies or other Dipteran pests, such as houseflies, stableflies, soldierflies, hornflies, deerflies, horseflies, midges, punkies, blackflies, or mosquitoes.

Compounds of the invention may be used in the following applications and on the following pests including arthropods, especially insects or mites, nematodes, or helminth or protozoan pests:

In the protection of stored products, for example cereals, including grain or flour, groundnuts, animal feedstuffs, timber or household goods, e.g. carpets and textiles, compounds of the invention are useful against attack by arthropods, more especially beetles, including weevils, moths or mites, for example Ephestia spp. (flour moths), Anthrenus spp. (carpet beetles), Tribolium spp. (flour beetles), Sitophilus spp. (grain weevils) or Acarus spp. (mites).

In the control of cockroaches, ants or termites or similar arthropod pests in infested domestic or industrial premises or in the control of mosquito larvae in waterways, wells, reservoirs or other running or standing water.

For the treatment of foundations, structures or soil in the prevention of the attack on building by termites, for example, Reticulitermes spp., Heterotermes spp., Coptotermes spp..

In agriculture against adults, larvae and eggs of Lepidoptera (butterflies and moths), e.g. Heliothis spp. such as Heliothis virescens (tobacco budworm), Heliothis armigera and Heliothis zea, Spodoptera spp. such as S. exempta, S. frugiperda, S. exiqua, S. littoralis (Egyptian cotton worm), S. eridania (southern army worm), and Mamestra configurata (bertha army worm); Earias spp. e.g. E. insulana (Egyptian bollworm), Pectinophora spp. e.g. Pectinophora gossypiella (pink bollworm), Ostrinia spp. such as O. nubilalis - (European cornborer), Trichoplusia ni (cabbage looper), Artogeia spp. (cabbage worms), Laphygma spp. (army worms), Agrotis and Amathes spp. (cutworms), Wiseana spp. (porina moth), Chilo spp. (rice stem borer), Tryporyza spp. and Diatraea spp. (sugar cane borers and rice borers), Sparganothis pilleriana (grape berry moth), Cydia pomonella (codling moth), Archips spp. (fruit tree tortrix moth), Plutella xylostella - (diamond back moth), Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Euxoa spp., Feltia brassicae, Panolis flammea, Prodenia litura, Carpocapsa pomonella, Pyrausta nubilalis, Ephestia kuehniella, Galleria mellonella, Tineola bisselliella, Tinea pellionella, Hofmannophila pseudospretella, Cacoecia podana, Capus reticulana, Choristoneura fumiferana, Clysia ambiguellis, Homona magnanime and Tortix viridana.

Against adults and larvae of Coleoptera (beetles) e.g. Hypothenemus hampei (coffee berry borer), Hylesinus spp. (bark beetles), Anthonomus spp. e.g. grandis (cotton boll weevil), Acalymma spp. (cucumber beetles), Lema spp., Psylliodes spp., Leptinotarsa decemlineata (Colorado potato beetle), Diabrotica spp. (corn rootworms), Gonocephalum spp. (false wire worms), Agriotes spp., Limonius spp. (wireworms), Dermolepida spp., Popillia spp., Heteronychus spp. (white grubs), Phaedon cochleariae (mustard beetle), Epitrix spp. (flea beetles), Lissorhoptrus oryzophilus (rice water weevil), Meligethes spp. (pollen beetles), Ceutorhynchus spp., Rhynchophorus and Cosmopolites spp. (root weevils), Anobium punctatum, Rhizopertha dominica, Bruchidius obtectus, Acanthoscelides obtectus, Hylotrupes bajulus, Agelastica alni, Psylliodes

chrysocephala, Epilachna varivestis, Atomaria spp., Oryzaephilus surinamensis, Sitophilus spp., Otiorrhynchus sulcatus, Cosmoplites sordidus, Ceuthorrhynchus assimilis, Hypera postica, Dermestes spp., Trogoderma spp., Anthrenus spp., Attagenus spp., Lyctus spp., Maligethes aeneus, Ptinus spp., Niptus hololeucrus, Gibbium psylloides, Tribolium spp., Tenebrio molitor, Conoderus spp., Melolontha melolontha, Amphimallon solstitialis and Costelytra zealandica.

Against Heteroptera (Hemiptera and Homoptera) e.g. Psylla spp., Bemisia spp., Trialeurodes spp., Aphis spp., Myzus spp., Megoura viciae, Phylloxera spp., Adelges spp., Phorodon humuli (hop damson aphid), Aeneolamia spp., Nephotettix spp. (rice leaf hoppers), Empoasca spp., Nilaparvata spp., Perkinsiella spp., Pyrilla spp., Aonidiella spp. (red scales), Coccus spp., Pseucoccus spp., Helopeltis spp. (mosquito bugs), Lygus spp., Dysdercus spp., Oxycarenus spp., Nezara spp., Eurygaster spp., Piesma quadrata, Cimex lectularius, Rhodnius prolixus and Triatoma spp. Aspidiotus hederae, Aeurodes brassicae, Brevicoryne brassicae, Cryptomyzus ribis, Doralis fabae, Doralis pomi., Eriosoma lanigerum, Hyalopterus arundinis, Macrosiphum avenae, Phorodon humuli, Rhopalosiphum padi, Euscelis bilobatus, Nephotettix cincticeps, Lecanium corni, Saissetia oleae, Laodelphax striatellus.

Against Hymenoptera e.g. Athalia spp. and Cephus spp. (saw flies), Atta spp. (leaf cutting ants), Diprion spp., Hopolocampa spp., Lasius spp., Monomorium spp., Polistes spp., Vespa spp., Vespula spp., and Solenopsis spp..

Against Diptera e.g. Delia spp. (root maggots), Atherigona spp. and Chlorops spp., Sarcophaga spp., Musca spp, Phormia spp., Aedes spp., Anopheles spp., Simulium spp., (shoot flies), Phytomyza spp. (leaf miners), Ceratitis spp. (fruit flies), Culex spp., Drosophila melanogaster, Ceratitis capitata, Dacus oleae, Tipula paludosa, Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Fannia spp., Bibio hortulanus, Oscinella frit, Phorbia spp., Pegomyia hyoscyani.

Against Thysanoptera such as Thrips tabaci, Hercinothrips femoralis, and Frankliniella spp..

Against Orthoptera such as Locusta and Schistocerca spp., (locusts and crickets) e.g. Gryllus spp., and Acheta spp. for example, Blatta orientalis, Periplaneta americana, Leucophaea maderae, Blatella germanica, Acheta domesticus, Gryllotalpa spp., Locusta migratoria migratorioides, Melanoplus differentialis and Schistocerca gregaria.

Against Collembola e.g. Sminthurus spp. and Onychiurus spp. (springtails); Periplaneta spp. and Blattela spp. (roaches).

Against Isoptera e.g. Odontotermes spp., Reticuletermes spp., Coptotermes spp. (termites).

Against Dermaptera e.g. Forticula sp. (earwigs).

Against arthropods of agricultural significance such as Acari (mites) e.g. Tetranychus spp., Panonychus spp., Bryobia spp. (spider mites), Ornithonyssus spp. (fowl mites), Eriophyes spp. (gall mites), and Polyphadotarsonemus supp..

Against Thysanura, for example Lepisma saccharia.

Against Anoplura for example, Phylloxera vastatrix, Pemphigus spp., Pediculus humanus corporis, Haematopinus spp. and Linognathus spp..

Against Mallophaga, for example, Trichodectes spp. and Damalinea spp..

Against Siphonoptera, for example, Xenopsylla cheopis and Ceratophyllus spp..

Against other arthopods, such as Blaniulus spp. (millipedes), Scutigerella spp. (symphilids), Oniscus spp. (woodlice) and Triops spp. (crustacea).

Against Isopoda, for example, Oniseus asellus, Armadillidium vulgare and Porcellio scaber.

Against Chilopoda, for example, Geophilus carpophagus and Scutigera spex..

Against nematodes which attack plants or trees of importance to agriculture, forestry or horticulture either directly or by spreading bacterial, viral, mycoplasma or fungal diseases of the plants. For example root-knot nematodes such as Meloidogyne spp. (e.g. M. incognita): cyst nematodes such as Globodera spp. (e.g. G. rostochiensis); Heterodera spp. (e.g. H. avenae); Radopholus spp. (e.g. R. similis; lesion nematodes such as Pratylenchus spp. (e.g. P. pratensis); Belonolaimus spp. (eg. B. gracilis); Tylenchulus spp. (e.g. T. semipenetrans); Rotylenchulus spp. (e.g. R. reniformis); Rotylenchus spp. (R. robustus); Helicotylenchus spp. (e.g. H. multicinctus); Hemicycliophora spp. (e.g. H. gracilis); Criconemoides spp. (e.g. C. similis); Trichodorus spp. (e.g. T. primitivus); dagger nematodes such as Xiphinema spp. (e.g. X. diversicaudatum), Longidorus spp. (e.g. L. elongatus); Hoplolaimus spp. (e.g. H. coronatus); Aphelenchoides spp. (e.g. A. ritzema-bosi, A. besseyi); stem and bulb eelworm such as Ditylenchus spp. (e.g. D. dipsaci).

In the field of veterinary medicine or livestock husbandry or in the maintenance of public health against arthropods, helminths or protozoa which are parasitic internally or externally upon vertebrates, particularly warm-blooded vertebrates, for example man or domestic animals, e.g. cattle, sheep, goats, equines, swine, poultry, dogs and cats, for example Acarina, including ticks (e.g. Ixodes spp., Boophilus spp. e.g. Boophilus

microplus, Amblyomma spp., Hyalomma spp., Rhipicephalus spp. e.g. Rhipicephalus appendiculatus, Haemaphysalis spp., Dermacentor spp., Ornithodorus spp. (e.g. Ornithodorus moubata) and mites (e.g. Damalinia spp., Dermahyssus gallinae, Sarcoptes spp. e.g. Sarcoptes scabiei, Psoroptes spp., Chorioptes spp;, Demodex spp., Eutrombicula spp.,); Diptera (e.g. Aedes spp., Anopheles spp., Musca spp., Hypoderma spp., Gasterophilus spp., Simulium spp); Hemiptera (e.g. Triatoma spp); Phthirapter (e.g. Damalinia spp., Linognathus spp.); Siphonaptera (e.g. Ctenocephalides spp.); Dictyoptera (e.g. Periplaneta spp., Blatella spp.); Hymenoptera (e.g. Monomorium pharaonis); for example against infections of the gastro-intestinal tract caused by parasitic nematode worms, for example members of the family Trichostrongylidae, Nippostrongylus brasiliensis, Trichinella spiralis, Haemonchus contortus, Trichostrongylus colubriformis, Nematodirus batus, Ostertagis circumcincta, Trichostrongylus axei, Cooperia spp. and Hymenolepis nana; in the control and treatment of protozoal diseases caused by, for example, Eimeria spp. e.g. Eimeria tenella, Eimeria acervulina, Eimeria brunetti, Eimeria maxima and Eimeria necatrix, Trypanosoms cruzi, Leishaminia spp., Plasmodium spp., Babesis spp., Trichomonadidae spp., Histomanas spp., Giardia spp., Toxoplasma spp., Entamoeba histolytica and Theileria spp..

The invention, as previously described, provides methods of control of pests via application or administration of an effective amount of compounds of formula (I) or (II) at a locus which comprises treatment of the locus.

In practical use for the control of arthropods, especially insects or mites, or nematode pests of plants, a method, for example, comprises applying to the plants or to the medium in which they grow an effective amount of a compound of the invention. For such a method, the active compound is generally applied to the locus in which the arthropod or nematode infestation is to be controlled at an effective rate in the range of about 0.005 kg to about 15 kg of the active compound per hectare of locus treated. Under ideal conditions, depending on the pest to be controlled, a lower rate may offer adequate protection. On the other hand, adverse weather conditions, resistance of the pest or other factors may require that the active ingredient be used at higher rates. The optimum rate depends usually upon a number of factors, for example, the type of pest being controlled, the type or the growth stage of the infested plant, the row spacing or also the method of application. More preferably an effective rate range of the active compound is from about 0.01 kg/ha to to about 2 kg/ha.

When a pest is soil-borne, the active compound generally in a formulated composition, is distributed evenly over the area to be treated (ie. for example broadcast or band treatment) in any convenient manner. Application may be made, if desired, to the field or crop-growing area generally or in close proximity to the seed or plant to be protected from attack. The active component can be washed into the soil by spraying with water over the area or can be left to the natural action of rainfall. During or after application, the formulated compound can, if desired, be distributed mechanically in the soil, for example by ploughing, disking, or use of drag chains. Application can be prior to planting, at planting, after planting but before sprouting has taken place, or after sprouting. Additionally, a method of control may also comprise treatment of the seed prior to planting with subsequent control effected after planting the seed.

Methods of control of pests also consist of application to or treatment of the foliage of plants to control arthropods, especially insects or mites, or nematodes attacking the aerial parts of the plants. In addition, methods of control of pests by the invention compounds are provided to control pests which feed on parts of the plant remote from the point of application, e.g.. leaf feeding insects which are controlled via systemic action of the active compound when applied for example to the roots of a plant or to the plant seed prior to planting. Furthermore, the compounds of the invention may reduce attacks on a plant by means of antifeeding or repellent effects.

The compounds of the invention and methods of control of pests therewith are of particular value in the protection of field, forage, plantation, glasshouse, orchard or vineyard crops, of ornamentals, or of plantation or forest trees, for example; cereals (such as maize, wheat, rice, or sorghum), cotton, tobacco, vegetables (such as beans, cole crops, curcurbits, lettuce, onions, tomatoes or peppers), held crops (such as potatoes, sugar beets, ground nuts, soybeans, or oil seed rape), sugar cane, grassland or forage crops (such as maize, sorghum, or lucerne), plantations (such as tea, coffee, cocoa, banana, palm oil, coconut, rubber, or spices), orchards or groves (such as of stone or pit fruit, citrus, kiwifruit, avocado, mango, olives or walnuts), vineyards, ornamental plants, flowers or vegetables or shrubs under glass or in gardens or parks, or forest trees (both deciduous and evergreen) in forests, plantations or nurseries.

They are also valuable in the protection of timber (standing, felled, converted, stored or structural) from attack, for example, by sawflies or beetles or termites.

They have applications in the protection of stored products such as grains, fruits, nuts, spices or tobacco, whether whole, milled or compounded into products, from moth, beetle, mite or grain weevil attack. Also protected are stored animal products such as skins, hair, wool or feathers in natural or converted form

(e.g. as carpets or textiles) from moth or beetle attack as well as stored meat, fish or grains from beetle, mite or fly attack.

Additionally, the compounds of the invention and methods of use thereof are of particular value in the control of arthropods, helminths or protozoa which are injurious to, or spread or act as vectors of diseases in man and domestic animals, for example those hereinbefore mentioned, and more especially in the control of ticks, mites, lice, fleas, midges, or biting, nuisance or myiasis flies. The compounds of the invention are particularly useful in controlling arthropods, helminths or protozoa which are present inside domestic host animals or which feed in or on the skin or suck the blood of the animal, for which purpose they may be administered orally, parenterally, percutaneously or topically.

Furthermore, compounds of the invention may be useful for coccidiosis, a disease caused by infections from protozoan parasites of the genus Eimeria. It is an important potential cause of economic loss in domestic animals and birds, particularly those raised or kept under intensive conditions. For example, cattle, sheep, pigs or rabbits may be affected, but the disease is especially important in poultry, particularly in chickens. Administration of a small amount of a compound of the invention, preferably by a combination with feed is effective in preventing or greatly reducing the incidence of coccidiosis. The compounds are effective against both the cecal form and the intestinal forms. Furthermore, the compounds of the invention may also exert an inhibiting effect on oocysts by greatly reducing the number and sporulation of those produced. The poultry disease is generally spread by the birds picking up the infectious organism in droppings in or on contaminated litter, ground, food, or drinking water. The disease is manifested by hemorrhage, accumulation of blood in the ceca, passage of blood to the droppings, weakness and digestive disturbances. The disease often terminates in the death of the animal, but the fowl which survive severe infections have had their market value subtantially reduced as a result of the infection.

The compositions hereinafter described for application to growing crops or crop growing loci or as a seed dressing may, in general, alternatively be employed for topical application to man or animals or in the protection of stored products, household goods, property or areas of the general environment. Suitable means of applying the compounds of the invention include:

to growing crops as foliar sprays, dusts, granules, fogs or foams or also as suspensions of finely divided or encapsulated compositions as soil or root treatments by liquid drenches, dusts granules, smokes or foams; to seeds of crops via application as seed dressings by liquid slurries or dusts;

to persons or animals infested by or exposed to infestation by arthropods, helminths or protozoa, by parenteral, oral or topical application of compositions in which the active ingredient exhibits an immediate and/or prolonged action over a period of time against the arthropods, helminths or protozoa, for example by incorporation in feed or suitable orally-ingestible pharmaceutical formulations, edible baits, salt licks, dietary supplements, pour-on formulations, sprays, baths, dips, showers, jets, dusts, greases, shampoos, creams, wax smears or livestock self-treatment systems;

to the environment in general or to specific locations where pests may lurk, including stored products, timber, household goods, or domestic or industrial premises, as sprays, fogs, dusts, smokes, wax-smears, lacquers, granules or baits, or in tricklefeeds, to waterways, wells, reservoirs or other running or standing water;

to domestic animals in feed to control fly larvae feeding in their feces;

In practice, the compounds of the invention most frequently form parts of compositions. These compositions can be employed to control: arthopods, especially insects or mites; nematodes; or helminth or protozoan pest. The compositions may be of any type known in the art suitable for application to the desired pest in any premises or indoor or outdoor area or by internal or external administration to vertebrates. These compositions contain at least one compound of the invention, such as described earlier, as the active ingredient in combination or association with one or more other compatible components which are for example, solid or liquid carriers or diluents, adjuvants, surface-active-agents, or the like appropriate for the intended use and which are agronomically or medicinally acceptable. These compositions, which may be prepared by any manner known in the art, likewise form a part of this invention.

These compositions may also contain other kinds or ingredients such as protective colloids, adhesives, thickeners, thixotropic agents, penetrating agents, spray oils (especially for acaridical use), stabilizers, preservative agents (especially mold preservatives) sequestering agents, or the like, as well as other known active ingredients with pesticidal properties (particularly insecticidal, miticidal, nematicidal, or fungicidal) or with properties regulating the growth of plants. More generally, the compounds employed in the invention may be combined with all the solid or liquid additives corresponding to the usual techniques of formulation.

Composition, suitable for applications in agriculture, horticulture, or the like include formulations suitable for use as, for example, sprays, dusts, granules, fogs, foams, emulsion, or the like.

Compositions suitable for administration to vertebrates or man, include preparations suitable for oral, parental, percutaneous, e.g. pour-on, or topical administration.

Compositions for oral administration comprise one or more of the compounds of general formula(I) in association with pharmaceutically acceptable carriers or coatings and include, for example, tablets, pills, capsules, pastes, gels, drenches, medicated feeds, medicated drinking water, medicated dietary supplements, slow-release boluses or other slow-release devices intended to be retained within the gastrointestinal tract. Any of these may incorporate the active ingredient contained within microcapsules or coated with acid-labile or alkali-labile or other pharmaceutically acceptable enteric coatings. Feed premixes or concentrates containing compounds of the present invention for use in preparation of medicated diets, drinking water or other materials for consumption by animals may also be used.

Compositions for parenteral administration include solutions, emulsions or suspensions in any suitable pharmaceutically acceptable vehicle or solid or semisolid subcutaneous implants or pellets designed to release the active ingredient over a protracted period of time and may be prepared and made sterile in any appropriate manner known to the art.

Compositions for percutaneous and topical administration include sprays, dusts, baths, dips, showers, jets, greases, shampoos, creams, wax-smears, or pour-on preparations or devices (e.g. ear tags attached externally to animals in such a way as to provide local or systemic arthropod control).

Solid or liquid baits, suitable for controlling arthropods, comprise one or more compounds of general formula(I) and a carrier or diluent which may include a food substance or some other substance to induce consumption by the arthropod.

The effective use doses of the compounds employed in the invention can vary within wide limits, particularly depending on the nature of the pest to be eliminated or degree of infestation, for example, of crops with these pests. In general, the compositions according to the invention usually contain about 0.05 to about 95% (by weight) of one or more active ingredients according to the invention, about 1 to about 95% of one or more solid or liquid carriers and, optionally, about 0.1 to about 50% of one or more other compatible components, such as surface-active agents or the like.

In the present account, the term "carrier" denotes an organic or inorganic ingredient, natural or synthetic, with which the active ingredient is combined to facilitate its application, for example, to the plant, to seeds or to the soil. This carrier is therefore generally inert and it must be acceptable (for example, agronomically acceptable, particularly to the treated plant).

The carrier may be a solid, for example, clays, natural or synthetic silicates, silica, resins, waxes, solid fertilizers (for example ammonium salts), ground natural minerals, such as kaolins, clays, talc, chalk, quartz, attapulgite, montmorillonite, bentonite or diatomaceous earth, or ground synthetic minerals, such as silica, alumina, or silicates especially aluminium or magnesium silicates. As solid carriers for granules the following are suitable: crushed or fractionated natural rocks such as calcite, marble, pumice, sepiolite and dolomite; synthetic granules of inorganic or organic meals; granules of organic material such as sawdust, coconut shells, corn cobs, corn husks or tobacco stalks; kieselguhr, tricalcium phosphate, powdered cork, or absorbent carbon black; water soluble polymers, resins, waxes; or solid fertilizers. Such solid compositions may, if desired, contain one or more compatible wetting, dispersing, emulsifying or colouring agents which, when solid, may also serve as a diluent.

The carrier may also be liquid, for example: water; alcohols, particularly butanol or glycol, as well as their ethers or esters, particularly methylglycol acetate; ketones, particularly acetone, cyclohexanone, methylethyl ketone, methylisobutylketone, or isophorone; petroleum fractions such as paraffinic or aromatic hydrocarbons, particularly xylenes or alkyl naphthalenes; mineral or vegetable oils; aliphatic chlorinated hydrocarbons, particularly trichloroethane or methylene chloride; aromatic chlorinated hydrocarbons, particularly chlorobenzenes; water-soluble or strongly polar solvents such as dimethylformamide, dimethyl sulphoxide, or N-methylpyrrolidone; liquefied gases; or the like or a mixture thereof.

The surface-active agent may be an emulsifying agent, dispersing agent or wetting agent of the ionic or non-ionic type or a mixture of such surface-active agents. Amongst these are e.g., salts of polyacrylic acids, salts of lignosulphonic acids, salts of phenolsulphonic or naphthalenesulphonic acids, polycondensates of ethylene oxide with fatty alcohols or fatty acids or fatty esters or fatty amines, substituted phenols (particularly alkylphenols or arylphenols), salts of sulphosuccinic acid esters, taurine derivatives (particularly alkyltaurates), phosphoric esters of alcohols or of polycondensates of ethylene oxide with phenols, esters of fatty acids with polyols, or sulphate, sulphonate or phosphate functional derivatives of the above compounds. The presence of at least one surface-active agent is generally essential when the active ingredient and/or the inert carrier are only slightly water soluble or are not water soluble and the carrier agent of the composition for application is water.

Compositions of the invention may further contain different other additives such adhesives or colorants. Adhesives such as carboxymethylcellulose or natural or synthetic polymers in the form of powders, granules or lattices, such as arabic gum, polyvinyl alcohol or polyvinyl acetate, natural phospholipids, such as cephalins or lecithins, or synthetic phospholipids can be used in the formulations. It is possible to use colorants such as inorganic pigments, for example: iron oxides, titanium oxides or Prussian Blue; organic dyestuffs, such as alizarin dyestuffs, azo dyestuffs or metal phthalocyanine dyestuffs; or trace nutrients such as salts of iron, manganese, boron, copper, cobalt, molybdenum or zinc.

Compositions containing compounds of general formula(I) which may be applied to control arthropod, plant nematode, helminth or protozoan pests, may also contain synergists (e.g. piperonyl butoxide or sesamex), stabilizing substances, other insecticides, acaricides, plant nematocides, anthelmintics or anticoccidials, fungicides (agricultural or veterinary as appropriate, e.g. benomyl and iprodione), bactericides, arthropod or vertebrate attractants or repellents or pheromones, deodorants, flavouring agents, dyes, or auxiliary therapeutic agents, e.g. trace elements. These may be designed to improve potency, persistence, safety, uptake where desired, spectrum of pests controlled or to enable the composition to perform other useful functions in the same animal or area treated.

Examples of other pesticidally-active compounds which may be included in, or used in conjunction with the compositions of the present invention are: acephate, chlorpyrifos, demeton-S-methyl, disulfoton, ethoprofos, fenitrothion, malathion, monocrotophos, parathion, phosalone, pirimiphos-methyl, triazophos, cyfluthrin, cyermethrin, deltamethrin, fenpropathrin, fenvalerate, permethrin, aldicarb, carbosulfan, methomyl, oxamyl pirimicarb, bendiocarb, teflubenzuron, dicofol, endosulfan, lindane, benzoximate, cartap, cyhexatin, tetradifon, avermectins, ivermectins, milbemycins, thiophanate, trichlorfon, dichlorvos, diaveridine or dimetriadazole.

For their agricultural application, the compounds of the formula(I) are therefore generally in the form of compositions, which are in various solid or liquid forms.

Solid forms of compositions which can be used are dusting powders (with a content of the compound of formula(I) ranging up to 80%), wettable powders or granules (including water dispersible granules), particularly those obtained by extrusion, compacting, impregnation of a granular carrier, or granulation starting from a powder (the content of the compound of formula(I) in these wettable powders or granules being between about 0.5 and about 80%). Solid homogenous or heterogenous compositions containing one or more compounds of general formula(I) for example granules, pellets, briquettes or capsules, may be used to treat standing or running water over a period of time. A similar effect may be achieved using trickle or intermittent feeds of water dispersible concentrates as described herein.

Liquid compositions, for example, include aqueous or non-aqueous solutions or suspensions (such as emulsifiable concentrates, emulsions, flowables, dispersions, or solutions) or aerosols. Liquid compositions also include, in particular, emulsifiable concentrates, dispersions, emulsions, flowables, aerosols, wettable powders (or powder for spraying), dry flowables or pastes as forms of compositions which are liquid or intended to form liquid compositions when applied, for example as aqueous sprays (including low and ultra-low volume) or as fogs or aerosols.

Liquid compositions, for example, in the form of emulsifiable or soluble concentrates most frequently comprise about 5 to about 80% by weight of the active ingredient, while the emulsions or solutions which are ready for application contain, in their case, about 0.01 to about 20% of the active ingredient. Besides the solvent, the emulsifiable or soluble concentrates may contain, when required, about 2 to about 50% of suitable additives, such as stabilizers, surface-active agents, penetrating agents, corrosion inhibitors, colorants or adhesives. Emulsions of any required concentration, which are particularly suitable for application, for example, to plants, may be obtained from these concentrates by dilution with water. These compositions are included within the scope of the compositions which may be employed in the present invention. The emulsions may be in the form of water-in-oil or oil-in-water type and they may have a thick consistency.

The liquid compositions of this invention may, in addition to normal agricultural use applications be used for example to treat substrates or sites infested or liable to infestation by arthropods (or other pests controlled by compounds of this invention) including premises, outdoor or indoor storage or processing areas, containers or equipment or standing or running water.

All these aqueous dispersions or emulsions or spraying mixtures can be applied, for example, to crops by any suitable means, chiefly by spraying, at rates which are generally of the order of about 100 to about 1,200 liters of spraying mixture per hectare, but may be higher or lower (eg. low or ultra-low volume) depending upon the need or application technique. The compounds or compositions according to the invention are conveniently applied to vegetation and in particular to roots or leaves having pests to be eliminated. Another method of application of the compounds or compositions according to the invention is

by chemigation, that is to say, the addition of a formulation containing the active ingredient to irrigation water. This irrigation may be sprinkler irrigation for foliar pesticides or it can be ground irrigation or underground irrigation for soil or for systemic pesticides.

The concentrated suspensions, which can be applied by spraying, are prepared so as to produce a stable fluid product which does not settle (fine grinding) and usually contain from about 10 to about 75% by weight of active ingredient, from about 0.5 to about 30% of surface-active agents, from about 0.1 to about 10% of thixotropic agents, from about 0 to about 30% of suitable additives, such as anti-foaming agents, corrosion inhibitors, stabilizers, penetrating agents, adhesives and, as the carrier, water or an organic liquid in which the active ingredient is poorly soluble or insoluble Some organic solids or inorganic salts may be dissolved in the carrier to help prevent settling or as antifreezes for water.

The wettable powers (or powder for spraying) are usually prepared so that they contain from about 10 to about 80% by weight of active ingredient, from about 20 to about 90% of a solid carrier, from about 0 to about 5% of a wetting agent, from about 3 to about 10% of a dispersing agent and, when necessary, from about 0 to about 80% of one or more stabilizers and/or other additives, such as penetrating agents, adhesives, anti-caking agents, colorants, or the like. To obtain these wettable powders, the active ingredient(s) is(are) thoroughly mixed in a suitable blender with additional substances which may be impregnated on the porous filler and is(are) ground using a mill or other suitable grinder. This produces wettable powders, the wettability and the suspendability of which are advantageous. They may be suspended in water to give any desired concentration and this suspension can be employed very advantageously in particular for application to plant foliage.

The "water dispersible granules (WG)" (granules which are readily dispersible in water) have compositions which are substantially close to that of the wettable powders. They may be prepared by granulation of formulations described for the wettable powders, either by a wet route (contacting finely divided active ingredient with the inert filler and a little water, e.g. 1 to 20% by weight, or with an aqueous solution of a dispersing agent or binder, followed by drying and screening), or by a dry route (compacting followed by grinding and screening).

The application dose (effective dose) of active ingredient, also as a formulated composition, is generally between about 0.005 and about 15 kg/ha, preferably between about 0.01 and about 2 kg/ha. Therefore, the rates and concentrations of the formulated compositions may vary according to the method of application or the nature of the compositions or use thereof. Generally speaking, the compositions for application to control arthropod, plant nematode, helminth or protozoan pests usually contain from about 0.00001% to about 95%, more particularly from about 0.0005% to about 50% by weight of one or more compounds of general formula(I) or of total active ingredients (that is to say the compound(s) of general formula(I) together with: other substances toxic to arthropods or plant nematodes, anthelmintics, anticoccidials, synergists, trace elements or stabilizers). The actual compositions employed and their rate of application will be selected to achieve the desired effect(s) by the farmer, livestock producer, medical or veterinary practitioner, pest control operator or other person skilled in the art.

Solid or liquid compositions for application topically to animals, timber, stored products or household goods usually contain from about 0.00005% to about 90%, more particularly from about 0.001% to about 10%, by weight of one or more compounds of general formula(I). For administration to animals orally or parenterally, including percutaneously solid or liquid compositions, these normally contain from about 0.1% to about 90% by weight of one or more compounds of general formula(I). Medicated feedstuffs normally contain from about 0.001% to about 3% by weight of one or more compounds of general formula(I). Concentrates or supplements for mixing with feedstuffs normally contain from about 5% to about 90%, preferably from about 5% to about 50%, by weight of one or more compounds of general formula(I). Mineral salt licks normally contain from about 0.1% to about 10% by weight of one or more compounds of general formula(I).

Dusts or liquid compositions for application to livestock, persons, goods, premises or outdoor areas may contain from about 0.0001% to about 15%, more especially from about 0.005% to about 2.0%, by weight, of one or more compounds of general formula(I). Suitable concentrations in treated waters are between about 0.0001 ppm and about 20 ppm, more particularly about 0.001 ppm to about 5.0 ppm. of one or more compounds of general formula(I) and may be used therapeutically in fish farming with appropriate exposure times. Edible baits may contain from about 0.01% to about 5%, preferably from about 0.01% to about 1.0%, by weight, of one or more compounds of general formula(I).

When administered to vertebrates parenterally, orally or by percutaneous or other means, the dosage of compounds of general formula(I) will depend upon the species, age, or health of the vertebrate and upon the nature and degree of its actual or potential infestation by arthropod, helminth or protozoan pests. A single dose of about 0.1 to about 100 mg, preferably about 2.0 to about 20.0 mg, per kg body weight of the

animal or doses of about 0.01 to about 20.0 mg, preferably about 0.1 to about 5.0 mg, per kg body weight of the animal per day, for sustained medication, are generally suitable by oral or parenteral administration. By use of sustained release formulations or devices, the daily doses required over a period of months may be combined and administered to animals on a single occasion.

The following composition **EXAMPLES 166-177** illustrate compositions for use against arthropods, especially mites or insects, plant nematodes, or helminth or protozoan pests which comprise, as active ingredient, compounds of general formula(I), especially compounds according to formula (II), such as those described in preparative **EXAMPLES 1 to 164**. The compositions described in **EXAMPLES 166 to 171** can each be diluted in water to give a sprayable compositon at concentrations suitable for use in the field. Generic chemical descriptions of the ingredients (for which all of the following percentages are in weight percent), used in the composition **EXAMPLES 166-177** exemplified below, are as follows:

| Trade Name | Chemical Description |
|---|---|
| Ethylan BCP | Nonylphenol ethylene oxide condensate |
| Soprophor BSU | Tristyrylphenol ethylene oxide condensate |
| Arylan CA | A 70% w/v solution of calcium dodecylbenzenesulfonate |
| Solvesso 150 | Light $C_{10}$ aromatic solvent |
| Arylan S | Sodium dodecylbenzenesulfonate |
| Darvan No2 | Sodium lignosulphonate |
| Celite PF | Synthetic magnesium silicate carrier |
| Sopropon T36 | Sodium salts of polycarboxylic acids |
| Rhodigel 23 | Polysaccharide xanthan gum |
| Bentone 38 | Organic derivative of magnesium montmorillonite |
| Aerosil | Microfine silicon dioxide |

**EXAMPLE 166**

A water soluble concentrate is prepared with the composition as follows:

| | |
|---|---|
| Active ingredient | 7% |
| Ethylan BCP | 10% |
| N-methylpyrrolidone | 83% |

To a solution of Ethylan BCP dissolved in a portion of N-methylpyrrolidone is added the active ingredient with heating and stirring until dissolved. The resulting solution is made up to volume with the remainder of the solvent.

**EXAMPLE 167**

An emulsifiable concentrate (EC) is prepared with the composition as follows:

| | |
|---|---|
| Active ingredient | 7% |
| Soprophor BSU | 4% |
| Arylan CA | 4% |
| N-methylpyrrolidone | 50% |
| Solvesso 150 | 35% |

The first three components are dissolved in N-methylpyrrolidone and to this is then added the Solvesso 150 to give the final volume.

**EXAMPLE 168**

A wettable powder (WP) is prepared with the composition as follows:

| | |
|---|---|
| Active ingredient | 40% |
| Arylan S | 2% |
| Darvan No2 | 5% |
| Celite PF | 53% |

The ingredients are mixed and ground in a hammer-mill to a powder with a particle size of less than 50 microns.

**EXAMPLE 169**

An aqueous-flowable formulation is prepared with the composition as follows:

| | |
|---|---|
| Active ingredient | 40.00% |
| Ethylan BCP | 1.00% |
| Sopropon T360. | 0.20% |
| Ethylene glycol | 5.00% |
| Rhodigel 230. | 0.15% |
| Water | 53.65% |

The ingredients are intimately mixed and are ground in a bead mill until a mean particle size of less than 3 microns is obtained.

**EXAMPLE 170**

An emulsifiable suspension concentrate is prepared with the composition as follows;

| | |
|---|---|
| Active ingredient | 30.0% |
| Ethylan BCP | 10.0% |
| Bentone 38 | 0.5% |
| Solvesso 150 | 59.5% |

The ingredients are intimately mixed and ground in a beadmill until a mean particle size of less than 3 microns is obtained.

**EXAMPLE 171**

A water dispersible granule is prepared with the composition as follows:

| | |
|---|---|
| Active ingredient | 30% |
| Darvan No 2 | 15% |
| Arylan S | 8% |
| Celite PF | 47% |

The ingredients are mixed, micronized in a fluid-energy mill and then granulated in a rotating pelletizer by spraying with water (up to 10%). The resulting granules are dried in a fluid-bed drier to remove excess water.

**EXAMPLE 172**

A dusting powder is prepared with the composition as follows:

| | |
|---|---|
| Active ingredient | 1 to 10% |
| Talc powder-superfine | 99 to 90% |

The ingredients are intimately mixed and further ground as necessary to achieve a fine powder. This powder may be appplied to a locus of arthropod infestation, for example refuse dumps, stored products or household goods or animals infested by, or at risk of infestation by, arthropods to control the arthropods by oral ingestion. Suitable means for distributing the dusting powder to the locus of arthropod infestation include mechanical blowers, handshakers or livestock self treatment devices.

**EXAMPLE 173**

An edible bait is prepared with the composition as follows:

| | |
|---|---|
| Active ingredient | 0.1 to 1.0% |
| Wheat flour | 80% |
| Molasses | 19.9 to 19% |

The ingredients are intimately mixed and formed as required into a bait form. This edible bait may be distributed at a locus, for example domestic or industrial premises, e.g. kitchens, hospitals or stores, or outdoor areas, infested by arthropods, for example ants, locusts, cockroaches or flies, to control the arthropods by oral ingestion.

**EXAMPLE 174**

A solution formulation is prepared with a composition as follows:

| | |
|---|---|
| Active ingredient | 15% |
| Dimethyl sulfoxide | 85% |

The active ingredient is dissolved in dimethyl sulfoxide with mixing and or heating as required. This solution may be applied percutaneously as a pour-on application to domestic animals infested by arthropods or, after sterilization by filtration through a polytetrafluoroethylene membrane (0.22 micrometer pore size), by parenteral injection, at a rate of application of from 1.2 to 12 ml of solution per 100 kg of animal body weight.

**EXAMPLE 175**

A wettable powder is prepared with the composition as follows:

| | |
|---|---|
| Active ingredient | 50% |
| Ethylan BCP | 5% |
| Aerosil | 5% |
| Celite PF | 40% |

The Ethylan BCP is absorbed onto the Aerosil which is then mixed with the other ingredients and ground in a hammer-mill to give a wettable powder, which may be diluted with water to a concentration of from 0.001% to 2% by weight of the active compound and applied to a locus of infestation by arthropods, for example, dipterous larvae or plant nematodes, by spraying, or to domestic animals infested by, or at risk of infection by arthropods, helminths or protozoa, by spraying or dipping, or by oral administration in drinking water, to control the arthropods, helminths or protozoa.

**EXAMPLE 176**

A slow release bolus composition is formed from granules containing the following components in varying percentages(similar to those described for the previous compositions) depending upon need:

Active ingredient
Density agent
Slow-release agent
Binder

The intimately mixed ingredients are formed into granules which are compressed into a bolus with a specific gravity of 2 or more. This can be administered orally to ruminant domestic animals for retention within the reticulo-rumen to give a continual slow release of active compound over an extended period of time to control infestation of the ruminant domestic animals by arthropods, helminths or protozoa.

**EXAMPLE 177**

A slow release composition in the form of granules, pellets, brickettes or the like can be prepared with compositions as follows:

| | |
|---|---|
| Active ingredient | 0.5 to 25% |
| Polyvinyl chloride | 75 to 99.5% |
| Dioctyl phthalate (plasticizer) | catalytic amount |

The components are blended and then formed into suitable shapes by melt-extrusion or molding. These composition are useful, for example, for addition to standing water or for fabrication into collars or eartags for attachment to domestic animals to control pests by slow release.

**Claims**

**Claims for the following Contracting States : AT, BE, CH, LI, DE, DK, FR, GB, GR, IT, LU, NL, SE**

**1.** A compound of formula (I)

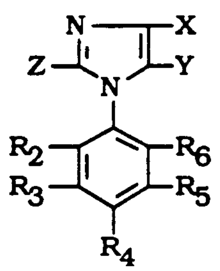

(I)

wherein:

X is a group selected from haloalkyl or haloalkoxy or an unsubstituted or halo-substituted group selected from alkylsulfenyl, alkylsulfinyl or alkylsulfonyl, wherein the defined alkyl and alkoxy moieties of each group are a linear or branched chain, containing one to four carbon atoms, and the halo-substitution of each group comprises one or more halogen atoms, which are the same or different, up to full substitution of the alkyl or alkoxy moiety;

Y and Z are each individually selected from: a hydrogen or a halogen atom; a group selected from nitro, cyano, hydroxyl and acceptable salts thereof, sulfhydryl and acceptable salts thereof, formyl, hydroxycarbonyl and acceptable salts thereof, alkoxycarbonyl, aminocarbonyl, alkylaminocarbonyl, dialkylaminocarbonyl, amino, alkylamino, dialkylamino, a trialkylammonium salt, cyanoalkyl, alkoxycarbonylamino, arylcarbonylamino, alkylaminocarbonylamino, dialkylaminocarbonylamino, aminosulfonyl, alkylaminosulfonyl, dialkylaminosulfonyl, or alkoxyalkylideneimino, in which the defined alkyl and alkoxy moieties of each group are a linear or branched chain containing one to four carbon atoms; a

linear or branched chain alkenyl or alkynyl group containing two to four carbon atoms; or a group selected from an unsubstituted or halo-substituted alkyl, alkoxy, alkylcarbonyl, alkylcarbonylamino, alkylsulfenyl, alkylsulfinyl or alkylsulfonyl, in which the defined alkyl and alkoxy moieties of each group are a linear or branched chain containing one to four carbon atoms and the halo-substitution consists of one or more halogen atoms, which are the same or different, up to full substitution of the alkyl or alkoxy moiety; and only one of Y and Z is a sulfur containing group; and $R_2$, $R_3$, $R_4$, $R_5$, and $R_6$, are individually selected from: a hydrogen or a halogen atom; a group selected from nitro, cyano, amino, alkylamino or dialkylamino, in which the alkyl moiety of each group is a linear or branched chain containing one to four carbon atoms; a linear or branched chain alkenyl or alkynyl group containing two to four carbon atoms, which may be substituted by one or more halogen atoms, which are the same or different, up to full substitution; or a group selected from an unsubstituted or halo-substituted alkyl, alkoxy, alkylsulfenyl, alkylsulfinyl or alkylsulfonyl, in which the defined alkyl and alkoxy moieties of each group are a linear or branched chain containing one to four carbon atoms and the halo-substitution comprises one or more halogen atoms, which are the same or different, up to full substitution of the alkyl or alkoxy moiety; provided that (i) X is other than trifluoromethyl when $R_3$ represents methyl, $R_4$ represents nitro or amino and the other symbols represent hydrogen and (ii) X is other than chloromethyl when $R_4$ represents methyl, methoxy or ethoxy and the other symbols represent hydrogen.

**2.** The compound of claim 1 wherein X is haloalkoxy.

**3.** The compound of Claim 1, wherein X is $S(O)_nR_1$, having the formula (II)

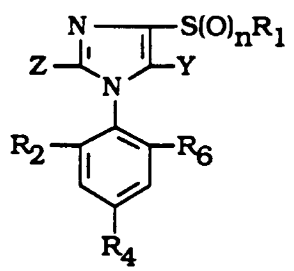

(II)

wherein:

| | |
|---|---|
| Y and Z | are individually selected from a hydrogen or halogen atom; a group selected from nitro, cyano, hydroxyl, sulfhydryl, amino, alkylamino or dialkylamino, in which the defined alkyl moiety of each group is a linear or branched chain containing one to four carbon atoms; or a group selected from an unsubstituted or fully halo-substituted alkyl, alkoxy, alkylcarbonyl, alkylcarbonylamino, alkylsulfenyl, alkylsulfinyl or alkylsulfonyl, in which the defined alkyl and alkoxy moieties of each group are a linear or branched chain, containing one to four carbon atoms, and the full halo-substitution of the alkyl or alkoxy moiety is by the same or different halogen atoms; and only one of Y and Z is a sulfur containing group; |
| $R_1$ | is a linear or branched alkyl group of one to four carbon atoms which are unsubstituted or halo-substituted by one or more halogen atoms, which are the same or different; |
| $R_2$ | is a hydrogen or a halogen atom or an alkyl, alkoxy, methylsulfenyl, methylsulfinyl or methylsulfonyl group; |
| $R_4$ | is selected from a halogen atom or a group selected from trifluoromethyl, trifluoromethoxy, difluoromethoxy, trifluoromethylsulfenyl, trifluoromethylsulfinyl, trifluoromethylsulfonyl, or a linear or branched chain alkyl group containing one to four carbon atoms; |
| $R_6$ | is a halogen atom; and |
| n | is 0, 1 or 2. |

**4.** The compound of Claim 3 wherein:

- Y is a hydrogen atom, a halogen atom, amino, hydroxy, alkoxy of one to four carbon atoms, methylsulfenyl, methylsulfinyl or methylsulfonyl;
- Z is a hydrogen atom, a halogen atom, or a linear or branched alkyl group of one to four carbon atoms which is optionally fully substituted by halogen atoms which are the same or different;
- $R_1$ is a methyl group fully substituted by halogen atoms which are the same or different;
- $R_2$ is a hydrogen atom, a halogen atom or methylsulfenyl;
- $R_4$ is a halogen atom, trifluoromethyl or trifluoromethoxy; and
- $R_6$ is a fluorine, chlorine or bromine atom.

**5.** The compound of Claim 4 wherein:

- Y is a hydrogen atom, a chlorine atom, a bromine atom, methylsulfenyl, methylsulfinyl or methoxy;
- Z is a hydrogen atom, a chlorine atom, a bromine atom or methyl;
- $R_1$ is trifluoromethyl, dichlorofluoromethyl or chlorodifluoromethyl;
- $R_2$ is a hydrogen atom, a chlorine atom, a bromine atom or methylsulfenyl;
- $R_4$ is a chlorine atom, a bromine atom, a fluorine atom, trifluoromethyl or trifluoromethoxy; and
- $R_6$ is a chlorine or bromine atom.

**6.** The compound of Claim 5 which is:

1-(2,6-dichloro-4-trifluoromethylphenyl)-2-chloro-4-trifluoromethylsulfenylimidazole;
1-(2,6-dichloro-4-trifluoromethylphenyl)-2-chloro-4-trifluoromethylsulfinylimidazole;
1-(2,6-dichloro-4-trifluoromethylphenyl)-5-bromo-4-dichlorofluoromethylsulfenylimidazole;
1-(2,6-dichloro-4-trifluoromethylphenyl)-2-chloro-4-dichlorofluoromethylsulfenylimidazole;
1-(2,6-dichloro-4-trifluoromethylphenyl)-2-bromo-4-dichlorofluoromethylsulfenylimidazole;
1-(2,6-dichloro-4-trifluoromethylphenyl)-4-trifluoromethylsulfinylimidazole;
1-(2,6-dichloro-4-trifluoromethylphenyl)-2-chloro-4-dichlorofluoromethylsulfinylimidazole;
1-(2,6-dichloro-4-trifluoromethylphenyl)-2-chloro-4-dichlorofluoromethylsulfonylimidazole;
1-(2,6-dichloro-4-trifluoromethylphenyl)-2-bromo-4-chlorodifluoromethylsulfinylimidazole;
1-(2,6-dichloro-4-trifluoromethylphenyl)-2-bromo-4-chlorodifluoromethylsulfenylimidazole;
1-(2,6-dichloro-4-trifluoromethylphenyl)-2-chloro-4-chlorodifluoromethylsulfenylimidazole;
1-(6-chloro-2-methylsulfenyl-4-trifluoromethylphenyl)-2-bromo-4-chlorodifluoromethylsulfonyl-imidazole;
1-(6-chloro-2-methylsulfenyl-4-trifluoromethylphenyl)-2-chloro-4-dichlorofluoromethylsulfonylimidazole;
1-(2,6-dichloro-4-trifluoromethylphenyl)-4-dichlorofluoromethylsulfenylimidazole;
1-(2,6-dichloro-4-trifluoromethylphenyl)-4-dichlorofluoromethylsulfinylimidazole;
1-(2,6-dichloro-4-trifluoromethylphenyl)-4-dichlorofluoromethylsulfonylimidazole;
1-(2,6-dichloro-4-trifluoromethylphenyl)-2-methyl-4-dichlorofluoromethylsulfenylimidazole;
1-(2,6-dichloro-4-trifluoromethylphenyl)-2-methyl-4-dichlorofluoromethylsulfinylimidazole;
1-(2,6-dichloro-4-trifluoromethylphenyl)-2-methyl-4-dichlorofluoromethylsulfonylimidazole;
1-(2,4,6-trichlorophenyl)-4-dichlorofluoromethylsulfenylimidazole;
1-(2,4,6-trichlorophenyl)-4-dichlorofluoromethylsulfinylimidazole;
1-(2,6-dichloro-4-trifluoromethoxyphenyl)-4-dichlorofluoromethylsulfinylimidazole;
1-(2,6-dichloro-4-trifluoromethylphenyl)-4-chlorodifluoromethylsulfinylimidazole;
1-(2,6-dichloro-4-trifluoromethylphenyl)-2-methyl-4-chlorodifluoromethylsulfinylimidaxole;
1-(2,6-dichloro-4-trifluoromethylphenyl)-2-methyl-4-chlorodifluoromethylsulfonylimidazole;
1-(2,4,6-trichlorophenyl)-4-chlorodifluoromethylsulfenylimidazole; or
1-(2,4,6-trichlorophenyl)-4-chlorodifluoromethylsulfinylimidizole.

**7.** The compound of Claim 5 which is:

1-(2,6-dichloro-4-trifluoromethylphenyl)-2-chloro-4-trifluoromethylsulfonylimidazole;
1-(2,6-dichloro-4-trifluoromethylphenyl)-5-methylsulfenyl-4-dichlorofluoromethylsulfenylimidazole;
1-(2,6-dichloro-4-trifluoromethylphenyl)-4-trifluoromethylsulfonylimidazole;
1-(2,6-dichloro-4-trifluoromethylphenyl)-5-bromo-4-trifluoromethylsulfonylimidazole;
1-(2,6-dichloro-4-trifluoromethylphenyl)-2-bromo-4-dichlorofluoromethylsulfinylimidazole;
1-(2,6-dichloro-4-trifluoromethylphenyl)-5-bromo-4-dichlorofluoromethylsulfonylimidazole;
1-(2,6-dichloro-4-trifluoromethylphenyl)-2-bromo-4-dichlorofluoromethylsulfonylimidazole;

1-(2,6-dichloro-4-trifluoromethylphenyl)-2-bromo-4-chlorodifluoromethylsulfonylimidazole;
1-(2,6-dichloro-4-trifluoromethylphenyl)-2-chloro-4-chlorodifluoromethylsulfinylimidazole;
1-(2,6-dichloro-4-trifluoromethylphenyl)-2-chloro-4-chlorodifluoromethylsulfonylimidazole;
1-(2,6-dichloro-4-trifluoromethylphenyl)-5-methylsulfonyl-4-dichlorofluoromethylsulfinylimidazole;
1-(2,6-dichloro-4-trifluoromethyl)-5-methylsulfinyl-4-dichlorofluoromethylsulfinylimidazole;
1-(6-chloro-2-methylsulfenyl-4-trifluoromethylphenyl)-2-chloro-4-dichlorofluoromethylsulfenylimidazole;
1-(6-chloro-2-methylsulfenyl-4-trifluoromethylphenyl)-2-chloro-4-dichlorofluoromethylsulfinylimidazole;
1-(2,6-dichloro-4-trifluoromethylphenyl)-2-chloro-5-methylsulfenyl-4-trifluoromethylsulfenylimidazole;
1-(2,6-dichloro-4-trifluoromethylphenyl)-5-bromo-4-trifluoromethylsulfenylimidazole;
1-(2,6-dichloro-4-trifluoromethylphenyl)-2-methyl-4-chlorodifluoromethylsulfenylimidazole;
1-(2,6-dichloro-4-trifluoromethylphenyl)-5-chloro-4-trifluoromethylsulfenylimidazole;
1-(2,6-dichloro-4-trifluoromethylphenyl)-2,5-dichloro-4-dichlorofluoromethylsulfenylimidazole;
1-(2,6-dichloro-4-trifluoromethylphenyl)-4-chlorodifluoromethylsulfenylimidazole;
1-(2-chloro-4-trifluoromethylphenyl)-4-dichlorofluoromethylsulfinylimidazole;
1-(2-chloro-4-trifluoromethylphenyl)-4-trifluoromethylsulfinylimidazole;
1-(2-chloro-4-trifluoromethylphenyl)-4-trifluoromethylsulfonylimidazole;
1-(2,6-dichloro-4-bromophenyl)-4-dichlorofluoromethylsulfenylimidazole;
1-(2,6-dichloro-4-bromophenyl)-4-dichlorofluoromethylsulfinylimidazole;
1-(2,6-dichloro-4-bromophenyl)-4-chlorodifluoromethylsulfenylimidazole;
1-(2,6-dichloro-4-trifluoromethoxyphenyl)-4-trifluoromethylsulfinylimidazole;
1-(2,6-dichloro-4-trifluoromethoxyphenyl)-4-chlorodifluoromethylsulfenylimidazole;
1-(2,6-dichloro-4-trifluoromethoxyphenyl)-4-chlorodifluoromethylsulfinylimidazole;
1-(2,6-dichloro-4-trifluoromethoxyphenyl)-4-dichlorofluoromethylsulfonylimidazole; or
1-(2,6-dichloro-4-trifluoromethoxyphenyl)-2-bromo-4-dichlorofluoromethylsulfenylimidazole.

**8.** A process for the preparation of a compound of formula I:
(A) conforming to the formula:

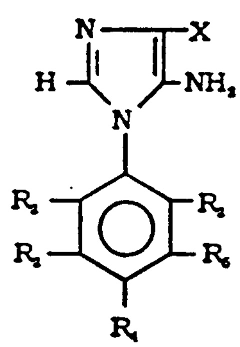

(Ia)

wherein $R_2$, $R_3$, $R_4$, $R_5$ and $R_6$ are as defined for formula (I) in Claim 1 and X is alkylsulfenyl, haloalkylsulfenyl. alkylsulfinyl, haloalkylsulfinyl, alkylsulfonyl, haloalkylsulfonyl, haloalkyl or haloalkoxy, wherein a compound of formula (5),

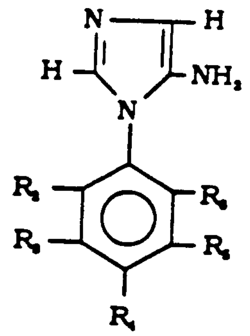

5

in which amino is optionally protected as required:

a) is first reacted with a sulfenyl halide, $R_1SHalo$ in which $R_1$ is alkyl or haloalkyl, in an organic reaction medium, optionally in the presence of an acid acceptor such as a tertiary amine to obtain a compound of formula (Ia), wherein X is alkylsulfenyl or haloalkylsulfenyl, which is then optionally oxidized by known methods such as by a peroxide, to obtain a compound of formula (Ia), wherein X is $S(O)_nR_1$ in which n is 1 or 2 and $R_1$ is as defined above, that is to say X is alkylsulfinyl, haloalkylsulfinyl, alkylsulfonyl or haloalkylsulfonyl;

b) is first reacted with a tris(alkylthio)methane or tris(arylthio)methane in an organic reaction medium in the presence of a Lewis Acid and optionally in the presence of an acid acceptor, then the obtained intermediate compound of formula (Ia) in which X is bis(alkylthio)methyl or bis-(arylthio)methyl is reacted in an organic reaction medium with a suitable alkylnitrite followed by a hydrolysis procedure to obtain an intermediate compound of formula (Ia), in which X is formyl, then is followed by known reduction procedures to give the intermediate compound, in which X is hydroxymethyl, and then is finally halogenated by known procedures to give a compound of formula (Ia), in which X is haloalkyl, more specifically halomethyl;

c) is first formylated by well known procedures such as that of Vilsmeier-Haack and the like to give the compound of formula (Ia), in which X is formyl, then is reacted following the procedures above in part b) to likewise obtain the compound of formula (Ia) , in which X is haloalkyl;

d) is reacted by the procedures of part b) or c) above to obtain an intermediate compound of formula (Ia), in which X is formyl, which compound may be optionally oxidized to an intermediate compound of formula (Ia), in which X is carboxyl, then finally the intermediate compound, in which X is formyl, is reacted with a halogenating agent such as diethylaminosulfur trifluoride, or the intermediate compound, in which X is carboxyl, is reacted with sulfur tetrafluoride to give a compound of formula (Ia), in which X is haloalkyl; more specifically difluoromethyl or trifluoromethyl; or

e) is first halogenated by well known procedures to obtain an intermediate compound, in which X is halogen, from which an organomagnesium or -lithium derivative is prepared, then said organometallic is reacted with oxodiperoxymolybdenum(pyridine)(hexamethylphosphoric triamide) or a trialkyl borate and an oxidizing agent such as hydrogen peroxide to obtain an intermediate compound of formula (Ia), in which X is hydroxy, then is finally reacted by known haloalkylating procedures to obtain a compound of formula (Ia) in which X is haloalkoxy; or

(B) conforming to the formula:

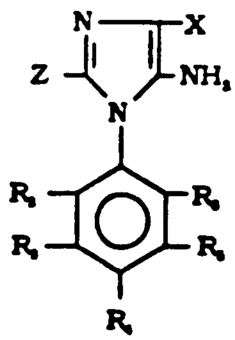

(Ib)

wherein X, $R_2$, $R_3$, $R_4$, $R_5$ and $R_6$ are as defined for formula (I) in Claim 1 and Z is aminosulfonyl, alkylaminosulfonyl, dialkylaminosulfonyl, nitro, amino, halogen, alkynyl, alkyl, hydroxy and salts thereof, alkoxy, haloalkoxy, formyl, alkylsulfenyl, haloalkylsulfenyl, alkylsulfinyl, haloalkylsulfinyl, alkylsulfonyl, haloalkylsulfonyl, or sulfhydryl and salts thereof, wherein a compound of formula (Ia) ,

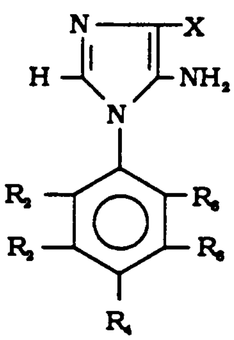

(Ia)

in which X and amino are optionally protected as required:

a) is first reacted with chlorosulfonic or dichlorosulfonic acid to give an intermediate compound, wherein Z is chlorosulfonyl, which compound is reacted with ammonia, an alkylamine or dialkylamine to give a compound of formula (Ib), in which Z is aminosulfonyl, alkylaminosulfonyl or dialkylaminosulfonyl;

b) is halogenated or nitrated by known procedures to give a compound of formula (Ib), in which Z is halogen or nitro, then the compound in which Z is nitro is optionally reduced to Z is amino by known procedures, or optionally the compound, in which Z is halogen, is treated by known procedures with a copper acetylide to give the compound in which Z is alkynyl;

c) is reacted with a strong base such as an organolithium reagent to give an intermediate organometallic carbanion, which is then quenched with an alkylating agent to give a compound of formula (Ib), in which Z is alkyl, or optionally the carbanion is reacted in a manner similar to that described in (A)e above to first give a compound of formula (1b), in which Z is hydroxyl or salts thereof, or then optionally the compound in which Z is hydroxy is converted to a compound wherein Z is alkoxy or haloalkoxy by known alkylation or haloalkylation procedures;

d) is reacted by formylation procedures similar to those described in (A)b or (A)c above, wherein the compound, in which Z is formyl, is prepared directly by conditions such as the Vilsmeier-Haack Reaction or via hydrolysis of an intermediate compound in which Z is bis(alkythio)methyl or bis(arylthio)methyl;

e) is first reacted with a mixture of bromine and a metal thiocyanate to give an intermediate compound of formula (Ib), in which Z is thiocyano, which then is treated with an alkylating agent, optionally in the presence of a base to directly give a compound of formula (Ib), in which Z is alkylsulfenyl or haloalkylsulfenyl, or optionally the intermediate compound in which Z is thiocyano

is first oxidized to a corresponding intermediate disulfide compound which is then reacted with a perhaloalkane, optionally in the presence of a reducing agent, to give a compound of formula (Ib) in which Z is haloalkylsulfenyl, particularly perhaloalkylsulfenyl, finally the compound, in which Z is alkylsulfenyl or haloalkylsulfenyl, is optionally oxidized by known methods similar to those of (A)a above to give a compound of formula (Ib), in which Z is alkylsulfinyl, haloalkylsulfinyl. alkylsulfonyl or haloalkylsulfonyl; or

f) is first reacted as above in part e) to give the intermediate compound, in which Z is thiocyano, which is then cleaved by a free radical promoter, such as potassium ferricyanide, to give a compound of formula (Ib), in which Z is sulfhydryl or salts thereof; or

(C) conforming to the formula:

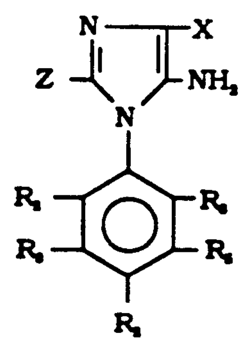

(Ib)

wherein X, $R_2$, $R_3$, $R_4$, $R_5$ and $R_6$ are as defined for formula (I) in Claim 1 and Z is amino, alkyl, cyano, carboxyl and salts thereof, aminocarbonyl, alkylaminocarbonyl, dialkylaminocarbonyl, alkoxycarbonyl, haloalkyl, cyanoalkyl, alkenyl, alkynyl, alkylcarbonyl or haloalkylcarbonyl, wherein a compound of formula (Ib), in which Z is formyl, prepared via procedures described in (B)d above, and in which X and amino are optionally protected as required:

a) is reduced to a compound of formula (Ib), in which Z is alkyl, particularly methyl, by known reducing agents such as sodium borohydride or p-toluenesulfonylhydrazine and sodium cyanoborohydride;

b) is reacted with a standard known oxidizing agent to give a compound of formula (Ib), in which Z is carboxyl or salts thereof, then optionally the carboxyl compound can be converted to a compound of formula (Ib), in which Z is amino, by a Curtius rearrangement via an intermediate acid halide, aside, and isocyanate or optionally the compound in which Z is carboxyl is treated with isophthalonitrile to give a compound of formula (Ib), in which Z is cyano, or optionally the compound in which Z is formyl is reacted with hydroxylamine to give an intermediate aldoxime compound which is then dehydrated by standard procedures to give the compound of formula (Ib), in which Z is cyano;

c) is converted to the compound in which Z is carboxyl by the procedure b) above, then the carboxyl is converted by standard procedures to an intermediate acid halide compound, which then is reacted with ammonia, an alkylamine, dialkylamine or alkyl alcohol to give a compound of formula (Ib), in which Z is aminocarbonyl, dialkylaminocarbonyl or alkoxycarbonyl;

d) is first reduced to an intermediate hydroxymethyl compound by procedures similar to those in (A)b above, followed by halogenation procedures also similar to those of (A)b above, to give a compound of formula (Ib), in which Z is haloalkyl, more specifically halomethyl, or optionally the haloalkyl compound, specifically halomethyl, is treated with a metal cyanide to give a compound of formula (Ib), in which Z is cyanoalkyl, more specifically cyanomethyl;

e) is reacted in a Wittig or modified Wittig reaction to give a compound of formula (Ib), in which Z is alkenyl or alkynyl;

f) is reacted with a Grignard reagent or alkyllithium reagent to give an intermediate compound of formula (Ib), in which Z is α-hydroxyalkyl, then is oxidized with known reagents to give a compound of formula (Ib), in which Z is alkylcarbonyl, then the alkylcarbonyl compound is optionally halogenated to a compound of formula (Ib), in which Z is haloalkylcarbonyl; or

g) is first converted according to the procedures above in parts b) and c) to the acid chloride intermediate, obtained via the compound in which Z is carboxyl, then by conventional Curtius

rearrangement procedures, the acid halide intermediate is converted via azide and isocyanate intermediates to the compound of formula (Ib), in which Z is amino; or

(D) conforming to the formula:

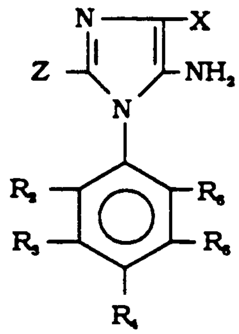

(Ib)

wherein X, $R_2$, $R_3$, $R_4$, $R_5$ and $R_6$ are as defined for formula (I) in Claim 1 and Z is alkylamino, dialkylamino, trialkylammonium salt, alkoxycarbonylamino, alkylaminocarbonylamino, dialkylaminocarbonylamino, alkoxyalkylideneimino, alkylcarbonylamino, haloalkylcarbonylamino or arylcarbonylamino, wherein a compound of formula (Ib), in which Z is amino, prepared via procedures described in (b)b or (B)g above and in which X and Y is amino are optionally protected as required:

a) is first reacted with phosgene to give an intermediate compound of formula (Ib), in which Z is chlorocarbonylamino or isocyanato, which then is reacted with an alkyl alcohol, alkylamine or dialkylamine to give a compound of formula (Ib), in which Z is alkoxycarbonylamino, alkylaminocarbonylamino or dialkylaminocarbonylamino;

b) is reacted with an alkylating agent, such as an alkyl iodide or dialkyl sulfate or optionally by known reductive methylation using formaldehyde and formic acid to give a compound of formula (Ib), in which Z is alkylamino, dialkylamino or trialkylammonium salt;

c) is reacted with an alkyl orthoformate to give a compound of formula (Ib), in which Z is alkoxyalkylideneimino, particularly alkoxymethylideneimino; or

d) is reacted with an alkyl-, haloalkyl- or arylcarbonyl halide, optionally in the presence of an acid acceptor, to give a compound of formula (Ib), in which Z is alkylcarbonylamino, haloalkylcarbonylamino or arylcarbonylamino; or

(E) conforming to the formula:

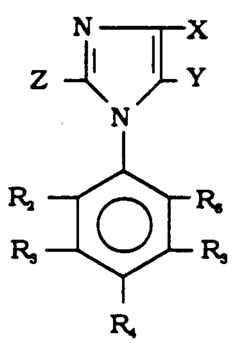

(I)

wherein X, Z, $R_2$, $R_3$, $R_4$, $R_5$ and $R_6$ are as defined for formula (I) in Claim 1 and Y is hydrogen, amino, halogen, alkylsulfenyl, haloalkylsulfenyl, alkylsulfinyl, haloalkylsulfinyl, alkylsulfonyl, haloalkylsulfonyl, cyano or nitro, wherein a compound of formula (Ib),

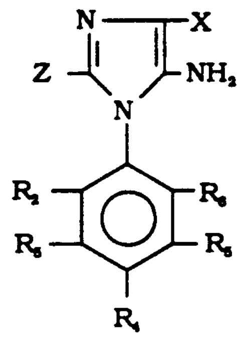

(Ib)

in which X, Z and $R_2$ to $R_6$ are as defined above and in which X, Z, and amino are optionally protected as required:

a) is deaminated by known procedures, such as with an alkylnitrite to convert the compound, in which Y is amino, into its corresponding diazonium salt, followed by quenching the diazonium salt with a quenching agent according to known procedures to obtain a compound of formula (I), in which Y is hydrogen, halogen, cyano, nitro, alkylsulfenyl, or haloalkylsulfenyl and then the compound, in which Y is alkylsulfenyl or haloalkylsulfenyl is optionally oxidized to a compound of formula (I), in which Y is alkylsulfinyl, haloalkylsulfinyl, alkylsulfonyl or haloalkylsulfonyl; or

(F) conforming to the formula:

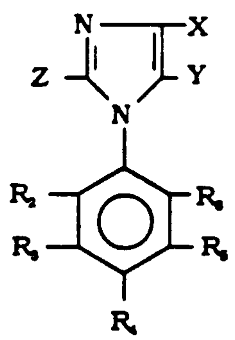

(I)

wherein X, Z, $R_2$, $R_3$, $R_4$, $R_5$ and $R_6$ are as defined for formula (I) in Claim 1 and Y is alkoxycarbonylamino, alkylaminocarbonylamino, dialkylaminocarbonylamino, alkoxyalkylideneimino, alkylcarbonylamino, haloalkylcarbonylamino, arylcarbonylamino, alkylamino, dialkylamino or trialkylammonium salt, wherein a compound of formula (Ib),

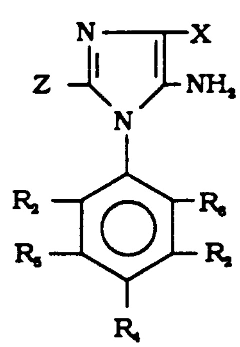

(Ib)

in which X, Z and $R_2$ to $R_6$ are as defined above and in which X, Z and amino are optionally protected as required:

a) is reacted in a manner similar to that described in (D)a above via a chlorocarbonylamino or isocyanato intermediate, obtained by reaction with phosgene, which then is by reacted with an alkyl alcohol, alkylamine, or dialkylamine to give a compound of formula (I), in which Y is alkoxycarbonylamino, alkylaminocarbonylamino or dialkylaminocarbonylamino;

b) is reacted in a manner similar to that described in (D)c above with an alkylorthoformate to give a compound of formula (I), in which Y is alkoxyalkylideneimino, particularly alkoxymethylideneimino;

c) is reacted in a similar manner to that described in (D)b above by alkylation or reductive methylation to give a compound of formula (I), in which Y is alkylamino, dialkylamino or trialkylammonium salt; or

d) is reacted in a similar manner to that described in (D)d above with an alkyl-, haloalkyl- or arylcarbonyl halide to give a compound of formula (I), in which Y is alkylcarbonylamino, haloalkylcarbonylamino or arylcarbonylamino; or

(G) conforming to the formula:

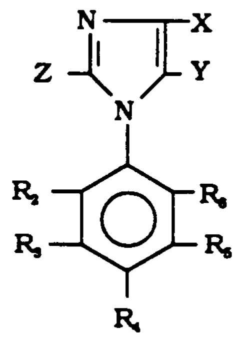

(I)

wherein X, Z, $R_2$, $R_3$, $R_4$, $R_5$ and $R_6$ are as defined for formula (I) in Claim 1 and Y is nitro, sulfhydryl and salts thereof, hydroxyl and salts thereof, alkoxy, haloalkoxy, aminosulfonyl, alkylaminosulfonyl, dialkylaminosulfonyl, alkyl, haloalkyl, alkenyl, alkynyl, cyanoalkyl or formyl, wherein a compound of formula (Ib),

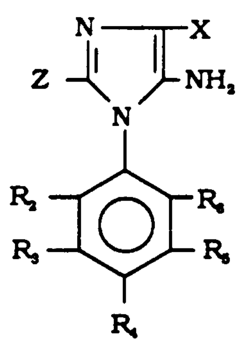

(Ib)

in which X, Z and $R_2$ to $R_6$ are as defined above and in which X and Z are optionally protected as required is deaminated according to the procedures described in (E) above to give a compound of formula (I), in which Y is hydrogen, then said compound, in which X and Z are optionally protected as required:

a) is nitrated by procedures similar to those described in Claim (B)b to give a compound of formula (I), in which Y is nitro;

b) is reacted in a similar manner by the procedures described in (B)f above to first give an intermediate compound, in which Y is thiocyano, which is then reacted to give a compound of formula (I), in which Y is sulfhydryl and salts thereof;

c) is first reacted with a strong base such as an organolithium reagent to give an intermediate metal carbanion, which is then quenched with an electrophile to give a compound of formula (I),

in which Y is alkyl, haloalkyl, alkenyl, alkynyl, cyanoalkyl or formyl;

d) is converted to the carbanion, as above in part c), and then quenched with sulfuryl chloride to give an intermediate compound, in which Y is chlorosulfonyl, which then is reacted with ammonia or an alkyl- or dialkylamine to give a compound of formula (I), in which Y is aminosulfonyl, alkylaminosulfonyl or dialkylaminosulfonyl;

e) is converted to the carbanion as above in part c) or optionally the carbanion is prepared via the compound in which Y is halogen, obtained by the procedure of (E) above and then the carbanion is reacted in a similar manner to the procedure described in (B)c above to give a compound of formula (I), in which Y is hydroxyl and salts thereof, alkoxy or haloalkoxy; or

(H) conforming to the formula:

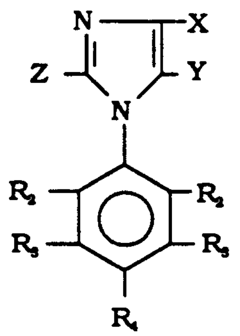

(I)

wherein X, Z, $R_2$, $R_3$, $R_4$, $R_5$ and $R_6$ are as defined for formula (I) in Claim 1 and Y is carboxyl and salts thereof, cyano, aminocarbonyl, alkylaminocarbonyl, dialkylaminocarbonyl, alkoxycarbonyl, haloalkyl, alkenyl, alkynyl, alkylcarbonyl or haloalkylcarbonyl, wherein a compound of formula (Ib),

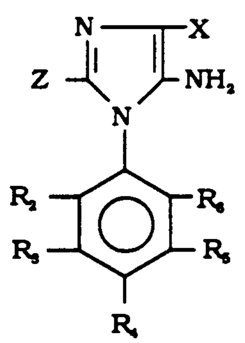

(Ib)

in which X, Z and $R_2$ to $R_6$ are as defined above is first deaminated according to the procedures described in (E) above to give a compound, in which Y is hydrogen, which is then converted by procedures described in (G)c above to give a compound of formula (I), in which Y is formyl, then said formyl compound, in which X and Z are optionally protected as required:

a) is reacted by similar procedures described in C(b) above to give a compound of formula (I), in which Y is carboxyl and salts thereof or cyano;

b) is reacted by similar procedures described in C(c) above to give a compound of formula (I), in which Y is aminocarbonyl, alkylaminocarbonyl, dialkylaminocarbonyl or alkoxycarbonyl;

c) is reacted by similar procedures described in C(d) above to give a compound of formula (I), in which Y is haloalkyl. more specifically halomethyl;

d) is reacted by similar procedures described in (B)b above and 19e to give a compound of formula (I), in which Y is alkenyl or alkynyl; or

e) is reacted by similar procedures described in (C)f above to give a compound of formula (I), in which Y is alkylcarbonyl or haloalkylcarbonyl; or

(I) conforming to the formula:

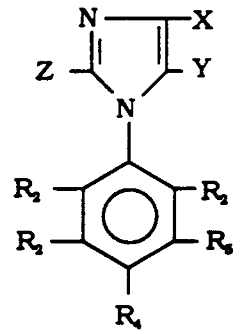

(I)

wherein Y, Z, $R_2$, $R_3$, $R_4$, $R_5$ and $R_6$ are as defined for formula (I) in Claim 1 and X is alkylsulfenyl, haloalkylsulfenyl, alkylsulfinyl, haloalkylsulfinyl, alkylsulfonyl or haloalkylsulfonyl, wherein a compound of formula (Ic),

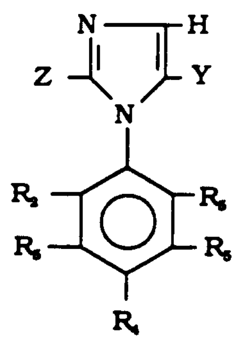

(Ic)

in which Y and Z are optionally protected as required:

a) is first reacted according to procedures similar to those described in (B)c above to convert a compound of formula (Ic), in which X is hydrogen, to an intermediate compound of formula (I), in which X is successively thiocyano and then a disulfide, then also by procedures similar to those described in (B)c above , the thiocyano or disulfide intermediate is converted to a compound of formula (I), in which X is alkylsulfenyl or haloalkylsulfenyl, particularly perhaloalkylsulfenyl, which compound then is optionally oxidized by procedures similar to those in (B)c above, to obtain the sulfoxide or sulfone analog, that is a compound of formula (I), in which X is alkylsulfinyl, haloalkylsulfinyl, preferably perhaloalkylsulfinyl, alkylsulfonyl or haloalkylsulfonyl, particularly perhaloalkylsulfonyl; or

b) is first reacted according to procedures similar to those described in (B)a above to convert the compound of formula (Ic), in which X is hydrogen to an intermediate compound of formula (I), in which X is chlorosulfonyl, then the chlorosulfonyl compound is reacted with a reducing agent such as triphenylphosphine to give the same disulfide intermediate described above in part a), then finally the disulfide is converted by the procedures described above in part a) to give a compound of formula (I), in which X is alkylsulfenyl or haloalkylsulfenyl, particularly perhaloalkylsulfenyl or optionally the sulfenyl compound is oxidized to give a compound of formula (I), in which X is alkylsulfinyl, haloalkylsulfinyl, particularly perhaloalkylsulfinyl, alkylsulfonyl or haloalkylsulfonyl, particularly perhaloalkylsulfonyl; or

(J) conforming to the formula:

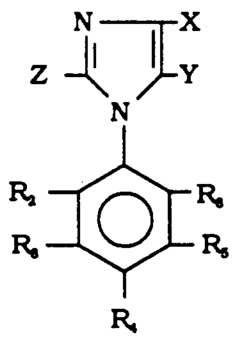

(IV)

wherein:

| | |
|---|---|
| $R_2$, $R_3$, $R_4$, $R_5$ and $R_6$ | are as defined in Claim 1; |
| X | is hydrogen or haloalkyl, particularly trifluoromethyl; |
| Y | is amino; hydroxy, optionally existing in its isomeric keto form when X is hydrogen; alkoxy; or haloalkoxy; and |
| Z | is hydrogen; halogen; alkyl; haloalkyl; hydroxy, optionally existing in its isomeric keto form when X is hydrogen and Y is imino; alkoxy; or haloalkoxy; |

whereby a compound of formula (III),

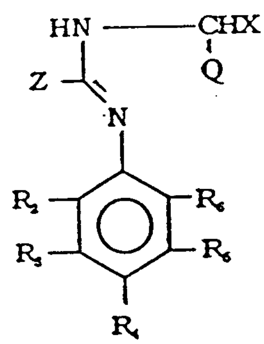

(III)

wherein:

| | |
|---|---|
| $R_2$, $R_3$, $R_4$, $R_5$ and $R_6$ | are as defined above; |
| X | is hydrogen or haloalkyl, particularly trifluoromethyl; |
| Z | is hydrogen, halogen, alkyl, haloalkyl or hydroxy, optionally existing in its isomeric keto form; and |
| Q | is cyano or lower alkoxycarbonyl; |

is reacted with a basic agent in a suitable reaction medium to give a compound of formula (IV), which when Y or Z is hydroxy, is then optionally alkylated or haloalkylated to Y or Z is alkoxy or haloalkoxy.

**9.** The process of preparation of a compound of formula (IV), according to Claim 8J, wherein the compound of formula (IV) is:

a) a compound of formula (5), depicted in Claim 8 in which X and Z are each hydrogen and Y is amino;

b) a compound of formula (17),

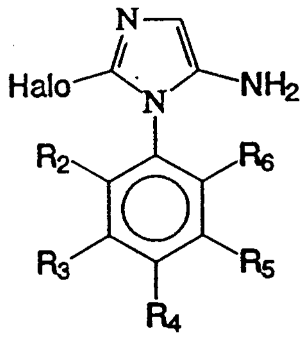

(17)

in which Halo means halogen, particularly chlorine;

c) a compound of formula (22),

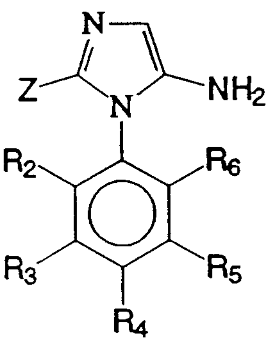

(22)

in which Z is alkyl or haloalkyl;

d) a compound of formula (27),

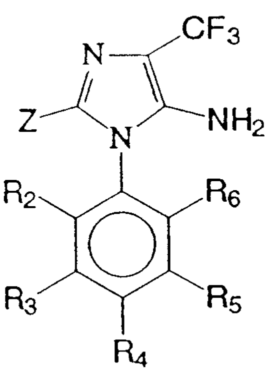

(27)

in which Z is halogen, alkyl, or haloalkyl;

e) a compound of formula (30), in which X is hydrogen and Y is hydroxyl optionally existing in its isomeric keto form (29),

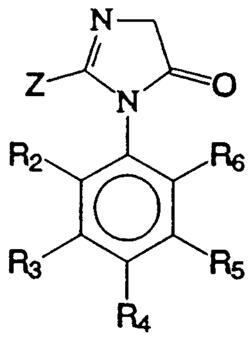

(29)

in which Z is halogen, alkyl or haloalkyl; or

f) a compound of formula (37), in which Y is amino and Z is hydroxy, optionally existing in its isomeric keto-imino form (34),

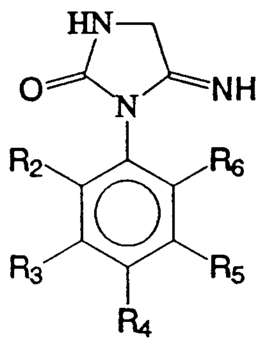

(34)

**10.** A process of preparation of a compound of formula (I):

(A) wherein X, Y, Z, $R_2$, $R_3$, $R_4$, $R_5$ and $R_6$ are as defined in Claim 1, wherein a compound of formula (5) depicted in Claim 8 is reacted according to the process of preparation of Claim 8 for introduction of the X, Y and Z substituents;

(B) wherein X, Y, $R_2$, $R_3$, $R_4$, $R_5$ and $R_6$ are as defined in Claim 1 and Z is halogen, whereby a compound of formula (17) depicted in Claim 9 is reacted according to the process of preparation of Claim 8 for introduction of the X and Y substituents;

(C) wherein X, Y, $R_2$, $R_3$, $R_4$, $R_5$ and $R_6$ are as defined in Claim 1 and Z is alkyl or haloalkyl, whereby a compound of formula (22) depicted in Claim 9 is reacted according to the process of preparation of Claim 8 for introduction of the X and Y substituents;

(D) wherein Y, $R_2$, $R_3$, $R_4$, $R_5$ and $R_6$ are as defined in Claim 1, X is haloalkyl, particularly trifluoromethyl, and Z is halogen, alkyl or haloalkyl, whereby a compound of formula (27) is reacted according to the process of Claim 8 for introduction of the Y substituent;

(E) wherein X, $R_2$, $R_3$, $R_4$, $R_5$ and $R_6$ are as defined in Claim 1, Y is hydroxy, alkoxy or haloalkoxy, and Z is halogen, alkyl or haloalkyl, whereby a compound of formula (30), optionally existing in its isomeric keto form (29), depicted in Claim 9 in which Y is hydroxy, optionally alkylated to Y is alkoxy or haloalkoxy, is reacted according to the process of Claim 8 for introduction of the X substituent; or

(F) wherein X, Y, $R_2$, $R_3$, $R_4$, $R_5$ and $R_6$ are as defined in Claim 1 and Z is hydroxy, alkoxy, haloalkoxy or halogen, whereby a compound of formula (37), optionally existing in its isomeric keto-imino form (34), depicted in Claim 9 in which Z is hydroxy, optionally alkylated to Z is alkoxy or haloalkoxy or optionally halogenated to Z is halogen, is reacted according to the process of claim 8 for introduction of the X and Y substitutents.

**11.** A compound of formula (Ia), (Ib), (Ic), (IV), (5), (17), (22), (27), (30)/(29), or (37)/(34), depicted in Claim 8 or 9 wherein the substituents X, Y, Z, $R_2$, $R_3$, $R_4$, $R_5$ and $R_6$ are as hereinbefore defined in Claim 8, 9

or 10.

**12.** A method for the control of: arthropods, particularly insects, aphids or mites; nematodes; or helminth or protozoan pests at a locus which comprises treatment of the locus with an effective amount of a compound of formula (I), wherein the various substitutents X, Y, Z, $R_2$, $R_3$, $R_4$, $R_5$ and $R_6$ are as defined in any one of Claims 1 to 7, but including compounds wherein (i) X is trifluoromethyl, when $R_3$ represents methyl, $R_4$ represents nitro or amino and the other symbols represent hydrogen and (ii) X is chloromethyl when $R_4$ represents methyl, methoxy or ethoxy and the other symbols represent hydrogen, with the exclusion of a method of treatment of the human or animal body carried out by a medical or veterinary practitioner.

**13.** The method of claim 12 wherein the compound of formula (I) is a compound named in claim 5 or 6.

**14.** The method of Claim 12 or 13 wherein the locus comprises agricultural or horticultural plants or a medium in which the plants grow and the pests are arthropod or nematode pests of the plants, and the treatment is by applying to the plants or to the medium in which they grow an effective amount of the compound of formula (I).

**15.** The method of Claim 14, wherein the compound is applied to the locus, in which the arthropod or nematode pests are controlled, at a rate of about 0.005 kg to about 15 kg of compound per hectare of locus treated, preferably at a rate of about 0.02 kg to about 2 kg of compound per hectare.

**16.** The method of Claim 15, wherein said pests are mites, aphids, insects or plant nematodes or combinations thereof, which comprises incorporating the compound into soil in which the plants are planted or are to be planted, or applying the compound to the plant seeds or to the plant's roots, or treating by foliar application.

**17.** The method of Claim 16, wherein said insects are soil insects in the Coleoptera order, particularly Diabrotica species, said mites are in the subclass Acari, and said aphids are in the super family Aphidoidea.

**18.** The method of Claim 12, wherein said method is employed in the field of veterinary medicine or livestock husbandry or in the maintenance of public health against: arthropods, particularly insects in the Diptera order or mites in the subclass Acari or both; or helminths or protozoa which are parasitic internally or externally upon warm-blooded vertebrates.

**19.** A composition for the control of arthropod, nematode, helminth, or protozoan pests comprising: an effective amount, preferably about 0.05% to about 95% by weight, of a compound of formula (I) according to any of Claims 1 to 6 but including compounds wherein (i) X is trifluoromethyl when $R_3$ represents methyl, $R_4$ represents nitro or amino and the other symbols represent hydrogen and (ii) X is chloromethyl when $R_4$ represents methyl, methoxy or ethoxy and the other symbols represent hydrogen as an active ingredient and one or more agronomically or medicinally compatible components preferably comprising about 1% to about 95%, by weight, of one or more solid or liquid carriers and about 0.1% to about 50%, by weight, or one or more additional components such as surface active agents.

**20.** A composition particularly useful in veterinary medicine and livestock husbandry or in the maintenance of public health comprising one or more compatible components and as an active ingredient at least one compound of formula (I) as defined in Claim 19.

**Claims for the following Contracting State : ES**

**1.** A process for the preparation of a compound of formula (I)

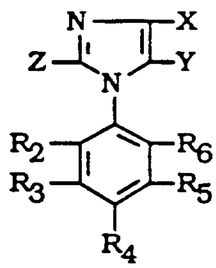

(I)

wherein:

X is a group selected from haloalkyl or haloalkoxy or an unsubstituted or halo-substituted group selected from alkylsulfenyl, alkylsulfinyl or alkylsulfonyl, wherein the defined alkyl and alkoxy moieties of each group are a linear or branched chain, containing one to four carbon atoms, and the halo-substitution of each group comprises one or more halogen atoms, which are the same or different, up to full substitution of the alkyl or alkoxy moiety;

Y and Z are each individually selected from: a hydrogen or a halogen atom; a group selected from nitro, cyano, hydroxyl and acceptable salts thereof, sulfhydryl and acceptable salts thereof, formyl, hydroxycarbonyl and acceptable salts thereof, alkoxycarbonyl, aminocarbonyl, alkylaminocarbonyl, dialkylaminocarbonyl, amino, alkylamino, dialkylamino, a trialkylammonium salt, cyanoalkyl, alkoxycarbonylamino, arylcarbonylamino, alkylaminocarbonylamino, dialkylaminocarbonylamino, aminosulfonyl, alkylaminosulfonyl, dialkylaminosulfonyl, or alkoxyalkylideneimino, in which the defined alkyl and alkoxy moieties of each group are a linear or branched chain containing one to four carbon atoms; a linear or branched chain alkenyl or alkynyl group containing two to four carbon atoms; or a group selected from an unsubstituted or halo-substituted alkyl, alkoxy, alkylcarbonyl, alkylcarbonylamino, alkylsulfenyl, alkylsulfinyl or alkylsulfonyl, in which the defined alkyl and alkoxy moieties of each group are a linear or branched chain containing one to four carbon atoms and the halo-substitution consists of one or more halogen atoms, which are the same or different, up to full substitution of the alkyl or alkoxy moiety; and only one of Y and Z is a sulfur containing group; and $R_2$, $R_3$, $R_4$, $R_5$, and $R_6$, are individually selected from: a hydrogen or a halogen atom; a group selected from nitro, cyano, amino, alkylamino or dialkylamino, in which the alkyl moiety of each group is a linear or branched chain containing one to four carbon atoms; a linear or branched chain alkenyl or alkynyl group containing two to four carbon atoms, which may be substituted by one or more halogen atoms, which are the same or different, up to full substitution; or a group selected from an unsubstituted or halo-substituted alkyl, alkoxy, alkylsulfenyl, alkylsulfinyl or alkylsulfonyl, in which the defined alkyl and alkoxy moieties of each group are a linear or branched chain containing one to four carbon atoms and the halo-substitution comprises one or more halogen atoms, which are the same or different, up to full substitution of the alkyl or alkoxy moiety; provided that (i) X is other than trifluoromethyl when $R_3$ represents methyl, $R_4$ represents nitro or amino and the other symbols represent hydrogen and (ii) X is other than chloromethyl when $R_4$ represents methyl, methoxy or ethoxy and the other symbols represent hydrogen:

(A) conforming to the formula:

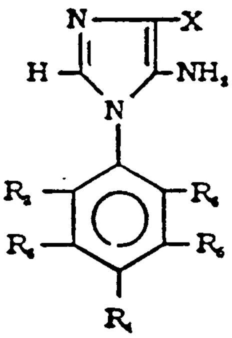

(Ia)

wherein $R_2$, $R_3$, $R_4$, $R_5$ and $R_6$ are as defined for formula (I)

and X is alkylsulfenyl, haloalkylsulfenyl, alkylsulfinyl, haloalkylsulfinyl, alkylsulfonyl, haloalkylsulfonyl, haloalkyl or haloalkoxy, wherein a compound of formula (5),

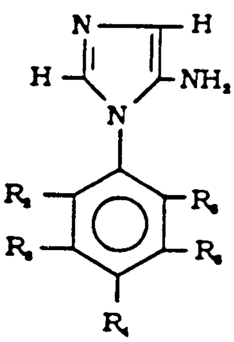

5

in which amino is optionally protected as required:

a) is first reacted with a sulfenyl halide, $R_1$SHalo in which $R_1$ is alkyl or haloalkyl, in an organic reaction medium, optionally in the presence of an acid acceptor such as a tertiary amine to obtain a compound of formula (Ia), wherein X is alkylsulfenyl or haloalkylsulfenyl, which is then optionally oxidized by known methods such as by a peroxide, to obtain a compound of formula (Ia), wherein X is $S(O)_nR_1$ in which n is 1 or 2 and $R_1$ is as defined above, that is to say X is alkylsulfinyl, haloalkylsulfinyl, alkylsulfonyl or haloalkylsulfonyl;

b) is first reacted with a tris(alkylthio)methane or tris(arylthio)methane in an organic reaction medium in the presence of a Lewis Acid and optionally in the presence of an acid acceptor, then the obtained intermediate compound of formula (Ia) in which X is bis(alkylthio)methyl or bis(arylthio)methyl is reacted in an organic reaction medium with a suitable alkylnitrite followed by a hydrolysis procedure to obtain an intermediate compound of formula (Ia), in which X is formyl, then is followed by known reduction procedures to give the intermediate compound, in which X is hydroxymethyl, and then is finally halogenated by known procedures to give a compound of formula (Ia), in which X is haloalkyl, more specifically halomethyl;

c) is first formylated by well known procedures such as that of Vilsmeier-Haack and the like to give the compound of formula (Ia), in which X is formyl, then is reacted following the procedures above in part b) to likewise obtain the compound of formula (Ia) , in which X is haloalkyl;

d) is reacted by the procedures of part b) or c) above to obtain an intermediate compound of formula (Ia), in which X is formyl, which compound may be optionally oxidized to an intermediate compound of formula (Ia), in which X is carboxyl, then finally the intermediate compound, in which X is formyl, is reacted with a halogenating agent such as diethylaminosulfur trifluoride, or the intermediate compound, in which X is carboxyl, is reacted with sulfur tetrafluoride to give a compound of formula (Ia), in which X is haloalkyl, more specifically difluoromethyl or

trifluoromethyl; or

e) is first halogenated by well known procedures to obtain an intermediate compound, in which X is halogen, from which an organomagnesium or -lithium derivative is prepared, then said organometallic is reacted with oxodiperoxymolybdenum(pyridine)(hexamethylphosphoric triamide) or a trialkyl borate and an oxidizing agent such as hydrogen peroxide to obtain an intermediate compound of formula (Ia), in which X is hydroxy, then is finally reacted by known haloalkylating procedures to obtain a compound of formula (Ia) in which X is haloalkoxy; or

(B) conforming to the formula:

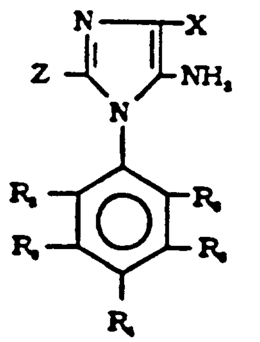

(Ib)

wherein X, $R_2$, $R_3$, $R_4$, $R_5$ and $R_6$ are as defined for formula (I)

and Z is aminosulfonyl, alkylaminosulfonyl, dialkylaminosulfonyl, nitro, amino, halogen, alkynyl, alkyl, hydroxy and salts thereof, alkoxy, haloalkoxy, formyl, alkylsulfenyl, haloalkylsulfenyl, alkylsulfinyl, haloalkylsulfinyl, alkylsulfonyl, haloalkylsulfonyl, or sulfhydryl and salts thereof, wherein a compound of formula (Ia) ,

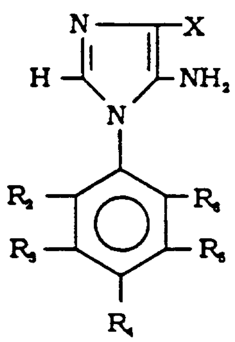

(Ia)

in which X and amino are optionally protected as required:

a) is first reacted with chlorosulfonic or dichlorosulfonic acid to give an intermediate compound, wherein Z is chlorosulfonyl, which compound is reacted with ammonia, an alkylamine or dialkylamine to give a compound of formula (Ib), in which Z is aminosulfonyl, alkylaminosulfonyl or dialkylaminosulfonyl;

b) is halogenated or nitrated by known procedures to give a compound of formula (Ib), in which Z is halogen or nitro, then the compound in which Z is nitro is optionally reduced to Z is amino by known procedures, or optionally the compound, in which Z is halogen, is treated by known procedures with a copper acetylide to give the compound in which Z is alkynyl;

c) is reacted with a strong base such as an organolithium reagent to give an intermediate organometallic carbanion, which is then quenched with an alkylating agent to give a compound of formula (Ib), in which Z is alkyl, or optionally the carbanion is reacted in a manner similar to that described in (A)e above to first give a compound of formula (Ib), in which Z is hydroxyl or salts thereof, or then optionally the compound in which Z is hydroxy is converted to a compound wherein Z is alkoxy or haloalkoxy by known alkylation or haloalkylation procedures;

d) is reacted by formylation procedures similar to those described in (A)b or (A)c above, wherein the compound, in which Z is formyl, is prepared directly by conditions such as the Vilsmeier-Haack Reaction or via hydrolysis of an intermediate compound in which Z is bis(alkythio)methyl or bis(arylthio)methyl;

e) is first reacted with a mixture of bromine and a metal thiocyanate to give an intermediate compound of formula (Ib), in which Z is thiocyano, which then is treated with an alkylating agent, optionally in the presence of a base to directly give a compound of formula (Ib), in which Z is alkylsulfenyl or haloalkylsulfenyl, or optionally the intermediate compound in which Z is thiocyano is first oxidized to a corresponding intermediate disulfide compound which is then reacted with a perhaloalkane, optionally in the presence of a reducing agent, to give a compound of formula (Ib) in which Z is haloalkylsulfenyl, particularly perhaloalkylsulfenyl, finally the compound, in which Z is alkylsulfenyl or haloalkylsulfenyl, is optionally oxidized by known methods similar to those of (A)a above to give a compound of formula (Ib), in which Z is alkylsulfinyl, haloalkylsulfinyl, alkylsulfonyl or haloalkylsulfonyl; or

f) is first reacted as above in part e) to give the intermediate compound, in which Z is thiocyano, which is then cleaved by a free radical promoter, such as potassium ferricyanide, to give a compound of formula (Ib), in which Z is sulfhydryl or salts thereof; or

(C) conforming to the formula:

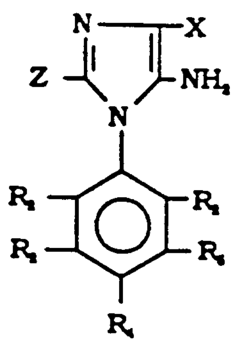

(Ib)

wherein X, $R_2$, $R_3$, $R_4$, $R_5$ and $R_6$ are as defined for formula (I)

and Z is amino, alkyl, cyano, carboxyl and salts thereof, aminocarbonyl, alkylaminocarbonyl, dialkylaminocarbonyl, alkoxycarbonyl, haloalkyl, cyanoalkyl, alkenyl, alkynyl, alkylcarbonyl or haloalkylcarbonyl, wherein a compound of formula (Ib), in which Z is formyl, prepared via procedures described in (B)d above, and in which X and amino are optionally protected as required:

a) is reduced to a compound of formula (Ib), in which Z is alkyl, particularly methyl, by known reducing agents such as sodium borohydride or p-toluenesulfonylhydrazine and sodium cyanoborohydride;

b) is reacted with a standard known oxidizing agent to give a compound of formula (Ib), in which Z is carboxyl or salts thereof, then optionally the carboxyl compound can be converted to a compound of formula (Ib), in which Z is amino, by a Curtius rearrangement via an intermediate acid halide, azide, and isocyanate or optionally the compound in which Z is carboxyl is treated with isophthalonitrile to give a compound of formula (Ib), in which Z is cyano, or optionally the compound in which Z is formyl is reacted with hydroxylamine to give an intermediate aldoxime compound which is then dehydrated by standard procedures to give the compound of formula (Ib), in which Z is cyano;

c) is converted to the compound in which Z is carboxyl by the procedure b) above, then the carboxyl is converted by standard procedures to an intermediate acid halide compound, which then is reacted with ammonia, an alkylamine, dialkylamine or alkyl alcohol to give a compound of formula (Ib), in which Z is aminocarbonyl, dialkylaminocarbonyl or alkoxycarbonyl;

d) is first reduced to an intermediate hydroxymethyl compound by procedures similar to those in (A)b above, followed by halogenation procedures also similar to those of (A)b above, to give a compound of formula (Ib), in which Z is haloalkyl, more specifically halomethyl, or optionally the haloalkyl compound, specifically halomethyl, is treated with a metal cyanide to give a compound of formula (Ib), in which Z is cyanoalkyl, more specifically cyanomethyl;

e) is reacted in a Wittig or modified Wittig reaction to give a compound of formula (Ib), in which Z is alkenyl or alkynyl;

f) is reacted with a Grignard reagent or alkyllithium reagent to give an intermediate compound of formula (Ib), in which Z is α-hydroxyalkyl, then is oxidized with known reagents to give a compound of formula (Ib), in which Z is alkylcarbonyl, then the alkylcarbonyl compound is optionally halogenated to a compound of formula (Ib), in which Z is haloalkylcarbonyl; or

g) is first converted according to the procedures above in parts b) and c) to the acid chloride intermediate, obtained via the compound in which Z is carboxyl, then by conventional Curtius rearrangement procedures, the acid halide intermediate is converted via azide and isocyanate intermediates to the compound of formula (Ib), in which Z is amino; or

(D) conforming to the formula:

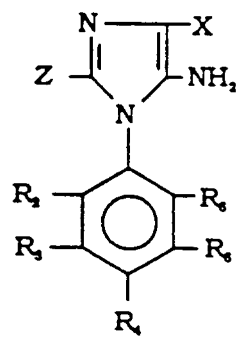

(Ib)

wherein X, $R_2$, $R_3$, $R_4$, $R_5$ and $R_6$ are as defined for formula (I)

and Z is alkylamino, dialkylamino, trialkylammonium salt, alkoxycarbonylamino, alkylaminocarbonylamino, dialkylaminocarbonylamino, alkoxyalkylideneimino, alkylcarbonylamino, haloalkylcarbonylamino or arylcarbonylamino, wherein a compound of formula (Ib), in which Z is amino, prepared via procedures described in (b)b or (B)g above and in which X and Y is amino are optionally protected as required:

a) is first reacted with phosgene to give an intermediate compound of formula (Ib), in which Z is chlorocarbonylamino or isocyanato, which then is reacted with an alkyl alcohol, alkylamine or dialkylamine to give a compound of formula (Ib), in which Z is alkoxycarbonylamino, alkylaminocarbonylamino or dialkylaminocarbonylamino;

b) is reacted with an alkylating agent, such as an alkyl iodide or dialkyl sulfate or optionally by known reductive methylation using formaldehyde and formic acid to give a compound of formula (Ib), in which Z is alkylamino, dialkylamino or trialkylammonium salt;

c) is reacted with an alkyl orthoformate to give a compound of formula (Ib), in which Z is alkoxyalkylideneimino, particularly alkoxymethylideneimino; or

d) is reacted with an alkyl-, haloalkyl- or arylcarbonyl halide, optionally in the presence of an acid acceptor, to give a compound of formula (Ib), in which Z is alkylcarbonylamino, haloalkylcarbonylamino or arylcarbonylamino; or

(E) conforming to the formula:

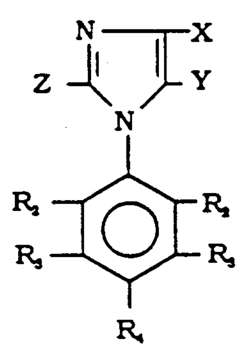

(I)

wherein X, Z, $R_2$, $R_3$, $R_4$, $R_5$ and $R_6$ are as defined for formula (I)

and Y is hydrogen, amino, halogen, alkylsulfenyl, haloalkylsulfenyl, alkylsulfinyl, haloalkylsulfinyl, alkylsulfonyl, haloalkylsulfonyl, cyano or nitro, wherein a compound of formula (Ib),

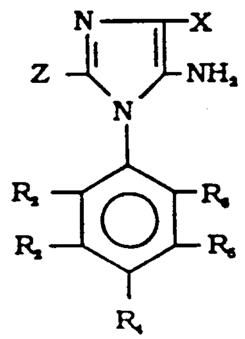

(Ib)

in which X, Z and $R_2$ to $R_6$ are as defined above and in which X, Z, and amino are optionally protected as required:

a) is deaminated by known procedures, such as with an alkylnitrite to convert the compound, in which Y is amino, into its corresponding diazonium salt, followed by quenching the diazonium salt with a quenching agent according to known procedures to obtain a compound of formula (I), in which Y is hydrogen, halogen, cyano, nitro, alkylsulfenyl, or haloalkylsulfenyl and then the compound, in which Y is alkylsulfenyl or haloalkylsulfenyl is optionally oxidized to a compound of formula (I), in which Y is alkylsulfinyl, haloalkylsulfinyl, alkylsulfonyl or haloalkylsulfonyl; or

(F) conforming to the formula:

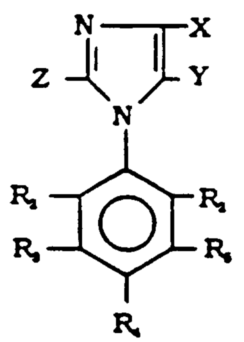

(I)

wherein X, Z, $R_2$, $R_3$, $R_4$, $R_5$ and $R_6$ are as defined for formula (I)

and Y is alkoxycarbonylamino, alkylaminocarbonylamino, dialkylaminocarbonylamino, alkoxyalkylideneimino, alkylcarbonylamino, haloalkylcarbonylamino, arylcarbonylamino, alkylamino, dialkylamino or trialkylammonium salt, wherein a compound of formula (Ib),

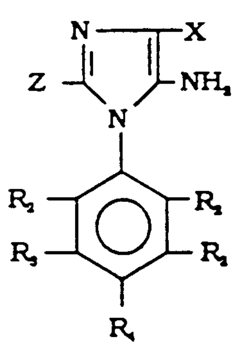

(Ib)

in which X, Z and $R_2$ to $R_6$ are as defined above and in which X, Z and amino are optionally protected as required:

a) is reacted in a manner similar to that described in (D)a above via a chlorocarbonylamino or isocyanato intermediate, obtained by reaction with phosgene, which then is by reacted with an alkyl alcohol, alkylamine, or dialkylamine to give a compound of formula (I), in which Y is alkoxycarbonylamino, alkylaminocarbonylamino or dialkylaminocarbonylamino;

b) is reacted in a manner similar to that described in (D)c above with an alkylorthoformate to give a compound of formula (I), in which Y is alkoxyalkylideneimino, particularly alkoxymethylideneimino;

c) is reacted in a similar manner to that described in (D)b above by alkylation or reductive methylation to give a compound of formula (I), in which Y is alkylamino, dialkylamino or trialkylammonium salt; or

d) is reacted in a similar manner to that described in (D)d above with an alkyl-, haloalkyl- or arylcarbonyl halide to give a compound of formula (I), in which Y is alkylcarbonylamino, haloalkylcarbonylamino or arylcarbonylamino ; or

(G) conforming to the formula:

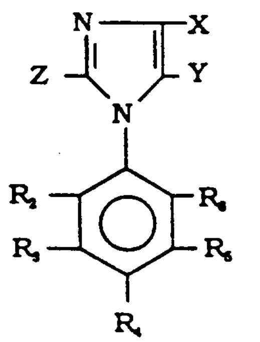

(I)

wherein X, Z, $R_2$, $R_3$, $R_4$, $R_5$ and $R_6$ are as defined for formula (I)

and Y is nitro, sulfhydryl and salts thereof, hydroxyl and salts thereof, alkoxy, haloalkoxy, aminosulfonyl, alkylaminosulfonyl, dialkylaminosulfonyl, alkyl, haloalkyl, alkenyl, alkynyl, cyanoalkyl or formyl, wherein a compound of formula (Ib),

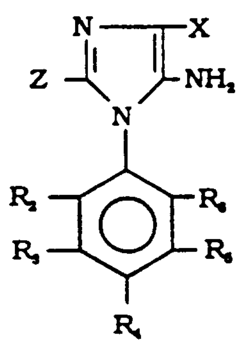

(Ib)

in which X, Z and $R_2$ to $R_6$ are as defined above and in which X and Z are optionally protected as required is deaminated according to the procedures described in (E) above to give a compound of formula (I), in which Y is hydrogen, then said compound, in which X and Z are optionally protected as required:

a) is nitrated by procedures similar to those described in Claim (B)b to give a compound of formula (I), in which Y is nitro;

b) is reacted in a similar manner by the procedures described in (B)f above to first give an intermediate compound, in which Y is thiocyano, which is then reacted to give a compound of

formula (I), in which Y is sulfhydryl and salts thereof;

c) is first reacted with a strong base such as an organolithium reagent to give an intermediate metal carbanion, which is then quenched with an electrophile to give a compound of formula (I), in which Y is alkyl, haloalkyl, alkenyl, alkynyl, cyanoalkyl or formyl;

d) is converted to the carbanion, as above in part c), and then quenched with sulfuryl chloride to give an intermediate compound, in which Y is chlorosulfonyl, which then is reacted with ammonia or an alkyl- or dialkylamine to give a compound of formula (I), in which Y is aminosulfonyl, alkylaminosulfonyl or dialkylaminosulfonyl;

e) is converted to the carbanion as above in part c) or optionally the carbanion is prepared via the compound in which Y is halogen, obtained by the procedure of (E) above and then the carbanion is reacted in a similar manner to the procedure described in (B)c above to give a compound of formula (I), in which Y is hydroxyl and salts thereof, alkoxy or haloalkoxy ; or

(H) conforming to the formula:

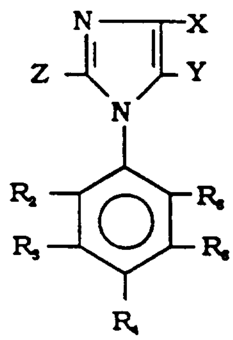

(I)

wherein X, Z, $R_2$, $R_3$ $R_4$, $R_5$ and $R_6$ are as defined for formula (I)

and Y is carboxyl and salts thereof, cyano, aminocarbonyl, alkylaminocarbonyl, dialkylaminocarbonyl, alkoxycarbonyl, haloalkyl, alkenyl, alkynyl, alkylcarbonyl or haloalkylcarbonyl, wherein a compound of formula (Ib),

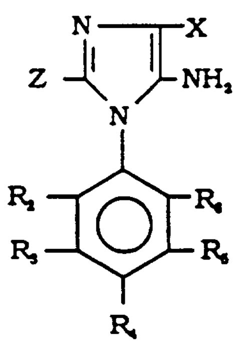

(Ib)

in which X, Z and $R_2$ to $R_6$ are as defined above is first deaminated according to the procedures described in (E) above to give a compound, in which Y is hydrogen, which is then converted by procedures described in (G)c above to give a compound of formula (I), in which Y is formyl, then said formyl compound, in which X and Z are optionally protected as required:

a) is reacted by similar procedures described in C(b) above to give a compound of formula (I), in which Y is carboxyl and salts thereof or cyano;

b) is reacted by similar procedures described in C(c) above to give a compound of formula (I), in which Y is aminocarbonyl, alkylaminocarbonyl, dialkylaminocarbonyl or alkoxycarbonyl;

c) is reacted by similar procedures described in C(d) above to give a compound of formula (I), in which Y is haloalkyl, more specifically halomethyl;

d) is reacted by similar procedures described in (B)b above and 19e to give a compound of formula (I), in which Y is alkenyl or alkynyl; or

e) is reacted by similar procedures described in (C)f above to give a compound of formula (I), in which Y is alkylcarbonyl or haloalkylcarbonyl; or

(I) conforming to the formula:

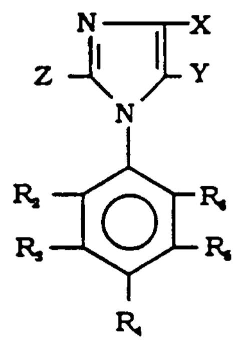

(I)

wherein Y, Z, $R_2$, $R_3$, $R_4$, $R_5$ and $R_6$ are as defined for formula (I)

and X is alkylsulfenyl, haloalkylsulfenyl, alkylsulfinyl, haloalkylsulfinyl, alkylsulfonyl or haloalkylsulfonyl, wherein a compound of formula (Ic),

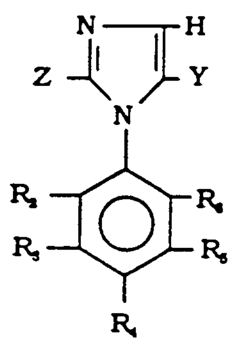

(Ic)

in which Y and Z are optionally protected as required:

a) is first reacted according to procedures similar to those described in (B)c above to convert a compound of formula (Ic), in which X is hydrogen, to an intermediate compound of formula (I), in which X is successively thiocyano and then a disulfide, then also by procedures similar to those described in (B)c above , the thiocyano or disulfide intermediate is converted to a compound of formula (I), in which X is alkylsulfenyl or haloalkylsulfenyl, particularly perhaloalkylsulfenyl, which compound then is optionally oxidized by procedures similar to those in (B)c above, to obtain the sulfoxide or sulfone analog, that is a compound of formula (I), in which X is alkylsulfinyl, haloalkylsulfinyl, preferably perhaloalkylsulfinyl, alkylsulfonyl or haloalkylsulfonyl, particularly perhaloalkylsulfonyl; or

b) is first reacted according to procedures similar to those described in (B)a above to convert the compound of formula (Ic), in which X is hydrogen to an intermediate compound of formula (I), in which X is chlorosulfonyl, then the chlorosulfonyl compound is reacted with a reducing agent such as triphenylphosphine to give the same disulfide intermediate described above in part a), then finally the disulfide is converted by the procedures described above in part a) to give a compound of formula (I), in which X is alkylsulfenyl or haloalkylsulfenyl, particularly perhaloalkylsulfenyl or optionally the sulfenyl compound is oxidized to give a compound of formula (I), in which X is alkylsulfinyl, haloalkylsulfinyl, particularly perhaloalkylsulfinyl, alkylsulfonyl or haloalkylsulfonyl, particularly perhaloalkylsulfonyl; or

(J) conforming to the formula:

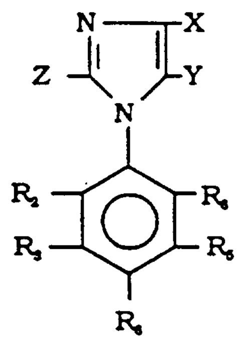

(IV)

wherein:

| | |
|---|---|
| $R_2$, $R_3$, $R_4$, $R_5$ and $R_6$ | are as hereinbefore defined; |
| X | is hydrogen or haloalkyl, particularly trifluoromethyl; |
| Y | is amino; hydroxy, optionally existing in its isomeric keto form when X is hydrogen; alkoxy; or haloalkoxy; and |
| Z | is hydrogen; halogen; alkyl; haloalkyl; hydroxy, optionally existing in its isomeric keto form when X is hydrogen and Y is imino; alkoxy; or haloalkoxy; |

whereby a compound of formula (III),

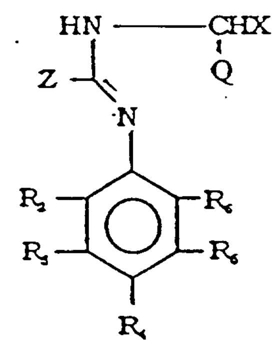

(III)

wherein

| | |
|---|---|
| $R_2$, $R_3$, $R_4$, $R_5$ and $R_6$ | are as defined above; |
| X | is hydrogen or haloalkyl, particularly trifluoromethyl; |
| Z | is hydrogen, halogen, alkyl, haloalkyl or hydroxy, optionally existing in its isomeric keto form; and |
| Q | is cyano or lower alkoxycarbonyl; |

is reacted with a basic agent in a suitable reaction medium to give a compound of formula (IV), which when Y or Z is hydroxy, is then optionally alkylated or haloalkylated to Y or Z is alkoxy or haloalkoxy.

**2.** The process of preparation of a compound of formula (IV), according to Claim 1j wherein the compound of formula (IV) is:

a) a compound of formula (5), depicted in Claim 1 in which X and Z are each hydrogen and Y is amino;

b) a compound of formula (17),

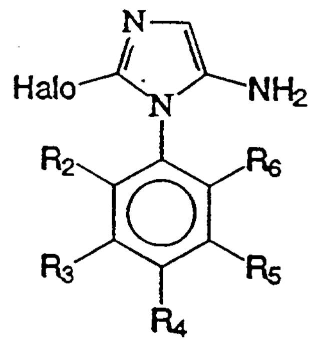

(17)

in which Halo means halogen, particularly chlorine;

c) a compound of formula (22),

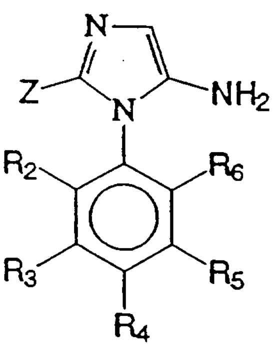

(22)

in which Z is alkyl or haloalkyl;

d) a compound of formula (27),

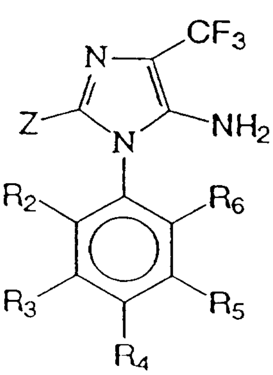

(27)

in which Z is halogen, alkyl, or haloalkyl;

e) a compound of formula (30), in which X is hydrogen and Y is hydroxyl optionally existing in its isomeric keto form (29),

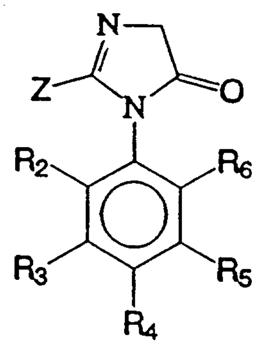

(29)

in which Z is halogen, alkyl or haloalkyl; or

f) a compound of formula (37), in which Y is amino and Z is hydroxy, optionally existing in its isomeric keto-imino form (34),

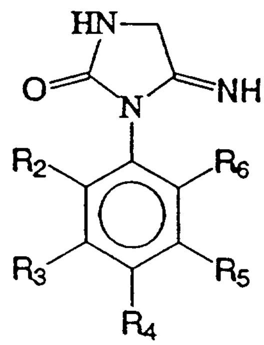

(34)

**3.** A process of preparation of a compound of formula (I):

(A) wherein X, Y, Z, $R_2$, $R_3$, $R_4$, $R_5$ and $R_6$ are as defined in Claim 1, wherein a compound of formula (5) depicted in Claim 1 is reacted according to the process of preparation of Claim 1 for introduction of the X, Y and Z substituents;

(B) wherein X, Y, $R_2$, $R_3$, $R_4$, $R_5$ and $R_6$ are as defined in Claim 1 and Z is halogen, whereby a compound of formula (17) depicted in Claim 2 is reacted according to the process of preparation of Claim 1 for introduction of the X and Y substituents;

(C) wherein X, Y, $R_2$, $R_3$, $R_4$, $R_5$ and $R_6$ are as defined in Claim 1 and Z is alkyl or haloalkyl, whereby a compound of formula (22) depicted in Claim 2 is reacted according to the process of preparation of Claim 1 for introduction of the X and Y substituents;

(D) wherein Y, $R_2$, $R_3$, $R_4$, $R_5$ and $R_6$ are as defined in Claim 1, X is haloalkyl, particularly trifluoromethyl, and Z is halogen, alkyl or haloalkyl, whereby a compound of formula (27) is reacted according to the process of Claim 1 for introduction of the Y substituent;

(E) wherein X, $R_2$, $R_3$, $R_4$, $R_5$ and $R_6$ are as defined in Claim 1, Y is hydroxy, alkoxy or haloalkoxy, and Z is halogen, alkyl or haloalkyl, whereby a compound of formula (30), optionally existing in its isomeric keto form (29), depicted in Claim 2 in which Y is hydroxy, optionally alkylated to Y is alkoxy or haloalkoxy, is reacted according to the process of Claim 1 for introduction of the X substituent; or

(F) wherein X, Y, $R_2$, $R_3$, $R_4$, $R_5$ and $R_6$ are as defined in Claim 1 and Z is hydroxy, alkoxy, haloalkoxy or halogen, whereby a compound of formula (37), optionally existing in its isomeric keto-imino form (34), depicted in Claim 2 in which Z is hydroxy, optionally alkylated to Z is alkoxy or haloalkoxy or optionally halogenated to Z is halogen, is reacted according to the process of Claim 1 for introduction of the X and Y substitutents.

**4.** A process according to claim 1, wherein X is haloalkoxy.

**5.** A process according to claim 1, wherein X is $S(O)_nR_1$, having the formula (II)

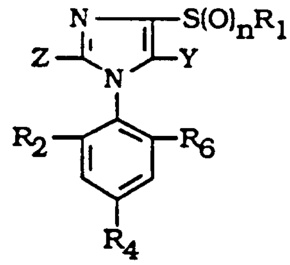

(II)

wherein:

| | |
|---|---|
| Y and Z | are individually selected from a hydrogen or halogen atom; a group selected from nitro, cyano, hydroxyl, sulfhydryl, amino, alkylamino or dialkylamino, in which the defined alkyl moiety of each group is a linear or branched chain containing one to four carbon atoms; or a group selected from an unsubstituted or fully halo-substituted alkyl, alkoxy, alkylcarbonyl, alkylcarbonylamino, alkylsulfenyl, alkylsulfinyl or alkylsulfonyl, in which the defined alkyl and alkoxy moieties of each group are a linear or branched chain, containing one to four carbon atoms, and the full halo-substitution of the alkyl or alkoxy moiety is by the same or different halogen atoms; and only one of Y and Z is a sulfur containing group; |
| $R_1$ | is a linear or branched alkyl group of one to four carbon atoms which are unsubstituted or halo-substituted by one or more halogen atoms, which are the same or different; |
| $R_2$ | is a hydrogen or a halogen atom or an alkyl, alkoxy, methylsulfenyl, methylsulfinyl or methylsulfonyl group; |
| $R_4$ | is selected from a halogen atom or a group selected from trifluoromethyl, trifluoromethoxy, difluoromethoxy, trifluoromethylsulfenyl, trifluoromethylsulfinyl, trifluoromethylsulfonyl, or a linear or branched chain alkyl group containing one to four carbon atoms; |
| $R_6$ | is a halogen atom; and |
| n | is 0, 1 or 2. |

**6.** A process according to Claim 5 wherein:

| | |
|---|---|
| Y | is a hydrogen atom, a halogen atom, amino, hydroxy, alkoxy of one to four carbon atoms, methylsulfenyl, methylsulfinyl or methylsulfonyl; |
| Z | is a hydrogen atom, a halogen atom, or a linear or branched alkyl group of one to four carbon atoms which is optionally fully substituted by halogen atoms which are the same or different; |
| $R_1$ | is a methyl group fully substituted by halogen atoms which are the same or different; |
| $R_2$ | is a hydrogen atom, a halogen atom or methylsulfenyl; |
| $R_4$ | is a halogen atom, trifluoromethyl or trifluoromethoxy; and |
| $R_6$ | is a fluorine, chlorine or bromine atom. |

**7.** A process according to Claim 6 wherein:

| | |
|---|---|
| Y | is a hydrogen atom, a chlorine atom, a bromine atom, methylsulfenyl, methylsulfinyl or methoxy; |
| Z | is a hydrogen atom, a chlorine atom, a bromine atom or methyl; |
| $R_1$ | is trifluoromethyl, dichlorofluoromethyl or chlorodifluoromethyl; |
| $R_2$ | is a hydrogen atom, a chlorine atom, a bromine atom or methylsulfenyl; |
| $R_4$ | is a chlorine atom, a bromine atom, a fluorine atom, trifluoromethyl or trifluoromethoxy; and |
| $R_6$ | is a chlorine or bromine atom. |

**8.** A process according to Claim 7, for the preparation of a compound of formula I which is

1-(2,6-dichloro-4-trifluoromethylphenyl)-2-chloro-4-trifluoromethylsulfenylimidazole;

1-(2,6-dichloro-4-trifluoromethylphenyl)-2-chloro-4-trifluoromethylsulfinylimidazole;

1-(2,6-dichloro-4-trifluoromethylphenyl)-5-bromo-4-dichlorofluoromethylsulfenylimidazole;

1-(2,6-dichloro-4-trifluoromethylphenyl)-2-chloro-4-dichlorofluoromethylsulfenylimidazole;
1-(2,6-dichloro-4-trifluoromethylphenyl)-2-bromo-4-dichlorofluoromethylsulfenylimidazole;
1-(2,6-dichloro-4-trifluoromethylphenyl)-4-trifluoromethylsulfinylimidazole;
1-(2,6-dichloro-4-trifluoromethylphenyl)-2-chloro-4-dichlorofluoromethylsulfinylimidazole;
1-(2,6-dichloro-4-trifluoromethylphenyl)-2-chloro-4-dichlorofluoromethylsulfonylimidazole;
1-(2,6-dichloro-4-trifluoromethylphenyl)-2-bromo-4-chlorodifluoromethylsulfinylimidazole;
1-(2,6-dichloro-4-trifluoromethylphenyl)-2-bromo-4-chlorodifluoromethylsulfenylimidazole;
1-(2,6-dichloro-4-trifluoromethylphenyl)-2-chloro-4-chlorodifluoromethylsulfenylimidazole;
1-(6-chloro-2-methylsulfenyl-4-trifluoromethylphenyl)-2-bromo-4-chlorodifluoromethylsulfonylimidazole;
1-(6-chloro-2-methylsulfenyl-4-trifluoromethylphenyl)-2-chloro-4-dichlorofluoromethylsulfonylimidazole;
1-(2,6-dichloro-4-trifluoromethylphenyl)-4-dichlorofluoromethylsulfenylimidazole;
1-(2,6-dichloro-4-trifluoromethylphenyl)-4-dichlorofluoromethylsulfinylimidazole;
1-(2,6-dichloro-4-trifluoromethylphenyl)-4-dichlorofluoromethylsulfonylimidazole;
1-(2,6-dichloro-4-trifluoromethylphenyl)-2-methyl-4-dichlorofluoromethylsulfenylimidazole;
1-(2,6-dichloro-4-trifluoromethylphenyl)-2-methyl-4-dichlorofluoromethylsulfinylimidazole;
1-(2,6-dichloro-4-trifluoromethylphenyl)-2-methyl-4-dichlorofluoromethylsulfonylimidazole;
1-(2,4,6-trichlorophenyl)-4-dichlorofluoromethylsulfenylimidazole;
1-(2,4,6-trichlorophenyl)-4-dichlorofluoromethylsulfinylimidazole;
1-(2,6-dichloro-4-trifluoromethoxyphenyl)-4-dichlorofluoromethylsulfinylimidazole;
1-(2,6-dichloro-4-trifluoromethylphenyl)-4-chlorodifluoromethylsulfinylimidazole;
1-(2,6-dichloro-4-trifluoromethylphenyl)-2-methyl-4-chlorodifluoromethylsulfinylimidazole;
1-(2,6-dichloro-4-trifluoromethylphenyl)-2-methyl-4-chlorodifluoromethylsulfonylimidazole;
1-(2,4,6-trichlorophenyl)-4-chlorodifluoromethylsulfenylimidazole; or
1-(2,4,6-trichlorophenyl)-4-chlorodifluoromethylsulfinylimidizole.

**9.** A process according to Claim 7 for the preparation of a compound of formula (I) which is:
1-(2,6-dichloro-4-trifluoromethylphenyl)-2-chloro-4-trifluoromethylsulfonylimidazole;
1-(2,6-dichloro-4-trifluoromethylphenyl)-5-methylsulfenyl-4-dichlorofluoromethylsulfenylimidazole;
1-(2,6-dichloro-4-trifluoromethylphenyl)-4-trifluoromethylsulfonylimidazole;
1-(2,6-dichloro-4-trifluoromethylphenyl)-5-bromo-4-trifluoromethylsulfonylimidazole;
1-(2,6-dichlor-4-trifluoromethylphenyl)-2-bromo-4-dichlorofluoromethylsulfinylimidazole;
1-(2,6-dichloro-4-trifluoromethylphenyl)-5-bromo-4-dichlorofluoromethylsulfonylimidazole;
1-(2,6-dichloro-4-trifluoromethylphenyl)-2-bromo-4-dichlorofluoromethylsulfonylimidazole;
1-(2,6-dichloro-4-trifluoromethylphenyl)-2-bromo-4-chlorodifluoromethylsulfonylimidazole;
1-(2,6-dichloro-4-trifluoromethylphenyl)-2-chloro-4-chlorodifluoromethylsulfinylimidazole;
1-(2,6-dichloro-4-trifluoromethylphenyl)-2-chloro-4-chlorodifluoromethylsulfonylimidazole;
1-(2,6-dichloro-4-trifluoromethylphenyl)-5-methylsulfonyl-4-dichlorofluoromethylsulfinylimidazole;
1-(2,6-dichloro-4-trifluoromethyl)-5-methylsulfinyl-4-dichlorofluoromethylsulfinylimidazole;
1-(6-chloro-2-methylsulfenyl-4-trifluoromethylphenyl)-2-chloro-4-dichlorofluoromethylsulfenylimidazole;
1-(6-chloro-2-methylsulfenyl-4-trifluoromethylphenyl)-2-chloro-4-dichlorofluoromethylsulfinylimidazole;
1-(2,6-dichloro-4-trifluoromethylphenyl)-2-chloro-5-methylsulfenyl-4-trifluoromethylsulfenylimidazole;
1-(2,6-dichloro-4-trifluoromethylphenyl)-5-bromo-4-trifluoromethylsulfenylimidazole;
1-(2,6-dichloro-4-trifluoromethylphenyl)-2-methyl-4-chlorodifluoromethylsulfenylimidazole;
1-(2,6-dichloro-4-trifluoromethylphenyl)-5-chloro-4-trifluoromethylsulfenylimidazole;
1-(2,6-dichloro-4-trifluoromethylphenyl)-2,5-dichloro-4-dichlorofluoromethylsulfenylimidazole;
1-(2,6-dichloro-4-trifluoromethylphenyl)-4-chlorodifluoromethylsulfenylimidazole;
1-(2-chloro-4-trifluoromethylphenyl)-4-dichlorofluoromethylsulfinylimidazole;
1-(2-chloro-4-trifluoromethylphenyl)-4-trifluoromethylsulfinylimidazole;
1-(2-chloro-4-trifluoromethylphenyl)-4-trifluoromethylsulfonylimidazole;
1-(2,6-dichloro-4-bromophenyl)-4-dichlorofluoromethylsulfenylimidazole;
1-(2,6-dichloro-4-bromophenyl)-4-dichlorofluoromethylsulfinylimidazole;
1-(2,6-dichloro-4-bromophenyl)-4-chlorodifluoromethylsulfenylimidazole;
1-(2,6-dichloro-4-trifluoromethoxyphenyl)-4-trifluoromethylsulfinylimidazole;
1-(2,6-dichloro-4-trifluoromethoxyphenyl)-4-chlorodifluoromethylsulfenylimidazole;

1-(2,6-dichloro-4-trifluoromethoxyphenyl)-4-chlorodifluoromethylsulfinylimidazole;
1-(2,6-dichloro-4-trifluoromethoxyphenyl)-4-dichlorofluoromethylsulfonylimidazole; or
1-(2,6-dichloro-4-trifluoromethoxyphenyl)-2-bromo-4-dichlorofluoromethylsulfenylimidazole.

**10.** A process for the preparation of a composition for the control of arthropod, nematode, helminth, or protozoan pests which comprises formulating an effective amount, preferably about 0.05% to about 95% by weight, of a compound of formula (I) as defined in any one of Claims 1 to 9 but including compounds wherein (i) X is trifluoromethyl when $R_3$ represents methyl, $R_4$ represents nitro or amino and the other symbols represent hydrogen and (ii) X is chloromethyl when $R_4$ represents methyl, methoxy or ethoxy and the other symbols represent hydrogen as an active ingredient and one or more agronomically or medicinally compatible components preferably comprising about 1% to about 95%, by weight, of one or more solid or liquid carriers and about 0.1% to about 50%, by weight, or one or more additional components such as surface active agents.

**11.** A process for the preparation of a composition particularly useful in veterinary medicine and livestock husbandry or in the maintenance of public health comprising formulating one or more compatible components and as an active ingredient at least one compound of formula (I) as defined in Claim 10.

**Patentansprüche**
**Patentansprüche für folgende Vertragsstaaten : AT, BE, CH, LI, DE, DK, FR, GB, GR, IT, LU, NL, SE**

**1.** Verbundung der Formel (I)

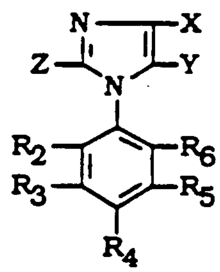

(I)

worin:

X eine aus Halogenalkyl oder Halogenalkoxy gewählte Gruppe oder eine aus Alkylsulfenyl, Alkylsulfinyl oder Alkylsulfonyl gewählte, unsubstituierte oder Halogensubstituierte Gruppe ist, worin die angegebenen Alkyl- und Alkoxyteile jeder Gruppe eine lineare oder verzweigte Kette mit ein bis vier Kohlenstoffatomen darstellen und die Halogensubstitution jeder Gruppe ein oder mehrere Halogenatome, welche gleich oder verschieden sind, bis zur vollständigen Substitution des Alkyl- oder Alkoxyteils umfaßt;

Y und Z jeweils individuell gewählt sind aus: einem Wasserstoff- oder einem Halogenatom; einer aus Nitro, Cyano, Hydroxyl gewählten Gruppe und annehmbaren Salzen davon, Sulfhydryl und annehmbaren Salzen davon, Formyl, Hydroxycarbonyl und annehmbaren Salzen davon, Alkoxycarbonyl, Aminocarbonyl, Alkylaminocarbonyl, Dialkylaminocarbonyl, Amino, Alkylamino, Dialkylamino, einem Trialkylammoniumsalz, Cyanoalkyl, Alkoxycarbonylamino, Arylcarbonylamino, Alkylaminocarbonylamino, Dialkylaminocarbonylamino, Aminosulfonyl, Alkylaminosulfonyl, Dialkylaminosulfonyl oder Alkoxyalkylidenimino, wobei die angegebenen Alkyl- und Alkoxyteile in jeder Gruppe lineare oder verzweigte Ketten mit ein bis vier Kohlenstoffatomen sind; einer linearen oder verzweigtkettigen Alkenyl- oder Alkynylgruppe mit zwei bis vier Kohlenstoffatomen; oder einer aus einem unsubstituierten oder Halogen-substituierten Alkyl, Alkoxy, Alkylcarbonyl, Alkylcarbonylamino, Alkylsulfenyl, Alkylsulfinyl oder Alkylsulfonyl gewählte Gruppe, wobei die angegebenen Alkyl- und Alkoxyteile jeder Gruppe eine lineare oder verzweigte Kette mit ein bis vier Kohlenstoffatomen bedeuten und die Halogen-Substitution aus einem oder mehreren Halogenatomen, welche gleich oder verschieden sind, bis zur vollständigen Substitution des Alkyl- oder Alkoxyteils besteht; und nur eines von Y und Z eine schwefelhaltige Gruppe ist; und $R_2$, $R_3$, $R_4$, $R_5$ und $R_6$ individuell ausgewählt sind aus:

einem Wasserstoff- oder Halogenatom; einer aus Nitro, Cyano, Amino, Alkylamino oder Dialkylamino gewählten Gruppe, wobei der Alkylteil jeder Gruppe eine lineare oder verzweigte Kette mit ein bis vier Kohlenstoffatomen ist; eine lineare oder verzweigtkettige Alkenyl- oder Alkynylgruppe mit zwei bis vier Kohlenstoffatomen, welche mit ein oder mehreren Halogenatomen, die gleich oder verschieden sind, bis zur vollständigen Substitution substituiert sein kann; oder eine aus einem unsubstituierten oder Halogen-substituierten Alkyl, Alkoxy, Alkylsulfenyl, Alkylsulfinyl oder Alkylsulfonyl gewählten Gruppe, wobei die definierten Alkyl- und Alkoxyteile in jeder Gruppe eine lineare oder verzweigte Kette mit ein bis vier Kohlenstoffatomen ist und die Halogen-Substitution ein oder mehrere Halogenatome, die gleich oder verschieden sind, bis zur vollständigen Substitution des Alkyl- oder Alkoxyteils umfaßt; mit der Maßgabe, daß (i) X etwas anderes als Trifluormethyl bedeutet, wenn $R_3$ für Methyl steht, $R_4$ Nitro oder Amino bedeutet und die anderen Symbole Wasserstoff repräsentieren und (ii) X etwas anderes als Chlormethyl ist, wenn $R_4$ Methyl, Methoxy oder Ethoxy bedeutet und die anderen Symbole Wasserstoff repräsentieren.

**2.** Verbindung nach Anspruch 1, worin X Halogenalkoxy ist.

**3.** Verbindung nach Anspruch 1, worin X $S(O)_nR_1$ ist mit der Formel (II)

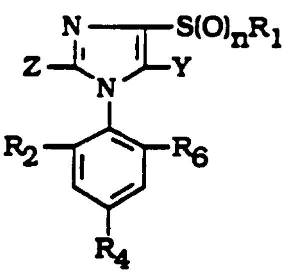

(II)

worin:

| | |
|---|---|
| Y und Z | individuell gewählt sind aus einem Wasserstoff- oder Halogenatom; einer aus Nitro, Cyano, Hydroxyl, Sulfhydryl, Amino, Alkylamino oder Dialkylamino gewählten Gruppe, wobei der angegebene Alkylteil jeder Gruppe eine lineare oder verzweigte Kette mit ein bis vier Kohlenstoffatomen ist; oder einer aus einem unsubstituierten oder vollständig Halogen-substituierten Alkyl, Alkoxy, Alkylcarbonyl, Alkylcarbonylamino, Alkylsulfenyl, Alkylsulfinyl oder Alkylsulfonyl gewählten Gruppe, worin die definierten Alkyl- und Alkoxyteile jeder Gruppe eine lineare oder verzweigte Kette mit ein bis vier Kohlenstoffatomen bedeuten und die vollständige Halogen-Substitution des Alkyl- oder Alkoxyteils in gleichen oder unterschiedlichen Halogenatomen besteht; und wobei nur eines von Y und Z eine schwefelhaltige Gruppe ist; |
| $R_1$ | eine lineare oder verzweigte Alkylgruppe mit ein bis vier Kohlenstoffatomen ist, welche unsubstituiert oder halogensubstituiert mit ein oder mehreren Halogenatomen, welche gleich oder verschieden sind, ist; |
| $R_2$ | ein Wasserstoff- oder ein Halogenatom oder eine Alkyl-, Alkoxy-, Methylsulfenyl-, Methylsulfinyl- oder Methylsulfonylgruppe ist; |
| $R_4$ | aus einem Halogenatom gewählt ist oder eine aus Trifluormethyl, Trifluormethoxy, Difluormethoxy, Trifluormethylsulfenyl, Trifluormethylsulfinyl, Trifluormethylsulfonyl gewählte Gruppe ist oder eine lineare oder verzweigtkettige Alkylgruppe mit ein bis vier Kohlenstoffatomen ist; |
| $R_6$ | ein Halogenatom ist; und |
| n | 0, 1 oder 2 ist. |

**4.** Verbindung nach Anspruch 3, worin:

| | |
|---|---|
| Y | ein Wasserstoffatom, ein Halogenatom, Amino, Hydroxy, Alkoxy mit ein bis vier Kohlenstoffatomen, Methylsulfenyl, Methylsulfinyl oder Methylsulfonyl ist; |
| Z | ein Wasserstoffatom, ein Halogenatom oder eine lineare oder verzweigte Alkylgruppe mit ein |

bis vier Kohlenstoffatomen, welche gegebenenfalls vollständig durch Halogenatome, welche gleich oder verschieden sein können, substituiert ist, bedeutet;

$R_1$ eine vollständig durch Halogenatome, welche gleich oder verschieden sind, substituierte Methylgruppe ist;

$R_2$ ein Wasserstoffatom, ein Halogenatom oder eine Methylsulfenylgruppe ist;

$R_4$ ein Halogenatom, Trifluormethyl oder Trifluormethoxy; und

$R_6$ ein Fluor-, Chlor- oder Bromatom ist.

**5.** Verbindung nach Anspruch 4, worin:

Y ein Wasserstoffatom, ein Chloratom, ein Bromatom, Methylsulfenyl, Methylsulfinyl oder Methoxy ist;

Z ein Wasserstoffatom, ein Chloratom, ein Bromatom oder Methyl ist;

$R_1$ Trifluormethyl, Dichlorfluormethyl oder Chlordifluormethyl ist;

$R_2$ ein Wasserstoffatom, ein Chloratom, ein Bromatom oder Methylsulfenyl ist;

$R_4$ ein Chloratom, ein Bromatom, ein Fluoratom, Trifluormethyl oder Trifluormethoxy ist; und

$R_6$ ein Chlor- oder Bromatom ist.

**6.** Verbindung nach Anspruch 5, welche ist:

1-(2,6-Dichlor-4-trifluormethylphenyl)-2-chlor-4-trifluormethylsulfenylimidazol;
1-(2,6-Dichlor-4-trifluormethylphenyl)-2-chlor-4-trifluormethylsulfinylimidazol;
1-(2,6-Dichlor-4-trifluormethylphenyl)-5-brom-4-dichlorfluormethylsulfenylimidazol;
1-(2,6-Dichlor-4-trifluormethylphenyl)-2-chlor-4-dichlorfluormethylsulfenylimidazol;
1-(2,6-Dichlor-4-trifluormethylphenyl)-2-brom-4-dichlorfluormethylsulfenylimidazol;
1-(2,6-Dichlor-4-trifluormethylphenyl)-4-chlor-4-trifluormethylsulfinylimidazol;
1-(2,6-Dichlor-4-trifluormethylphenyl)-2-chlor-4-dichlorfluormethylsulfinylimidazol;
1-(2,6-Dichlor-4-trifluormethylphenyl)-2-chlor-4-dichlorfluormethylsulfonylimidazol;
1-(2,6-Dichlor-4-trifluormethylphenyl)-2-brom-4-chlordifluormethylsulfinylimidazol;
1-(2,6-Dichlor-4-trifluormethylphenyl)-2-brom-4-chlordifluormethylsulfenylimidazol;
1-(2,6-Dichlor-4-trifluormethylphenyl)-2-chlor-4-chlordifluormethylsulfenylimidazol;
1-(6-Chlor-2-methylsulfenyl-4-trifluormethylphenyl)-2-brom-4-chlordifluormethylsulfonylimidazol;
1-(6-Chlor-2-methylsulfenyl-4-trifluormethylphenyl)-2-chlor-4-dichlorfluormethylsulfonylimidazol;
1-(2,6-Dichlor-4-trifluormethylphenyl)-4-dichlorfluormethylsulfenylimidazol;
1-(2,6-Dichlor-4-trifluormethylphenyl)-4-dichlorfluormethylsulfinylimidazol;
1-(2,6-Dichlor-4-trifluormethylphenyl)-4-dichlorfluormethylsulfonylimidazol;
1-(2,6-Dichlor-4-trifluormethylphenyl)-2-methyl-4-dichlorfluormethylsulfenylimidazol;
1-(2,6-Dichlor-4-trifluormethylphenyl)-2-methyl-4-dichlorfluormethylsulfinylimidazol;
1-(2,6-Dichlor-4-trifluormethylphenyl)-2-methyl-4-dichlorfluormethylsulfonylimidazol;
1-(2,4,6-Trichlorphenyl)-4-dichlorfluormethylsulfenylimidazol;
1-(2,4,6-Trichlorphenyl)-4-dichlorfluormethylsulfinylimidazol;
1-(2,6-Dichlor-4-trifluormethoxyphenyl)-4-dichlorfluormethylsulfinylimidazol;
1-(2,6-Dichlor-4-trifluormethylphenyl)-4-chlordifluormethylsulfinylimidazol;
1-(2,6-Dichlor-4-trifluormethylphenyl)-2-methyl-4-chlordifluormethylsulfinylimidazol;
1-(2,6-Dichlor-4-trifluormethylphenyl)-2-methyl-4-chlordifluormethylsulfonylimidazol;
1-(2,4,6-Trichlorphenyl)-4-chlordifluormethylsulfenylimidazol; oder
1-(2,4,6-Trichlorphenyl)-4-chlordifluormethylsufinylimidazol;

**7.** Verbindung nach Anspruch 5, welche ist:

1-(2,6-Dichlor-4-trifluormethylphenyl)-2-chlor-4-trifluormethylsulfonylimidazol;
1-(2,6-Dichlor-4-trifluormethylphenyl)-5-methylsulfenyl-4-dichlorfluormethylsulfenylimidazol;
1-(2,6-Dichlor-4-trifluormethylphenyl)-4-trifluormethylsulfonylimidazol;
1-(2,6-Dichlor-4-trifluormethylphenyl)-5-brom-4-trifluormethylsulfonylimidazol;
1-(2,6-Dichlor-4-trifluormethylphenyl)-2-brom-4-dichlorfluormethylsulfinylimidazol;
1-(2,6-Dichlor-4-trifluormethylphenyl)-5-brom-4-dichlorfluormethylsulfonylimidazol;
1-(2,6-Dichlor-4-trifluormethylphenyl)-2-brom-4-dichlorfluormethylsulfonylimidazol;
1-(2,6-Dichlor-4-trifluormethylphenyl)-2-brom-4-chlordifluormethylsulfonylimidazol;
1-(2,6-Dichlor-4-trifluormethylphenyl)-2-chlor-4-chlordifluormethylsulfinylimidazol;
1-(2,6-Dichlor-4-trifluormethylphenyl)-2-chlor-4-chlordifluormethylsulfonylimidazol;
1-(2,6-Dichlor-4-trifluormethylphenyl)-5-methylsulfonyl-4-dichlorfluormethylsulfinylimidazol;

1-(2,6-Dichlor-4-trifluormethylphenyl)-5-methylsulfinyl)-4-dichlorfluormethylsulfinylimidazol;
1-(6-Chlor-2-methylsulfenyl-4-trifluormethylphenyl)-2-chlor-4-dichlorfluormethylsulfenylimidazol;
1-(6-Chlor-2-methylsulfenyl-4-trifluormethylphenyl)-2-chlor-4-dichlorfluormethylsulfinylimidazol;
1-(2,6-Dichlor-4-trifluormethylphenyl)-2-chlor-5-methylsulfenyl-4-trifluormethylsulfenylimidazol;
1-(2,6-Dichlor-4-trifluormethylphenyl)-5-brom-4-trifluormethylsulfenylimidazol;
1-(2,6-Dichlor-4-trifluormethylphenyl)-2-methyl-4-chlordifluormethylsulfenylimidazol;
1-(2,6-Dichlor-4-trifluormethylphenyl)-5-chlor-4-trifluormethylsulfenylimidazol;
1-(2,6-Dichlor-4-trifluormethylphenyl)-2,5-dichlor-4-dichlorfluormethylsulfenylimidazol;
1-(2,6-Dichlor-4-trifluormethylphenyl)-4-chlordifluormethylsulfenylimidazol;
1-(2-Chlor-4-trifluormethylphenyl)-4-dichlorfluormethylsulfinylimidazol;
1-(2-Chlor-4-trifluormethylphenyl)-4-trifluormethylsulfinylimidazol;
1-(2-Chlor-4-trifluormethylphenyl)-4-trifluormethylsulfonylimidazol;
1-(2,6-Dichlor-4-bromphenyl)-4-dichlorfluormethylsulfenylimidazol;
1-(2,6-Dichlor-4-bromphenyl)-4-dichlorfluormethylsulfinylimidazol;
1-(2,6-Dichlor-4-bromphenyl)-4-chlordifluormethylsulfenylimidazol;
1-(2,6-Dichlor-4-trifluormethoxyphenyl)-4-trifluormethylsulfinylimidazol;
1-(2,6-Dichlor-4-trifluormethoxyphenyl)-4-chlordifluormethylsulfenylimidazol;
1-(2,6-Dichlor-4-trifluormethoxyphenyl)-4-chlordifluormethylsulfinylimidazol;
1-(2,6-Dichlor-4-trifluormethoxyphenyl)-4-dichlorfluormethylsulfonylimidazol; oder
1-(2,6-Dichlor-4-trifluormethoxyphenyl)-2-brom-4-dichlorfluormethylsulfenylimidazol.

**8.** Verfahren zur Herstellung einer Verbindung der Formel I:
(A) entsprechend der Formel:

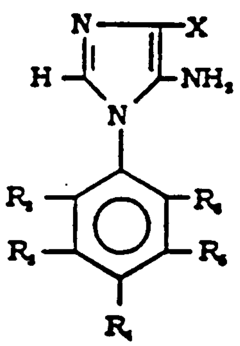

(Ia)

worin $R_2$, $R_3$, $R_4$, $R_5$ und $R_6$ die gleiche Bedeutung wie für die Formel (I) in Anspruch 1 besitzen und X Alkylsulfenyl, Halogenalkylsulfenyl, Alkylsulfinyl, Halogenalkylsulfinyl, Alkylsulfonyl, Halogenalkylsulfonyl, Halogenalkyl oder Halogenalkoxy ist, wobei eine Verbindung der Formel (5)

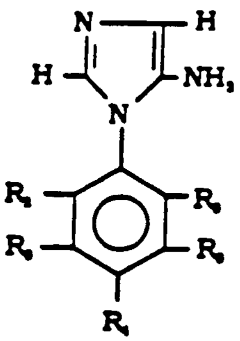

5

worin Amino gegebenenfalls wie erforderlich geschützt ist:
a) zuerst mit einem Sulfenylhalogenid, $R_1$SHalogen, worin $R_1$ Alkyl oder Halogenalkyl ist, in einem organischen Reaktionsmedium, gegebenenfalls in Gegenwart eines Säureakzeptors, wie eines tertiären Amins, umgesetzt wird, um eine Verbindung der Formel (Ia) zu erhalten, worin X

Alkylsulfenyl oder Halogenalkylsulfenyl ist, welches dann gegebenenfalls durch bekannte Verfahren, wie durch ein Peroxid, oxidiert wird, um eine Verbindung der Formel (Ia) zu erhalten, worin X $S(O)_nR_1$ ist, worin n 1 oder 2 ist und $R_1$ die gleiche Bedeutung wie oben hat, d.h. X Alkylsulfinyl, Halogenalkylsulfinyl, Alkylsulfonyl oder Halogenalkulsulfonyl ist;

b) zuerst mit einem Tris(alkylthio)methan oder Tris(arylthio)methan in einem organischen Reaktionsmedium in Gegenwart einer Lewis-Säure und gegebenenfalls in Gegenwart eines Säureakzeptors umgesetzt wird, dann die erhaltene Zwischenverbindung der Formel (Ia), in der X Bis-(alkylthio)methyl oder Bis(arylthio)methyl ist, in einem organischen Reaktionsmedium mit einem geeigneten Alkylnitrid gefolgt von einem Hydrolysevorgang umgesetzt wird, um eine Zwischenverbindung der Formel (Ia) zu erhalten, in der X Formyl ist, wonach bekannte Reduktionsvorgänge durchgeführt werden, um die Zwischenverbindung zu erhalten, in der X Hydroxymethyl ist und welche dann schließlich mittels einer bekannten Prozedur halogeniert wird, um eine Verbindung der Formel (Ia) zu erhalten, in der X Halogenalkyl, insbesondere Halogenmethyl, ist;

c) zuerst mittels eines bekannten Verfahrens, wie das nach Vilsmeier-Haack und dergleichen, formyliert wird, um eine Verbindung der Formel (Ia) zu erhalten, in der X Formyl ist, welche dann unter Nachvollziehen der in Teil b) obenstehenden Prozedur umgesetzt wird, um gleichfalls die Verbindung der Formel (Ia) zu erhalten, in der X Halogenalkyl ist;

d) nach der Prozedur des obigen Abschnitts b) oder c) umgesetzt wird, um eine Zwischenverbindung der Formel (Ia) zu erhalten, in der X Formyl ist, welche Verbindung gegebenenfalls zu einer Zwischenverbindung der Formel (Ia), in der X Carboxyl ist, oxidiert werden kann, wonach schließlich die Zwischenverbindung, in der X Formyl ist, mit einem Halogenierungsmittel, wie Diethylaminoschwefeltrifluorid, umgesetzt wird, oder die Zwischenverbindung, in der X Carboxyl ist, mit Schwefeltetrafluorid umgesetzt wird, um eine Verbindung der Formel (Ia), in der X Halogenalkyl, insbesondere Difluormethyl oder Trifluormethyl, ist, zu erhalten; oder

e) zuerst nach einem gängigen Verfahren halogeniert wird, um eine Zwischenverbindung, in der X Halogen ist, zu erhalten, aus der ein Organomagnesium- oder -lithium-Derivat hergestellt wird, wonach die organometallische Verbindung mit Oxodiperoxymolybdän(pyridin)-(hexamethylphosphorsäuretriamid) oder einem Trialkylborat und einem Oxidationsmittel, wie Wasserstoffperoxid, umgesetzt wird, um eine Zwischenverbindung der Formel (Ia), in der X Hydroxy ist, zu erhalten, welche dann mittels eines bekannten Halogenalkylierungsverfahrens umgesetzt wird, um eine Verbindung der Formel (Ia), in der X Halogenalkoxy ist, zu erhalten; oder

(B) entsprechend der Formel:

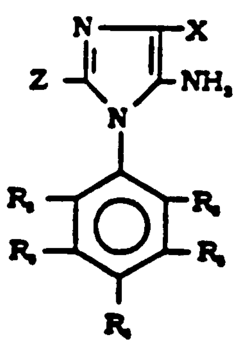

(Ib)

worin X, $R_2$, $R_3$, $R_4$, $R_5$ und $R_6$ wie für Formel (I) in Anspruch 1 definiert sind und Z Aminosulfonyl, Alkylaminosulfonyl, Dialkylaminosulfonyl, Nitro, Amino, Halogen, Alkynyl, Alkyl, Hydroxy und Salze davon, Alkoxy, Halogenalkoxy, Formyl, Alkylsulfenyl, Halogenalkylsulfenyl, Alkylsulfinyl, Halogenalkylsulfinyl, Alkylsulfonyl, Halogenalkylsulfonyl oder Sulfhydryl und Salze davon ist, wobei eine Verbindung der Formel (Ia)

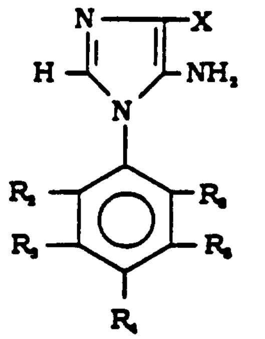

(Ia)

in der X und Amino gegebenenfalls nach Bedarf geschützt sind:

a) zuerst mit Chlorsulfon- oder Dichlorsulfonsäure umgesetzt wird, um eine Zwischenverbindung, worin Z Chlorsulfonyl ist, zu erhalten, welche Verbindung mit Ammoniak, einem Alkylamin oder Dialkylamin umgesetzt wird, um eine Verbindung der Formel (Ib) zu erhalten, in der Z Aminosulfonyl, Alkylaminosulfonyl oder Dialkylaminosulfonyl ist;

b) durch bekannte Verfahren halogeniert oder nitriert wird, um eine Verbindung der Formel (Ib), in der Z Halogen oder Nitro ist, zu erhalten, wonach die Verbindung, in der Z Nitro ist, gegebenenfalls durch ein bekanntes Verfahren reduziert wird, damit Z Amino ist, oder wonach gegebenenfalls die Verbindung, in der Z Halogen ist, mittels eines bekannten Verfahrens mit einem Kupferacetylid behandelt wird, um die Verbindung, in der Z Alkynyl ist, zu erhalten;

c) mit einer starken Base, wie einem Organolithiumreagenz umgesetzt wird, um ein organometallisches Carbanionintermediat zu erhalten, welches dann mit einem Alkylierungsmittel gequenscht wird, um eine Verbindung der Formel (Ib), in der Z Alkyl ist, zu erhalten, oder gegebenenfalls das Carbanion in einer Weise umgesetzt, die der in (A)e, obenstehend, beschriebenen ähnlich ist, um zuerst eine Verbindung der Formel (Ib), in der Z Hydroxyl oder ein Salz davon ist, zu erhalten und dann gegebenenfalls die Verbindung, in der Z Hydroxy ist, zu einer Verbindung umzuwandeln, worin Z Alkoxy oder Halogenalkoxy ist, und zwar durch bekannte Alkylierungs- oder Halogenalkylierungsverfahren;

d) mittels Formylierungsverfahren, die den in (A)b oder (A)c obenstehend beschriebenen ähnlich sind, umgesetzt wird, wobei die Verbindung, in der Z Formyl ist, direkt durch Bedingungen, wie der Vilsmeier-Haack-Reaktion, hergestellt wird oder über die Hydrolyse einer Zwischenverbindung, in der Z Bis(alkylthio)methyl oder Bis(arylthio)methyl ist;

e) zuerst mit einer Mischung aus Brom und einem Metallthiocyanat umgesetzt wird, um eine Zwischenverbindung der Formel (Ib), in der Z Thiocyano ist, zu erhalten, welche dann mit einem Alkylierungsmittel, gegebenenfalls in Gegenwart einer Base, behandelt wird, um direkt eine Verbindung der Formel (Ib), in der Z Alkylsulfenyl oder Halogenalkylsulfenyl ist, zu erhalten, oder gegebenenfalls wird die Zwischenverbindung, in der Z Thiocyano ist, zuerst zu einer entsprechenden Disulfidzwischenverbindung oxidiert, welche dann mit einem Perhalogenalkan gegebenenfalls in Gegenwart eines Reduktionsmittels umgesetzt wird, um eine Verbindung der Formel (Ib), in der Z Halogenalkylsulfenyl ist, insbesondere Perhalogenalkysulfenyl, ist, zu erhalten, wobei schließlich die Verbindung, in der Z Alkylsulfenyl oder Halogenalkylsulfenyl ist, gegebenenfalls durch bekannte Verfahren, die denen von (A)a, obenstehend, ähnlich sind, oxidiert wird, um eine Verbindung der Formel (Ib) zu erhalten, in der Z Alkylsulfinyl, Halogenalkylsulfinyl, Alkylsulfonyl oder Halogenalkylsulfonyl ist; oder

f) zuerst wie obenstehend in Abschnitt e) umgesetzt wird, um eine Zwischenverbindung, in der Z Thiocyano ist, zu erhalten, welche dann durch einen freien Radikalpromotor, wie Kaliumferricyanid, gespalten wird, um eine Verbindung der Formel (Ib), in der Z Sulfhydryl oder Salze davon bedeutet, zu erhalten; oder

(C) entsprechend der Formel:

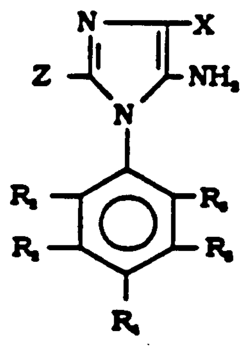

(Ib)

worin X, $R_2$, $R_3$, $R_4$, $R_5$ und $R_6$ wie für Formel (I) in Anspruch 1 definiert sind und Z Amino, Alkyl, Cyano, Carboxyl und Salze davon, Aminocarbonyl, Alkylaminocarbonyl, Dialkylaminocarbonyl, Alkoxycarbonyl, Halogenalkyl, Cyanoalkyl, Alkenyl, Alkynyl, Alkylcarbonyl oder Halogenalkylcarbonyl ist, wobei eine Verbindung der Formel (Ib), in der Z Formyl ist, hergestellt mittels dem obenstehend in (B)d beschriebenen Verfahren, und in der X und Amino gegebenenfalls nach Bedarf geschützt sind:

a) zu einer Verbindung der Formel (Ib), in der Z Alkyl, insbesondere Methyl, ist, mittels bekannter Reduktionsmittel, wie Natriumborhydrid oder p-Toluolsulfonylhydrazin und Natriumcyanoborhydrid reduziert wird;

b) mit einem standardmäßigen bekannten Oxidationsmittel umgesetzt wird, um eine Verbindung der Formel (Ib), in der Z Carboxyl oder Salze davon bedeutet, zu erhalten, wonach die Carboxylverbindung gegebenenfalls zu einer Verbindung der Formel (Ib), in der Z Amino ist, durch eine Curtius-Umlagerung über ein Säurehalogenid-, Azid- und Isocyanat-Zwischenprodukt umgewandelt werden kann oder gegebenenfalls die Verbindung, in der Z Carboxyl ist, mit Isophthalonitril behandelt wird, um eine Verbindung der Formel (Ib), in der Z Cyano ist, zu erhalten, oder gegebenenfalls wird die Verbindung, in der Z Formyl ist, mit Hydroxylamin umgesetzt, um eine Aldoximzwischenverbindung zu erhalten, welche dann durch Standardverfahren dehydratisiert wird, um die Verbindung der Formel (Ib), in der Z Cyano ist, zu erhalten;

c) mittels der obenstehenden Prozedur b) zu der Verbindung, in der Z Carboxyl ist, umgewandelt wird, dann das Carboxyl durch Standardverfahren zu einer Säurehalogenidzwischenverbindung umgewandelt wird, welche dann mit Ammoniak, einem Alkylamin, Dialkylamin oder Alkylalkohol umgesetzt wird, um eine Verbindung der Formel (Ib), in der Z Aminocarbonyl, Dialkylaminocarbonyl oder Alkoxycarbonyl ist, zu erhalten;

d) zuerst mittels Verfahren, die denen in (A)b, obenstehend, ähnlich sind, zu einer Hydroxymethylzwischenverbindung reduziert wird, gefolgt von Halogenierungsvorgängen,die ebenfalls denen von (A)b, obenstehend, ähnlich sind, um eine Verbindung der Formel (Ib), in der Z Halogenalkyl, insbesondere Halogenmethyl, ist, zu erhalten, oder gegebenenfalls wird die Halogenalkylverbindung, insbesondere Halogenmethyl, mit einem Metallcyanid behandelt, um eine Verbindung der Formel (Ib), in der Z Cyanoalkyl, insbesondere Cyanomethyl, ist, zu erhalten;

e) in einer Wittig- oder modifizierten Wittig-Reaktion umgesetzt wird, um eine Verbindung der Formel (Ib), in der Z Alkenyl oder Alkynyl ist, zu erhalten;

f) mit einem Grignard-Reagenz oder Alkyllithiumreagenz umgesetzt wird, um eine Zwischenverbindung der Formel (Ib), in der Z $\alpha$-Hydroxyalkyl ist, zu erhalten, welche dann mittels bekannter Reagenzien oxidiert wird, um eine Verbindung der Formel (Ib), in der Z Alkylcarbonyl ist, zu erhalten, wobei dann die Alkylcarbonylverbindung gegebenenfalls zu einer Verbindung der Formel (Ib) halogeniert wird, in der Z Halogenalkylcarbonyl ist; oder

g) zuerst gemäß dem obigen Verfahren in den Teilen b) und c) zu dem Säurechloridintermediat, erhalten über die Verbindung, in der Z Carboxyl ist, umgewandelt wird, wonach durch herkömmliche Curtius-Umlagerungsvorgänge das Säurehalogenidintermediat über Azid- und Isocyanatintermediate zu der Verbindung der Formel (Ib), in der Z Amino ist, umgewandelt wird; oder

(D) entsprechend der Formel:

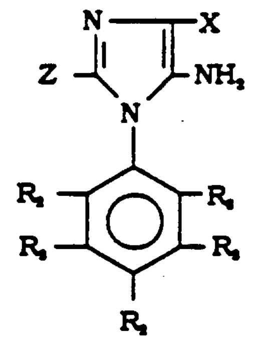

(Ib)

worin X, $R_2$, $R_3$, $R_4$, $R_5$ und $R_6$ wie für Formel (I) in Anspruch I definiert sind und Z Alkylamino, Dialkylamino, Trialkylammoniumsalz, Alkoxycarbonylamino, Alkylaminocarbonylamino, Dialkylaminocarbonylamino, Alkoxyalkylidenimino, Alkylcarbonylamino, Halogenalkylcarbonylamino oder Arylcarbonylamino ist, worin eine Verbindung der Formel (Ib), in der Z Amino ist, hergestellt über die in (B)-b oder (B)g, obenstehend, beschriebenen Verfahrensschritte hergestellt, und in der X und Amino gegebenenfalls nach Bedarfgeschützt sind:

a) zuerst mit Phosgen umgesetzt wird, um eine Zwischenverbindung der Formel (Ib), in der Z Chlorcarbonylamino oder Isocyanat ist, zu erhalten, welche dann mit einem Alkylalkohol, Alkylamin oder Dialkylamin umgesetzt wird, um eine Verbindung der Formel (Ib), in der Z Alkoxycarbonylamino, Alkylaminocarbonylamino oder Dialkylaminocarbonylamino ist, zu erhalten;

b) mit einem Alkylierungsmittel, wie einem Alkyliodid oder Dialkylsulfat, oder gegebenenfalls mittels einer bekannten reduktiven Methylierung unter Verwendung von Formaldehyd und Ameisensäure umgesetzt wird, um eine Verbindung der Formel (Ib), in der Z Alkylamino, Dialkylamino oder Trialkylammoniumsalz ist, zu erhalten;

c) mit einem Alkylorthoformiat umgesetzt wird, um eine Verbindung der Formel (Ib), in der Z Alkoxyalkylidenimino, insbesondere Alkoxymethylidenimino, ist, zu erhalten; oder

d) mit einem Alkyl-, Halogenalkyl- oder Arylcarbonylhalogenid, gegebenenfalls in Gegenwart eines Säureakzeptors umgesetzt wird, um eine Verbindung der Formel (Ib), in der Z Alkylcarbonylamino, Halogenalkylcarbonylamino oder Arylcarbonylamino ist, zu erhalten; oder

(E) entsprechend der Formel:

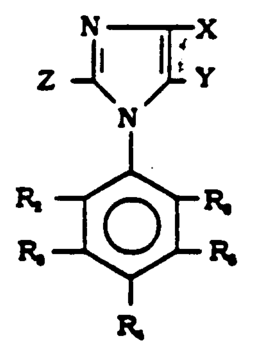

(I)

worin X, Z, $R_2$, $R_3$, $R_4$, $R_5$ und $R_6$ wie in Formel (I) in Anspruch 1 definiert sind und Y Wasserstoff, Amino, Halogen, Alkylsulfenyl, Halogenalkylsulfenyl, Alkylsulfinyl, Halogenalkylsulfinyl, Alkylsulfonyl, Halogenalkylsulfonyl, Cyano oder Nitro ist, worin eine Verbindung der Formel (Ib)

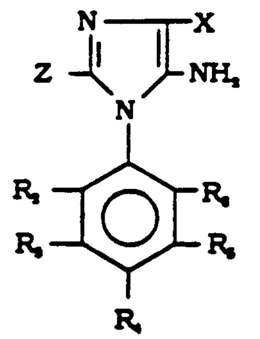

(Ib)

in der X, Z und $R_2$ bis $R_6$ wie obenstehend definiert sind, und in der X, Z und Amino gegebenenfalls nach Bedarf geschützt sind:

a) durch bekannte Verfahren, wie mit einem Alkylnitrit, desaminiert wird, um die Verbindung, in der Y Amino ist, in ihr entsprechendes Diazoniumsalz umzuwandeln, gefolgt von Quenschen des Diazoniumsalzes mit einem Quenschmittel gemäß den gängigen Verfahren, um eine Verbindung der Formel (I), in der Y Wasserstoff Halogen, Cyano, Nitro, Alkylsulfenyl oder Halogenalkylsulfenyl ist, zu erhalten, und wonach die Verbindung, in der Y Alkylsulfenyl oder Halogenalkylsulfenyl ist, gegebenenfalls zu einer Verbindung der Formel (I), in der Y Alkylsulfinyl, Halogenalkylsulfinyl, Alkylsulfonyl oder Halogenalkylsulfonyl ist, zu oxidieren; oder

(F) entsprechend der Formel:

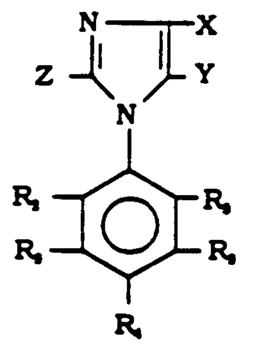

(I)

worin X, Z, $R_2$, $R_3$, $R_4$, $R_5$ und $R_6$ die gleiche Bedeutung wie für Formel (I) in Anspruch 1 besitzen und Y Alkoxycarbonylamino, Alkylaminocarbonylamino, Dialkylaminocarbonylamino, Alkoxyalkylidenimino, Alkylcarbonylamino, Halogenalkylcarbonylamino, Arylcarbonylamino, Alkylamino, Dialkylamino oder Trialkylammoniumsalz ist, worin eine Verbindung der Formel (Ib)

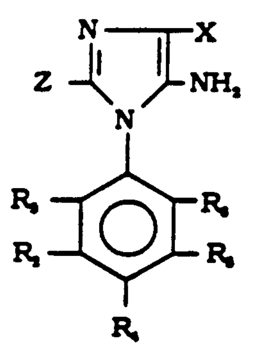

(Ib)

in der X, Z und $R_2$ bis $R_6$ wie oben definiert sind, und in der X, Z und Amino gegebenenfalls nach Bedarf geschützt sind:

a) in einer zu der in (D)a, obenstehend, beschriebenen ähnlichen Weise über ein Chlorcarbonylamino- oder Isocyanatintermediat, erhalten durch die Reaktion mit Phosgen, umgesetzt wird, welches dann mit einem Alkylalkohol, Alkylamin oder Dialkylamin umgesetzt wird, um eine Verbindung der Formel (I), in der Y Alkoxycarbonylamino, Alkylaminocarbonylamino oder Dialkylaminocarbonylamino ist, zu erhalten;

b) in einer ähnlichen Weise wie der in (D)c, obenstehend, beschriebenen mit einem Alkylorthoformiat umgesetzt wird, um eine Verbindung der Formel (I), in der Y Alkoxyalkylidenimino, insbesondere Alkoxymethylidenimino, ist, zu erhalten;

c) in einer ähnlichen Weise wie der in (D)b, obenstehend, beschriebenen durch Alkylierung oder reduktive Methylierung umgesetzt wird, um eine Verbindung der Formel (I), in der Y Alkylamino, Dialkylamino oder Trialkylammoniumsalz ist, zu erhalten; oder

d) in einer ähnlichen Weise wie der in (D)d, obenstehend, beschriebenen mit einem Alkyl-, Halogenalkyl- oder Arylcarbonylhalogenid umgesetzt wird, um eine Verbindung der Formel (I), in der Y Alkylcarbonylamino, Halogenalkylcarbonylamino oder Arylcarbonylamino ist, zu erhalten; oder

(G) entsprechend der Formel:

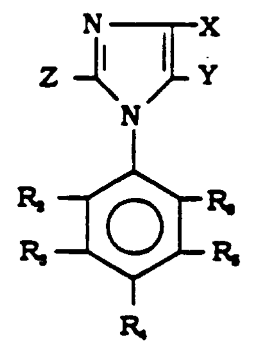

(I)

worin X, Z, $R_2$, $R_3$, $R_4$, $R_5$ und $R_6$ wie für Formel (I) in Anspruch 1 definiert sind und Y Nitro, Sulfhydryl und Salze davon, Hydroxyl und Salze davon, Alkoxy, Halogenalkoxy, Aminosulfonyl, Alkylaminosulfonyl, Dialkylaminosulfonyl, Alkyl, Halogenalkyl, Alkenyl, Alkynyl, Cyanoalkyl oder Formyl ist, worin eine Verbindung der Formel (Ib)

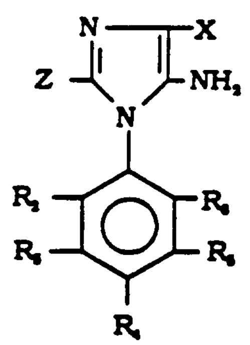

(Ib)

in der X, Z und $R_2$ bis $R_6$ die gleiche Bedeutung wie oben haben, und in der X und Z gegebenenfalls nach Bedarf geschützt sind, gemäß den in (E) obenstehenden Verfahren desaminiert wird, um eine Verbindung der Formel (I), in der Y Wasserstoff ist, zu erhalten, wonach dann die Verbindung, in der X und Z gegebenenfalls nach Bedarf geschützt sind:

a) mittels den in Anspruch (B)b beschriebenen ähnlichen Verfahren nitriert wird, um eine Verbindung der Formel (I), in der Y Nitro ist, zu erhalten;

b) in einer ähnlichen Weise durch das obenstehend in (B)f beschriebene Verfahren umgesetzt wird, um zuerst eine Zwischenverbindung, in der Y Thiocyano ist, zu erhalten, welche dann umgesetzt wird, um eine Verbindung der Formel (I), in der Y Sulfhydryl und Salze davon ist, zu

erhalten;

c) zuerst mit einer starken Base, wie einem Organolithiumreagenz, umgesetzt wird, um ein Metallcarbanion-Intermediat zu erhalten, welches dann mit einem Elektrophilen gequenscht wird, um eine Verbindung der Formel (I), in der Y Alkyl, Halogenalkyl, Alkenyl, Alkynyl, Cyanoalkyl oder Formyl ist, zu erhalten;

d) zu dem Carbanion, wie oben in Abschnitt c), umgewandelt wird, und dann mit Sulfurylchlorid gequenscht wird, um eine Zwischenverbindung, in der Y Chlorsulfonyl ist, zu erhalten, welche dann mit Ammoniak oder einem Alkyl- oder Dialkylamin umgesetzt wird, um eine Verbindung der Formel (I), in der Y Aminosulfonyl, Alkylaminosulfonyl oder Dialkylaminosulfonyl ist, zu erhalten;

e) zu dem Carbanion, wie obenstehend in Abschnitt c), umgewandelt wird, oder gegebenenfalls das Carbanion über die Verbindung, in der Y Halogen ist, erhalten durch das obenstehende Verfahren von (E), hergestellt wird, und dann das Carbanion in einer dem in (B)c obenstehend beschriebenen Verfahren ähnlichen Weise umgesetzt wird, um eine Verbindung der Formel (I), in der Y Hydroxyl und Salze davon, Alkoxy oder Halogenalkoxy ist, zu erhalten; oder

(H) entsprechend der Formel:

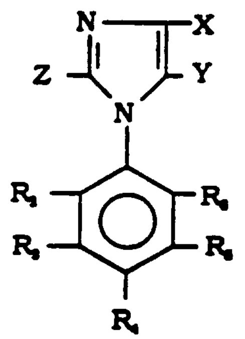

(I)

worin X, Z, $R_2$, $R_3$, $R_4$, $R_5$ und $R_6$ wie für Formel (I) in Anspruch 1 definiert sind und Y Carboxyl oder Salze davon, Cyano, Aminocarbonyl, Alkylaminocarbonyl, Dialkylaminocarbonyl, Alkoxycarbonyl, Halogenalkyl, Alkenyl, Alkynyl, Alkylcarbonyl oder Halogenalkylcarbonyl ist, worin eine Verbindung der Formel (Ib)

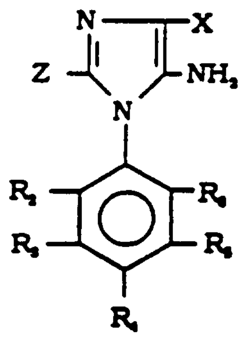

(Ib)

in der X, Z und $R_2$ bis $R_6$ die gleiche Bedeutung wie oben haben, zuerst gemäß den in (E) obenstehend beschriebenen Verfahren desaminiert wird, um eine Verbindung, in der Y Wasserstoff ist, zu erhalten, welche dann durch die in (G)c obenstehend beschriebenen Verfahren umgewandelt wird, um eine Verbindung der Formel (I), in der Y Formyl ist, zu erhalten, wonach die Formylverbindung, in der X und Z gegebenenfalls nach Bedarf geschützt sind:

a) durch ähnliche Verfahren, wie den obenstehend in C(b) beschriebenen, umgesetzt wird, um eine Verbindung der Formel (I), in der Y Carboxyl und Salze davon oder Cyano ist, zu erhalten;

b) durch ähnliche Verfahren, wie den obenstehend in C(c) beschriebenen, umgesetzt wird, um eine Verbindung der Formel (I), in der Y Aminocarbonyl, Alkylaminocarbonyl, Dialkylaminocarbonyl oder Alkoxycarbonyl ist, zu erhalten;

c) durch ähnliche Verfahren, wie den obenstehend in C(d) beschriebenen, umgesetzt wird, um eine Verbindung der Formel (I), in der Y Halogenalkyl, insbesondere Halogenmethyl, ist, zu erhalten;

d) durch ähnliche Verfahren, wie den obenstehend in (B)b und 19e beschriebenen, umgesetzt wird, um eine Verbindung der Formel (I), in der Y Alkenyl oder Alkynyl ist, zu erhalten; oder

e) durch ähnliche Verfahren, wie den obenstehend in (C)f beschriebenen, umgesetzt wird, um eine Verbindung der Formel (I), in der Y Alkylcarbonyl oder Halogenalkylcarbonyl ist, zu erhalten; oder

(I) entsprechend der Formel:

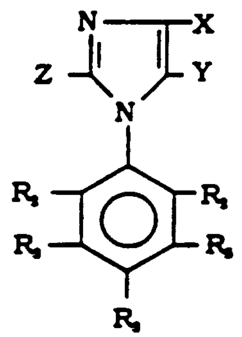

(I)

worin Y, Z, $R_2$, $R_3$, $R_4$, $R_5$ und $R_6$ die gleiche Bedeutung wie für Formel (I) in Anspruch 1 besitzen und X Alkylsulfenyl, Halogenalkylsulfenyl, Alkylsulfinyl, Halogenalkylsulfinyl, Alkylsulfonyl oder Halogenalkylsulfonyl ist, wobei eine Verbindung der Formel (Ic)

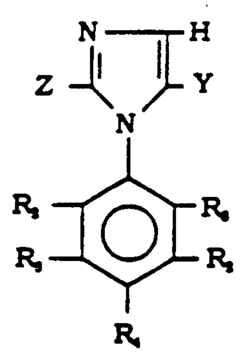

(Ic)

in der Y und Z gegebenenfalls nach Bedarf geschützt sind:

a) zuerst gemäß den Verfahren, die den obenstehend in (B)c beschriebenen ähnlich sind, umgesetzt, um eine Verbindung der Formel (Ic), in der X Wasserstoff ist, zu einer Zwischenverbindung der Formel (I), in der X nacheinander Thiocyano und dann ein Disulfid ist, umzuwandeln, wonach ebenfalls durch Verfahren, die den obenstehend in (B)c beschriebenen ähnlich sind, das Thiocyano- oder Disulfid-Intermediat zu einer Verbindung der Formel (I), in der X Alkylsulfenyl oder Halogenalkylsulfenyl, insbesondere Perhalogenalkylsulfenyl, ist, umzuwandeln, wobei die Verbindung dann gegebenenfalls mittels Verfahren, die den in (B)c obenstehend beschriebenen ähnlich sind, oxidiert wird, um das Sulfoxid- oder Sulfonanaloge, d.h. eine Verbindung der Formel (I), in der X Alkylsulfinyl, Halogenalkylsulfinyl, vorzugsweise Perhalogenalkylsulfinyl, Alkylsulfonyl oder Halogenalkylsulfonyl, insbesondere Perhalogenalkylsulfonyl ist, zu erhalten; oder

b) gemäß Verfahren, die den obenstehend in (B)a beschriebenen ähnlich sind, zuerst umgesetzt wird, um die Verbindung der Formel (Ic), in der X Wasserstoff ist, zu einer Zwischenverbindung der Formel (I), in der X Chlorsulfonyl ist, umzuwandeln, wonach die Chlorsulfonylverbindung mit einem Reduktionsmittel, wie Triphenylphosphin umgesetzt wird, um das gleiche Disulfid-Intermediat, obenstehend in Abschnitt a) beschrieben, zu erhalten, wonach schließlich das Disulfid durch die obenstehend in Abschnitt a) beschriebenen Verfahren umgewandelt wird, um eine Verbindung der Formel (I), in der X Alkylsulfenyl oder Halogenalkylsulfenyl, insbesondere Perhalogenalkylsulfenyl ist, zu erhalten, oder wobei gegebenenfalls die Sulfenylverbindung oxidiert wird, um eine

Verbindung der Formel (I), in der X Alkylsulfinyl, Halogenalkylsulfinyl, insbesondere Perhalogenakylsulfinyl, Alkylsulfonyl oder Halogenalkylsulfonyl, insbesondere Perhalogenalkylsulfonyl, ist, zu erhalten; oder

(J) entsprechend der Formel

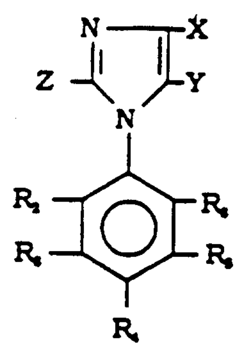

(IV)

worin:

| | |
|---|---|
| $R_2$, $R_3$, $R_4$, $R_5$ und $R_6$ | die gleiche Bedeutung wie in Anspruch 1 haben; |
| X | Wasserstoff oder Halogenalkyl, insbesondere Trifluormethyl ist; |
| Y | Amino; Hydroxy, gegebenenfalls in seiner isomeren Keto-Form, wenn X Wasserstoff ist, vorliegend; Alkoxy oder Halogenalkoxy ist; und |
| Z | Wasserstoff Halogen; Alkyl; Halogenalkyl; Hydroxy, gegebenenfalls in seiner isomeren Keto-Form, wenn X Wasserstoff ist, vorliegend, ist und Y Imino; Alkoxy oder Halogenalkoxy ist; |

wobei eine Verbindung der Formel (III)

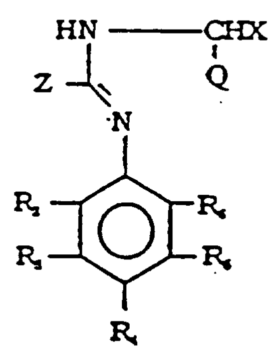

(III)

worin:

| | |
|---|---|
| $R_2$, $R_3$, $R_4$, $R_5$ und $R_6$ | wie oben definiert sind; |
| X | Wasserstoff oder Halogenalkyl, insbesondere Trifluormethyl, ist; |
| Z | Wasserstoff Halogen, Alkyl, Halogenalkyl oder Hydroxy, gegebenenfalls in seiner isomeren Keto-Form vorliegend, ist; und |
| Q | Cyano oder niederes Alkoxycarbonyl ist; |

mit einem basischen Mittel in einem geeigneten Reaktionsmedium umgesetzt wird, um eine Verbindung der Formel (IV), welche, wenn Y oder Z Hydroxy ist, anschließend gegebenenfalls alkyliert oder halogenalkyliert wird, damit Y oder Z Alkoxy oder Halogenalkoxy ist.

**9.** Verfahren zur Herstellung einer Verbindung der Formel (IV) gemäß Anspruch 8J, wobei die Verbindung der Formel (IV) folgendes ist:

a) eine Verbindung der in Anspruch 8 aufgeführten Formel (5), in der X und Z jeweils Wasserstoff sind und Y Amino ist;

b) eine Verbindung der Formel (17)

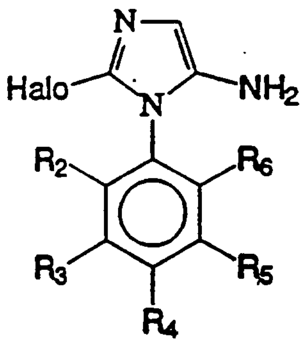

(17)

in der Halo für Halogen, insbesondere Chlor, steht;
c) eine Verbindung der Formel (22)

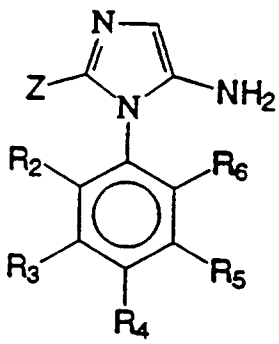

(22)

in der Z Alkyl oder Halogenalkyl ist;
d) eine Verbindung der Formel (27)

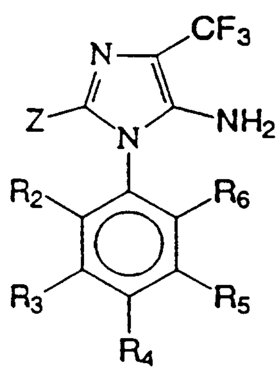

(27)

in der Z Halogen, Alkyl oder Halogenalkyl ist;
e) eine Verbindung der Formel (30), in der X Wasserstoff ist und Y Hydroxyl, gegebenenfalls in seiner isomeren Keto-Form (29) vorliegend, ist

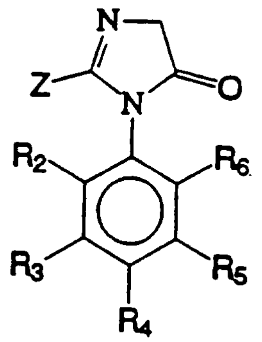

(29)

worin Z Halogen, Alkyl oder Halogenalkyl ist; oder

f) eine Verbindung der Formel (37), in der Y Amino ist und Z Hydroxy ist, gegebenenfalls in seiner isomeren Keto-Imino-Form (34) vorliegend, ist

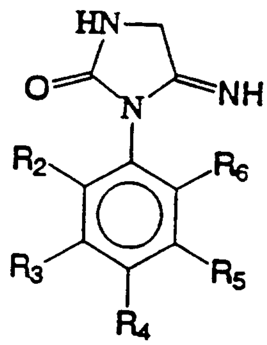

(34)

**10.** Verfahren zur Herstellung einer Verbindung der Formel (I):

(A) worin X, Y, Z, $R_2$, $R_3$, $R_4$, $R_5$ und $R_6$ wie in Anspruch 1 definiert sind, wobei eine Verbindung der in Anspruch 8 aufgeführten Formel (5) gemäß dem Herstellungsverfahren von Anspruch 8 zur Einführung der X-, Y- und Z-Substituenten umgesetzt wird;

(B) worin X, Y, $R_2$, $R_3$, $R_4$, $R_5$ und $R_6$ wie in Anspruch 1 definiert sind und Z Halogen ist, wobei eine Verbindung der in Anspruch 9 aufgeführten Formel (17) gemäß dem Herstellungsverfahren von Anspruch 8 zur Einführung der X- und Y-Substituenten umgesetzt wird;

(C) worin X, Y, $R_2$, $R_3$, $R_4$, $R_5$ und $R_6$ wie in Anspruch 1 definiert sind und Z Alkyl oder Halogenalkyl ist, wobei eine Verbindung der in Anspruch 9 aufgeführten Formel (22) gemäß dem Herstellungsverfahren von Anspruch 8 zur Einführung der X- und Y-Substituenten umgesetzt wird;

(D) wobei Y, $R_2$, $R_3$, $R_4$, $R_5$ und $R_6$ wie in Anspruch 1 definiert sind, X Halogenalkyl, insbesondere Trifluormethyl, ist und Z Halogen, Alkyl oder Halogenalkyl ist, wobei eine Verbindung der Formel (27) gemäß dem Verfahren von Anspruch 8 zur Einführung des Y-Substituenten umgesetzt wird;

(E) wobei X, $R_2$, $R_3$, $R_4$, $R_5$ und $R_6$ wie in Anspruch 1 definiert sind, Y Hydroxy, Alkoxy oder Halogenalkoxy ist, und Z Halogen, Alkyl oder Halogenalkyl ist, wobei eine Verbindung der Formel (30), gegebenenfalls in ihrer isomeren Keto-Form (29) vorliegend, aufgeführt in Anspruch 9, in der Y Hydroxy ist, gegebenenfalls so alkyliert, daß Y Alkoxy oder Halogenalkoxy ist, gemäß dem Verfahren von Anspruch 8 zur Einführung des X-Substituenten umgesetzt wird; oder

(F) worin X, Y, $R_2$, $R_3$, $R_4$, $R_5$ und $R_6$ wie in Anspruch 1 definiert sind und Z Hydroxy, Alkoxy, Halogenalkoxy oder Halogen ist, wobei eine Verbindung der Formel (37), gegebenenfalls in ihrer isomeren Keto-Imino-Form (34) vorliegend, aufgeführt in Anspruch 9, in der Z Hydroxy ist, gegebenenfalls so alkyliert, daß Z Alkoxy oder Halogenalkoxy ist oder gegebenenfalls so halogeniert, daß Z Halogen ist, gemäß dem Verfahren von Anspruch 8 zur Einführung der X- und Y-Substituenten umgesetzt wird.

**11.** Verbindung der Formel (Ia), (Ib), (Ic), (IV), (5), (17), (22), (27), (30)/(29) oder (37)/(34), aufgeführt in Anspruch 8 oder 9, worin die Substituenten X, Y, Z, $R_2$, $R_3$, $R_4$, $R_5$ und $R_6$ die gleiche Bedeutung wie vorstehend in Anspruch 8, 9 oder 10 haben.

**12.** Verfahren zur Bekämpfung von: Arthropoden, insbesondere Insekten, Aphiden oder Milben; Nemathoden; Helminthen oder Protozoen-Schädlingen an einer Stelle, welche das Behandeln der Stelle mit einer wirksamen Menge einer Verbindung der Formel (I) umfaßt, wobei die verschiedenen Substituenten X, Z, Y, $R_2$, $R_3$, $R_4$, $R_5$ und $R_6$ wie in den Ansprüchen 1 bis 7 definiert sind, wobei jedoch Verbindungen eingeschlossen sind, worin (i) X Trifluormethyl ist, wenn $R_3$ für Methyl steht, $R_4$ für Nitro oder Amino steht und die anderen Symbole Wasserstoff repräsentieren, und (ii) X Chlormethyl ist, wenn $R_4$ für Methyl, Methoxy oder Ethoxy steht und die anderen Symbole Wasserstoff repräsentieren, mit der Ausschließung eines Verfahrens der Behandlung des menschlichen oder tierischen Körpers, die von einem Arzt oder Veterinärmediziner durchgeführt wird.

**13.** Verfahren nach Anspruch 12, worin die Verbindung der Formel (I) eine in Anspruch 5 oder 6 genannte Verbindung ist.

**14.** Verfahren nach Anspruch 12 oder 13, wobei die Stelle bzw. Ort landwirtschaftliche oder gartenbauliche Pflanzen oder ein Medium, in dem die Pflanzen wachsen, und die Schädlinge arthropode oder nematode Schädlinge der Pflanzen sind, umfaßt, und die Behandlung das Auftragen einer wirksamen Menge der Verbindung der Formel (I) auf die Pflanzen oder auf das Medium, in dem sie wachsen, ist.

**15.** Verfahren nach Anspruch 14, wobei die Verbindung auf die Stelle, in der die anthropoden oder nematoden Schädlinge bekämpft werden, in einer Menge von etwa 0,005 kg bis etwa 15 kg Verbindung pro Hektar der behandelten Stelle, vorzugsweise in einer Menge von etwa 0,02 kg bis etwa 2 kg, Verbindung pro Hektar, aufgetragen wird.

**16.** Verfahren nach Anspruch 15, wobei die Schädlinge Milben, Amphiden, Insekten oder Pflanzennematoden oder Kombinationen davon sind, welches das Einbringen der Verbindung in die Erde, in der die Pflanzen gepflanzt sind oder gepflanzt bzw. gesät werden sollen, oder das Aufbringen der Verbindung auf die Pflanzensamen oder die Pflanzenwurzeln oder das Behandeln mittels Blattauftragung umfaßt.

**17.** Verfahren nach Anspruch 16, wobei die Insekten Bodeninsekten der Ordnung Coleoptera, insbesondere Diabrotica-Arten, sind, wobei die Milben zu der Unterklasse Acari und die Aphiden zu der Oberfamilie Aphidiodea gehören.

**18.** Verfahren nach Anspruch 12, wobei das Verfahren auf dem Gebiet der Veterinärmedizin oder Tierhaltung oder zur Erhaltung der allgemeinen Gesundheit gegen Arthropoden, insbesondere Insekten, der Ordnung Diptera oder Milben der Unterklasse Acari oder beidem; oder Helminthen oder Protozoen, welche im inneren oder extern auf warmblütigen Wirbeltieren parasitisch sind, angewandt wird.

**19.** Zusammensetzung zur Bekämpung von Arthiopoden, Nematoden, Helminthen oder Protozoen-Schädlingen, umfassend: eine wirksame Menge, vorzugsweise etwa 0,05 bis etwa 95 Gew.-% einer Verbindung der Formel (I) gemäß einem der Ansprüche 1 bis 6, aber einschließlich der Verbindungen, worin (i) X Trifluormethyl ist, wenn $R_3$ Methyl bedeutet, $R_4$ Nitro oder Amino bedeutet und die anderen Symbole Wasserstoffrepräsentieren, und (ii) X Chlormethyl ist, wenn $R_4$ Methyl, Methoxy oder Ethoxy bedeutet und die anderen Symbole Wasserstoff repräsentieren, als ein aktiver Bestandteil, und eine oder mehrere landwirtschaftlich oder medizinisch verträgliche Komponenten, umfassend vorzugsweise etwa 1 bis etwa 95 Gew.-% eines oder mehrerer fester oder flüssiger Träger und etwa 0,1 bis etwa 50 Gew.-% eines oder mehrerer zusätzlicher Komponenten, wie oberflächenaktive Mittel.

**20.** Zusammensetzung, die insbesondere in der Veterinärmedizin und der Tierhaltung oder bei der Aufrechterhaltung der allgemeinen Gesundheit brauchbar ist, umfassend eine oder mehrere kompatible Komponenten und als einen aktiven Bestandteil mindestens eine Verbindung der Formel (I), wie in Anspruch 19 definiert.

**Patentansprüche für folgenden Vertragsstaat : ES**

1. Verfahren zur Herstellung einer Verbindung der Formel (I)

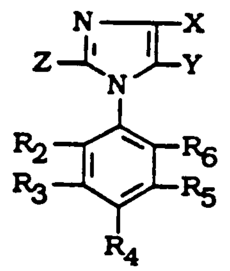

(I)

worin:

X eine aus Halogenalkyl oder Halogenalkoxy gewählte Gruppe oder eine aus Alkylsulfenyl, Alkylsulfinyl oder Alkylsulfonyl gewählte, unsubstituierte oder Halogensubstituierte Gruppe ist, worin die angegebenen Alkyl- und Alkoxyteile jeder Gruppe eine lineare oder verzweigte Kette mit ein bis vier Kohlenstoffatomen darstellen und die Halogensubstitution jeder Gruppe ein oder mehrere Halogenatome, welche gleich oder verschieden sind, bis zur vollständigen Substitution des Alkyl- oder Alkoxyteils umfaßt;

Y und Z jeweils individuell gewählt sind aus: einem Wasserstoff- oder einem Halogenatom; einer aus Nitro, Cyano, Hydroxyl gewählten Gruppe und annehmbaren Salzen davon, Sulfhydryl und annehmbaren Salzen davon, Formyl, Hydroxycarbonyl und annehmbaren Salzen davon, Alkoxycarbonyl, Aminocarbonyl, Alkylaminocarbonyl, Dialkylaminocarbonyl, Amino, Alkylamino, Dialkylamino, einem Trialkylammoniumsalz, Cyanoalkyl, Alkoxycarbonylamino, Arylcarbonylamino, Alkylaminocarbonylamino, Dialkylaminocarbonylamino, Aminosulfonyl, Alkylaminosulfonyl, Dialkylaminosulfonyl oder Alkoxyalkylidenimino, wobei die angegebenen Alkyl- und Alkoxyteile in jeder Gruppe lineare oder verzweigte Ketten mit ein bis vier Kohlenstoffatomen sind; einer linearen oder verzweigtkettigen Alkenyl- oder Alkynylgruppe mit zwei bis vier Kohlenstoffatomen; oder einer aus einem unsubstituierten oder Halogen-substituierten Alkyl, Alkoxy, Alkylcarbonyl, Alkylcarbonylamino, Alkylsulfenyl, Alkylsulfinyl oder Alkylsulfonyl gewählte Gruppe, wobei die angegebenen Alkyl- und Alkoxyteile jeder Gruppe eine lineare oder verzweigte Kette mit ein bis vier Kohlenstoffatomen bedeuten und die Halogen-Substitution aus einem oder mehreren Halogenatomen, welche gleich oder verschieden sind, bis zur vollständigen Substitution des Alkyl- oder Alkoxyteils besteht; und nur eines von Y und Z eine schwefelhaltige Gruppe ist; und $R_2$, $R_3$, $R_4$, $R_5$ und $R_6$ individuell ausgewählt sind aus: einem Wasserstoff- oder Halogenatom; einer aus Nitro, Cyano, Amino, Alkylamino oder Dialkylamino gewählten Gruppe, wobei der Alkylteil jeder Gruppe eine lineare oder verzweigte Kette mit ein bis vier Kohlenstoffatomen ist; eine lineare oder verzweigtkettige Alkenyl- oder Alkynylgruppe mit zwei bis vier Kohlenstoffatomen, welche mit ein oder mehreren Halogenatomen, die gleich oder verschieden sind, bis zur vollständigen Substitution substituiert sein kann; oder eine aus einem unsubstituierten oder Halogensubstituierten Alkyl, Alkoxy, Alkylsulfenyl, Alkylsulfinyl oder Alkylsulfonyl gewählten Gruppe, wobei die definierten Alkyl- und Alkoxyteile in jeder Gruppe eine lineare oder verzweigte Kette mit ein bis vier Kohlenstoffatomen ist und die Halogen-Substitution ein oder mehrere Halogenatome, die gleich oder verschieden sind, bis zur vollständigen Substitution des Alkyl- oder Alkoxyteils umfaßt; mit der Maßgabe, daß (i) X etwas anderes als Trifluormethyl bedeutet, wenn $R_3$ für Methyl steht, $R_4$ Nitro oder Amino bedeutet und die anderen Symbole Wasserstoff repräsentieren und (ii) X etwas anderes als Chlormethyl ist, wenn $R_4$ Methyl, Methoxy oder Ethoxy bedeutet und die anderen Symbole Wasserstoff repräsentieren:

(A) entsprechend der Formel:

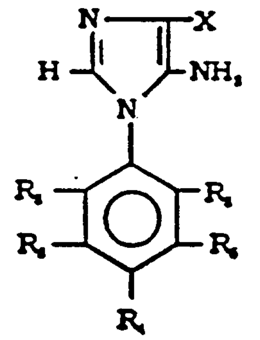

(Ia)

worin $R_2$, $R_3$, $R_4$, $R_5$ und $R_6$ die gleiche Bedeutung wie für die Formel (I) besitzen und X Alkylsulfenyl, Halogenalkylsulfenyl, Alkylsulfinyl, Halogenalkylsulfinyl, Alkylsulfonyl, Halogenalkylsulfonyl, Halogenalkyl oder Halogenalkoxy ist, wobei eine Verbindung der Formel (5)

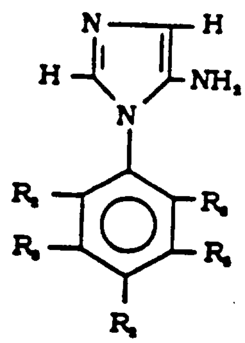

5

worin Amino gegebenenfalls wie erforderlich geschützt ist:

a) zuerst mit einem Sulfenylhalogenid, $R_1$SHalogen, worin $R_1$ Alkyl oder Halogenalkyl ist, in einem organischen Reaktionsmedium, gegebenenfalls in Gegenwart eines Säureakzeptors, wie eines tertiären Amins, umgesetzt wird, um eine Verbindung der Formel (Ia) zu erhalten, worin X Alkylsulfenyl oder Halogenalkylsulfenyl ist, welches dann gegebenenfalls durch bekannte Verfahren, wie durch ein Peroxid, oxidiert wird, um eine Verbindung der Formel (Ia) zu erhalten, worin X $S(O)_nR_1$ ist, worin n 1 oder 2 ist und $R_1$ die gleiche Bedeutung wie oben hat, d.h. X Alkylsulfinyl, Halogenalkylsulfinyl, Alkylsulfonyl oder Halogenalkylsulfonyl ist;

b) zuerst mit einem Tris(alkylthio)methan oder Tris(arylthio)methan in einem organischen Reaktionsmedium in Gegenwart einer Lewis-Säure und gegebenenfalls in Gegenwart eines Säureakzeptors umgesetzt wird, dann die erhaltene Zwischenverbindung der Formel (Ia), in der X Bis-(alkylthio)methyl oder Bis(arylthio)methyl ist, in einem organischen Reaktionsmedium mit einem geeigneten Alkylnitrid gefolgt von einem Hydrolysevorgang umgesetzt wird, um eine Zwischenverbindung der Formel (Ia) zu erhalten, in der X Formyl ist, wonach bekannte Reduktionsvorgänge durchgeführt werden, um die Zwischenverbindung zu erhalten, in der X Hydroxymethyl ist und welche dann schließlich mittels einer bekannten Prozedur halogeniert wird, um eine Verbindung der Formel (Ia) zu erhalten, in der X Halogenalkyl, insbesondere Halogenmethyl, ist;

c) zuerst mittels eines bekannten Verfahrens, wie das nach Vilsmeier-Haack und dergleichen, formyliert wird, um eine Verbindung der Formel (Ia) zu erhalten, in der X Formyl ist, welche dann unter Nachvollziehen der in Teil b) obenstehenden Prozedur umgesetzt wird, um gleichfalls die Verbindung der Formel (Ia) zu erhalten, in der X Halogenalkyl ist;

d) nach der Prozedur des obigen Abschnitts b) oder c) umgesetzt wird, um eine Zwischenverbindung der Formel (Ia) zu erhalten, in der X Formyl ist, welche Verbindung gegebenenfalls zu einer Zwischenverbindung der Formel (Ia), in der X Carboxyl ist, oxidiert werden kann, wonach schließlich die Zwischenverbindung, in der X Formyl ist, mit einem Halogenierungsmittel, wie Diethylaminoschwefeltrifluorid, umgesetzt wird, oder die Zwischenverbindung, in der X Carboxyl

ist, mit Schwefeltetrafluorid umgesetzt wird, um eine Verbindung der Formel (Ia), in der X Halogenalkyl, insbesondere Difluormethyl oder Trifluormethyl, ist, zu erhalten; oder

e) zuerst nach einem gängigen Verfahren halogeniert wird, um eine Zwischenverbindung, in der X Halogen ist, zu erhalten, aus der ein Organomagnesium- oder -lithium-Derivat hergestellt wird, wonach die organometallische Verbindung mit Oxodiperoxymolybdän(pyridin)-(hexamethylphosphorsäuretriamid) oder einem Trialkylborat und einem Oxidationsmittel, wie Wasserstoffperoxid, umgesetzt wird, um eine Zwischenverbindung der Formel (Ia), in der X Hydroxy ist, zu erhalten, welche dann mittels eines bekannten Halogenalkylierungsverfahrens umgesetzt wird, um eine Verbindung der Formel (Ia), in der X Halogenalkoxy ist, zu erhalten; oder

(B) entsprechend der Formel:

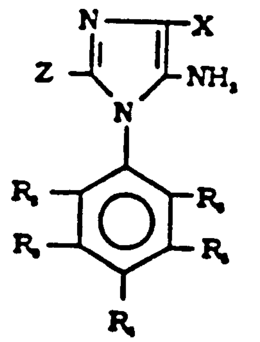

(Ib)

worin X, $R_2$, $R_3$, $R_4$, $R_5$ und $R_6$ wie für Formel (I) definiert sind und Z Aminosulfonyl, Alkylaminosulfonyl, Dialkylaminosulfonyl, Nitro, Amino, Halogen, Alkynyl, Alkyl, Hydroxy und Salze davon, Alkoxy, Halogenalkoxy, Formyl, Alkylsulfenyl, Halogenalkylsulfenyl, Alkylsulfinyl, Halogenalkylsulfinyl, Alkylsulfonyl, Halogenalkylsulfonyl oder Sulfhydryl und Salze davon ist, wobei eine Verbindung der Formel (Ia)

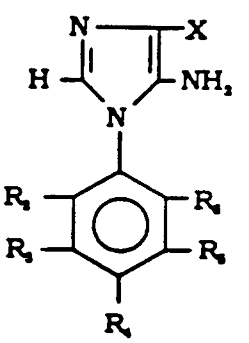

(Ia)

in der X und Amino gegebenenfalls nach Bedarf geschützt sind:

a) zuerst mit Chlorsulfon- oder Dichlorsulfonsäure umgesetzt wird, um eine Zwischenverbindung, worin Z Chlorsulfonyl ist, zu erhalten, welche Verbindung mit Ammoniak, einem Alkylamin oder Dialkylamin umgesetzt wird, um eine Verbindung der Formel (Ib) zu erhalten, in der Z Aminosulfonyl, Alkylaminosulfonyl oder Dialkylaminosulfonyl ist;

b) durch bekannte Verfahren halogeniert oder nitriert wird, um eine Verbindung der Formel (Ib), in der Z Halogen oder Nitro ist, zu erhalten, wonach die Verbindung, in der Z Nitro ist, gegebenenfalls durch ein bekanntes Verfahren reduziert wird, damit Z Amino ist, oder wonach gegebenenfalls die Verbindung, in der Z Halogen ist, mittels eines bekannten Verfahrens mit einem Kupferacetylid behandelt wird, um die Verbindung, in der Z Alkynyl ist, zu erhalten;

c) mit einer starken Base, wie einem Organolithiumreagenz umgesetzt wird, um ein organometallisches Carbanionintermediat zu erhalten, welches dann mit einem Alkylierungsmittel gequenscht wird, um eine Verbindung der Formel (Ib), in der Z Alkyl ist, zu erhalten, oder gegebenenfalls das Carbanion in einer Weise umgesetzt, die der in (A)e, obenstehend, beschriebenen ähnlich ist, um zuerst eine Verbindung der Formel (Ib), in der Z Hydroxyl oder ein Salz davon ist, zu erhalten

und dann gegebenenfalls die Verbindung, in der Z Hydroxy ist, zu einer Verbindung umzuwandeln, worin Z Alkoxy oder Halogenalkoxy ist, und zwar durch bekannte Alkylierungs- oder Halogenalkylierungsverfahren;

d) mittels Formylierungsverfahren, die den in (A)b oder (A)c obenstehend beschriebenen ähnlich sind, umgesetzt wird, wobei die Verbindung, in der Z Formyl ist, direkt durch Bedingungen, wie der Vilsmeier-Haack-Reaktion, hergestellt wird oder über die Hydrolyse einer Zwischenverbindung, in der Z Bis(alkylthio)methyl oder Bis(arylthio)methyl ist;

e) zuerst mit einer Mischung aus Brom und einem Metallthiocyanat umgesetzt wird, um eine Zwischenverbindung der Formel (Ib), in der Z Thiocyano ist, zu erhalten, welche dann mit einem Alkylierungsmittel, gegebenenfalls in Gegenwart einer Base, behandelt wird, um direkt eine Verbindung der Formel (Ib), in der Z Alkylsulfenyl oder Halogenalkylsulfenyl ist, zu erhalten, oder gegebenenfalls wird die Zwischenverbindung, in der Z Thiocyano ist, zuerst zu einer entsprechenden Disulfidzwischenverbindung oxidiert, welche dann mit einem Perhalogenalkan gegebenenfalls in Gegenwart eines Reduktionsmittels umgesetzt wird, um eine Verbindung der Formel (Ib), in der Z Halogenalkylsulfenyl ist, insbesondere Perhalogenalkylsulfenyl, ist, zu erhalten, wobei schließlich die Verbindung, in der Z Alkylsulfenyl oder Halogenalkylsulfenyl ist, gegebenenfalls durch bekannte Verfahren, die denen von (A)a, obenstehend, ähnlich sind, oxidiert wird, um eine Verbindung der Formel (Ib) zu erhalten, in der Z Alkylsulfinyl, Halogenalkylsulfinyl, Alkylsulfonyl oder Halogenalkylsulfonyl ist; oder

f) zuerst wie obenstehend in Abschnitt e) umgesetzt wird, um eine Zwischenverbindung, in der Z Thiocyano ist, zu erhalten, welche dann durch einen freien Radikalpromotor, wie Kaliumferricyanid, gespalten wird, um eine Verbindung der Formel (Ib), in der Z Sulfhydryl oder Salze davon bedeutet, zu erhalten; oder

(C) entsprechend der Formel:

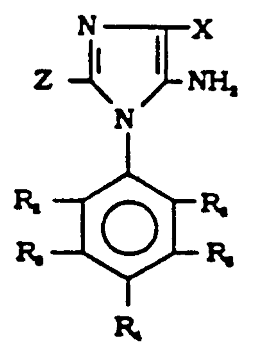

(Ib)

worin X, $R_2$, $R_3$, $R_4$, $R_5$ und $R_6$ wie für Formel (I) definiert sind und Z Amino, Alkyl, Cyano, Carboxyl und Salze davon, Aminocarbonyl, Alkylaminocarbonyl, Dialkylaminocarbonyl, Alkoxycarbonyl, Halogenalkyl, Cyanoalkyl, Alkenyl, Alkynyl, Alkylcarbonyl oder Halogenalkylcarbonyl ist, wobei eine Verbindung der Formel (Ib), in der Z Formyl ist, hergestellt mittels dem obenstehend in (B)d beschriebenen Verfahren, und in der X und Amino gegebenenfalls nach Bedarf geschützt sind:

a) zu einer Verbindung der Formel (Ib), in der Z Alkyl, insbesondere Methyl, ist, mittels bekannter Reduktionsmittel, wie Natriumborhydrid oder p-Toluolsulfonylhydrazin und Natriumcyanoborhydrid reduziert wird;

b) mit einem standardmäßigen bekannten Oxidationsmittel umgesetzt wird, um eine Verbindung der Formel (Ib), in der Z Carboxyl oder Salze davon bedeutet, zu erhalten, wonach die Carboxylverbindung gegebenenfalls zu einer Verbindung der Formel (Ib), in der Z Amino ist, durch eine Curtius-Umlagerung über ein Säurehalogenid-, Azid- und Isocyanat-Zwischenprodukt umgewandelt werden kann oder gegebenenfalls die Verbindung, in der Z Carboxyl ist, mit Isophthalonitril behandelt wird, um eine Verbindung der Formel (Ib), in der Z Cyano ist, zu erhalten, oder gegebenenfalls wird die Verbindung, in der Z Formyl ist, mit Hydroxylamin umgesetzt, um eine Aldoximzwischenverbindung zu erhalten, welche dann durch Standardverfahren dehydratisiert wird, um die Verbindung der Formel (Ib), in der Z Cyano ist, zu erhalten;

c) mittels der obenstehenden Prozedur b) zu der Verbindung, in der Z Carboxyl ist, umgewandelt wird, dann das Carboxyl durch Standardverfahren zu einer Säurehalogenidzwischenverbindung umgewandelt wird, welche dann mit Ammoniak, einem Alkylamin, Dialkylamin oder Alkylalkohol

umgesetzt wird, um eine Verbindung der Formel (Ib), in der Z Aminocarbonyl, Dialkylaminocarbonyl oder Alkoxycarbonyl ist, zu erhalten;

d) zuerst mittels Verfahren, die denen in (A)b, obenstehend, ähnlich sind, zu einer Hydroxymethylzwischenverbindung reduziert wird, gefolgt von Halogenierungsvorgängen,die ebenfalls denen von (A)b, obenstehend, ähnlich sind, um eine Verbindung der Formel (Ib), in der Z Halogenalkyl, insbesondere Halogenmethyl, ist, zu erhalten, oder gegebenenfalls wird die Halogenalkylverbindung, insbesondere Halogenmethyl, mit einem Metallcyanid behandelt, um eine Verbindung der Formel (Ib), in der Z Cyanoalkyl, insbesondere Cyanomethyl, ist, zu erhalten;

e) in einer Wittig- oder modifizierten Wittig-Reaktion umgesetzt wird, um eine Verbindung der Formel (Ib), in der Z Alkenyl oder Alkynyl ist, zu erhalten;

f) mit einem Grignard-Reagenz oder Alkyllithiumreagenz umgesetzt wird, um eine Zwischenverbindung der Formel (Ib), in der Z α-Hydroxyalkyl ist, zu erhalten, welche dann mittels bekannter Reagenzien oxidiert wird, um eine Verbindung der Formel (Ib), in der Z Alkylcarbonyl ist, zu erhalten, wobei dann die Alkylcarbonylverbindung gegebenenfalls zu einer Verbindung der Formel (Ib) halogeniert wird, in der Z Halogenalkylcarbonyl ist; oder

g) zuerst gemäß dem obigen Verfahren in den Teilen b) und c) zu dem Säurechloridintermediat, erhalten über die Verbindung, in der Z Carboxyl ist, umgewandelt wird, wonach durch herkömmliche Curtius-Umlagerungsvorgänge das Säurehalogenidintermediat über Azid- und Isocyanatintermediate zu der Verbindung der Formel (Ib), in der Z Amino ist, umgewandelt wird; oder

(D) entsprechend der Formel:

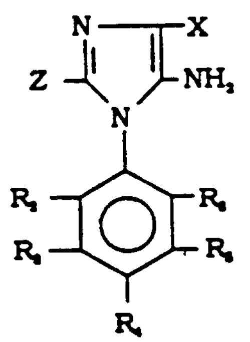

(Ib)

worin X, $R_2$, $R_3$, $R_4$, $R_5$ und $R_6$ wie für Formel (I) definiert sind und Z Alkylamino, Dialkylamino, Trialkylammoniumsalz, Alkoxycarbonylamino, Alkylaminocarbonylamino, Dialkylaminocarbonylamino, Alkoxyalkylidenimino, Alkylcarbonylamino, Halogenalkylcarbonylamino oder Arylcarbonylamino ist, worin eine Verbindung der Formel (Ib), in der Z Amino ist, hergestellt über die in (B)b oder (B)g, obenstehend, beschriebenen Verfahrensschritte hergestellt, und in der X und Amino gegebenenfalls nach Bedarf geschützt sind:

a) zuerst mit Phosgen umgesetzt wird, um eine Zwischenverbindung der Formel (Ib), in der Z Chlorcarbonylamino oder Isocyanat ist, zu erhalten, welche dann mit einem Alkylalkohol, Alkylamin oder Dialkylamin umgesetzt wird, um eine Verbindung der Formel (Ib), in der Z Alkoxycarbonylamino, Alkylaminocarbonylamino oder Dialkylaminocarbonylamino ist, zu erhalten;

b) mit einem Alkylierungsmittel, wie einem Alkyliodid oder Dialkylsulfat, oder gegebenenfalls mittels einer bekannten reduktiven Methylierung unter Verwendung von Formaldehyd und Ameisensäure umgesetzt wird, um eine Verbindung der Formel (Ib), in der Z Alkylamino, Dialkylamino oder Trialkylammoniumsalz ist, zu erhalten;

c) mit einem Alkylorthoformiat umgesetzt wird, um eine Verbindung der Formel (Ib), in der Z Alkoxyalkylidenimino, insbesondere Alkoxymethylidenimino, ist, zu erhalten; oder

d) mit einem Alkyl-, Halogenalkyl- oder Arylcarbonylhalogenid, gegebenenfalls in Gegenwart eines Säureakzeptors umgesetzt wird, um eine Verbindung der Formel (Ib), in der Z Alkylcarbonylamino, Halogenalkylcarbonylamino oder Arylcarbonylamino ist, zu erhalten; oder

(E) entsprechend der Formel:

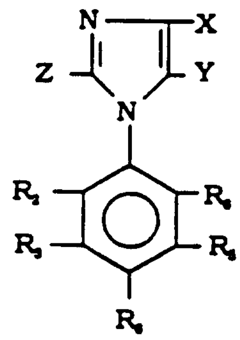

(I)

worin X, Z, $R_2$, $R_3$, $R_4$, $R_5$ und $R_6$ wie in Formel (I) definiert sind und Y Wasserstoff, Amino, Halogen, Alkylsulfenyl, Halogenalkylsulfenyl, Alkylsulfinyl, Halogenalkylsulfinyl, Alkylsulfonyl, Halogenalkylsulfonyl, Cyano oder Nitro ist, worin eine Verbindung der Formel (Ib)

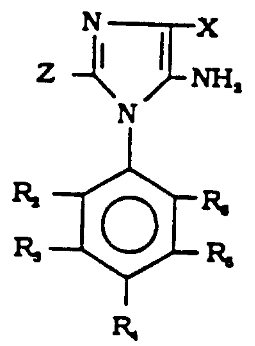

(Ib)

in der X, Z und $R_2$ bis $R_6$ wie obenstehend definiert sind, und in der X, Z und Amino gegebenenfalls nach Bedarf geschützt sind:

a) durch bekannte Verfahren, wie mit einem Alkylnitrit, desaminiert wird, um die Verbindung, in der Y Amino ist, in ihr entsprechendes Diazoniumsalz umzuwandeln, gefolgt von Quenschen des Diazoniumsalzes mit einem Quenschmittel gemäß den gängigen Verfahren, um eine Verbindung der Formel (I), in der Y Wasserstoff, Halogen, Cyano, Nitro, Alkylsulfenyl oder Halogenalkylsulfenyl ist, zu erhalten, und wonach die Verbindung, in der Y Alkylsulfenyl oder Halogenalkylsulfenyl ist, gegebenenfalls zu einer Verbindung der Formel (I), in der Y Alkylsulfinyl, Halogenalkylsulfinyl, Alkylsulfonyl oder Halogenalkylsulfonyl ist, zu oxidieren; oder

(F) entsprechend der Formel:

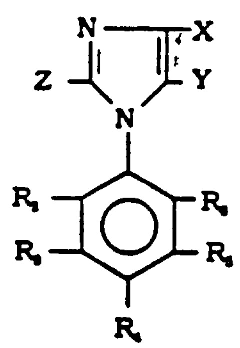

(I)

worin X, Z, $R_2$, $R_3$, $R_4$, $R_5$ und $R_6$ die gleiche Bedeutung wie für Formel (I) besitzen und Y Alkoxycarbonylamino, Alkylaminocarbonylamino, Dialkylaminocarbonylamino, Alkoxyalkylidenimino,

Alkylcarbonylamino, Halogenalkylcarbonylamino, Arylcarbonylamino, Alkylamino, Dialkylamino oder Trialkylammoniumsalz ist, worin eine Verbindung der Formel (Ib)

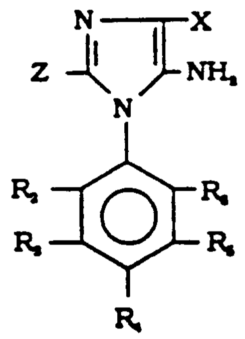

(Ib)

in der X, Z und $R_2$ bis $R_6$ wie oben definiert sind, und in der X, Z und Amino gegebenenfalls nach Bedarf geschützt sind:

a) in einer zu der in (D)a, obenstehend, beschriebenen ähnlichen Weise über ein Chlorcarbonylamino- oder Isocyanatintermediat, erhalten durch die Reaktion mit Phosgen, umgesetzt wird, welches dann mit einem Alkylalkohol, Alkylamin oder Dialkylamin umgesetzt wird, um eine Verbindung der Formel (I), in der Y Alkoxycarbonylamino, Alkylaminocarbonylamino oder Dialkylaminocarbonylamino ist, zu erhalten;

b) in einer ähnlichen Weise wie der in (D)c, obenstehend, beschriebenen mit einem Alkylorthoformiat umgesetzt wird, um eine Verbindung der Formel (I), in der Y Alkoxyalkylidenimino, insbesondere Alkoxymethylidenimino, ist, zu erhalten;

c) in einer ähnlichen Weise wie der in (D)b, obenstehend, beschriebenen durch Alkylierung oder reduktive Methylierung umgesetzt wird, um eine Verbindung der Formel (I), in der Y Alkylamino, Dialkylamino oder Trialkylammoniumsalz ist, zu erhalten; oder

d) in einer ähnlichen Weise wie der in (D)d, obenstehend, beschriebenen mit einem Alkyl-, Halogenalkyl- oder Arylcarbonylhalogenid umgesetzt wird, um eine Verbindung der Formel (I), in der Y Alkylcarbonylamino, Halogenalkylcarbonylamino oder Arylcarbonylamino ist, zu erhalten; oder

(G) entsprechend der Formel:

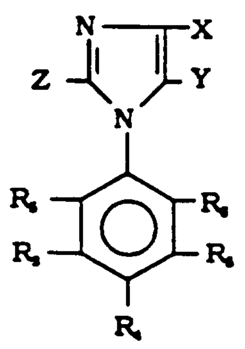

(I)

worin X, Z, $R_2$, $R_3$, $R_4$, $R_5$ und $R_6$ wie für Formel (I) definiert sind und Y Nitro, Sulfhydryl und Salze davon, Hydroxyl und Salze davon, Alkoxy, Halogenalkoxy, Aminosulfonyl, Alkylaminosulfonyl, Dialkylaminosulfonyl, Alkyl, Halogenalkyl, Alkenyl, Alkynyl, Cyanoalkyl oder Formyl ist, worin eine Verbindung der Formel (Ib)

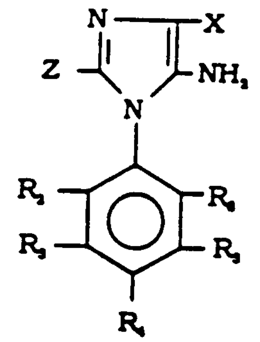

(Ib)

in der X, Z und $R_2$ bis $R_6$ die gleiche Bedeutung wie oben haben, und in der X und Z gegebenenfalls nach Bedarf geschützt sind, gemäß den in (E) obenstehenden Verfahren desaminiert wird, um eine Verbindung der Formel (I), in der Y Wasserstoff ist, zu erhalten, wonach dann die Verbindung, in der X und Z gegebenenfalls nach Bedarf geschützt sind:

a) mittels den in Anspruch (B)b beschriebenen ähnlichen Verfahren nitriert wird, um eine Verbindung der Formel (I), in der Y Nitro ist, zu erhalten;

b) in einer ähnlichen Weise durch das obenstehend in (B)f beschriebene Verfahren umgesetzt wird, um zuerst eine Zwischenverbindung, in der Y Thiocyano ist, zu erhalten, welche dann umgesetzt wird, um eine Verbindung der Formel (I), in der Y Sulfhydryl und Salze davon ist, zu erhalten;

c) zuerst mit einer starken Base, wie einem Organolithiumreagenz, umgesetzt wird, um ein Metallcarbanion-Intermediat zu erhalten, welches dann mit einem Elektrophilen gequenscht wird, um eine Verbindung der Formel (I), in der Y Alkyl, Halogenalkyl, Alkenyl, Alkynyl, Cyanoalkyl oder Formyl ist, zu erhalten;

d) zu dem Carbanion, wie oben in Abschnitt c), umgewandelt wird, und dann mit Sulfurylchlorid gequenscht wird, um eine Zwischenverbindung, in der Y Chlorsulfonyl ist, zu erhalten, welche dann mit Ammoniak oder einem Alkyl- oder Dialkylamin umgesetzt wird, um eine Verbindung der Formel (I), in der Y Aminosulfonyl, Alkylaminosulfonyl oder Dialkylaminosulfonyl ist, zu erhalten;

e) zu dem Carbanion, wie obenstehend in Abschnitt c), umgewandelt wird, oder gegebenenfalls das Carbanion über die Verbindung, in der Y Halogen ist, erhalten durch das obenstehende Verfahren von (E), hergestellt wird, und dann das Carbanion in einer dem in (B)c obenstehend beschriebenen Verfahren ähnlichen Weise umgesetzt wird, um eine Verbindung der Formel (I), in der Y Hydroxyl und Salze davon, Alkoxy oder Halogenalkoxy ist, zu erhalten; oder

(H) entsprechend der Formel:

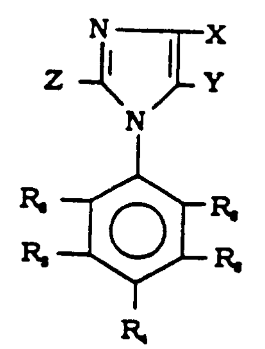

(I)

worin X, Z, $R_2$, $R_3$, $R_4$, $R_5$ und $R_6$ wie für Formel (I) definiert sind und Y Carboxyl oder Salze davon, Cyano, Aminocarbonyl, Alkylaminocarbonyl, Dialkylaminocarbonyl, Alkoxycarbonyl, Halogenalkyl, Alkenyl, Alkynyl, Alkylcarbonyl oder Halogenalkylcarbonyl ist, worin eine Verbindung der Formel (Ib)

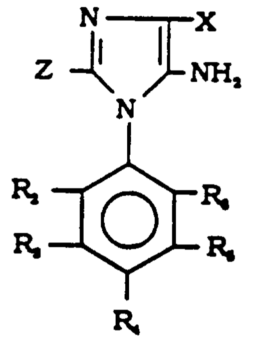

(Ib)

in der X, Z und $R_2$ bis $R_6$ die gleiche Bedeutung wie oben haben, zuerst gemäß den in (E) obenstehend beschriebenen Verfahren desaminiert wird, um eine Verbindung, in der Y Wasserstoff ist, zu erhalten, welche dann durch die in (G)c obenstehend beschriebenen Verfahren umgewandelt wird, um eine Verbindung der Formel (I), in der Y Formyl ist, zu erhalten, wonach die Formylverbindung, in der X und Z gegebenenfalls nach Bedarf geschützt sind:

a) durch ähnliche Verfahren, wie den obenstehend in C(b) beschriebenen, umgesetzt wird, um eine Verbindung der Formel (I), in der Y Carboxyl und Salze davon oder Cyano ist, zu erhalten;

b) durch ähnliche Verfahren, wie den obenstehend in C(c) beschriebenen, umgesetzt wird, um eine Verbindung der Formel (I), in der Y Aminocarbonyl, Alkylaminocarbonyl, Dialkylaminocarbonyl oder Alkoxycarbonyl ist, zu erhalten;

c) durch ähnliche Verfahren, wie den obenstehend in C(d) beschriebenen, umgesetzt wird, um eine Verbindung der Formel (I), in der Y Halogenalkyl, insbesondere Halogenmethyl, ist, zu erhalten;

e) durch ähnliche Verfahren, wie den obenstehend in (C)f beschriebenen, umgesetzt wird, um eine Verbindung der Formel (I), in der Y Alkylcarbonyl oder Halogenalkylcarbonyl ist, zu erhalten; oder

(I) entsprechend der Formel:

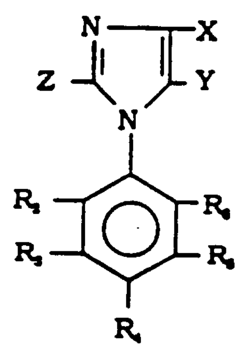

(I)

worin Y, Z, $R_2$, $R_3$, $R_4$, $R_5$ und $R_6$ die gleiche Bedeutung wie für Formel (I) besitzen und X Alkylsulfenyl, Halogenalkylsulfenyl, Alkylsulfinyl, Halogenalkylsulfinyl, Alkylsulfonyl oder Halogenalkylsulfonyl ist, wobei eine Verbindung der Formel (Ic)

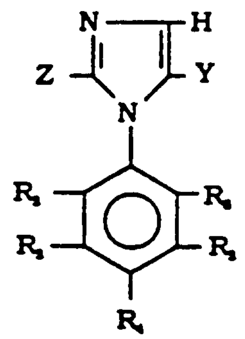

(Ic)

in der Y und Z gegebenenfalls nach Bedarf geschützt sind:

a) zuerst gemäß den Verfahren, die den obenstehend in (B)c beschriebenen ähnlich sind, umgesetzt, um eine Verbindung der Formel (Ic), in der X Wasserstoff ist, zu einer Zwischenverbindung der Formel (I), in der X nacheinander Thiocyano und dann ein Disulfid ist, umzuwandeln, wonach ebenfalls durch Verfahren, die den obenstehend in (B)c beschriebenen ähnlich sind, das Thiocyano- oder Disulfid-Intermediat zu einer Verbindung der Formel (I), in der X Alkylsulfenyl oder Halogenalkylsulfenyl, insbesondere Perhalogenalkylsulfenyl, ist, umzuwandeln, wobei die Verbindung dann gegebenenfalls mittels Verfahren, die den in (B)c obenstehend beschriebenen ähnlich sind, oxidiert wird, um das Sulfoxid- oder Sulfonanaloge, d.h. eine Verbindung der Formel (I), in der X Alkylsulfinyl, Halogenalkylsulfinyl, vorzugsweise Perhalogenalkylsulfinyl, Alkylsulfonyl oder Halogenalkylsulfonyl, insbesondere Perhalogenalkylsulfonyl ist, zu erhalten; oder

b) gemäß Verfahren, die den obenstehend in (B)a beschriebenen ähnlich sind, zuerst umgesetzt wird, um die Verbindung der Formel (Ic), in der X Wasserstoff ist, zu einer Zwischenverbindung der Formel (I), in der X Chlorsulfonyl ist, umzuwandeln, wonach die Chlorsulfonylverbindung mit einem Reduktionsmittel, wie Triphenylphosphin umgesetzt wird, um das gleiche Disulfid-Intermediat, obenstehend in Abschnitt a) beschrieben, zu erhalten, wonach schließlich das Disulfid durch die obenstehend in Abschnitt a) beschriebenen Verfahren umgewandelt wird, um eine Verbindung der Formel (I), in der X Alkylsulfenyl oder Halogenalkylsulfenyl, insbesondere Perhalogenalkylsulfenyl ist, zu erhalten, oder wobei gegebenenfalls die Sulfenylverbindung oxidiert wird, um eine Verbindung der Formel (I), in der X Alkylsulfinyl, Halogenalkylsulfinyl, insbesondere Perhalogenalkylsulfinyl, Alkylsulfonyl oder Halogenalkylsulfonyl, insbesondere Perhalogenalkylsulfonyl, ist, zu erhalten; oder

(J) entsprechend der Formel

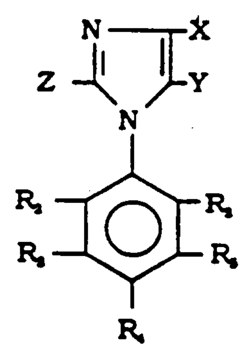

(IV)

worin:

| | |
|---|---|
| $R_2$, $R_3$, $R_4$, $R_5$ und $R_6$ | die gleiche Bedeutung haben, wie es obenstehend definiert ist; |
| X | Wasserstoff oder Halogenalkyl, insbesondere Trifluormethyl ist; |
| Y | Amino; Hydroxy, gegebenenfalls in seiner isomeren Keto-Form, wenn X Wasserstoff ist, vorliegend; Alkoxy oder Halogenalkoxy ist; und |
| Z | Wasserstoff; Halogen; Alkyl; Halogenalkyl; Hydroxy, gegebenenfalls in seiner isomeren Keto-Form, wenn X Wasserstoff ist, vorliegend, ist und Y Imino; Alkoxy oder Halogenalkoxy ist; |

wobei eine Verbindung der Formel (III)

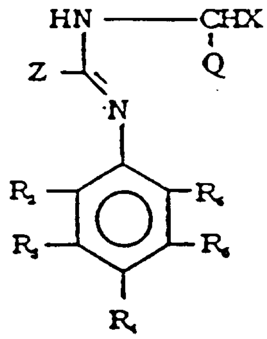

(III)

worin:

| | |
|---|---|
| $R_2$, $R_3$, $R_4$, $R_5$ und $R_6$ | wie oben definiert sind; |
| X | Wasserstoff oder Halogenalkyl, insbesondere Trifluormethyl, ist; |
| Z | Wasserstoff Halogen, Alkyl, Halogenalkyl oder Hydroxy, gegebenenfalls in seiner isomeren Keto-Form vorliegend, ist; und |
| Q | Cyano oder niederes Alkoxycarbonyl ist; |

mit einem basischen Mittel in einem geeigneten Reaktionsmedium umgesetzt wird, um eine Verbindung der Formel (IV), welche, wenn Y oder Z Hydroxy ist, anschließend gegebenenfalls alkyliert oder halogenalkyliert wird, damit Y oder Z Alkoxy oder Halogenalkoxy ist.

**2.** Verfahren zur Herstellung einer Verbindung der Formel (IV) gemäß Anspruch 1J, wobei die Verbindung der Formel (IV) folgendes ist:

a) eine Verbindung der in Anspruch 1 aufgeführten Formel <u>(5)</u>, in der X und Z jeweils Wasserstoff sind und Y Amino ist;

b) eine Verbindung der Formel <u>(17)</u>

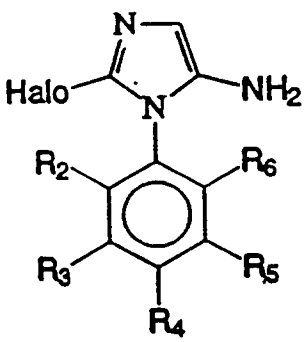

(17)

in der Halo für Halogen, insbesondere Chlor, steht;

c) eine Verbindung der Formel (22)

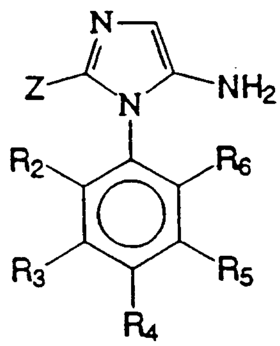

(22)

in der Z Alkyl oder Halogenalkyl ist;

d) eine Verbindung der Formel (27)

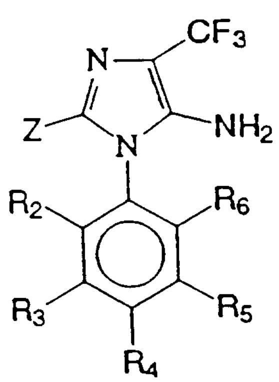

(27)

in der Z Halogen, Alkyl oder Halogenalkyl ist;

e) eine Verbindung der Formel (30), in der X Wasserstoff ist und Y Hydroxyl, gegebenenfalls in seiner isomeren Keto-Form (29) vorliegend, ist

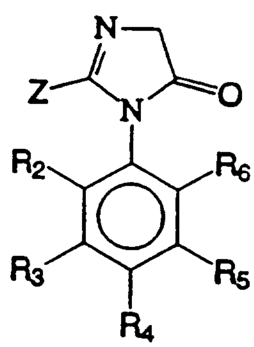

(29)

worin Z Halogen, Alkyl oder Halogenalkyl ist; oder

f) eine Verbindung der Formel (37), in der Y Amino ist und Z Hydroxy ist, gegebenenfalls in seiner isomeren Keto-Imino-Form (34) vorliegend, ist

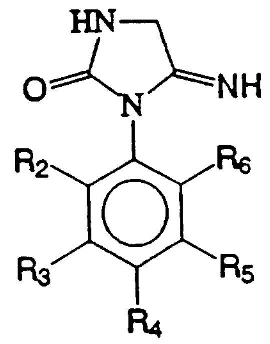

(34)

3. Verfahren zur Herstellung einer Verbindung der Formel (I):

(A) worin X, Y, Z, $R_2$, $R_3$, $R_4$, $R_5$ und $R_6$ wie in Anspruch 1 definiert sind, wobei eine Verbindung der in Anspruch 1 aufgeführten Formel (5) gemäß dem Herstellungsverfahren von Anspruch 1 zur Einführung der X-, Y- und Z-Substituenten umgesetzt wird;

(B) worin X, Y, $R_2$, $R_3$, $R_4$, $R_5$ und $R_6$ wie in Anspruch 1 definiert sind und Z Halogen ist, wobei eine Verbindung der in Anspruch 9 aufgeführten Formel (17) gemäß dem Herstellungsverfahren von Anspruch 1 zur Einführung der X- und Y-Substituenten umgesetzt wird;

(C) worin X, Y, $R_2$, $R_3$, $R_4$, $R_5$ und $R_6$ wie in Anspruch 1 definiert sind und Z Alkyl oder Halogenalkyl ist, wobei eine Verbindung der in Anspruch 2 aufgeführten Formel (22) gemäß dem Herstellungsverfahren von Anspruch 1 zur Einführung der X- und Y-Substituenten umgesetzt wird;

(D) wobei Y, $R_2$, $R_3$, $R_4$, $R_5$ und $R_6$ wie in Anspruch 1 definiert sind, X Halogenalkyl, insbesondere Trifluormethyl, ist und Z Halogen, Alkyl oder Halogenalkyl ist, wobei eine Verbindung der Formel (27) gemäß dem Verfahren von Anspruch 1 zur Einführung des Y-Substituenten umgesetzt wird;

(E) wobei X, $R_2$, $R_3$, $R_4$, $R_5$ und $R_6$ wie in Anspruch 1 definiert sind, Y Hydroxy, Alkoxy oder Halogenalkoxy ist, und Z Halogen, Alkyl oder Halogenalkyl ist, wobei eine Verbindung der Formel (30), gegebenenfalls in ihrer isomeren Keto-Form (29) vorliegend, aufgeführt in Anspruch 2, in der Y Hydroxy ist, gegebenenfalls so alkyliert, daß Y Alkoxy oder Halogenalkoxy ist, gemäß dem Verfahren von Anspruch 1 zur Einführung des X-Substituenten umgesetzt wird; oder

(F) worin X, Y, $R_2$, $R_3$, $R_4$, $R_5$ und $R_6$ wie in Anspruch 1 definiert sind und Z Hydroxy, Alkoxy, Halogenalkoxy oder Halogen ist, wobei eine Verbindung der Formel (37), gegebenenfalls in ihrer isomeren Keto-Imino-Form (34) vorliegend, aufgeführt in Anspruch 2, in der Z Hydroxy ist, gegebenenfalls so alkyliert, daß Z Alkoxy oder Halogenalkoxy ist oder gegebenenfalls so halogeniert, daß Z Halogen ist, gemäß dem Verfahren von Anspruch 1 zur Einführung der X- und Y-Substituenten umgesetzt wird.

4. Verbindung nach Anspruch 1, worin X Halogenalkoxy ist.

5. Verbindung nach Anspruch 1, worin X $S(O)_nR_1$ ist mit der Formel (II)

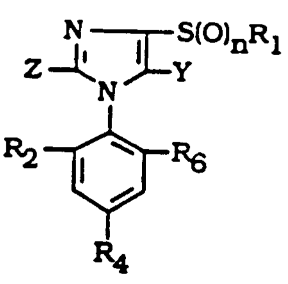

(II)

worin:

Y und Z individuell gewählt sind aus einem Wasserstoff- oder Halogenatom; einer aus Nitro, Cyano, Hydroxyl, Sulfhydryl, Amino, Alkylamino oder Dialkylamino gewählten Gruppe, wobei der angegebene Alkylteil jeder Gruppe eine lineare oder verzweigte Kette mit ein bis vier Kohlenstoffatomen ist; oder einer aus einem unsubstituierten oder vollständig

Halogen-substituierten Alkyl, Alkoxy, Alkylcarbonyl, Alkylcarbonylamino, Alkylsulfenyl, Alkylsulfinyl oder Alkylsulfonyl gewählten Gruppe, worin die definierten Alkyl- und Alkoxyteile jeder Gruppe eine lineare oder verzweigte Kette mit ein bis vier Kohlenstoffatomen bedeuten und die vollständige Halogen-Substitution des Alkyl- oder Alkoxyteils in gleichen oder unterschiedlichen Halogenatomen besteht; und wobei nur eines von Y und Z eine schwefelhaltige Gruppe ist;

$R_1$ eine lineare oder verzweigte Alkylgruppe mit ein bis vier Kohlenstoffatomen ist, welche unsubstituiert oder halogensubstituiert mit ein oder mehreren Halogenatomen, welche gleich oder verschieden sind, ist;

$R_2$ ein Wasserstoff- oder ein Halogenatom oder eine Alkyl-, Alkoxy-, Methylsulfenyl-, Methylsulfinyl- oder Methylsulfonylgruppe ist;

$R_4$ aus einem Halogenatom gewählt ist oder eine aus Trifluormethyl, Trifluormethoxy, Difluormethoxy, Trifluormethylsulfenyl, Trifluormethylsulfinyl, Trifluormethylsulfonyl gewählte Gruppe ist oder eine lineare oder verzweigtkettige Alkylgruppe mit ein bis vier Kohlenstoffatomen ist;

$R_6$ ein Halogenatom ist; und

n 0, 1 oder 2 ist.

**6.** Verfahren nach Anspruch 5, worin:

Y ein Wasserstoffatom, ein Halogenatom, Amino, Hydroxy, Alkoxy mit ein bis vier Kohlenstoffatomen, Methylsulfenyl, Methylsulfinyl oder Methylsulfonyl ist;

Z ein Wasserstoffatom, ein Halogenatom oder eine lineare oder verzweigte Alkylgruppe mit ein bis vier Kohlenstoffatomen, welche gegebenenfalls vollständig durch Halogenatome, welche gleich oder verschieden sein können, substituiert ist, bedeutet;

$R_1$ eine vollständig durch Halogenatome, welche gleich oder verschieden sind, substituierte Methylgruppe ist;

$R_2$ ein Wasserstoffatom, ein Halogenatom oder eine Methylsulfenylgruppe ist;

$R_4$ ein Halogenatom, Trifluormethyl oder Trifluormethoxy; und

$R_6$ ein Fluor-, Chlor- oder Bromatom ist.

**7.** Verfahren nach Anspruch 6, worin:

Y ein Wasserstoffatom, ein Chloratom, ein Bromatom, Methylsulfenyl, Methylsulfinyl oder Methoxy ist;

Z ein Wasserstoffatom, ein Chloratom, ein Bromatom oder Methyl ist;

$R_1$ Trifluormethyl, Dichlorfluormethyl oder Chlordifluormethyl ist;

$R_2$ ein Wasserstoffatom, ein Chloratom, ein Bromatom oder Methylsulfenyl ist;

$R_4$ ein Chloratom, ein Bromatom, ein Fluoratom, Trifluormethyl oder Trifluormethoxy ist; und

$R_6$ ein Chlor- oder Bromatom ist.

**8.** Verfahren nach Anspruch 7 zur Herstellung einer Verbindung der Formel I, welche ist:

1-(2,6-Dichlor-4-trifluormethylphenyl)-2-chlor-4-trifluormethylsulfenylimidazol;
1-(2,6-Dichlor-4-trifluormethylphenyl)-2-chlor-4-trifluormethylsulfinylimidazol;
1-(2,6-Dichlor-4-trifluormethylphenyl)-5-brom-4-dichlorfluormethylsulfenylimidazol;
1-(2,6-Dichlor-4-trifluormethylphenyl)-2-chlor-4-dichlorfluormethylsulfenylimidazol;
1-(2,6-Dichlor-4-trifluormethylphenyl)-2-brom-4-dichlorfluormethylsulfenylimidazol;
1-(2,6-Dichlor-4-trifluormethylphenyl)-4-chlor-4-trifluormethylsulfinylimidazol;
1-(2,6-Dichlor-4-trifluormethylphenyl)-2-chlor-4-dichlorfluormethylsulfinylimidazol;
1-(2,6-Dichlor-4-trifluormethylphenyl)-2-chlor-4-dichlorfluormethylsulfonylimidazol;
1-(2,6-Dichlor-4-trifluormethylphenyl)-2-bromo-4-chlordifluormethylsulfinylimidazol;
1-(2,6-Dichlor-4-trifluormethylphenyl)-2-brom-4-chlordifluormethylsulfenylimidazol;
1-(2,6-Dichlor-4-trifluormethylphenyl)-2-chlor-4-chlordifluormethylsulfenylimidazol;
1-(6-Chlor-2-methylsulfenyl-4-trifluormethylphenyl)-2-brom-4-chlordifluormethylsulfonylimidazol;
1-(6-Chlor-2-methylsulfenyl-4-trifluormethylphenyl)-2-chlor-4-dichlorfluormethylsulfonylimidazol;
1-(2,6-Dichlor-4-trifluormethylphenyl)-4-dichlorfluormethylsulfenylimidazol;
1-(2,6-Dichlor-4-trifluormethylphenyl)-4-dichlorfluormethylsulfinylimidazol;
1-(2,6-Dichlor-4-trifluormethylphenyl)-4-dichlorfluormethylsulfonylimidazol;
1-(2,6-Dichlor-4-trifluormethylphenyl)-2-methyl-4-dichlorfluormethylsulfenylimidazol;
1-(2,6-Dichlor-4-trifluormethylphenyl)-2-methyl-4-dichlorfluormethylsulfinylimidazol;

1-(2,6-Dichlor-4-trifluormethylphenyl)-2-methyl-4-dichlorfluormethylsulfonylimidazol;
1-(2,4,6-Trichlorphenyl)-4-dichlorfluormethylsulfenylimidazol;
1-(2,4,6-Trichlorphenyl)-4-dichlorfluormethylsulfinylimidazol;
1-(2,6-Dichlor-4-trifluormethoxyphenyl)-4-dichlorfluormethylsulfinylimidazol;
1-(2,6-Dichlor-4-trifluormethylphenyl)-4-chlordifluormethylsulfinylimidazol;
1-(2,6-Dichlor-4-trifluormethylphenyl)-2-methyl-4-chlordifluormethylsulfinylimidazol;
1-(2,6-Dichlor-4-trifluormethylphenyl)-2-methyl-4-chlordifluormethylsulfonylimidazol;
1-(2,4,6-Trichlorphenyl)-4-chlordifluormethylsulfenylimidazol; oder
1-(2,4,6-Trichlorphenyl)-4-chlordifluormethylsulfinylimidazol;

**9.** Verfahren nach Anspruch 7 zur Herstellung einer Verbindung der Formel (I), welche ist:
1-(2,6-Dichlor-4-trifluormethylphenyl)-2-chlor-4-trifluormethylsulfonylimidazol;
1-(2,6-Dichlor-4-trifluormethylphenyl)-5-methylsulfenyl-4-dichlorfluormethylsulfenylimidazol;
1-(2,6-Dichlor-4-trifluormethylphenyl)-4-trifluormethylsulfonylimidazol;
1-(2,6-Dichlor-4-trifluormethylphenyl)-5-brom-4-trifluormethylsulfonylimidazol;
1-(2,6-Dichlor-4-trifluormethylphenyl)-2-brom-4-dichlorfluormethylsulfinylimidazol;
1-(2,6-Dichlor-4-trifluormethylphenyl)-5-brom-4-dichlorfluormethylsulfonylimidazol;
1-(2,6-Dichlor-4-trifluormethylphenyl)-2-brom-4-dichlorfluormethylsulfonylimidazol;
1-(2,6-Dichlor-4-trifluormethylphenyl)-2-brom-4-chlordifluormethylsulfonylimidazol;
1-(2,6-Dichlor-4-trifluormethylphenyl)-2-chlor-4-chlordifluormethylsulfinylimidazol;
1-(2,6-Dichlor-4-trifluormethylphenyl)-2-chlor-4-chlordifluormethylsulfonylimidazol;
1-(2,6-Dichlor-4-trifluormethylphenyl)-5-methylsulfonyl-4-dichlorfluormethylsulfinylimidazol;
1-(2,6-Dichlor-4-trifluormethylphenyl)-5-methylsulfinyl-4-dichlorfluormethylsulfinylimidazol;
1-(6-Chlor-2-methylsulfenyl-4-trifluormethylphenyl)-2-chlor-4-dichlorfluormethylsulfenylimidazol;
1-(6-Chlor-2-methylsulfenyl-4-trifluormethylphenyl)-2-chlor-4-dichlorfluormethylsulfinylimidazol;
1-(2,6-Dichlor-4-trifluormethylphenyl)-2-chlor-5-methylsulfenyl-4-trifluormethylsulfenylimidazol;
1-(2,6-Dichlor-4-trifluormethylphenyl)-5-brom-4-trifluormethylsulfenylimidazol;
1-(2,6-Dichlor-4-trifluormethylphenyl)-2-methyl-4-chlordifluormethylsulfenylimidazol;
1-(2,6-Dichlor-4-trifluormethylphenyl)-5-chlor-4-trifluormethylsulfenylimidazol;
1-(2,6-Dichlor-4-trifluormethylphenyl)-2,5-dichlor-4-dichlorfluormethylsulfenylimidazol;
1-(2,6-Dichlor-4-trifluormethylphenyl)-4-chlordifluormethylsulfenylimidazol;
1-(2-Chlor-4-trifluormethylphenyl)-4-dichlorfluormethylsulfinylimidazol;
1-(2-Chlor-4-trifluormethylphenyl)-4-trifluormethylsulfinylimidazol;
1-(2-Chlor-4-trifluormethylphenyl)-4-trifluormethylsulfonylimidazol;
1-(2,6-Dichlor-4-bromphenyl)-4-dichlorfluormethylsulfenylimidazol;
1-(2,6-Dichlor-4-bromphenyl)-4-dichlorfluormethylsulfinylimidazol;
1-(2,6-Dichlor-4-bromphenyl)-4-chlordifluormethylsulfenylimidazol;
1-(2,6-Dichlor-4-trifluormethoxyphenyl)-4-trifluormethylsulfinylimidazol;
1-(2,6-Dichlor-4-trifluormethoxyphenyl)-4-chlordifluormethylsulfenylimidazol;
1-(2,6-Dichlor-4-trifluormethoxyphenyl)-4-chlordifluormethylsulfinylimidazol;
1-(2,6-Dichlor-4-trifluormethoxyphenyl)-4-dichlorfluormethylsulfonylimidazol; oder
1-(2,6-Dichlor-4-trifluormethoxyphenyl)-2-brom-4-dichlorfluormethylsulfenylimidazol.

**10.** Verfahren zur Herstellung einer Zusammensetzung zur Bekämpfung von Arthropoden-, Nemathoden-, Helminthen- oder Protozoen-Schädlingen, welches das Formulieren einer wirksamen Menge, vorzugsweise etwa 0,05 bis etwa 95 Gew.-%, einer Verbindung der Formel (I), wie sie in einem beliebigen der Ansprüche 1 bis 9 definiert ist, wobei jedoch Verbindungen eingeschlossen sind, worin (i) X Trifluormethyl ist, wenn $R_3$ für Methyl steht, $R_4$ für Nitro oder Amino steht und die anderen Symbole Wasserstoffrepräsentieren, und (ii) X Chlormethyl ist, wenn $R_4$ für Methyl, Methoxy oder Ethoxy steht und die anderen Symbole Wasserstoff repräsentieren, als einen aktiven Bestandteil, und einer oder mehrerer landwirtschaftlich oder medizinisch verträglicher Komponenten, umfassend vorzugsweise etwa 1 bis etwa 95 Gew.-% eines oder mehrerer fester oder flüssiger Träger, und etwa 0,1 bis etwa 50 Gew.-% einer oder mehrerer zusätzlicher Komponenten, wie oberflächenaktive Mittel, umfaßt.

**11.** Verfahren zur Herstellung einer Zusammensetzung die auf dem Gebiet der Veterinärmedizin und Tierhaltung oder zur Erhaltung der allgemeinen Gesundheit nützlich ist, umfassend das Formulieren einer oder mehrerer kompatibler Komponenten und als einen aktiven Bestandteil mindestens einer Verbindung der Formel (I), wie in Anspruch 10 definiert.

## Revendications

**Revendications pour les Etats contractants suivants : AT, BE, CH, LI, DE, DK, FR, GB, GR, IT, LU, NL, SE**

**1.** Composé de formule (I)

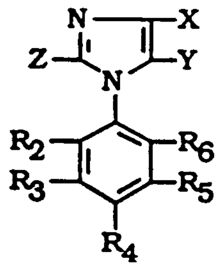

(I)

dans laquelle :

X est un groupe choisi entre halogénalkyle ou halogénalkoxy ou un groupe non substitué ou à substituant halogéno choisi entre alkylsulfényle, alkylsulfinyle ou alkylsulfonyle, dont les parties alkyle et alkoxy définies de chaque groupe sont à chaîne linéaire ou ramifiée, contenant un à quatre atomes de carbone, et la substitution halogéno de chaque groupe consiste en un ou plusieurs atomes d'halogènes, qui sont identiques ou différents, jusqu'à et y compris la substitution totale de la partie alkyle ou alkoxy ;

Y et Z sont choisis chacun, individuellement, entre : un atome d'hydrogène ou un atome d'halogène ; un groupe choisi entre nitro, cyano, hydroxyle et ses sels acceptables, sulfhydryle et ses sels acceptables, formyle, hydroxycarbonyle et ses sels acceptables, alkoxycarbonyle, aminocarbonyle, alkylaminocarbonyle, dialkylaminocarbonyle, amino, alkylamino, dialkylamino, sel de trialkylammonium, cyanalkyle, alkoxycarbonylamino, arylcarbonylamino, alkylaminocarbonylamino, dialkylaminocarbonylamino, aminosulfonyle, alkylaminosulfonyle, dialkylaminosulfonyle ou alkoxyalkylidène-imino, les parties alkyle et alkoxy définies de chaque groupe étant à chaîne linéaire ou à chaîne ramifiée contenant un à quatre atomes de carbone ; un groupe alcényle ou alcynyle à chaîne linéaire ou ramifiée contenant deux à quatre atomes de carbone ; ou un groupe choisi entre alkyle, alkoxy, alkylcarbonyle, alkylcarbonylamino, alkylsulfényle, alkylsulfinyle ou alkylsulfonyle non substitué ou à substituant halogéno, les parties alkyle et alkoxy définies de chaque groupe étant à chaîne linéaire ou à chaîne ramifiée contenant un à quatre atomes de carbone, et la substitution halogéno consistant en un ou plusieurs atomes d'halogènes, identiques ou différents, jusqu'à et y compris la substitution totale de la partie alkyle ou alkoxy ; et un un seul de Y et Z est un groupe contenant du soufre ; et

$R_2$, $R_3$, $R_4$, $R_5$ et $R_6$ sont choisis, individuellement, entre : un atome d'hydrogène ou un atome d'halogène ; un groupe choisi entre nitro, cyano, amino, alkylamino ou dialkylamino, la partie alkyle de chaque groupe étant à chaîne linéaire ou à chaîne ramifiée contenant un à quatre atome de carbone ; un groupe alcényle ou alcynyle à chaîne linéaire ou ramifiée contenant deux à quatre atomes de carbone, qui peut être substitué par un ou plusieurs atomes d'halogènes, identiques ou différents, jusqu'à et y compris la substitution totale ; ou un groupe choisi entre alkyle, alkoxy, alkylsulfényle, alkylsulfinyle ou alkylsulfonyle non substitué ou à substituant halogéno, les parties alkyle et alkoxy définies de chaque groupe étant à chaîne linéaire ou à chaîne ramifiée contenant un à quatre atomes de carbone et la substitution halogéno consistant en un ou plusieurs atomes d'halogènes, identiques ou différents, jusqu'à et y compris la substitution totale de la partie alkyle ou alkoxy, sous réserve que

(i) X représente autre chose qu'un groupe trifluorométhyle lorsque $R_3$ représente un groupe méthyle, $R_4$ représente un groupe nitro ou amino et les autres symboles représentent de l'hydrogène et
(ii) X représente autre chose qu'un groupe chlorométyle lorsque $R_4$ représente un groupe méthyle, méthoxy ou éthoxy et les autres symboles représentent de l'hydrogène.

2. Composé suivant la revendication 1, dans lequel X est un groupe halogénalkoxy.

3. Composé suivant la revendication 1, dans lequel X est un groupe $S(O)_nR_1$, de formule (II)

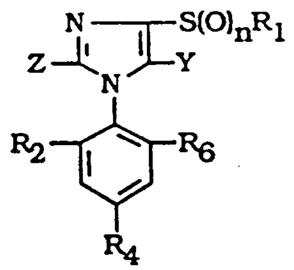

(II)

dans laquelle :

Y et Z sont choisis, individuellement, entre un atome d'hydrogène ou d'halogène ; un groupe choisi entre nitro, cyano, hydroxyle, sulfhydryle, amino, alkylamino ou dialkylamino, la partie alkyle définie de chaque groupe étant à chaîne linéaire ou à chaîne ramifiée contenant un à quatre atomes de carbone ; ou un groupe choisi entre alkyle, alkoxy, alkylcarbonyle, alkylcarbonylamino, alkylsulfényle, alkylsulfinyle ou alkylsulfonyle non substitué ou entièrement substitué par un halogène, les parties alkyle et alkoxy définies de chaque groupe étant à chaîne linéaire ou à chaîne ramifiée contenant un à quatre atomes de carbone, et la substitution totale par un halogène de la partie alkyle ou alkoxy est effectuée par le même atome d'halogène ou par des atomes d'halogènes différents ; et un seul de Y et Z est un groupe contenant du soufre ;

$R_1$ est un groupe alkyle à chaîne linéaire ou ramifiée ayant un à quatre atomes de carbone, non substitué ou substitué par un ou plusieurs atomes d'halogènes qui sont identiques ou différents;

$R_2$ est un atome d'hydrogène ou un atome d'halogène ou un groupe alkyle, alkoxy, méthylsulfényle, méthylsulfinyle ou méthylsulfonyle ;

$R_4$ est choisi entre un atome d'halogène ou un groupe choisi entre trifluorométhyle, trifluorométhoxy, difluorométhoxy, trifluorométhylsulfényle, trifluorométhylsulfinyle, trifluorométhylsulfonyle ou un groupe alkyle à chaîne linéaire ou ramifiée contenant un à quatre atomes de carbone ;

$R_6$ est un atome d'halogène ; et

n a la valeur 0, 1 ou 2.

4. Composé suivant la revendication 3, dans lequel :

Y est un atome d'hydrogène, un atome d'halogène, un groupe amino, hydroxy, alkoxy de un à quatre atomes de carbone, méthylsulfényle, méthylsulfinyle ou méthylsulfonyle ;

Z est un atome d'hydrogène, un atome d'halogène ou un groupe alkyle à chaîne linéaire ou à chaîne ramifiée ayant un à quatre atomes de carbone, qui est entièrement substitué, à titre facultatif par des atomes d'halogènes qui sont identiques ou différents ;

$R_1$ est un groupe méthyle entièrement substitué par des atomes d'halogènes qui sont identiques ou différents ;

$R_2$ est un atome d'hydrogène, un atome d'halogène ou un groupe méthylsulfényle ;

$R_4$ est un atome d'halogène, un groupe trifluorométhyle ou trifluorométhoxy ; et

$R_6$ est un atome de fluor, de chlore ou de brome.

**5.** Composé suivant la revendication 4, caractérisé en ce que :

Y est un atome d'hydrogène, un atome de chlore, un atome de brome, un groupe méthylsulfényle, méthylsulfinyle ou méthoxy ;

Z est un atome d'hydrogène, un atome de chlore, un atome de brome ou un groupe méthyle ;

$R_1$ est un groupe trifluorométhyle, dichlorofluorométhyle ou chlorodifluorométhyle ;

$R_2$ est un atome d'hydrogène, un atome de chlore, un atome de brome ou un groupe méthylsulfényle ;

$R_4$ est un atome de chlore, un atome de brome, un atome de fluor, un groupe trifluorométhyle ou trifluorométhoxy ; et

$R_6$ est un atome de chlore ou de brome.

**6.** Composé suivant la revendication 5, qui est :

le 1-(2,6-dichloro-4-trifluorométhylphényl)-2-chloro-4-trifluorométhylsulfénylimidazole ;
le 1-(2,6-dichloro-4-trifluorométhylphényl)-2-chloro-4-trifluorométhylsulfinylimidazole ;
le 1-(2,6-dichloro-4-trifluorométhylphényl)-5-bromo-4-dichlorofluorométhylsulfénylimidazole ;
le 1-(2,6-dichloro-4-trifluorométhylphényl)-2-chloro-4-dichlorofluorométhylsulfénylimidazole ;
le 1-(2,6-dichloro-4-trifluorométhylphényl)-2-bromo-4-dichlorofluorométhylsulfénylimidazole ;
le 1-(2,6-dichloro-4-trifluorométhylphényl)-4-trifluorométhylsulfinylimidazole ;
le 1-(2,6-dichloro-4-trifluorométhylphényl)-2-chloro-4-dichlorofluorométhylsulfinylimidazole ;
le 1-(2,6-dichloro-4-trifluorométhylphényl)-2-chloro-4-dichlorofluorométhylsulfonylimidazole ;
le 1-(2,6-dichloro-4-trifluorométhylphényl)-2-bromo-4-chlorodifluorométhylsulfinylimidazole ;
le 1-(2,6-dichloro-4-trifluorométhylphényl)-2-bromo-4-chlorodifluorométhylsulfénylimidazole ;
le 1-(2,6-dichloro-4-trifluorométhylphényl)-2-chloro-4-chlorodifluorométhylsulfénylimidazole ;
le 1-(6-chloro-2-méthylsulfényl-4-trifluorométhylphényl)-2-bromo-4-chlorodifluorométhylsulfonylimidazole ;
le 1-(6-chloro-2-méthylsulfényl-4-trifluorométhylphényl)-2-chloro-4-dichlorofluorométhylsulfonylimidazole ;
le 1-(2,6-dichloro-4-trifluorométhylphényl)-4-dichlorofluorométhylsulfénylimidazole ;
le 1-(2,6-dichloro-4-trifluorométhylphényl)-4-dichlorofluorométhylsulfinylimidazole ;
le 1-(2,6-dichloro-4-trifluorométhylphényl)-4-dichlorofluorométhylsulfonylimidazole ;
le 1-(2,6-dichloro-4-trifluorométhylphényl)-2-méthyl-4-dichlorofluorométhylsulfénylimidazole ;
le 1-(2,6-dichloro-4-trifluorométhylphényl)-2-méthyl-4-dichlorofluorométhylsulfinylimidazole ;
le 1-(2,6-dichloro-4-trifluorométhylphényl)-2-méthyl-4-dichlorofluorométhylsulfonylimidazole ;
le 1-(2,4,6-trichlorophényl)-4-dichlorofluorométhylsulfénylimidazole;
le 1-(2,4,6-trichlorophényl)-4-dichlorofluorométhylsulfinylimidazole ;
le 1-(2,6-dichloro-4-trifluorométhoxyphényl)-4-dichlorofluorométhylsulfinylimidazole ;
le 1-(2,6-dichloro-4-trifluorométhylphényl)-4-chlorodifluorométhylsulfinylimidazole ;
le 1-(2,6-dichloro-4-trifluorométhylphényl)-2-méthyl-4-chlorodifluorométhylsulfinylimidazole ;
le 1-(2,6-dichloro-4-trifluorométhylphényl)-2-méthyl-4-chlorodifluorométhylsulfonylimidazole ;
le 1-(2,4,6-trichlorophényl)-4-chlorodifluorométhylsulfénylimidazole; ou
le 1-(2,4,6-trichlorophényl)-4-chlorodifiuorométhylsulfinylimidazole.

**7.** Composé suivant la revendication 5, qui est :

le 1-(2,6-dichloro-4-trifluorométhylphényl)-2-chloro-4-trifluorométhylsulfonylimidazole ;
le 1-(2,6-dichloro-4-trifluorométhylphényl)-5-méthylsulfényl-4-dichlorofluorométhylsulfénylimidazole ;
le 1-(2,6-dichloro-4-trifluorométhylphényl)-4-trifluorométhylsulfonylimidazole ;
le 1-(2,6-dichloro-4-trifluorométhylphényl)-5-bromo-4-trifluorométhylsulfonylimidazole ;
le 1-(2,6-dichloro-4-trifluorométhylphényl)-2-bromo-4-dichlorofluorométhylsulfinylimidazole ;
le 1-(2,6-dichloro-4-trifluorométhylphényl)-5-bromo-4-dichlorofluorométhylsulfonylimidazole ;
le 1-(2,6-dichloro-4-trifluorométhylphényl)-2-bromo-4-dichlorofluorométhylsulfonylimidazole ;
le 1-(2,6-dichloro-4-trifluorométhylphényl)-2-bromo-4-chlorodifluorométhylsulfonylimidazole ;
le 1-(2,6-dichloro-4-trifluorométhylphényl)-2-chloro-4-chlorodifluorométhylsulfinylimidazole ;
le 1-(2,6-dichloro-4-trifluorométhylphenyl)-2-chloro-4-chlorodifluorométhylsulfonylimidazole ;
le 1-(2,6-dichloro-4-trifluorométhylphényl)-5-méthylsulfonyl-4-dichlorofluorométhylsulfinylimidazole ;
le 1-(2,6-dichloro-4-trifluorométhyl)-5-méthylsulfinyl-4-dichlorofluorométhylsulfinylimidazole ;
le 1-(6-chloro-2-méthylsulfényl-4-trifluorométhylphényl)-2-chloro-4-dichlorofluorométhylsulfénylimidazole ;
le 1-(6-chloro-2-méthylsulfényl-4-trifluorométhylphényl)-2-chloro-4-dichlorofluorométhylsulfinylimidazole ;
le 1-(2,6-dichloro-4-trifluorométhylphényl)-2-chloro-5-méthylsulfényl-4-trifluorométhylsulfénylimidazole ;
le 1-(2,6-dichloro-4-trifluorométhylphényl)-5-bromo-4-trifluorométhylsulfénylimidazole ;
le 1-(2,6-dichloro-4-trifluorométhylphényl)-2-méthyl-4-chlorodifluorométhylsulfénylimidazole ;
le 1-(2,6-dichloro-4-trifluorométhylphényl)-5-chloro-4-trifluorométhylsulfénylimidazole ;
le1-(2,6-dichloro-4-trifluorométhylphényl)-2,5-dichloro-4-dichlorofluorométhylsulfénylimidazole ;
le 1-(2,6-dichloro-4-trifluorométhylphényl)-4-chlorodifluorométhylsulfénylimidazole ;
le 1-(2-chloro-4-trifluorométhylphényl)-4-dichlorofluorométhylsulfinylimidazole ;
le 1-(2-chloro-4-trifluorométhylphényl)-4-trifluorométhylsulfinylimidazole ;
le 1-(2-chloro-4-trifluorométhylphényl)-4-trifluorométhylsulfonylimidazole ;
le 1-(2,6-dichloro-4-bromophényl)-4-dichlorofluorométhylsulfénylimidazole ;
le 1-(2,6-dichloro-4-bromophényl)-4-dichlorofluorométhylsulfinylimidazole ;
le 1-(2,6-dichloro-4-bromophényl)-4-chlorodifluorométhylsulfénylimidazole ;
le 1-(2,6-dichloro-4-trifluorométhoxyphényl)-4-trifluorométhylsulfinylimidazole ;
le 1-(2,6-dichloro-4-trifluorométhoxyphényl)-4-chlorodifluorométhylsulfénylimidazole ;
le 1-(2,6-dichloro-4-trifluorométhoxyphényl)-4-chlorodifluorométhylsulfinylimidazole ;
le 1-(2,6-dichloro-4-trifluorométhoxyphényl)-4-dichlorofluorométhylsulfonylimidazole ; ou
le 1-(2,6-dichloro-4-trifluorométhoxyphényl)-2-bromo-4-dichlorofluorométhylsulfénylimidazole.

**8.** Procédé de préparation d'un composé de formule (I) :
(A) répondant à la formule :

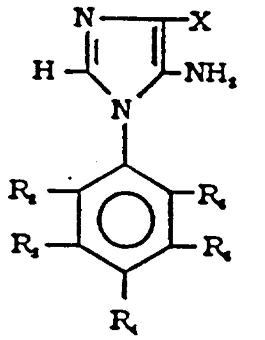

(Ia)

dans laquelle $R_2$, $R_3$, $R_4$, $R_5$ et $R_6$ ont les définitions données pour la formule (I) dans la revendication 1 et X est un groupe alkylsulfényle, halogénalkylsulfényle, alkylsulfinyle, halogénalkylsulfinyle, alkylsulfonyle, halogénalkylsulfonyle, halogénalkyle ou halogénalkoxy, dans lequel un composé de formule (5)

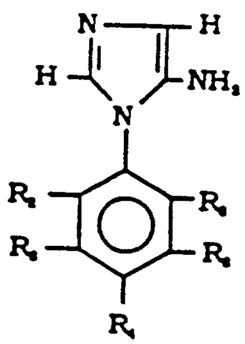

5

dans laquelle le groupe amino est facultativement protégé si nécessaire :
a) est tout d'abord amené à réagir avec un halogénure de sulfényle, $R_1$SHalo, dans lequel $R_1$ est un groupe alkyle ou halogénalkyle, dans un milieu réactionnel organique, facultativement en présence d'un accepteur d'acide tel qu'une amine tertiaire afin d'obtenir un composé de formule

(Ia) dans laquelle X est un groupe alkylsulfényle ou halogénalkylsulfényle, qui est ensuite facultativement oxydé par des opérations connues, par exemple à l'aide d'un peroxyde, pour obtenir un composé de formule (Ia) dans laquelle X est un groupe $S(O)_nR_1$ dans laquelle n a la valeur 1 ou 2 et $R_1$ est tel que défini ci-dessus, c'est-à-dire que X est un groupe alkylsulfinyle, halogénalkylsulfinyle, alkylsulfonyle ou halogénalkylsulfonyle ;

b) est tout d'abord amené à réagir avec un tris(alkylthio)méthane ou un tris(arylthio)méthane dans un milieu réactionnel organique en présence d'un acide de Lewis et, à titre facultatif, en présence d'un accepteur d'acide, puis le composé intermédiaire obtenu de formule (10) dans laquelle X est un groupe bis(alkylthio)méthyle ou bis(arylthio)méthyle est amené à réagir, dans un milieu réactionnel organique, avec un nitrite d'alkyle convenable, la réaction étant suivie d'une opération d'hydrolyse en vue d'obtenir un composé intermédiaire de formule (Ia) dans laquelle X est un groupe formyle, l'hydrolyse étant suivie d'opérations connues de réduction en vue d'obtenir un composé intermédiaire dans lequel X est un groupe hydroxyméthyle, ce composé étant finalement halogéné par des opérations connues en donnant un composé de formule (Ia) dans laquelle X est un groupe halogénalkyle, plus particulièrement un groupe halogénométhyle.

c) est tout d'abord formylé par des opérations bien connues telles que la réaction de Vilsmeier-Haack et une réaction similaire, avec formation du composé de formule (Ia) dans laquelle X est un groupe formyle, composé qui est ensuite amené à réagir selon les modes opératoires indiqués ci-dessus dans la partie b) en donnant de même le composé de formule (Ia) dans laquelle X est un groupe halogénalkyle ;

d) est amené à réagir selon les modes opératoires de la partie b) ou c) ci-dessus en donnant un composé intermédiaire de formule (Ia) dans laquelle X est un groupe formyle, ce composé pouvant être facultativement oxydé en un composé intermédiaire de formule (Ia) dans laquelle X est un groupe carboxyle, puis le composé intermédiaire dans lequel X est un groupe formyle est finalement amené à réagir avec un agent d'halogénation tel que le trifluorure de diéthylaminosoufre, ou bien le composé intermédiaire, dans lequel X est un groupe carboxyle, est amené à réagir avec le tétrafluorure de soufre en donnant un composé de formule (Ia) dans laquelle X est un groupe halogénalkyle, plus particulièrement le groupe difluorométhyle ou trifluorométhyle ; ou bien

e) est tout d'abord halogéné par des modes opératoires bien connus en donnant un composé intermédiaire dans lequel X est un halogène, à partir duquel on prépare un dérivé organomagnésien ou un dérivé organique de lithium, puis ledit composé organométallique est amené à réagir avec l'oxodiperoxymolybdène(pyridine)-(hexaméthylphosphorotriamide) ou un borate de trialkyle et un agent oxydant tel que le peroxyde d'hydrogène en donnant un composé intermédiaire de formule (Ia) dans laquelle X est un groupe hydroxy, et ce composé est finalement amené à réagir par des opérations connues d'halogénalkylation en donnant un composé de formule (Ia) dans laquelle X est un groupe halogénalkoxy ; ou

(B) répondant à la formule :

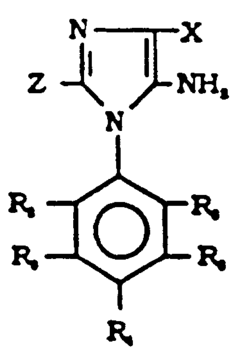

(Ib)

dans laquelle X, $R_2$, $R_3$, $R_4$, $R_5$ et $R_6$ sont tels que définis pour la formule (I) dans la revendication 1 et Z est un groupe aminosulfonyle, alkylaminosulfonyle, dialkylaminosulfonyle, nitro, amino, un halogène, un groupe alcynyle, alkyle, hydroxy et ses sels, alkoxy, halogénalkoxy, formyle, alkylsulfényle, halogénalkylsulfényle, alkylsulfinyle, halogénalkylsulfinyle, alkylsulfonyle, halogénalkylsulfonyle ou sulfhydryle et ses sels, dans lequel un composé de formule (Ia)

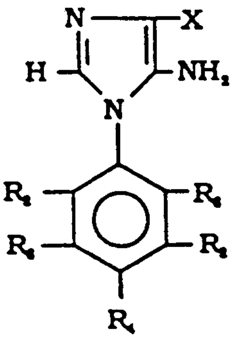

(Ia)

dans laquelle X et le groupe amino sont facultativement protégés si nécessaire :

a) est tout d'abord amené à réagir avec l'acide chlorosulfonique ou dichlorosulfonique en donnant un composé intermédiaire dans lequel Z est un groupe chlorosulfonyle, composé qui est amené à réagir avec l'ammoniac, une alkylamine ou une dialkylamine en donnant un composé de formule (Ib) dans laquelle Z est un groupe aminosulfonyle, alkylaminosulfonyle ou dialkylaminosulfonyle ;

b) est halogéné ou nitré par des opérations connues en donnant un composé de formule (Ib) dans laquelle Z est un halogène ou un groupe nitro, puis le composé dans lequel Z est un groupe nitro est facultativement réduit en composé dans lequel Z est un groupe amino par des opérations connues, ou bien à titre facultatif, le composé dans lequel Z est un halogène est traité selon des modes opératoires connus avec un acétylure de cuivre en donnant le composé dans lequel Z est un groupe alcynyle ;

c) est amené à réagir avec une base forte telle qu'un composé réactif organique de lithium en donnant un carbanion organométallique intermédiaire qui est ensuite désactivé avec un agent alkylant en donnant un composé de formule (Ib) dans laquelle Z est un groupe alkyle, ou bien à titre facultatif, le carbanion est amené à réagir d'une manière semblable à la réaction décrite en (A)e ci-dessus en donnant tout d'abord un composé de formule (Ib) dans laquelle Z est un groupe hydroxyle ou ses sels, puis à titre facultatif, le composé dans lequel Z est un groupe hydroxy est converti en un composé dans lequel Z est un groupe alkoxy ou halogénalkoxy par des opérations connues d'alkylation ou d'halogénalkylation ;

d) est amené à réagir par des modes opératoires de formylation analogues à ceux qui sont décrits en (A)b ou (A)c ci-dessus, où le composé dans lequel Z est un groupe formyle est préparé directement dans des conditions telles que la réaction de Vilsmeier-Haack ou par hydrolyse d'un composé intermédiaire dans lequel Z est un groupe bis(alkylthio)méthyle ou bis-(arylthio)méthyle ;

e) est tout d'abord amené à réagir avec un mélange de brome et d'un thiocyanate métallique en donnant un composé intermédiaire de formule (Ib) dans laquelle Z est un groupe thiocyano, qui est ensuite traité avec un agent d'alkylation, facultativement en présence d'une base, en donnant directement un composé de formule (Ib) dans laquelle Z est un groupe alkylsulfényle ou halogénalkylsulfényle, ou bien à titre facultatif, le composé intermédiaire dans lequel Z est un groupe thiocyano est tout d'abord oxydé en un disulfure intermédiaire correspondant qui est ensuite amené à réagir avec un perhalogénalcane, facultativement en présence d'un agent réducteur, en donnant un composé de formule (Ib) dans laquelle Z est un groupe halogénalkylsulfényle, en particulier un groupe perhalogénalkylsulfényle, finalement le composé dans lequel Z est un groupe alkylsulfényle ou halogénalkylsulfényle est facultativement oxydé par des opérations connues analogues à celles qui sont indiquées en a) dans la revendication 17 en donnant un composé de formule (Ib) dans laquelle Z est un groupe alkylsulfinyle, halogénalkylsulfinyle, alkylsulfonyle ou halogénalkylsulfonyle ; ou bien

f) est tout d'abord amené à réagir comme dans la partie e) ci-dessus en donnant le composé intermédiaire dans lequel Z est un groupe thiocyano, qui est ensuite clivé par un promoteur de radicaux libres tel que le ferricyanure de potassium, en donnant un composé de formule (Ib) dans laquelle Z est un groupe sulfhydryle ou ses sels ; ou

(C) répondant à la formule :

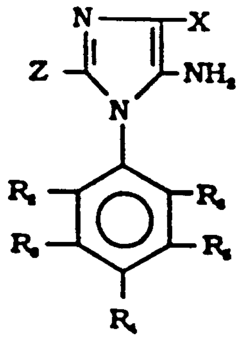

(Ib)

dans laquelle X, $R_2$, $R_3$, $R_4$, $R_5$ et $R_6$ ont la définition donnée pour la formule (I) dans la revendication 1 et Z est un groupe amino, alkyle, cyano, carboxyle et ses sels, aminocarbonyle, alkylaminocarbonyle, dialkylaminocarbonyle, alkoxycarbonyle, halogénalkyle, cyanalkyle, alcényle, alcynyle, alkylcarbonyle ou halogénalkylcarbonyle, caractérisé en ce qu'un composé de formule (Ib) dans laquelle Z est un groupe formyle, préparé selon les modes opératoires décrits en (B)d ci-dessus, et dans laquelle X et le groupe amino sont facultativement protégés si nécessaire :

a) est réduit en un composé de formule (Ib) dans laquelle Z est un groupe alkyle, en particulier le groupe méthyle, par des agents réducteurs connus tels que le borohydrure de sodium ou la p-toluènesulfonylhydrazine et le cyanoborohydrure de sodium ;

b) est amené à réagir avec un agent oxydant connu classique pour former un composé de formule (Ib) dans laquelle Z est un groupe carboxyle ou ses sels, puis à titre facultatif, le composé à groupe carboxyle peut être converti en un composé de formule (Ib) dans laquelle Z est un groupe amino, par une transposition de Curtius passant par un halogénure d'acide, un azide ou un isocyanate intermédiaire, ou bien à titre facultatif, le composé dans lequel Z est un groupe carboxyle est traité avec l'isophtalonitrile en donnant un composé de formule (Ib) dans laquelle Z est un groupe cyano, ou bien le composé dans lequel Z est un groupe formyle est facultativement amené à réagir avec l'hydroxylamine en donnant une aldoxime intermédiaire qui est ensuite déshydratée par des modes opératoires classiques en donnant le composé de formule (Ib) dans laquelle Z est un groupe cyano ;

c) est converti en le composé dans lequel Z est un groupe carboxyle par le mode opératoire b) ci-dessus, puis le groupe carboxyle est converti par des modes opératoires classiques en un halogénure d'acide intermédiaire qui est ensuite amené à réagir avec l'ammoniac, une alkylamine, une dialkylamine ou un alcanol en donnant un composé de formule (Ib) dans laquelle Z est un groupe aminocarbonyle, dialkylaminocarbonyle ou alkoxycarbonyle ;

d) est tout d'abord réduit en un composé hydroxyméthylique intermédiaire par des modes opératoires analogues à ceux qui sont indiqués en (A)b ci-dessus, la réduction étant suivie d'opérations d'halogénation également semblables à celles qui sont indiquées en (A)b ci-dessus, en donnant un composé de formule (Ib) dans laquelle Z est un groupe halogénalkyle, plus particulièrement un groupe halogénométhyle, ou bien à titre facultatif, le composé halogénalkylique, en particulier halogénométhylique, est traité avec un cyanure métallique, ce qui donne un composé de formule (Ib) dans laquelle Z est un groupe cyanalkyle, plus particulièrement un groupe cyanométhyle ;

e) est amené à réagir dans une réaction de Wittig ou une réaction de Wittig modifiée, avec formation d'un composé de formule (Ib) dans laquelle Z est un groupe alcényle ou alcynyle ;

f) est amené à réagir avec un réactif de Grignard ou un réactif du type alkyl-lithium en donnant un composé intermédiaire de formule (Ib) dans laquelle Z est un groupe $\alpha$-hydroxyalkyle, puis est oxydé avec des réactifs connus en donnant un composé de formule (Ib) dans laquelle Z est un groupe alkylcarbonyle, après quoi le composé alkylcarbonylique est facultativement halogéné en un composé de formule (Ib) dans laquelle Z est un groupe halogénalkylcarbonyle ; ou bien

g) est tout d'abord converti selon les modes opératoires indiqués ci-dessus dans les parties b) et c) en le chlorure d'acide intermédiaire, obtenu en passant par le composé dans lequel Z est un groupe carboxyle, puis par les processus classiques de transposition de Curtius, l'halogénure

d'acide intermédiaire est converti en passant par l'azide et l'isocyanate intermédiaires en le composé de formule (Ib) dans laquelle Z est un groupe amino ; ou

(D) répondant à la formule :

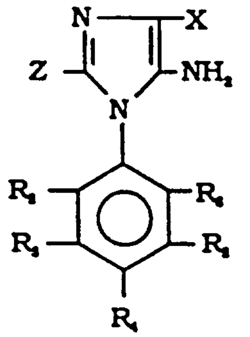

(Ib)

dans laquelle X, $R_2$, $R_3$, $R_4$, $R_5$ et $R_6$ sont tels que définis pour la formule (I) dans la revendication 1 et Z est un groupe alkylamino, dialkylamino, sel de trialkylammonium, alkoxycarbonylamino, alkylaminocarbonylamino, dialkylaminocarbonylamino, alkoxyalkylidène-imino, alkylcarbonylamino, halogénalkylcarbonylamino ou arylcarbonylamino, caractérisé en ce qu'un composé de formule (Ib) dans laquelle Z est un groupe amino, préparé selon des modes opératoires décrits en (B)b ou (B)g ci-dessus et X et Y, qui est un groupe amino, sont facultativement protégés si nécessaire :

a) est tout d'abord amené à réagir avec le phosgène pour former un composé intermédiaire de formule (Ib) dans laquelle Z est un groupe chlorocarbonylamino ou isocyanato, qui est ensuite amené à réagir avec un alcanol, une alkylamine ou une dialkylamine pour former un composé de formule (Ib) dans laquelle Z est un groupe alkoxycarbonylamino, alkylaminocarbonylamino ou dialkylaminocarbonylamino ;

b) est amené à réagir avec un agent d'alkylation tel qu'un iodure d'alkyle ou un sulfate de dialkyle ou bien, à titre facultatif, par une méthylation connue par voie de réduction en utilisant le formaldéhyde et l'acide formique pour former un composé (Ib) dans lequel Z est un groupe alkylamino, dialkylamino ou sel de trialkylammonium ;

c) est amené à réagir avec un orthoformiate d'alkyle en donnant un composé de formule (Ib) dans laquelle Z est un groupe alkoxyalkylidène-imino, notamment alkoxyméthylidène-imino ; ou bien

d) est amené à réagir avec un halogénure d'alkyl-, d'halogénalkyl- ou d'arylcarbonyle, facultativement en présence d'un accepteur d'acide, en donnant un composé de formule (Ib) dans laquelle Z est un groupe alkylcarbonylamino, halogénalkylcarbonylamino ou arylcarbonylamino ; ou

(E) répondant à la formule :

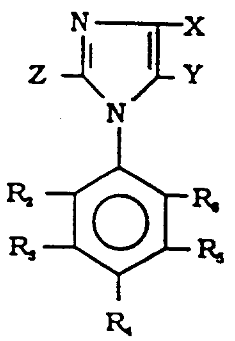

(I)

dans laquelle X, Z, $R_2$, $R_3$, $R_4$, $R_5$ et $R_6$ sont tels que définis pour la formule (I) dans la revendication 1 et Y est l'hydrogène, un groupe amino, un halogène, un groupe alkylsulfényle, halogénalkylsulfényle, alkylsulfinyle, halogénalkylsulfinyle, alkylsulfonyle, halogénalkylsulfonyle, cyano ou nitro, dans lequel un composé de formule (Ib)

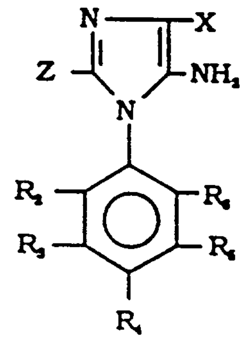

(Ib)

dans laquelle X, Z et $R_2$ à $R_6$ sont tels que définis ci-dessus et X, Z et le groupe amino sont facultativement protégés si nécessaire :

a) est déaminé par des opérations connues, par exemple avec un nitrite d'alkyle pour convertir le composé dans lequel Y est un groupe amino en son sel de diazonium correspondant, le sel de diazonium étant ensuite désactivé avec un agent de désactivation conformément à des modes opératoires connus pour obtenir un composé de formule (I) dans laquelle Y est l'hydrogène, un halogène, un groupe cyano, nitro, alkylsulfényle ou halogénalkylsulfényle, puis le composé dans lequel Y est un groupe alkylsulfényle ou halogénalkylsulfényle est facultativement oxydé en un composé de formule (I) dans laquelle Y est un groupe alkylsulfinyle, halogénalkylsulfinyle, alkylsulfonyle ou halogénalkylsulfonyle ; ou

(F) répondant à la formule :

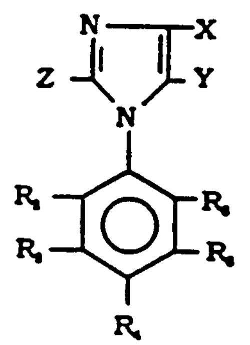

(I)

dans laquelle X, Z, $R_2$, $R_3$, $R_4$, $R_5$ et $R_6$ sont tels que définis pour la formule (I) dans la revendication 1 et Y est un groupe alkoxycarbonylamino, alkylaminocarbonylamino, dialkylaminocarbonylamino, alkoxyalkylidène-imino, alkylcarbonylamino, halogénalkylcarbonylamino, arylcarbonylamino, alkylamino, dialkylamino ou sel de trialkylammonium, dans lequel un composé de formule (Ib)

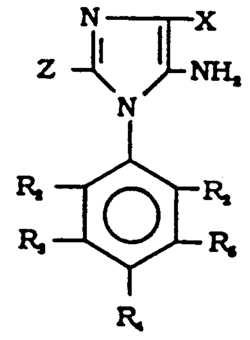

(Ib)

dans laquelle X, Z et $R_2$ à $R_6$ sont tels que définis ci-dessus et X, Z et le groupe amino sont facultativement protégés si nécessaire :

a) est amené à réagir par un mode opératoire analogue à celui qui est décrit en (D)a ci-dessus en passant par un composé intermédiaire chlorocarbonylaminé ou isocyanato, obtenu par réaction avec le phosgène, que l'on fait ensuite réagir avec un alcanol, une alkylamine ou une dialkylamine, afin d'obtenir un composé de formule (I) dans laquelle Y est un groupe alkoxycarbonylamino, alkylaminocarbonylamino ou dialkylaminocarbonylamino ;

b) est amené à réagir par un mode opératoire analogue à celui qui est décrit en (D)c ci-dessus avec un orthoformiate d'alkyle en donnant un composé de formule (I) dans laquelle Y est un groupe alkoxyalkylidène-imino, notamment alkoxyméthylidène-imino ;

c) est amené à réagir par un mode opératoire analogue à celui qui est décrit en (D)b ci-dessus par alkylation ou par méthylation par voie de réduction en donnant un composé de formule (I) dans laquelle Y est un groupe alkylamino, dialkylamino ou sel de trialkylammonium ; ou bien

d) est amené à réagir par un mode opératoire analogue à celui qui est décrit en (D)d ci-dessus avec un halogénure d'alkylcarbonyle, d'halogénalkylcarbonyle ou d'arylcarbonyle en donnant un composé de formule (I) dans laquelle Y est un groupe alkylcarbonylamino, halogénalkylcarbonylamino ou arylcarbonylamino ; ou

(G) répondant à la formule :

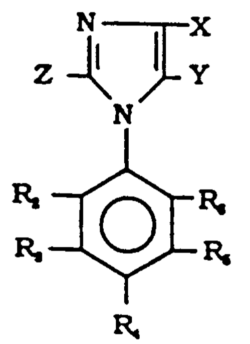

(I)

dans laquelle X, Z, $R_2$, $R_3$, $R_4$, $R_5$ et $R_6$ ont la définition donnée pour la formule (I) dans la revendication 1 et Y est un groupe nitro, sulfhydryle et ses sels, hydroxyle et ses sels, alkoxy, halogénalkoxy, aminosulfonyle, alkylaminosulfonyle, dialkylaminosulfonyle, alkyle, halogénalkyle, alcényle, alcynyle, cyanalkyle ou formyle, caractérisé en ce qu'un composé de formule (Ib)

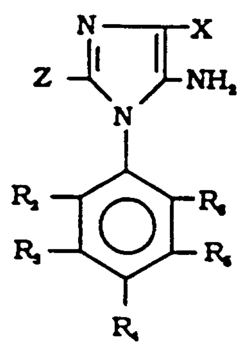

(Ib)

dans laquelle X, Z et $R_2$ à $R_6$ sont tels que définis ci-dessus et X et Z sont facultativement protégés si nécessaire, et déaminés conformément aux modes opératoires décrits en (E) ci-dessus en donnant un composé de formule (I) dans laquelle Y est l'hydrogène, puis ledit composé dans lequel X et Y sont facultativement protégés si nécessaire :

a) est nitré par des modes opératoires analogues à ceux qui sont décrits en b) dans la revendication 18 en donnant un composé de formule (I) dans laquelle Y est un groupe nitro ;

b) est amené à réagir par un mode opératoire analogue à ceux qui sont décrits en f) dans la revendication 18 en donnant tout d'abord un composé intermédiaire, dans lequel Y est un groupe

thiocyano, que l'on fait ensuite réagir pour obtenir un composé de formule (I) dans laquelle Y est un groupe sulfhydryle et ses sels ;

c) est tout d'abord amené à réagir avec une base forte telle qu'un réactif du type d'un composé organique de lithium pour former un carbanion métallique intermédiaire que l'on désactive ensuite avec un électrophile pour obtenir un composé de formule (I) dans laquelle Y est un groupe alkyle, halogénalkyle, alcényle, alcynyle, cyanalkyle ou formyle ;

d) est converti en le carbanion, comme dans la partie c) ci-dessus, puis désactivé avec du chlorure de sulfuryle, ce qui donne un composé intermédiaire dans lequel Y est un groupe chlorosulfonyle que l'on fait ensuite réagir avec l'ammoniac ou une alkylamine ou dialkylamine pour obtenir un composé de formule (I) dans laquelle Y est un groupe aminosulfonyle, alkylaminosulfonyle ou dialkylaminosulfonyle ;

e) est converti en le carbanion comme dans la partie c) ci-dessus ou bien, à titre facultatif, le carbanion est préparé, avec passage par le composé dans lequel Y est un halogène, obtenu selon le mode opératoire de l'Exemple (E) ci-dessus, après quoi le carbanion est amené à réagir par un mode opératoire analogue à celui qui est décrit en (B)c ci-dessus en donnant un composé de formule (I) dans laquelle Y est un groupe hydroxyle et ses sels, un groupe alkoxy ou un groupe halogénalkoxy ; ou

(H) répondant à la formule :

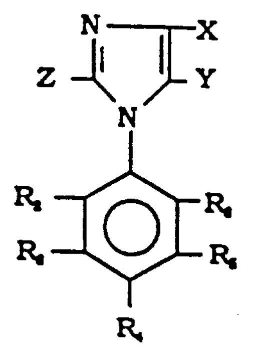

(I)

dans laquelle X, Z, $R_2$, $R_3$, $R_4$, $R_5$ et $R_6$ sont tels que définis pour la formule (I) dans la revendication 1 et Y est un groupe carboxyle et ses sels, un groupe cyano, aminocarbonyle, alkylaminocarbonyle, dialkylaminocarbonyle, alkoxycarbonyle, halogénalkyle, alcényle, alcynyle, alkylcarbonyle ou halogénalkylcarbonyle, caractérisé en ce qu'un composé de formule (Ib)

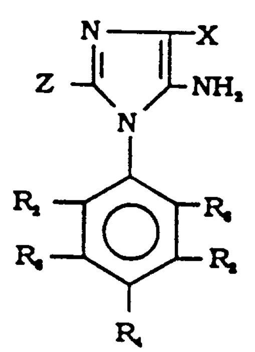

(Ib)

dans laquelle X, Z et $R_2$ à $R_6$ sont tels que définis ci-dessus, est tout d'abord déaminé conformément aux modes opératoires décrits en (E) ci-dessus en donnant un composé dans lequel Y est l'hydrogène, que l'on convertit ensuite par les modes opératoires décrits en (G)c ci-dessus pour obtenir un composé de formule (I) dans laquelle Y est un groupe formyle, après quoi ce composé formylique, dans lequel X et Z sont facultativement protégés si nécessaire :

a) est amené à réagir par des modes opératoires analogues à ceux qui sont décrits en (C)b ci-dessus, pour obtenir un composé de formule (I) dans laquelle Y est un groupe carboxyle et ses

sels ou un groupe cyano ;

b) est amené à réagir par des modes opératoires analogues à ceux qui sont décrits en (C)c ci-dessus en donnant un composé de formule (I) dans laquelle Y est un groupe aminocarbonyle, alkylaminocarbonyle, dialkylaminocarbonyle ou alkoxycarbonyle ;

c) est amené à réagir par des modes opératoires analogues à ceux qui sont décrits en (C)d ci-dessus en donnant un composé de formule (I) dans laquelle Y est un groupe halogénalkyle, plus particulièrement un groupe halogénométhyle ;

d) est amené à réagir par des modes opératoires analogues à ceux qui sont décrits en (B)b ci-dessus et en (C)e ci-dessus en donnant un composé de formule (I) dans laquelle Y est un groupe alcényle ou alcynyle ; ou

e) est amené à réagir par des modes opératoires analogues à ceux qui sont décrits en (C)f ci-dessus en donnant un composé de formule (I) dans laquelle Y est un groupe alkylcarbonyle ou halogénalkylcarbonyle ; ou

(I) répondant à la formule :

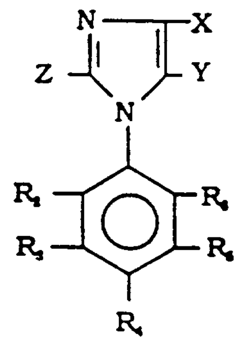

(I)

dans laquelle Y, Z, $R_2$, $R_3$, $R_4$, $R_5$ et $R_6$ ont les définitions données pour la formule (I) dans la revendication 1 et X est un groupe alkylsulfényle, halogénalkylsulfényle, alkylsulfinyle, halogénalkylsulfinyle, alkylsulfonyle ou halogénalkylsulfonyle, caractérisé en ce qu'un composé de formule (Ic)

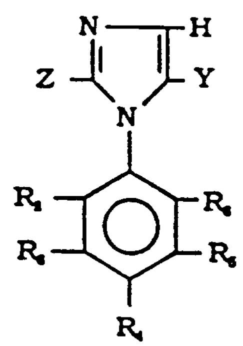

(Ic)

dans laquelle Y et Z sont facultativement progégés si nécessaire :

a) est tout d'abord amené à réagir conformément à des modes opératoires analogues à ceux qui sont décrits en (B)c ci-dessus pour convertir un composé de formule (Ic), dans laquelle X est l'hydrogène, en un composé intermédiaire de formule (I) dans laquelle X est successivement un groupe thiocyano puis un groupe disulfure, après quoi, également par des modes opératoires analogues à ceux qui sont décrits en (B)c ci-dessus, le composé intermédiaire thiocyano ou disulfure est converti en un composé de formule (I) dans laquelle X est un groupe alkylsulfényle ou halogénalkylsulfényle, en particulier un groupe perhalogénalkylsulfényle, ce composé est ensuite facultativement oxydé par des modes opératoires analogues à ceux qui sont décrits en (B)c ci-dessus en donnant l'analogue du type sulfoxyde ou sulfone, c'est-à-dire un composé de formule (I) dans laquelle X est un groupe alkylsulfinyle, halogénalkylsulfinyle, de préférence perhalogénalkylsulfinyle, un groupe alkylsulfonyle ou halogénalkylsulfonyle, notamment perhalogénalkylsulfonyle ; ou bien

b) est tout d'abord amené à réagir conformément à des modes opératoires analogues à ceux qui sont décrits en (B)a ci-dessus pour convertir le composé de formule (Ic), dans laquelle X est l'hydrogène, en un composé intermédiaire de formule (I) dans laquelle X est un groupe chlorosulfonyle, puis le composé chlorosulfonylique est amené à réagir avec un agent réducteur tel que la triphénylphosphine en donnant le même disulfure intermédiaire que celui qui a été décrit ci-dessus dans la partie a), après quoi, finalement, le disulfure est converti par les modes opératoires décrits dans la partie a) ci-dessus en donnant un composé de formule (I) dans laquelle X est un groupe alkylsulfényle ou halogénalkylsulfényle, notamment perhalogénalkylsulfényle, ou bien à titre facultatif, le composé sulfénylique est oxydé en donnant un composé de formule (I) dans laquelle X est un groupe alkylsulfinyle, halogénalkylsulfinyle, en particulier perhalogénalkylsulfinyle, alkylsulfonyle ou halogénalkylsulfonyle, en particulier perhalogénalkylsulfonyle ; ou

(J) répondant à la formule :

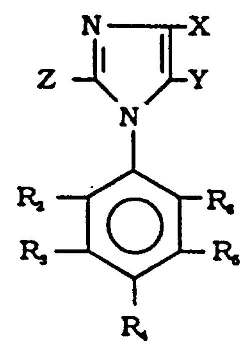

(IV)

dans laquelle :

| | |
|---|---|
| $R_2$, $R_3$, $R_4$, $R_5$ et $R_6$ | sont tels que définis dans la revendication 1 ; |
| X | est l'hydrogène ou un groupe halogénalkyle, notamment trifluorométhyle ; |
| Y | est un groupe amino ; hydroxy, existant facultativement sous sa forme isomérique céto lorsque X est l'hydrogène ; un groupe alkoxy ; ou un groupe halogénalkoxy ; et |
| Z | est l'hydrogène ; un halogène ; un groupe alkyle ; halogénalkyle ; hydroxy, existant facultativement sous sa forme céto-isomérique lorsque X est l'hydrogène et Y est un groupe imino ; alkoxy ; ou halogénalkoxy ; dans lequel un composé de formule (III) |

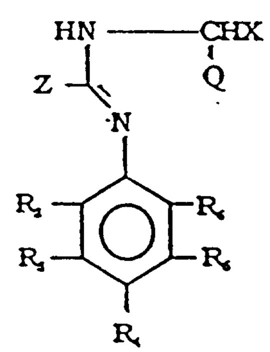

(III)

dans laquelle :

| | |
|---|---|
| $R_2$, $R_3$, $R_4$, $R_5$ et $R_6$ | ont la définition donnée ci-dessus ; |
| X | est l'hydrogène ou un groupe halogénalkyle, en particulier trifluorométhyle ; |
| Z | est l'hydrogène, un halogène, un groupe alkyle, halogénalkyle ou hy- |

droxy, existant facultativement sous sa forme céto-isomérique ; et

Q est un groupe cyano ou alkoxycarbonyle inférieur ;

est amené à réagir avec un agent basique dans un milieu réactionnel convenable pour former un composé de formule (IV) qui, lorsque Y ou Z est un groupe hydroxy, est ensuite facultativement alkylé ou halogénalkylé en composé dans lequel Y ou Z est un groupe alkoxy ou halogénalkoxy.

**9.** Procédé de préparation d'un composé de formule (IV) suivant la revendication 8(J), dans lequel le composé de formule (IV) est :

a) un composé de formule (5) représentée dans la revendication 8, dans laquelle X et Z sont chacun un atome d'hydrogène et Y est un groupe amino ;

b) un composé de formule (17)

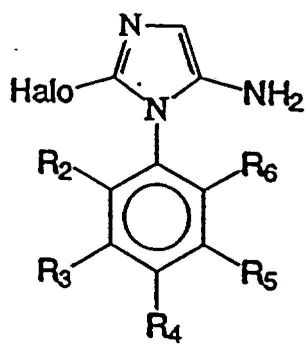

(17)

dans laquelle Halo désigne un halogène, en particulier le chlore ;

c) un composé de formule (22)

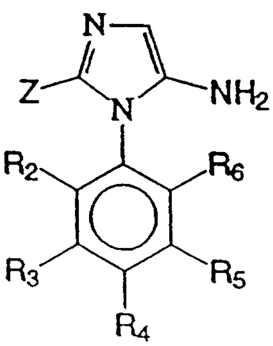

(22)

dans laquelle Z est un groupe alkyle ou halogénalkyle ;

d) un composé de formule (27)

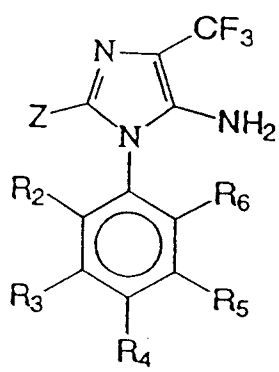

(27)

dans laquelle Z est un halogène, un groupe alkyle ou un groupe halogénalkyle ;

e) un composé de formule (30), dans laquelle X est de l'hydrogène et Y est un groupe hydroxyle, existant facultativement sous sa forme céto-isomérique (29),

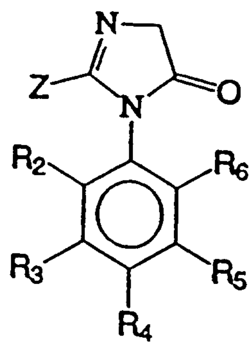

(29)

dans laquelle Z est un halogène, un groupe alkyle ou un groupe halogénalkyle ; ou bien

f) un composé de formule (37), dans laquelle Y est un groupe amino et Z est un groupe hydroxy, existant facultativement sous sa forme céto-imino isomérique (34)

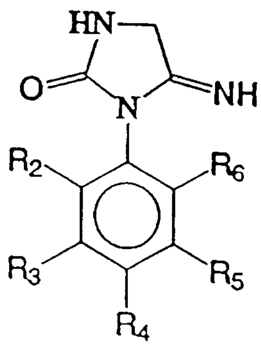

(34)

**10.** Procédé de préparation d'un composé de formule (I) :

(A) dans laquelle X, Y, Z, $R_2$, $R_3$, $R_4$, $R_5$ et $R_6$ ont les définitions données dans la revendication 1, dans lequel un composé de formule (5) représentée dans la revendication 8 est amené à réagir conformément au procédé de préparation de la revendication 8 pour l'introduction des substituants X, Y et Z ;

(B) dans laquelle X, Y, $R_2$, $R_3$, $R_4$, $R_5$ et $R_6$ sont tels que définis dans la revendication 1 et Z est un halogène, dans lequel un composé de formule (17) représentée dans la revendication 9 est amené à réagir conformément au procédé de préparation de la revendication 8 pour l'introduction des substituants X et Y ;

(C) dans laquelle X, Y, $R_2$, $R_3$, $R_4$, $R_5$ et $R_6$ ont les définitions données dans la revendication 1 et Z est un groupe alkyle ou halogénalkyle, dans lequel un composé de formule (22) représentée dans la revendication 9 est amené à réagir conformément au procédé de préparation de la revendication 8 pour l'introduction des substituants X et Y ;

(D) dans laquelle Y, $R_2$, $R_3$, $R_4$, $R_5$ et $R_6$ ont les définitions données dans la revendication 1, X est un groupe halogénalkyle, en particulier trifluorométhyle, et Z est un halogène, un groupe alkyle ou un groupe halogénealkyle, dans lequel un composé de formule (27) est amené à réagir conformément au procédé de la revendication 8 pour l'introduction du substituant Y ;

(E) dans laquelle X, $R_2$, $R_3$, $R_4$, $R_5$ et $R_6$ ont la définition donnée dans la revendication 1, Y est un groupe hydroxy, alkoxy ou halogénalkoxy et Z est un halogène, un groupe alkyle ou un groupe halogénalkyle, dans lequel un composé de formule (30), existant facultativement sous sa forme céto isomérique (29) représentée dans la revendication 9, dans laquelle Y est un groupe hydroxy, facultativement alkylé en un groupe Y alkoxy ou halogénealkoxy, est amené à réagir conformément au procédé de préparation de la revendication 8 pour l'introduction du substituant X ;

(F) dans laquelle X, Y, $R_2$, $R_3$, $R_4$, $R_5$ et $R_6$ ont les définitions données dans la revendication 1 et Z est un groupe hydroxy, alkoxy, halogénalkoxy ou un halogène, caractérisé en ce qu'un composé de formule (37), existant facultativement sous sa forme céto-imino isomérique (34) représentée dans la revendication 9, dans laquelle Z est un groupe hydroxy, facultativement alkylé en un groupe Z alkoxy ou halogénalkoxy ou facultativement halogéné de telle sorte que Z représente un halogène, est amené à réagir conformément au procédé de préparation de la revendication 8 pour l'introduction des substituants X et Y.

**11.** Composé de formule (Ia), (Ib), (Ic), (IV), (5), (17), (22), (27), (30)/(29) ou (37)/(34), représentée dans la revendication 8 ou 9, dans lequel les substituants X, Y, Z, $R_2$, $R_3$, $R_4$, $R_5$ et $R_6$ sont tels que définis ci-dessus dans la revendication 8, 9 ou 10.

**12.** Procédé pour combattre des arthropodes, en particulier des insectes, des pucerons ou des acariens ; des nématodes ; ou des helminthes ou protozoaires parasites en un site, qui comprend le traitement du site avec une quantité efficace d'un composé de formule (I), dans laquelle les divers substituants X, Y, Z, $R_2$, $R_3$, $R_4$, $R_5$ et $R_6$ sont tels que définis dans l'une quelconque des revendications 1 à 7, mais comprenant des composés dans lesquels (i) X est un groupe trifluorométhyle, lorsque $R_3$ est un groupe méthyle, $R_4$ est un groupe nitro ou amino et les autres symboles représentent l'hydrogène, et (ii) X est un groupe chlorométhyle lorsque $R_4$ représente un groupe méthyle, méthoxy ou éthoxy et les autres symboles représentent l'hydrogène, avec exclusion d'un procédé de traitement du corps humain ou animal accompli par un médecin ou vétérinaire praticien.

**13.** Procédé suivant la revendication 12, dans lequel le composé de formule (I) est un composé nommé dans la revendication 5 ou 6.

**14.** Procédé suivant la revendication 12 ou 13, dans lequel le site comprend des plantes cultivées en agriculture ou en horticulture ou un milieu dans lequel les plantes se développent, et les parasites sont des arthropodes ou des nématodes parasites des plantes, et le traitement consiste à appliquer aux plantes ou au milieu dans lequel elles se développent une quantité efficace du composé de formule (I).

**15.** Procédé suivant la revendication 14, dans lequel le composé est appliqué au site dans lequel les arthropodes ou nématodes parasites sont combattus, à un taux d'environ 0,005 kg à environ 15 kg de composé par hectare de site traité, de préférence à un taux d'environ 0,02 kg à environ 2 kg de composé par hectare.

**16.** Procédé suivant la revendication 15, dans lequel les parasites sont des acariens, des pucerons, d'autres insectes ou des nématodes des plantes ou plusieurs de ces parasites, qui consiste à incorporer le composé dans le sol dans lequel les végétaux sont plantés ou doivent l'être, ou à appliquer le composé aux semences ou aux racines des végétaux, ou à effectuer le traitement par application foliaire.

**17.** Procédé suivant la revendication 16, dans lequel les insectes sont des insectes du sol de l'ordre des coléoptères, en particulier des espèces du genre Diabrotica, les acariens appartiennent à la sous-classe des Acari et les pucerons appartiennent à la superfamille des Aphidoidea.

**18.** Procédé suivant la revendication 12, qui est utilisé dans le domaine de la médecine vétérinaire ou de l'exploitation du bétail ou dans le maintien de l'hygiène publique contre : des arthropodes, en particulier des insectes de l'ordre des diptères ou des acariens de la sous-classe des Acari ou les deux ; ou des helminthes ou des protozoaires qui sont des endoparasites ou des ectoparasites de vertébrés à sang chaud.

**19.** Composition destinée à combattre des arthropodes, nématodes, helminthes ou protozoaires parasites, caractérisée en ce qu'elle comprend une quantité efficace, de préférence environ 0,05 à environ 95 % en poids, d'un composé de formule (I) suivant l'une quelconque des revendications 1 à 6, mais comprenant des composés dans lesquels (i) X est un groupe trifluorométhyle lorsque $R_3$ représente un groupe méthyle, $R_4$ représente un groupe nitro ou amino et les autres symboles représentent l'hydrogène et (ii) X est un groupe chlorométhyle lorsque $R_4$ représente un groupe méthyle, méthoxy ou éthoxy et les autres symboles représentent l'hydrogène, comme ingrédient actif et un ou plusieurs

composants compatibles du point de vue agronomique ou médicinal, comprenant environ 1 % à environ 95 % en poids d'un ou plusieurs supports solides ou liquides et environ 0,1 à environ 50 % en poids d'un ou plusieurs composants additionnels tels que des agents tensio-actifs.

**20.** Composition destinée en particulier à être utilisée en médecine vétérinaire et dans l'exploitation du bétail ou dans le maintien de l'hygiène publique, comprenant un ou plusieurs composants compatibles et, comme ingrédient actif, au moins un composé de formule (I) suivant la revendication 19.

**Revendications pour l'Etat contractant suivant : ES**

**1.** Procédé de préparation d'un composé de formule (I)

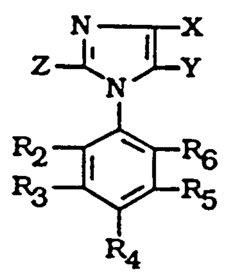

(I)

dans laquelle :

X est un groupe choisi entre halogénalkyle ou halogénalkoxy ou un groupe non substitué ou à substituant halogéno choisi entre alkylsulfényle, alkylsulfinyle ou alkylsulfonyle, dont les parties alkyle et alkoxy définies de chaque groupe sont à chaîne linéaire ou ramifiée, contenant un à quatre atomes de carbone, et la substitution halogéno de chaque groupe consiste en un ou plusieurs atomes d'halogènes, qui sont identiques ou différents, jusqu'à et y compris la substitution totale de la partie alkyle ou alkoxy ;

Y et Z sont choisis chacun, individuellement, entre : un atome d'hydrogène ou un atome d'halogène ; un groupe choisi entre nitro, cyano, hydroxyle et ses sels acceptables, sulfhydryle et ses sels acceptables, formyle, hydroxycarbonyle et ses sels acceptables, alkoxycarbonyle, aminocarbonyle, alkylaminocarbonyle, dialkylaminocarbonyle, amino, alkylamino, dialkylamino, sel de trialkylammonium, cyanalkyle, alkoxycarbonylamino, arylcarbonylamino, alkylaminocarbonylamino, dialkylaminocarbonylamino, aminosulfonyle, alkylaminosulfonyle, dialkylaminosulfonyle ou alkoxyalkylidène-imino, les parties alkyle et alkoxy définies de chaque groupe étant à chaîne linéaire ou à chaîne ramifiée contenant un à quatre atomes de carbone ; un groupe alcényle ou alcynyle à chaîne linéaire ou ramifiée contenant deux à quatre atomes de carbone ; ou un groupe choisi entre alkyle, alkoxy, alkylcarbonyle, alkylcarbonylamino, alkylsulfényle, alkylsulfinyle ou alkylsulfonyle non substitué ou à substituant halogéno, les parties alkyle et alkoxy définies de chaque groupe étant à chaîne linéaire ou à chaîne ramifiée contenant un à quatre atomes de carbone, et la substitution halogéno consistant en un ou plusieurs atomes d'halogènes, identiques ou différents, jusqu'à et y compris la substitution totale de la partie alkyle ou alkoxy ; et un un seul de Y et Z est un groupe contenant du soufre ; et

$R_2$, $R_3$, $R_4$, $R_5$ et $R_6$ sont choisis, individuellement, entre : un atome d'hydrogène ou un atome d'halogène ; un groupe choisi entre nitro, cyano, amino, alkylamino ou dialkylamino, la partie alkyle de chaque groupe étant à chaîne linéaire ou à chaîne ramifiée contenant un à quatre atome de carbone ; un groupe alcényle ou alcynyle à chaîne linéaire ou ramifiée contenant deux à quatre atomes de carbone, qui peut être substitué par un ou plusieurs atomes d'halogènes, identiques ou différents, jusqu'à et y compris la substitution totale ; ou un groupe choisi entre alkyle, alkoxy, alkylsulfényle, alkylsulfinyle

ou alkylsulfonyle non substitué ou à substituant halogéno, les parties alkyle et alkoxy définies de chaque groupe étant à chaîne linéaire ou à chaîne ramifiée contenant un à quatre atomes de carbone et la substitution halogéno consistant en un ou plusieurs atomes d'halogènes, identiques ou différents, jusqu'à et y compris la substitution totale de la partie alkyle ou alkoxy, sous réserve que (i) X représente autre chose qu'un groupe trifluorométhyle lorsque $R_3$ est un groupe méthyle, $R_4$ représente un groupe nitro ou amino et les autres symboles représentent l'hydrogène et (ii) X représente autre chose qu'un groupe chlorométhyle lorsque $R_4$ est un groupe méthyle, méthoxy ou éthoxy et les autres symboles représentent de l'hydrogène.

(A) répondant à la formule :

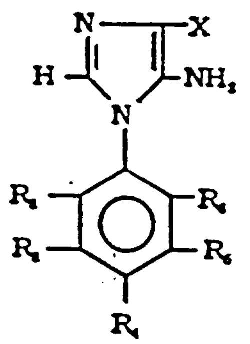

(Ia)

dans laquelle $R_2$, $R_3$, $R_4$, $R_5$ et $R_6$ ont les définitions données pour la formule (I) et X est un groupe alkylsulfényle, halogénalkylsulfényle, alkylsulfinyle, halogénalkylsulfinyle, alkylsulfonyle, halogénalkylsulfonyle, halogénalkyle ou halogénalkoxy, dans lequel un composé de formule (5)

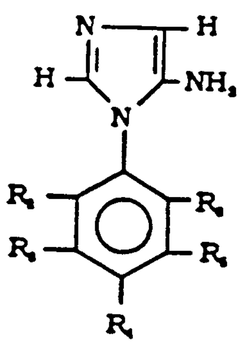

5

dans laquelle le groupe amino est facultativement protégé si nécessaire :

a) est tout d'abord amené à réagir avec un halogénure de sulfényle, $R_1$SHalo, dans lequel $R_1$ est un groupe alkyle ou halogénalkyle, dans un milieu réactionnel organique, facultativement en présence d'un accepteur d'acide tel qu'une amine tertiaire afin d'obtenir un composé de formule (Ia) dans laquelle X est un groupe alkylsulfényle ou halogénalkylsulfényle, qui est ensuite facultativement oxydé par des opérations connues, par exemple à l'aide d'un peroxyde, pour obtenir un composé de formule (Ia) dans laquelle X est un groupe $S(O)_nR_1$ dans laquelle n a la valeur 1 ou 2 et $R_1$ est tel que défini ci-dessus, c'est-à-dire que X est un groupe alkylsulfinyle, halogénalkylsulfinyle, alkylsulfonyle ou halogénalkylsulfonyle ;

b) est tout d'abord amené à réagir avec un tris(alkylthio)méthane ou un tris(arylthio)méthane dans un milieu réactionnel organique en présence d'un acide de Lewis et, à titre facultatif, en présence d'un accepteur d'acide, puis le composé intermédiaire obtenu de formule (10) dans laquelle X est un groupe bis(alkylthio)méthyle ou bis(arylthio)méthyle est amené à réagir, dans un milieu réactionnel organique, avec un nitrite d'alkyle convenable, la réaction étant suivie d'une opération d'hydrolyse en vue d'obtenir un composé intermédiaire de formule (Ia) dans laquelle X est un

groupe formyle, l'hydrolyse étant suivie d'opérations connues de réduction en vue d'obtenir un composé intermédiaire dans lequel X est un groupe hydroxyméthyle, ce composé étant finalement halogéné par des opérations connues en donnant un composé de formule (Ia) dans laquelle X est un groupe halogénalkyle, plus particulièrement un groupe halogénométhyle.

c) est tout d'abord formylé par des opérations bien connues telles que la réaction de Vilsmeier-Haack et une réaction similaire, avec formation du composé de formule (Ia) dans laquelle X est un groupe formyle, composé qui est ensuite amené à réagir selon les modes opératoires indiqués ci-dessus dans la partie b) en donnant de même le composé de formule (Ia) dans laquelle X est un groupe halogénalkyle ;

d) est amené à réagir selon les modes opératoires de la partie b) ou c) ci-dessus en donnant un composé intermédiaire de formule (Ia) dans laquelle X est un groupe formyle, ce composé pouvant être facultativement oxydé en un composé intermédiaire de formule (Ia) dans laquelle X est un groupe carboxyle, puis le composé intermédiaire dans lequel X est un groupe formyle est finalement amené à réagir avec un agent d'halogénation tel que le trifluorure de diéthylaminosoufre, ou bien le composé intermédiaire, dans lequel X est un groupe carboxyle, est amené à réagir avec le tétrafluorure de soufre en donnant un composé de formule (Ia) dans laquelle X est un groupe halogénalkyle, plus particulièrement le groupe difluorométhyle ou trifluorométhyle ; ou bien

e) est tout d'abord halogéné par des modes opératoires bien connus en donnant un composé intermédiaire dans lequel X est un halogène, à partir duquel on prépare un dérivé organomagnésien ou un dérivé organique de lithium, puis ledit composé organométallique est amené à réagir avec l'oxodiperoxymolybdène(pyridine-(hexaméthylphosphorotriamide) ou un borate de trialkyle et un agent oxydant tel que le peroxyde d'hydrogène en donnant un composé intermédiaire de formule (Ia) dans laquelle X est un groupe hydroxy, et ce composé est finalement amené à réagir par des opérations connues d'halogénalkylation en donnant un composé de formule (Ia) dans laquelle X est un groupe halogénalkoxy ; ou

(B) correspondant à la formule :

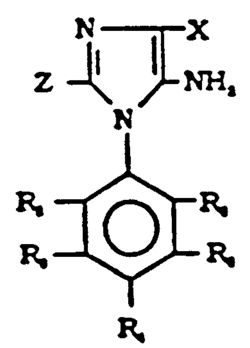

(IIb)

dans laquelle X, $R_2$, $R_3$, $R_4$, $R_5$ et $R_6$ sont tels que définis pour la formule (I) et Z est un groupe aminosulfonyle, alkylaminosulfonyle, dialkylaminosulfonyle, nitro, amino, un halogène, un groupe alcynyle, alkyle, hydroxy et ses sels, alkoxy, halogénalkoxy, formyle, alkylsulfényle, halogénalkylsulfényle, alkylsulfinyle, halogénalkylsulfinyle, alkylsulfonyle, halogénalkylsulfonyle ou sulfhydryle et ses sels, dans lequel un composé de formule (Ia)

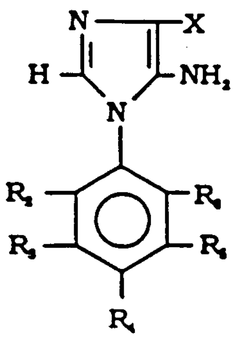

(Ia)

dans laquelle X et le groupe amino sont facultativement protégés si nécessaire :

a) est tout d'abord amené à réagir avec l'acide chlorosulfonique ou dichlorosulfonique en donnant un composé intermédiaire dans lequel Z est un groupe chlorosulfonyle, composé qui est amené à réagir avec l'ammoniac, une alkylamine ou une dialkylamine en donnant un composé de formule (Ib) dans laquelle Z est un groupe aminosulfonyle, alkylaminosulfonyle ou dialkylaminosulfonyle ;

b) est halogéné ou nitré par des opérations connues en donnant un composé de formule (Ib) dans laquelle Z est un halogène ou un groupe nitro, puis le composé dans lequel Z est un groupe nitro est facultativement réduit en composé dans lequel Z est un groupe amino par des opérations connues, ou bien à titre facultatif, le composé dans lequel Z est un halogène est traité selon des modes opératoires connus avec un acétylure de cuivre en donnant le composé dans lequel Z est un groupe alcynyle ;

c) est amené à réagir avec une base forte telle qu'un composé réactif organique de lithium en donnant un carbanion organométallique intermédiaire qui est ensuite désactivé avec un agent alkylant en donnant un composé de formule (Ib) dans laquelle Z est un groupe alkyle, ou bien à titre facultatif, le carbanion est amené à réagir d'une manière semblable à la réaction décrite en (A)e ci-dessus en donnant tout d'abord un composé de formule (Ib) dans laquelle Z est un groupe hydroxyle ou ses sels, puis à titre facultatif, le composé dans lequel Z est un groupe hydroxy est converti en un composé dans lequel Z est un groupe alkoxy ou halogénalkoxy par des opérations connues d'alkylation ou d'halogénalkylation ;

d) est amené à réagir par des modes opératoires de formylation analogues à ceux qui sont décrits en (A)b ou (A)c ci-dessus, où le composé dans lequel Z est un groupe formyle est préparé directement dans des conditions telles que la réaction de Vilsmeier-Haack ou par hydrolyse d'un composé intermédiaire dans lequel Z est un groupe bis(alkylthio)méthyle ou bis-(arylthio)méthyle ;

e) est tout d'abord amené à réagir avec un mélange de brome et d'un thiocyanate métallique en donnant un composé intermédiaire de formule (Ib) dans laquelle Z est un groupe thiocyano, qui est ensuite traité avec un agent d'alkylation, facultativement en présence d'une base, en donnant directement un composé de formule (Ib) dans laquelle Z est un groupe alkylsulfényle ou halogénalkylsulfényle, ou bien à titre facultatif, le composé intermédiaire dans lequel Z est un groupe thiocyano est tout d'abord oxydé en un disulfure intermédiaire correspondant qui est ensuite amené à réagir avec un perhalogénalcane, facultativement en présence d'un agent réducteur, en donnant un composé de formule (Ib) dans laquelle Z est un groupe halogénalkylsulfényle, en particulier un groupe perhalogénalkylsulfényle, finalement le composé dans lequel Z est un groupe alkylsulfényle ou halogénalkylsulfényle est facultativement oxydé par des opérations connues analogues à celles qui sont indiquées en A(a) ci-dessus en donnant un composé de formule (Ib) dans laquelle Z est un groupe alkylsulfinyle, halogénalkylsulfinyle, alkylsulfonyle ou halogénalkylsulfonyle ; ou bien

f) est tout d'abord amené à réagir comme dans la partie e) ci-dessus en donnant le composé intermédiaire dans lequel Z est un groupe thiocyano, qui est ensuite clivé par un promoteur de radicaux libres tel que le ferricyanure de potassium, en donnant un composé de formule (Ib) dans laquelle Z est un groupe sulfhydryle ou ses sels ; ou

(C) répondant à la formule :

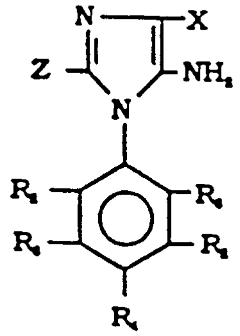

(Ib)

dans laquelle X, $R_2$, $R_3$, $R_4$, $R_5$ et $R_6$ ont la définition donnée pour la formule (I) et Z est un groupe amino, alkyle, cyano, carboxyle et ses sels, aminocarbonyle, alkylaminocarbonyle, dialkylaminocarbonyle, alkoxycarbonyle, halogénalkyle, cyanalkyle, alcényle, alcynyle, alkylcarbonyle ou halogénalkylcarbonyle, caractérisé en ce qu'un composé de formule (Ib) dans laquelle Z est un groupe formyle, préparé selon les modes opératoires décrits en (B)d ci-dessus, et dans laquelle X et le groupe amino sont facultativement protégés si nécessaire :

a) est réduit en un composé de formule (Ib) dans laquelle Z est un groupe alkyle, en particulier le groupe méthyle, par des agents réducteurs connus tels que le borohydrure de sodium ou la p-toluènesulfonylhydrazine et le cyanoborohydrure de sodium ;

b) est amené à réagir avec un agent oxydant connu classique pour former un composé de formule (Ib) dans laquelle Z est un groupe carboxyle ou ses sels, puis à titre facultatif, le composé à groupe carboxyle peut être converti en un composé de formule (Ib) dans laquelle Z est un groupe amino, par une transposition de Curtius passant par un halogénure d'acide, un azide ou un isocyanate intermédiaire, ou bien à titre facultatif, le composé dans lequel Z est un groupe carboxyle est traité avec l'isophtalonitrile en donnant un composé de formule (Ib) dans laquelle Z est un groupe cyano, ou bien le composé dans lequel Z est un groupe formyle est facultativement amené à réagir avec l'hydroxylamine en donnant une aldoxime intermédiaire qui est ensuite déshydratée par des modes opératoires classiques en donnant le composé de formule (Ib) dans laquelle Z est un groupe cyano ;

c) est converti en le composé dans lequel Z est un groupe carboxyle par le mode opératoire b) ci-dessus, puis le groupe carboxyle est converti par des modes opératoires classiques en un halogénure d'acide intermédiaire qui est ensuite amené à réagir avec l'ammoniac, une alkylamine, une dialkylamine ou un alcanol en donnant un composé de formule (Ib) dans laquelle Z est un groupe aminocarbonyle, dialkylaminocarbonyle ou alkoxycarbonyle ;

d) est tout d'abord réduit en un composé hydroxyméthylique intermédiaire par des modes opératoires analogues à ceux qui sont indiqués en (A)b ci-dessus, la réduction étant suivie d'opérations d'halogénation également semblables à celles qui sont indiquées en (A)b ci-dessus, en donnant un composé de formule (Ib) dans laquelle Z est un groupe halogénalkyle, plus particulièrement un groupe halogénométhyle, ou bien à titre facultatif, le composé halogénalkylique, en particulier halogénométhylique, est traité avec un cyanure métallique, ce qui donne un composé de formule (Ib) dans laquelle Z est un groupe cyanalkyle, plus particulièrement un groupe cyanométhyle ;

e) est amené à réagir dans une réaction de Wittig ou une réaction de Wittig modifiée, avec formation d'un composé de formule (Ib) dans laquelle Z est un groupe alcényle ou alcynyle ;

f) est amené à réagir avec un réactif de Grignard ou un réactif du type alkyl-lithium en donnant un composé intermédiaire de formule (Ib) dans laquelle Z est un groupe α-hydroxyalkyle, puis est oxydé avec des réactifs connus en donnant un composé de formule (Ib) dans laquelle Z est un groupe alkylcarbonyle, après quoi le composé alkylcarbonylique est facultativement halogéné en un composé de formule (Ib) dans laquelle Z est un groupe halogénalkylcarbonyle ; ou bien

g) est tout d'abord converti selon les modes opératoires indiqués ci-dessus dans les parties b) et c) en le chlorure d'acide intermédiaire, obtenu en passant par le composé dans lequel Z est un groupe carboxyle, puis par les processus classiques de transposition de Curtius, l'halogénure

d'acide intermédiaire est converti en passant par l'azide et l'isocyanate intermédiaires en le composé de formule (Ib) dans laquelle Z est un groupe amino ; ou

(D) répondant à la formule :

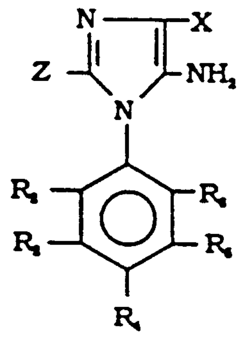

(Ib)

dans laquelle X, $R_2$, $R_3$, $R_4$, $R_5$ et $R_6$ sont tels que définis pour la formule (I) et Z est un groupe alkylamino, dialkylamino, sel de trialkylammonium, alkoxycarbonylamino, alkylaminocarbonylamino, dialkylaminocarbonylamino, alkoxyalkylidène-imino, alkylcarbonylamino, halogénalkylcarbonylamino ou arylcarbonylamino, dans lequel un composé de formule (Ib) dans laquelle Z est un groupe amino, préparé selon des modes opératoires décrits en (B)b ou (B)g ci-dessus et X et Y, qui est un groupe amino, sont facultativement protégés si nécessaire :

a) est tout d'abord amené à réagir avec le phosgène pour former un composé intermédiaire de formule (Ib) dans laquelle Z est un groupe chlorocarbonylamino ou isocyanato, qui est ensuite amené à réagir avec un alcanol, une alkylamine ou une dialkylamine pour former un composé de formule (Ib) dans laquelle Z est un groupe alkoxycarbonylamino, alkylaminocarbonylamino ou dialkylaminocarbonylamino ;

b) est amené à réagir avec un agent d'alkylation tel qu'un iodure d'alkyle ou un sulfate de dialkyle ou bien, à titre facultatif, par une méthylation connue par voie de réduction en utilisant le formaldéhyde et l'acide formique pour former un composé (Ib) dans lequel Z est un groupe alkylamino, dialkylamino ou sel de trialkylammonium ;

c) est amené à réagir avec un orthoformiate d'alkyle en donnant un composé de formule (Ib) dans laquelle Z est un groupe alkoxyalkylidène-imino, notamment alkoxyméthylidène-imino ; ou bien

d) est amené à réagir avec un halogénure d'alkyl-, d'halogénalkyl- ou d'arylcarbonyle, facultativement en présence d'un accepteur d'acide, en donnant un composé de formule (Ib) dans laquelle Z est un groupe alkylcarbonylamino, halogénalkylcarbonylamino ou arylcarbonylamino ; ou

(E) répondant à la formule :

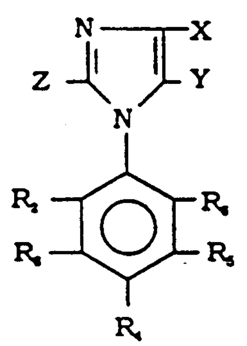

(I)

dans laquelle X, Z, $R_2$, $R_3$, $R_4$, $R_5$ et $R_6$ sont tels que définis pour la formule (I) et Y est l'hydrogène, un groupe amino, un halogène, un groupe alkylsulfényle, halogénalkylsulfényle, alkylsulfinyle, halogénalkylsulfinyle, alkylsulfonyle, halogénalkylsulfonyle, cyano ou nitro, dans lequel un composé de formule (Ib)

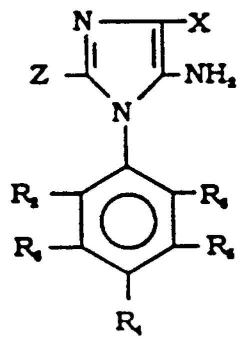

(Ib)

dans laquelle X, Z et $R_2$ à $R_6$ sont tels que définis ci-dessus et X, Z et le groupe amino sont facultativement protégés si nécessaire :

a) est déaminé par des opérations connues, par exemple avec un nitrite d'alkyle pour convertir le composé dans lequel Y est un groupe amino en son sel de diazonium correspondant, le sel de diazonium étant ensuite désactivé avec un agent de désactivation conformément à des modes opératoires connus pour obtenir un composé de formule (I) dans laquelle Y est l'hydrogène, un halogène, un groupe cyano, nitro, alkylsulfényle ou halogénalkylsulfényle, puis le composé dans lequel Y est un groupe alkylsulfényle ou halogénalkylsulfényle est facultativement oxydé en un composé de formule (I) dans laquelle Y est un groupe alkylsulfinyle, halogénalkylsulfinyle, alkylsulfonyle ou halogénalkylsulfonyle ; ou

(F) répondant à la formule :

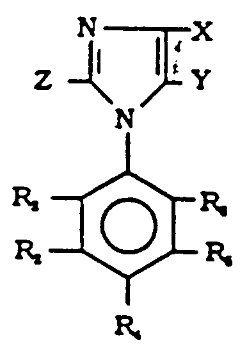

(I)

dans laquelle X, Z, $R_2$, $R_3$, $R_4$, $R_5$ et $R_6$ sont tels que définis pour la formule (I) et Y est un groupe alkoxycarbonylamino, alkylaminocarbonylamino, dialkylaminocarbonylamino, alkoxyalkylidène-imino, alkylcarbonylamino, halogénalkylcarbonylamino, arylcarbonylamino, alkylamino, dialkylamino ou sel de trialkylammonium, dans lequel un composé de formule (Ib)

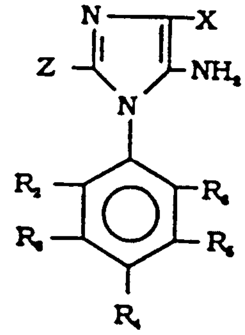

(Ib)

dans laquelle X, Z et $R_2$ à $R_6$ sont tels que définis ci-dessus et X, Z et le groupe amino sont

facultativement protégés si nécessaire :

a) est amené à réagir par un mode opératoire analogue à celui qui est décrit en (D) a ci-dessus en passant par un composé intermédiaire chlorocarbonylaminé ou isocyanato, obtenu par réaction avec le phosgène, que l'on fait ensuite réagir avec un alcanol, une alkylamine ou une dialkylamine, afin d'obtenir un composé de formule (I) dans laquelle Y est un groupe alkoxycarbonylamino, alkylaminocarbonylamino ou dialkylaminocarbonylamino ;

b) est amené à réagir par un mode opératoire analogue à celui qui est décrit en (D)c ci-dessus avec un orthoformiate d'alkyle en donnant un composé de formule (I) dans laquelle Y est un groupe alkoxyalkylidène-imino, notamment alkoxyméthylidène-imino ;

c) est amené à réagir par un mode opératoire analogue à celui qui est décrit en (D)b ci-dessus par alkylation ou par méthylation par voie de réduction en donnant un composé de formule (I) dans laquelle Y est un groupe alkylamino, dialkylamino ou sel de trialkylammonium ; ou bien

d) est amené à réagir par un mode opératoire analogue à celui qui est décrit en (D)d ci-dessus avec un halogénure d'alkylcarbonyle, d'halogénalkylcarbonyle ou d'arylcarbonyle en donnant un composé de formule (I) dans laquelle Y est un groupe alkylcarbonylamino, halogénalkylcarbonylamino ou arylcarbonylamino ; ou

(G) répondant à la formule :

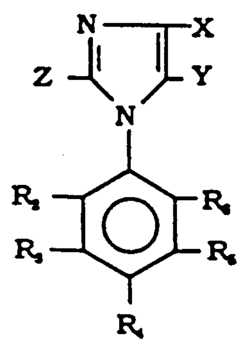

(I)

dans laquelle X, Z, $R_2$, $R_3$, $R_4$, $R_5$ et $R_6$ ont la définition donnée pour la formule (I) et Y est un groupe nitro, sulfhydryle et ses sels, hydroxyle et ses sels, alkoxy, halogénalkoxy, aminosulfonyle, alkylaminosulfonyle, dialkylaminosulfonyle, alkyle, halogénalkyle, alcényle, alcynyle, cyanalkyle ou formyle, dans lequel un composé de formule (Ib)

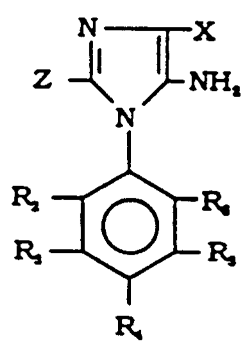

(Ib)

dans laquelle X, Z et $R_2$ à $R_6$ sont tels que définis ci-dessus et X et Z sont facultativement protégés si nécessaire, et déaminés conformément aux modes opératoires décrits en (E) ci-dessus en donnant un composé de formule (I) dans laquelle Y est l'hydrogène, puis ledit composé dans lequel X et Y sont facultativement protégés si nécessaire :

a) est nitré par des modes opératoires analogues à ceux qui sont décrits en (B)b en donnant un composé de formule (I) dans laquelle Y est un groupe nitro ;

b) est amené à réagir par un mode opératoire analogue à ceux qui sont décrits en (B)f en donnant tout d'abord un composé intermédiaire, dans lequel Y est un groupe thiocyano, que l'on fait ensuite réagir pour obtenir un composé de formule (I) dans laquelle Y est un groupe sulfhydryle et ses sels ;

c) est tout d'abord amené à réagir avec une base forte telle qu'un réactif du type d'un composé organique de lithium pour former un carbanion métallique intermédiaire que l'on désactive ensuite avec un électrophile pour obtenir un composé de formule (I) dans laquelle Y est un groupe alkyle, halogénalkyle, alcényle, alcynyle, cyanalkyle ou formyle ;

d) est converti en le carbanion, comme dans la partie c) ci-dessus, puis désactivé avec du chlorure de sulfuryle, ce qui donne un composé intermédiaire dans lequel Y est un groupe chlorosulfonyle que l'on fait ensuite réagir avec l'ammoniac ou une alkylamine ou dialkylamine pour obtenir un composé de formule (I) dans laquelle Y est un groupe aminosulfonyle, alkylaminosulfonyle ou dialkylaminosulfonyle ;

e) est converti en le carbanion comme dans la partie c) ci-dessus ou bien, à titre facultatif, le carbanion est préparé, avec passage par le composé dans lequel Y est un halogène, obtenu selon le mode opératoire de (E) ci-dessus, après quoi le carbanion est amené à réagir par un mode opératoire analogue à celui qui est décrit en (B)c ci-dessus en donnant un composé de formule (I) dans laquelle Y est un groupe hydroxyle et ses sels, un groupe alkoxy ou un groupe halogénalkoxy ; ou

(H) répondant à la formule :

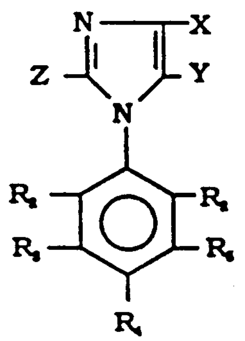

(I)

dans laquelle X, Z, $R_2$, $R_3$, $R_4$, $R_5$ et $R_6$ sont tels que définis pour la formule (I) et Y est un groupe carboxyle et ses sels, un groupe cyano, aminocarbonyle, alkylaminocarbonyle, dialkylaminocarbonyle, alkoxycarbonyle, halogénalkyle, alcényle, alcynyle, alkylcarbonyle ou halogénalkylcarbonyle, dans lequel un composé de formule (Ib)

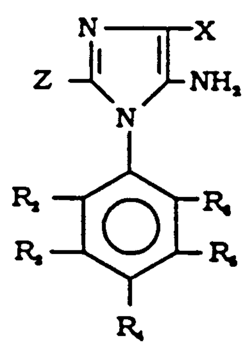

(Ib)

dans laquelle X, Z et $R_2$ à $R_6$ sont tels que définis ci-dessus, est tout d'abord déaminé conformément aux modes opératoires décrits en (E) ci-dessus en donnant un composé dans lequel Y est l'hydrogène, que l'on convertit ensuite par les modes opératoires décrits en (G)c ci-dessus pour obtenir un composé de formule (I) dans laquelle Y est un groupe formyle, après quoi ce composé formylique, dans lequel X et Z sont facultativement protégés si nécessaire :

a) est amené à réagir par des modes opératoires analogues à ceux qui sont décrits en (C)b ci-dessus, pour obtenir un composé de formule (I) dans laquelle Y est un groupe carboxyle et ses sels ou un groupe cyano :

b) est amené à réagir par des modes opératoires analogues à ceux qui sont décrits en (C)c ci-dessus en donnant un composé de formule (I) dans laquelle Y est un groupe aminocarbonyle,

alkylaminocarbonyle, dialkylaminocarbonyle ou alkoxycarbonyle :

c) est amené à réagir par des modes opératoires analogues à ceux qui sont décrits en (C)d ci-dessus en donnant un composé de formule (I) dans laquelle Y est un groupe halogénalkyle, plus particulièrement un groupe halogénométhyle :

d) est amené à réagir par des modes opératoires analogues à ceux qui sont décrits en (B)b ci-dessus et en (C)e ci-dessus en donnant un composé de formule (I) dans laquelle Y est un groupe alcényle ou alcynyle ; ou

e) est amené à réagir par des modes opératoires analogues à ceux qui sont décrits en (C)f ci-dessus en donnant un composé de formule (I) dans laquelle Y est un groupe alkylcarbonyle ou halogénalkylcarbonyle ; ou

(I) répondant à la formule :

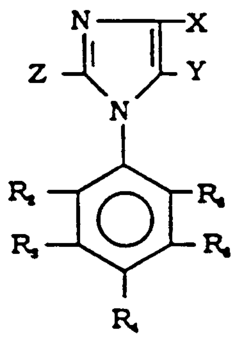

(I)

dans laquelle Y, Z, $R_2$, $R_3$, $R_4$, $R_5$ et $R_6$ ont les définitions données pour la formule (I) et X est un groupe alkylsulfényle, halogénalkylsulfényle, alkylsulfinyle, halogénalkylsulfinyle, alkylsulfonyle ou halogénalkylsulfonyle, dans lequel un composé de formule (Ic)

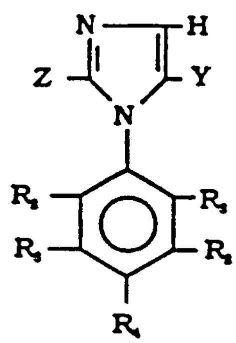

(Ic)

dans laquelle Y et Z sont facultativement protégés si nécessaire :

a) est tout d'abord amené à réagir conformément à des modes opératoires analogues à ceux qui sont décrits en (B)c ci-dessus pour convertir un composé de formule (Ic), dans laquelle X est l'hydrogène, en un composé intermédiaire de formule (I) dans laquelle X est successivement un groupe thiocyano puis un groupe disulfure, après quoi, également par des modes opératoires analogues à ceux qui sont décrits en (B)c ci-dessus, le composé intermédiaire thiocyano ou disulfure est converti en un composé de formule (I) dans laquelle X est un groupe alkylsulfényle ou halogénalkylsulfényle, en particulier un groupe perhalogénalkylsulfényle, ce composé est ensuite facultativement oxydé par des modes opératoires analogues à ceux qui sont décrits en (B)c ci-dessus en donnant l'analogue du type sulfoxyde ou sulfone, c'est-à-dire un composé de formule (I) dans laquelle X est un groupe alkylsulfinyle, halogénalkylsulfinyle, de préférence perhalogénalkylsulfinyle, un groupe alkylsulfonyle ou halogénalkylsulfonyle, notamment perhalogénalkylsulfonyle ; ou bien

b) est tout d'abord amené à réagir conformément à des modes opératoires analogues à ceux qui sont décrits en (B)a ci-dessus pour convertir le composé de formule (Ic), dans laquelle X est

l'hydrogène, en un composé intermédiaire de formule (I) dans laquelle X est un groupe chlorosulfonyle, puis le composé chlorosulfonylique est amené à réagir avec un agent réducteur tel que la triphénylphosphine en donnant le même disulfure intermédiaire que celui qui a été décrit ci-dessus dans la partie a), après quoi, finalement, le disulfure est converti par les modes opératoires décrits dans la partie a) ci-dessus en donnant un composé de formule (I) dans laquelle X est un groupe alkylsulfényle ou halogénalkylsulfényle, notamment perhalogénalkylsulfényle, ou bien à titre facultatif, le composé sulfénylique est oxydé en donnant un composé de formule (I) dans laquelle X est un groupe alkylsulfinyle, halogénalkylsulfinyle, en particulier perhalogénalkylsulfinyle, alkylsulfonyle ou halogénalkylsulfonyle, en particulier perhalogénalkylsulfonyle ; ou

(J) répondant à la formule :

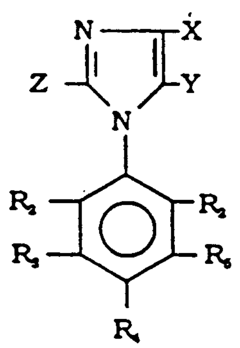

(IV)

dans laquelle :

| | |
|---|---|
| $R_2$, $R_3$, $R_4$, $R_5$ et $R_6$ | sont tels que définis ci-dessus ; |
| X | est l'hydrogène ou un groupe halogénalkyle, notamment trifluorométhyle ; |
| Y | est un groupe amino ; hydroxy, existant facultativement sous sa forme isomérique céto lorsque X est l'hydrogène ; un groupe alkoxy ; ou un groupe halogénalkoxy ; et |
| Z | est l'hydrogène ; un halogène ; un groupe alkyle ; halogénalkyle ; hydroxy, existant facultativement sous sa forme céto-isomérique lorsque X est l'hydrogène et Y est un groupe imino ; alkoxy ; ou halogénalkoxy ; dans lequel un composé de formule (III) |

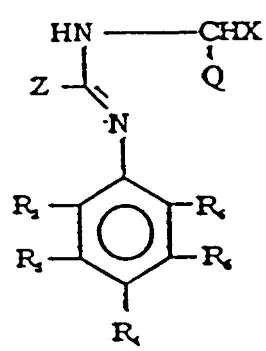

(III)

dans laquelle :

| | |
|---|---|
| $R_2$, $R_3$, $R_4$, $R_5$ et $R_6$ | ont la définition donnée ci-dessus ; |
| X | est l'hydrogène ou un groupe halogénalkyle, en particulier trifluorométhyle ; |
| Z | est l'hydrogène, un halogène, un groupe alkyle, halogénalkyle ou hydroxy, existant facultativement sous sa forme céto-isomérique ; et |
| Q | est un groupe cyano ou alkoxycarbonyle inférieur ; |

est amené à réagir avec un agent basique dans un milieu réactionnel convenable pour former un composé de formule (IV) qui, lorsque Y ou Z est un groupe hydroxy, est ensuite facultativement alkylé ou halogénalkylé en composé dans lequel Y ou Z est un groupe alkoxy ou halogénalkoxy.

**2.** Procédé de préparation d'un composé de formule (IV) suivant la revendication 1J, dans lequel le composé de formule (IV) est :

a) un composé de formule (5) représentée dans la revendication 1, dans laquelle X et Z sont chacun un atome d'hydrogène et Y est un groupe amino ;

b) un composé de formule (17)

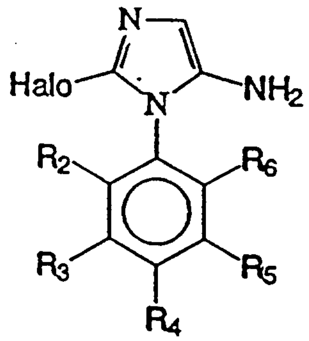

(17)

dans laquelle Halo désigne un halogène, en particulier le chlore ;

c) un composé de formule (22)

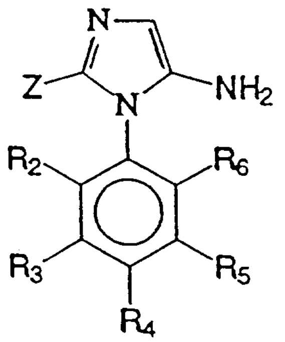

(22)

dans laquelle Z est un groupe alkyle ou halogénalkyle ;

d) un composé de formule (27)

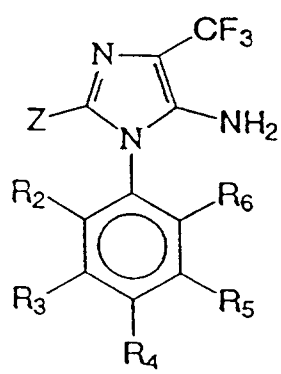

(27)

dans laquelle Z est un halogène, un groupe alkyle ou un groupe halogénalkyle ;

e) un composé de formule (30), dans laquelle X est de l'hydrogène et Y est un groupe hydroxyle, existant facultativement sous sa forme céto isomérique (29),

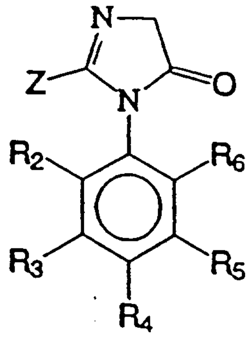

(29)

dans laquelle Z est un halogène, un groupe alkyle ou un groupe halogénalkyle ; ou bien

f) un composé de formule (37), dans laquelle Y est l'hydrogène et Z est un groupe hydroxy, existant facultativement sous sa forme céto-imino isomérique (34)

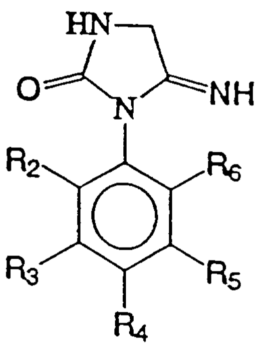

(34)

3. Procédé de préparation d'un composé de formule (I) :

(A) dans laquelle X, Y, Z, $R_2$, $R_3$, $R_4$, $R_5$ et $R_6$ ont les définitions données dans la revendication 1, dans lequel un composé de formule (5) représentée dans la revendication 1 est amené à réagir conformément au procédé de préparation selon la revendication 1 pour l'introduction des substituants X, Y et Z ;

(B) dans laquelle X, Y, $R_2$, $R_3$, $R_4$, $R_5$ et $R_6$ sont tels que définis dans la revendication 1 et Z est un halogène, dans lequel un composé de formule (17) représentée dans la revendication 2 est amené à réagir conformément au procédé suivant la revendication 1 pour l'introduction des substituants X et Y ;

(C) dans laquelle X, Y, $R_2$, $R_3$, $R_4$, $R_5$ et $R_6$ ont les définitions données dans la revendication 1 et Z est un groupe alkyle ou halogénalkyle, dans lequel un composé de formule (22) représentée dans la revendication 2 est amené à réagir conformément au procédé suivant la revendication 1 pour l'introduction des substituants X et Y ;

(D) dans laquelle Y, $R_2$, $R_3$, $R_4$, $R_5$ et $R_6$ ont les définitions données dans la revendication 1, X est un groupe halogénalkyle, en particulier trifluorométhyle, et Z est un halogène, un groupe alkyle ou un groupe halogénalkyle, dans lequel un composé de formule (27) est amené à réagir conformément au procédé suivant la revendication 1 pour l'introduction du substituant Y ;

(E) dans laquelle X, $R_2$, $R_3$, $R_4$, $R_5$ et $R_6$ ont la définition donnée dans la revendication 1, Y est un groupe hydroxy, alkoxy ou halogénalkoxy et Z est un halogène, un groupe alkyle ou un groupe halogénalkyle, dans lequel un composé de formule (30), existant facultativement sous sa forme céto isomérique (29) représentée dans la revendication 2, dans laquelle Y est un groupe hydroxy, facultativement alkylé en un groupe Y alkoxy ou halogénalkoxy, est amené à réagir conformément au procédé de la revendication 1 pour l'introduction du substituant X ; ou

(F) dans laquelle X, Y, $R_2$, $R_3$, $R_4$, $R_5$ et $R_6$ ont les définitions données dans la revendication 1 et Z est un groupe hydroxy, alkoxy, halogénalkoxy ou un halogène, caractérisé en ce qu'un composé de formule (37), existant facultativement sous sa forme céto-imino isomérique (34) représentée dans la revendication 2, dans laquelle Z est un groupe hydroxy, facultativement alkylé en un groupe Z

alkoxy ou halogénalkoxy ou facultativement halogéné de telle sorte que Z représente un halogène, est amené à réagir conformément au procédé de la revendication 1 pour l'introduction des substituants X et Y.

**4.** Procédé suivant la revendication 1, dans lequel X est un groupe halogénalkoxy.

**5.** Procédé suivant la revendication 1, dans lequel X est un groupe $S(O)_nR_1$, répondant à la formule (II)

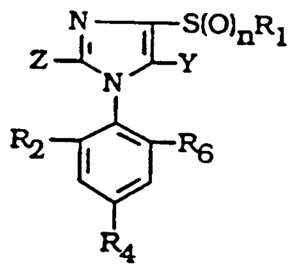

(II)

dans laquelle :

- Y et Z sont choisis, individuellement, entre un atome d'hydrogène ou d'halogène ; un groupe choisi entre nitro, cyano, hydroxyle, sulfhydryle, amino, alkylamino ou dialkylamino, la partie alkyle définie de chaque groupe étant à chaîne linéaire ou à chaîne ramifiée contenant un à quatre atomes de carbone ; ou un groupe choisi entre alkyle, alkoxy, alkylcarbonyle, alkylcarbonylamino, alkylsulfényle, alkylsulfinyle ou alkylsulfonyle non substitué ou entièrement substitué par un halogène, les parties alkyle et alkoxy définies de chaque groupe étant à chaîne linéaire ou à chaîne ramifiée contenant un à quatre atomes de carbone, et la substitution totale par un halogène de la partie alkyle ou alkoxy est effectuée par le même atome d'halogène ou par des atomes d'halogènes différents ; et un seul de Y et Z est un groupe contenant du soufre ;
- $R_1$ est un groupe alkyle à chaîne linéaire ou ramifiée ayant un à quatre atomes de carbone, non substitué ou substitué par un ou plusieurs atomes d'halogènes qui sont identiques ou différents :
- $R_2$ est un atome d'hydrogène ou un atome d'halogène ou un groupe alkyle, alkoxy, méthylsulfényle, méthylsulfinyle ou méthylsulfonyle ;
- $R_4$ est choisi entre un atome d'halogène ou un groupe choisi entre trifluorométhyle, trifluorométhoxy, difluorométhoxy, trifluorométhylsulfényle, trifluorométhylsulfinyle, trifluorométhylsulfonyle ou un groupe alkyle à chaîne linéaire ou ramifiée contenant un à quatre atomes de carbone ;
- $R_6$ est un atome d'halogène ; et
- n a la valeur 0, 1 ou 2.

**6.** Procédé suivant la revendication 5, dans lequel :

- Y est un atome d'hydrogène, un atome d'halogène, un groupe amino, hydroxy, alkoxy de un à quatre atomes de carbone, méthylsulfényle, méthylsulfinyle ou méthylsulfonyle ;
- Z est un atome d'hydrogène, un atome d'halogène ou un groupe alkyle à chaîne linéaire ou à chaîne ramifiée ayant un à quatre atomes de carbone, qui est entièrement substitué à titre facultatif par des atomes d'halogènes qui sont identiques ou différents ;
- $R_1$ est un groupe méthyle entièrement substitué par des atomes d'halogènes qui sont identiques ou différents ;
- $R_2$ est un atome d'hydrogène, un atome d'halogène ou un groupe méthylsulfényle ;
- $R_4$ est un atome d'halogène, un groupe trifluorométhyle ou trifluorométhoxy ; et
- $R_6$ est un atome de fluor, de chlore ou de brome.

**7.** Procédé suivant la revendication 6, dans lequel :

- Y est un atome d'hydrogène, un atome de chlore, un atome de brome, un groupe méthylsulfényle, méthylsulfinyle ou méthoxy ;

X est un atome d'hydrogène, un atome de chlore, un atome de brome ou un groupe méthyle ;
$R_1$ est un groupe trifluorométhyle, dichlorofluorométhyle ou chlorodifluorométhyle ;
$R_2$ est un atome d'hydrogène, un atome de chlore, un atome de brome ou un groupe méthylsulfényle ;
$R_4$ est un atome de chlore, un atome de brome, un atome de fluor, un groupe trifluorométhyle ou trifluorométhoxy ; et
$R_6$ est un atome de chlore ou de brome.

**8.** Procédé suivant la revendication 7, pour la préparation d'un composé de formule I qui est :
le 1-(2,6-dichloro-4-trifluorométhylphényl)-2-chloro-4-trifluorométhylsulfénylimidazole ;
le 1-(2,6-dichloro-4-trifluorométhylphényl)-2-chloro-4-trifluorométhylsulfinylimidazole ;
le 1-(2,6-dichloro-4-trifluorométhylphényl)-5-bromo-4-dichlorofluorométhylsulfénylimidazole ;
le 1-(2,6-dichloro-4-trifluorométhylphényl)-2-chloro-4-dichlorofluorométhylsulfénylimidazole ;
le 1-(2,6-dichloro-4-trifluorométhylphényl)-2-bromo-4-dichlorofluorométhylsulfénylimidazole ;
le 1-(2,6-dichloro-4-trifluorométhylphényl)-4-trifluorométhylsulfinylimidazole ;
le 1-(2,6-dichloro-4-trifluorométhylphényl)-2-chloro-4-dichlorofluorométhylsulfinylimidazole ;
le 1-(2,6-dichloro-4-trifluorométhylphényl)-2-chloro-4-dichlorofluorométhylsulfonylimidazole ;
le 1-(2,6-dichloro-4-trifluorométhylphényl)-2-bromo-4-chlorodifluorométhylsulfinylimidazole ;
le 1-(2,6-dichloro-4-trifluorométhylphényl)-2-bromo-4-chlorodifluorométhylsulfénylimidazole ;
le 1-(2,6-dichloro-4-trifluorométhylphenyl)-2-chloro-4-chlorodifluorométhylsulfénylimidazole ;
le 1-(6-chloro-2-méthylsulfényl-4-trifluorométhylphényl)-2-bromo-4-chlorodifluorométhylsulfonylimidazole ;
le 1-(6-chloro-2-méthylsulfényl-4-trifluorométhylphényl)-2-chloro-4-dichlorofluorométhylsulfonylimidazole ;
le 1-(2,6-dichloro-4-trifluorométhylphényl)-4-dichlorofluorométhylsulfénylimidazole ;
le 1-(2,6-dichloro-4-trifluorométhyiphényl)-4-dichlorofluorométhylsulfinylimidazole ;
le 1-(2,6-dichloro-4-trifluorométhylphényl)-4-dichlorofluorométhylsulfonylimidazole ;
le 1-(2,6-dichloro-4-trifluorométhylphényl)-2-méthyl-4-dichlorofluorométhylsulfénylimidazole ;
le 1-(2,6-dichloro-4-trifluorométhylphényl)-2-méthyl-4-dichlorofluorométhylsulfinylimidazole ;
le 1-(2,6-dichloro-4-trifluorométhylphényl)-2-méthyl-4-dichlorofluorométhylsulfonylimidazole ;
le 1-(2,4,6-trichlorophényl)-4-dichlorofluorométhylsulfénylimidazole;
le 1-(2,4,6-trichlorophényl)-4-dichlorofluorométhylsulfinylimidazole ;
le 1-(2,6-dichloro-4-trifluorométhoxyphényl)-4-dichlorofluorométhylsulfinylimdazole ;
le 1-(2,6-dichloro-4-trifluorométhylphényl)-4-chlorodifluorométhylsulfinylimidazole ;
le 1-(2,6-dichloro-4-trifluorométhylphényl)-2-méthyl-4-chlorodifluorométhylsulfinylimidazole ;
le 1-(2,6-dichloro-4-trifluorométhylphényl)-2-méthyl-4-chlorodifluorométhylsulfonylimidazole ;
le 1-(2,4,6-trichlorophényl)-4-chlorodifluorométhylsulfénylimidazole; ou
le 1-(2,4,6-trichlorophényl)-4-chlorodifluorométhylsulfinylimidazole.

**9.** Procédé suivant la revendication 7, pour la préparation d'un composé de formule (I) qui est :
le 1-(2,6-dichloro-4-trifluorométhylphényl)-2-chloro-4-trifluorométhylsulfonylimidazole ;
le 1-(2,6-dichloro-4-trifluorométhylphényl)-5-méthylsulfényl-4-dichlorofluorométhylsulfénylimidazole ;
le 1-(2,6-dichloro-4-trifluorométhylphényl)-4-trifluorométhylsulfonylimidazole ;
le 1-(2,6-dichloro-4-trifluorométhylphényl)-5-bromo-4-trifluorométhylsulfonylimidazole ;
le 1-(2,6-dichloro-4-trifluorométhylphényl)-2-bromo-4-dichlorofluorométhylsulfinylimidazole ;
le 1-(2,6-dichloro-4-trifluorométhylphényl)-5-bromo-4-dichlorofluorométhylsulfonylimidazole ;
le 1-(2,6-dichloro-4-trifluorométhylphényl)-2-bromo-4-dichlorofluorométhylsulfonylimidazole ;
le 1-(2,6-dichloro-4-trifluorométhylphényl)-2-bromo-4-chlorodifluorométhylsulfonylimidazole ;
le 1-(2,6-dichloro-4-trifluorométhylphényl)-2-chloro-4-chlorodifluorométhylsulfinylimidazole ;
le 1-(2,6-dichloro-4-trifluorométhylphényl)-2-chloro-4-chlorodifluorométhylsulfonylimidazole ;
le 1-(2,6-dichloro-4-trifluorométhylphényl)-5-méthylsulfonyl-4-dichlorofluorométhylsulfinylimidazole ;
le 1-(2,6-dichloro-4-trifluorométhyl)-5-méthylsulfinyl-4-dichlorofluorométhylsulfinylimidazole ;
le 1-(6-chloro-2-méthylsulfényl-4-trifluorométhylphényl)-2-chloro-4-dichlorofluorométhylsulfénylimidazole ;
le 1-(6-chloro-2-méthylsulfényl-4-trifluorométhylphényl)-2-chloro-4-dichlorofluorométhylsulfinylimidazole ;
le 1-(2,6-dichloro-4-trifluorométhylphényl)-2-chloro-5-méthylsulfényl-4-trifluorométhylsuifénylimidazole ;

le 1-(2,6-dichloro-4-trifluorométhylphényl)-5-bromo-4-trifluorométhylsulfénylimidazole ;
le 1-(2,6-dichloro-4-trifluorométhylphényl)-2-méthyl-4-chlorodifluorométhylsulfénylimidazole ;
le 1-(2,6-dichloro-4-trifluorométhylphényl)-5-chloro-4-trifluorométhylsulfénylimidazole ;
le 1-(2,6-dichloro-4-trifluorométhylphényl)-2,5-dichloro-4-dichlorofluorométhylsulfénylimidazole ;
le 1-(2,6-dichloro-4-trifluorométhylphényl)-4-chlorodifluorométhylsulfénylimidazole ;
le 1-(2-chloro-4-trifluorométhylphényl)-4-dichlorofluorométhylsulfinylimidazole ;
le 1-(2-chloro-4-trifluorométhylphényl)-4-trifluorométhylsulfinylimidazole ;
le 1-(2-chloro-4-trifluorométhylphényl)-4-trifluorométhylsulfonylimidazole ;
le 1-(2,6-dichloro-4-bromophényl)-4-dichlorofluorométhylsulfénylimidazole ;
le 1-(2,6-dichloro-4-bromophényl)-4-dichlorofluorométhylsulfinylimidazole ;
le 1-(2,6-dichloro-4-bromophényl)-4-chlorodifluorométhylsulfénylimidazole ;
le 1-(2,6-dichloro-4-trifluorométhoxyphényl)-4-trifluorométhylsulfinylimidazole ;
le 1-(2,6-dichloro-4-trifluorométhoxyphényl)-4-chlorodifluorométhylsulfénylimidazole ;
le 1-(2,6-dichloro-4-trifluorométhoxyphényl)-4-chlorodifluorométhylsulfinylimidazole ;
le 1-(2,6-dichloro-4-trifluorométhoxyphényl)-4-dichlorofluorométhylsulfonylimidazole ; ou
le 1-(2,6-dichloro-4-trifluorométhoxyphenyl)-2-bromo-4-dichlorofluorométhylsulfénylimidazole.

**10.** Procédé de préparation d'une composition destinée à combattre des arthropodes, nématodes, helminthes ou protozoaires parasites, qui comprend la formulation d'une quantité efficace, de préférence environ 0,05 à environ 95 % en poids, d'un composé de formule (I) suivant l'une quelconque des revendications 1 à 9, mais comprenant des composés dans lesquels (i) X est un groupe trifluorométhyle lorsque $R_3$ représente un groupe méthyle, $R_4$ représente un groupe nitro ou amino et les autres symboles représentent l'hydrogène et (ii) X est un groupe chlorométhyle lorsque $R_4$ représente un groupe méthyle, méthoxy ou éthoxy et les autres symboles représentent l'hydrogène, comme ingrédient actif et un ou plusieurs composants compatibles du point de vue agronomique ou médicinal, comprenant environ 1 % à environ 95 % en poids d'un ou plusieurs supports solides ou liquides et environ 0,1 à environ 50 % en poids d'un ou plusieurs composants additionnels tels que des agents tensio-actifs.

**11.** Procédé de préparation d'une composition destinée en particulier à être utilisée en médecine vétérinaire et dans l'exploitation du bétail ou dans le maintien de l'hygiène publique, comprenant la formulation d'un ou plusieurs composants compatibles et, comme ingrédient actif, d'au moins un composé de formule (I) suivant la revendication 10.